# EUROPEAN PATENT APPLICATION

(11) **EP 4 310 089 A1**
(43) Date of publication of application: **24.01.2024**
(21) Application number: 22770477.2
(22) Date of filing: 15.03.2022
(51) Int. Cl.: C07D 487/04, A61K 31/407, A61P 25/00, A61P 25/22, A61P 25/24

(54) **NITROGEN-CONTAINING HETEROCYCLIC POLYCYCLIC COMPOUND, PREPARATION METHOD THEREFOR, AND APPLICATION THEREOF**

(30) Priority: 16.03.2021 CN 202110280887; 28.05.2021 CN 202110591611; 13.08.2021 CN 202110934860
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: XIAO, Hualing, Shanghai 201203 (CN); LU, Xingyun, Shanghai 201203 (CN); SU, Yidong, Shanghai 201203 (CN); GONG, Zhen, Shanghai 201203 (CN); YU, Wensheng, Shanghai 201203 (CN)
(74) Representative: Cabinet Beau de Loménie
(86) International application number: PCT/CN2022/080829
(87) International publication number: WO 2022/194122

(57) **Abstract**

The present invention relates to a nitrogen-containing heterocyclic polycyclic compound, a preparation method therefor, and an application thereof. In particular, the present invention relates to a compound represented by general formula I, a preparation method for same, a pharmaceutical composition containing same, and an application of same as an Orexin receptor antagonist in the preparation of drugs related to nervous system diseases, wherein each substituent in general formula I is as defined in the description.

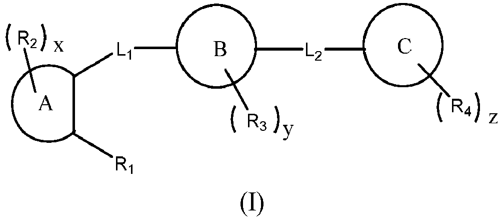

## Description

### FIELD OF THE INVENTION

The present invention belongs to the field of biomedicine, and specifically relates to a nitrogen-containing heterocyclic polycyclic compound, preparation method therefor, and application thereof.

### BACKGROUND OF THE INVENTION

The hypothalamus is the center for regulating diet and energy balance. Orexin is a neuropeptide synthesized and secreted by orexin neurons in the lateral hypothalamus (LH). It is named for its strong appetite-promoting effect. Orexin is classified into Orexin A and Orexin B. Both Orexin A and Orexin B act on the G protein-coupled receptors, Orexin receptor OX1R and Orexin receptor OX2R. OX1R and OX2R are widely expressed throughout the central nervous system. OX1R binds Orexin A more strongly than Orexin B, while OX2R binds Orexin A and Orexin B equally. There is an intricate relationship between Orexin and other neuropeptides that affect eating. It has a wide range of effects on increasing food intake, drinking water, regulating sleep-wake cycles, reproduction, body temperature, blood pressure, and sensation. For example, Orexin regulates wakefulness by regulating two distinct G protein-coupled receptors, OX1R and OX2R. Orexin receptor antagonists have potential therapeutic advantages in the treatment of neurological disorders, including insomnia, depression, anxiety, drug addiction, and the like.

OX1R and OX2R activate intracellular Ca²⁺ by phospholipase C. OX2R can also be coupled to Gi/Go to inhibit the production of cAMP by inhibiting adenylate cyclase. Studies have found that among OX1R and OX2R, OX2R is preferentially expressed in the paraventricular nucleus of the hypothalamus and participates in the regulation of the HPA axis. Nocturnal hypothalamic-pituitary-adrenal (HPA) hyperexcitation is the biggest difference between depressed patients and normal people. Down-regulation of the hyperexcitation of the HPA system will help improve depressive symptoms.

At present, there are several drugs targeting OX1/2R that are in the clinical stage or have been marketed, such as Suvoraxant developed by Merck, Lemborexant developed by Eisai, and the like. However, drugs that are Orexin 1/2 antagonists have antagonistic effects on both OX1R and OX2R receptors. Acting on OX1R will change the normal physiological structure of rapid eye movement sleep (NEM, brain activity is the same as awake) and non-rapid eye movement sleep (NEREM, deep sleep), that is, sacrificing NREM time and prolonging REM time, and then increasing the risk of drowsiness. Moreover, acting on OX1R does not have an antidepressant effect.

OX2R antagonists can play an antidepressant effect, and OX2R single receptor antagonists can also play a sufficient effect on insomnia. Therefore, selective OX2R antagonists can avoid various side effects such as lethargy caused by the action on OX1R. Currently, among OX2R antagonists, only Seltorexant developed by Janssen is in the clinical stage, and its main indications are major depressive disorder (MDD), primary and secondary insomnia, and the like.

Selective OX2R antagonists have the potential of treating neurological diseases such as insomnia, depression, and anxiety, and have huge clinical needs. Selective OX2R antagonists have good application prospects as drugs in the pharmaceutical industry.

### SUMMARY OF THE INVENTION

The present invention provides an OX2R antagonist with a novel structure. It is found that compounds with such structure will exhibit good activity, selectivity, brain-plasma ratio (ratio of drug concentration in brain to drug concentration in plasma, referred to as B/P), and less toxic and side effects. The present invention relates to a compound of formula I, a stereoisomer thereof or a pharmaceutically acceptable salt thereof: 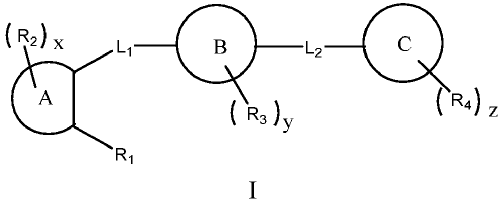 wherein:
ring A, ring B and ring C are each independently selected from the group consisting of cycloalkyl, heterocyclyl, aryl and heteroaryl, and ring B is preferably a monocyclic ring or a bicyclic ring;
in the present invention, ring C is not a 5-membered heteroaryl including thiazole, oxazole and the like;
R₁, R₂, R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
L₁, L₂ are each independently selected from the group consisting of a bond, -[C(R₅)₂]ₙ-, -[C(R₅)₂-O]ₙ-, -O-, -CO-, -COO-, -OOC-, -CON(R₅)-, -N(R₅)CO-, -N(R₅)-, -C(R₅)₂N(R₅)-;
each R₅ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₁, R₂, R₃, R₄, R₅ can be connected to form a cycloalkyl, heterocyclyl, aryl or heteroaryl, the cycloalkyl, heterocyclyl, aryl or heteroaryl can be optionally further substituted;
x is selected from the group consisting of 0, 1, 2, 3, 4 and 5;
y is selected from the group consisting of 0, 1, 2, 3, 4, 5, 6, 7, 8, 9, 10, 11, 12, 13 and 14;
z is selected from the group consisting of 0, 1, 2, 3, 4 and 5;
n is selected from the group consisting of 1, 2, 3, 4, 5 and 6.

In some embodiments, ring A is selected from the group consisting of C₃₋₁₂ cycloalkyl, 3 to 12 membered heterocyclyl, C₆₋₁₄ aryl and 5 to 14 membered heteroaryl;
preferably selected from the group consisting of 5 to 6 membered monocyclic heteroaryl and C₆₋₁₀ aryl;
more preferably selected from the group consisting of phenyl and naphthalene;
   and/or 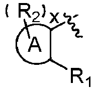 is selected from the group consisting of 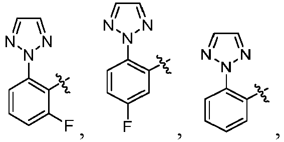 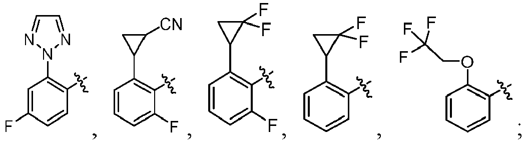 and/or 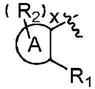 is selected from the group consisting of 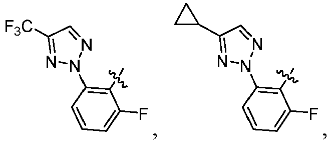 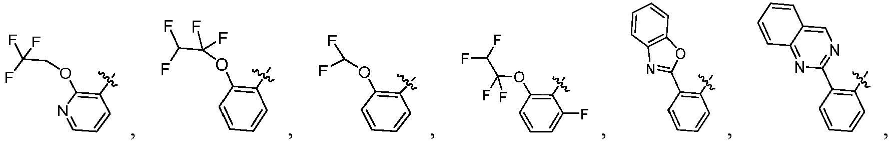 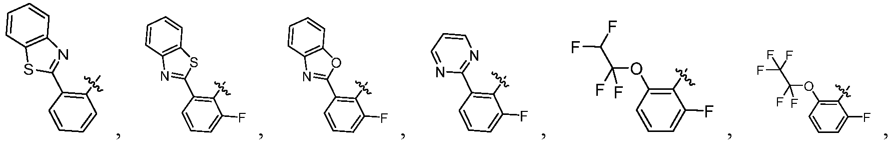 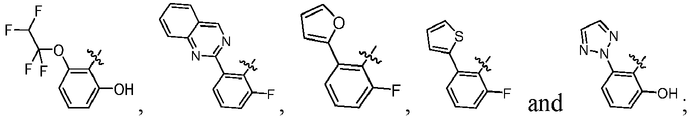 and/or 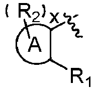 is selected from the group consisting of

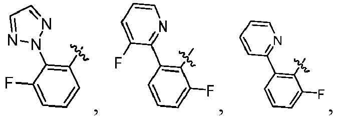

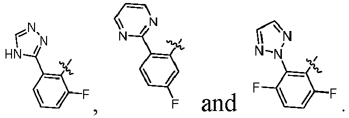

In some embodiments, ring B is selected from the group consisting of C₃₋₁₂ cycloalkyl, 3 to 12 membered heterocyclyl, C₆₋₁₄ aryl and 5 to 14 membered heteroaryl;
preferably selected from the group consisting of C₃₋₈ cycloalkyl and 3 to 12 membered heterocyclyl;
more preferably selected from the group consisting of cyclopropyl or 3 to 12 membered nitrogen-containing heterocyclyl;
further preferably selected from the group consisting of cyclopropyl, 4 to 7 membered monocyclic heterocyclyl containing 1 to 2 heteroatoms selected from the group consisting of N, O and S, 6 to 10 membered fused heterocyclyl containing 1 to 3 heteroatoms selected from the group consisting of N, O and S, 8 to 10 membered fused heterocyclyl containing 1 to 3 heteroatoms selected from the group consisting of N, O and S, and 8 to 10 membered bridged heterocyclyl containing 1 to 3 heteroatoms selected from the group consisting of N, O and S;
more further preferably selected from the group consisting of cyclopropyl, 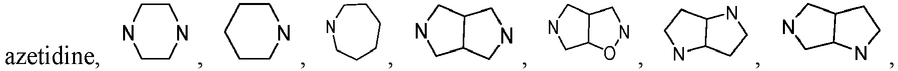 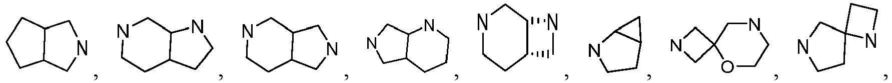 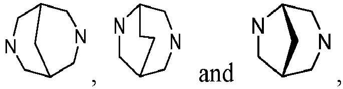 wherein the atoms connected to L₁ or L₂ are any two different carbon atoms or nitrogen atoms on the ring, and the N atom which is not a connecting atom bears H or R₃;
and/or, more further preferably selected from 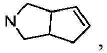 wherein the atoms connected to L₁ or L₂ are any two different carbon atoms or nitrogen atoms on the ring, and the N atom which is not a connecting atom bears H or R₃; preferably, the atoms connected to L₁ or L₂ are the nitrogen atom on the ring and the carbon atom farthest from the nitrogen atom on the ring.

In some embodiments, ring C is selected from the group consisting of C₃₋₁₂ cycloalkyl, 3 to 12 membered heterocyclyl, C₆₋₁₄ aryl and 5 to 14 membered heteroaryl, and ring C is not a 5 membered heteroaryl;
preferably selected from 6 membered heteroaryl containing 1 to 3 heteroatoms selected from the group consisting of N, O and S;
more preferably selected from the group consisting of pyrazinyl, pyridazinyl, pyridyl, pyrimidinyl, pyrazinyl;
more further preferably selected from the group consisting of pyrimidyl and pyridyl;
   and/or 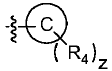 is selected from the group consisting of 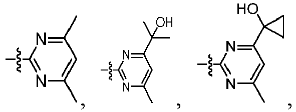 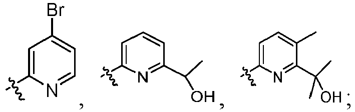 and/or 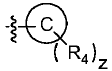 is selected from the group consisting of 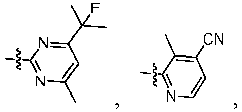 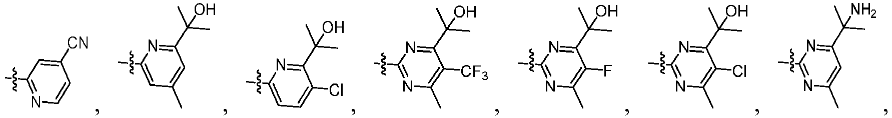 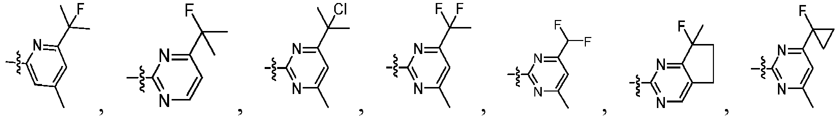 and 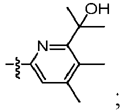 and/or 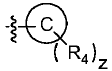 is selected from the group consisting of

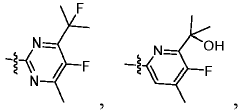

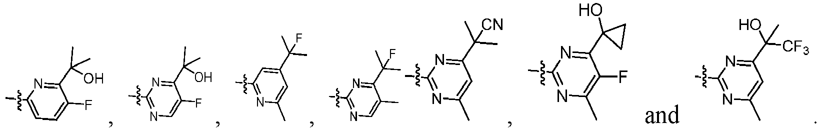

In some embodiments, each R₁ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, C₃₋₆ cycloalkyl, 5 to 6 membered heteroaryl and C₁₋₃ haloalkoxy, the 5 to 6 membered heteroaryl and C₁₋₃ haloalkoxy are each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
more further preferably selected from the group consisting of 1H-1,2,3-triazol-1-yl, 2H-1,2,3-triazol-2-yl, cyclopropyl, 2,2,2-trifluoroethoxy, 1H-1,2,3-triazol-1-yl, 2H-1,2,3-triazol-2-yl, cyclopropyl and 2,2,2-trifluoroethoxy, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
or, more further preferably selected from the group consisting of 4-(trifluoromethyl)-2H-1,2,3-triazol-2-yl, 4-cyclopropyl-2H-1,2,3-triazol-2-yl, 1,1,2,2-tetrafluoroethoxy, difluoromethoxy, benzoxazol-2-yl, quinazolin-2-yl, benzo[d]thiazol-2-yl, pyrimidin-2-yl, perfluoroethoxy, pyrimidin-2-yl, furan-2-yl, thiophen-2-yl, 4-(trifluoromethyl)-2H-1,2,3-triazol-2-yl, 4-cyclopropyl-2H-1,2,3-triazol-2-yl, 1,1,2,2-tetrafluoroethoxy, difluoromethoxy, benzoxazol-2-yl, quinazolin-2-yl, benzo[d]thiazol-2-yl, pyrimidin-2-yl, perfluoroethoxy, pyrimidin-2-yl, furan-2-yl and thiophen-2-yl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
or, more further preferably selected from the group consisting of 3-fluoropyridin-2-yl, pyridin-2-yl and 4H-1,2,4-triazol-3-yl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, each R₂ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen and halogen, the halogen is preferably fluorine;
optionally, R₂ is preferably located at the meta or para position of R₁;
optionally, R₂ is preferably located at the ortho position of L₁ or the ortho position of R₁.

In some embodiments, each R₃ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, methyl, ethyl, propyl, isopropyl, butyl and tert-butyl, the halogen is preferably fluorine.

In some embodiments, R₄ and R₅ are each independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, methyl, ethyl, propyl, isopropyl, butyl, tert-butyl, 2-hydroxyisopropyl, 1-hydroxycyclopropyl, 2-cyanocyclopropyl, 2,2-difluorocyclopropyl and 1-hydroxyethyl, the halogen is preferably selected from the group consisting of fluorine and bromine;
or, more preferably selected from the group consisting of 2-hydroxybutan-2-yl, 2-fluoropropan-2-yl, cyano, 2-aminopropan-2-yl, 2-fluoropropan-2-yl, 2-chloropropan-2-yl, 1,1-difluoroethyl, difluoromethyl and 1-fluorocyclopropyl;
or, more preferably selected from the group consisting of 2-cyanopropyl-2-yl and 1,1,1 -trifluoro-2-hydroxypropyl-2-yl.

In some embodiments, any two or more of R₁, R₂, R₃, R₄, R₅ can be connected to form a C₃₋₁₂ cycloalkyl, 3 to 12 membered heterocyclyl, C₆₋₁₄ aryl or 5 to 14 membered heteroaryl, the C₃₋₁₂ cycloalkyl, 3 to 12 membered heterocyclyl, C₆₋₁₄ aryl or 5 to 14 membered heteroaryl is optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably form a C₃₋₈ cycloalkyl, 3 to 8 membered heterocyclyl, C₆₋₁₀ aryl or 5 to 10 membered heteroaryl, the C₃₋₈ cycloalkyl, 3 to 8 membered heterocyclyl, C₆₋₁₀ aryl or 5 to 10 membered heteroaryl is optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
wherein, the C₃₋₈ cycloalkyl is preferably C₅ cycloalkyl.

The present invention further relates to a compound of formula II or formula II', a stereoisomer thereof or a pharmaceutically acceptable salt thereof: 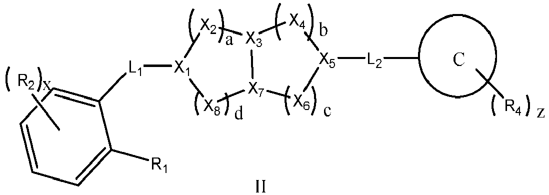 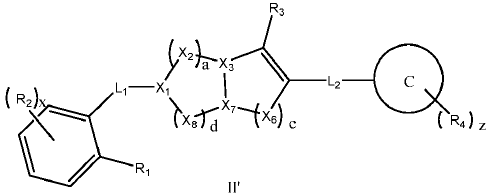 wherein:
X₁, X₃, X₅, X₇ are each independently selected from the group consisting of N and CR₃;
X₂, X₄, X₆, X₈ are each independently selected from the group consisting of -NR₃-, -C(R₃)₂-, -CO-, -O- and -S-;
ring C is selected from the group consisting of cycloalkyl, heterocyclyl, aryl and heteroaryl, and ring C is not a 5 membered heteroaryl;
L₁, L₂ are each independently selected from the group consisting of a bond, -[C(R₅)₂]ₙ-, -[C(R₅)₂-O]ₙ-, -O-, -CO-, -COO-, -OOC-, -CON(R₅)-, -N(R₅)CO-, -N(R₅)- and -C(R₅)₂N(R₅)-; L₁ is preferably selected from the group consisting of -CO- and -[C(R₅)₂]ₙ-; L₂ is preferably selected from the group consisting of a bond and -N(R₅)-;
preferably, in the fused ring comprising X₁, X₃, X₅, X₇, any one or more single bonds can be replaced by double bonds;
R₁, R₂, R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
each R₅ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₁, R₂, R₃, R₄, R₅ can be connected to form a cycloalkyl, heterocyclyl, aryl or heteroaryl, the cycloalkyl, heterocyclyl, aryl or heteroaryl can be optionally further substituted;
a is selected from the group consisting of 0, 1, 2 and 3;
b is selected from the group consisting of 0, 1, 2 and 3;
c is selected from the group consisting of 0, 1, 2 and 3;
d is selected from the group consisting of 0, 1, 2 and 3;
x is selected from the group consisting of 0, 1, 2, 3 and 4;
z is selected from the group consisting of 0, 1, 2, 3, 4 and 5;
n is selected from the group consisting of 1, 2, 3, 4, 5 and 6.

In some embodiments, each R₁ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, C₃₋₆ cycloalkyl, 5 to 6 membered heteroaryl and C₁₋₃ haloalkoxy, the 5 to 6 membered heteroaryl and C₁₋₃ haloalkoxy are each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
more further preferably selected from the group consisting of 1H-1,2,3-triazol-1-yl, 2H-1,2,3-triazol-2-yl, cyclopropyl, 2,2,2-trifluoroethoxy, 1H-1,2,3-triazol-1-yl, 2H-1,2,3-triazol-2-yl, cyclopropyl and 2,2,2-trifluoroethoxy, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
or, more further preferably selected from the group consisting of 4-(trifluoromethyl)-2H-1,2,3-triazol-2-yl, 4-cyclopropyl-2H-1,2,3-triazol-2-yl, 1,1,2,2-tetrafluoroethoxy, difluoromethoxy, benzoxazol-2-yl, quinazolin-2-yl, benzo[d]thiazol-2-yl, pyrimidin-2-yl, perfluoroethoxy, pyrimidin-2-yl, furan-2-yl, thiophen-2-yl, 4-(trifluoromethyl)-2H-1,2,3-triazol-2-yl, 4-cyclopropyl-2H-1,2,3-triazol-2-yl, 1,1,2,2-tetrafluoroethoxy, difluoromethoxy, benzoxazol-2-yl, quinazolin-2-yl, benzo[d]thiazol-2-yl, pyrimidin-2-yl, perfluoroethoxy, pyrimidin-2-yl, furan-2-yl and thiophen-2-yl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
or, more further preferably selected from the group consisting of 3-fluoropyridin-2-yl, pyridin-2-yl and 4H-1,2,4-triazol-3-yl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, each R₂ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen and halogen, the halogen is preferably selected from the group consisting of fluorine, chlorine, bromine and iodine;
optionally, R₂ is preferably located at the meta or para position of R₁;
optionally, R₂ is preferably located at the ortho position of L₁ or the ortho position of R₁.

In some embodiments, X₁, X₅ are each independently selected from N, X₃, X₇ are each independently selected from CR₃, X₂, X₄, X₆, X₈ are each independently selected from -C(R₃)₂-, a=1, b=1, c=1, d=1;
or
X₁, X₅ are each independently selected from N, X₃, X₇ are each independently selected from CR₃, X₂ is -O-, X₄, X₆, X₈ are each independently selected from -C(R₃)₂-, a=1, b=1, c=1, d=1;
   or
X₁, X₅ are each independently selected from N, X₃, X₇ are each independently selected from CR₃, X₆ is -O-, X₂, X₄, X₈ are each independently selected from -C(R₃)₂-, a=1, b=1, c=1, d=1;
   or
X₁, X₅ are each independently selected from N, X₃, X₇ are each independently selected from CR₃, X₂, X₆ are each independently selected from -C(R₃)₂-, a=2, b=0, c=2, d=0;
   or
X₁, X₅ are each independently selected from N, X₃, X₇ are each independently selected from CR₃, X₂, X₄, X₈ are each independently selected from -C(R₃)₂-, a=1, b=2, c=0, d=1;
   or
X₅ is independently selected from N, X₁, X₃, X₇ are each independently selected from CR₃, X₂, X₄, X₆, X₈ are each independently selected from -C(R₃)₂-, a=1, b=1, c=1, d= 1;
   or
X₁, X₅ are each independently selected from N, X₃, X₇ are each independently selected from CR₃, X₂, X₆, X₈ are each independently selected from -C(R₃)₂-, a=1, b=0, c=2, d=2;
   or
X₁, X₅ are each independently selected from N, X₃, X₇ are each independently selected from CR₃, X₂, X₄, X₆, X₈ are each independently selected from -C(R₃)₂-, a=1, b=1, c=1, d=2;
   or
X₁, X₅ are each independently selected from N, X₃, X₇ are each independently selected from CR₃, X₂, X₄, X₆, X₈ are each independently selected from -C(R₃)₂-, a=1, b=1, c=2, d=1;
   or
X₁, X₅ are each independently selected from N, X₃, X₇ are each independently selected from CR₃, X₂, X₄, X₆, X₈ are each independently selected from -C(R₃)₂-, a=1, b=1, c=3, d=0;
   or
X₁, X₅ are each independently selected from N, X₃, X₇ are each independently selected from CR₃, X₂, X₄, X₆, X₈ are each independently selected from -C(R₃)₂-, a=1, b=0, c=1, d=2;
   or
X₁, X₅ are each independently selected from N, X₃, X₇ are each independently selected from CR₃, X₂, X₄, X₆, X₈ are each independently selected from -C(R₃)₂-, a=1, b=0, c=2, d=2;
   or
X₁, X₅ are each independently selected from N, X₃, X₇ are each independently selected from CR₃, X₂, X₄, X₆, X₈ are each independently selected from -C(R₃)₂-, a=0, b=1, c=2, d=2;
   or
X₁, X₅ are each independently selected from N, X₃, X₇ are each independently selected from CR₃, X₂, X₄, X₆, X₈ are each independently selected from -C(R₃)₂-, a=1, b=1, c=2, d=1;
   or
X₁, X₅ are each independently selected from N, X₃, X₇ are each independently selected from CR₃, X₂, X₄, X₆, X₈ are each independently selected from -C(R₃)₂-, a=1, b=1, c=1, d=2;
   or
X₁, X₅ are each independently selected from N, X₃, X₇ are each independently selected from CR₃, X₂, X₄, X₆, X₈ are each independently selected from -C(R₃)₂-, a=0, b=1, c=1, d=3.

In some embodiments, in formula (II'), X₁ is each independently selected from N, X₃, X₇ are each independently selected from CR₃, X₂, X₆, X₈ are each independently selected from -C(R₃)₂-, a=1, c=1, d=1;
and/or
when X₁, X₂, X₃, X₄, X₅, X₆, X₇, X₈ have chirality, they are each independently R configuration or S configuration;
   and/or
each R₃ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, methyl, ethyl, propyl, isopropyl, butyl and tert-butyl, the halogen is preferably fluorine.

In some embodiments, ring C is selected from the group consisting of C₃₋₁₂ cycloalkyl, 3 to 12 membered heterocyclyl, C₆₋₁₄ aryl and 5 to 14 membered heteroaryl, and ring C is not a 5 membered heteroaryl;
preferably selected from 6 membered heteroaryl containing 1 to 3 heteroatoms selected from the group consisting of N, O and S;
more preferably selected from the group consisting of pyrazinyl, pyridazinyl, pyridyl, pyrimidinyl, pyrazinyl;
more further preferably selected from the group consisting of pyrimidyl and pyridine.

In some embodiments, R₄ and R₅ are each independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, methyl, ethyl, propyl, isopropyl, butyl, *tert*-butyl, 2-hydroxyisopropyl, 1-hydroxycyclopropyl, 2-cyanocyclopropyl, 2,2-difluorocyclopropyl and 1-hydroxyethyl, the halogen is preferably selected from the group consisting of fluorine and bromine;
or, more preferably selected from the group consisting of 2-hydroxybutan-2-yl, 2-fluoropropan-2-yl, cyano, 2-aminopropan-2-yl, 2-fluoropropan-2-yl, 2-chloropropan-2-yl, 1,1-difluoroethyl, difluoromethyl and 1-fluorocyclopropyl;
or, more preferably selected from 2-cyanopropyl-2-yl.

In some embodiments, the compound is further represented by the following formula: 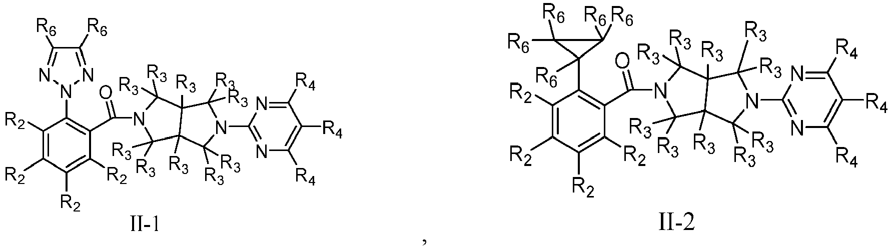 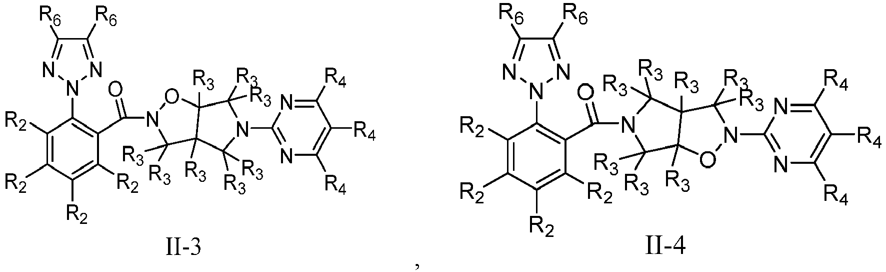 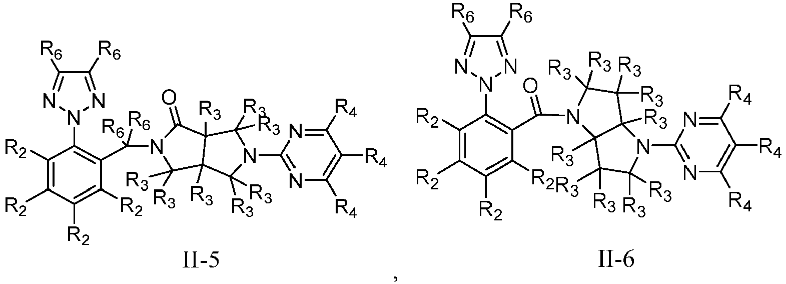 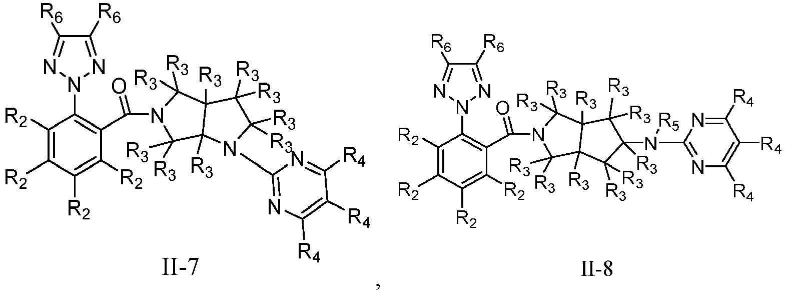 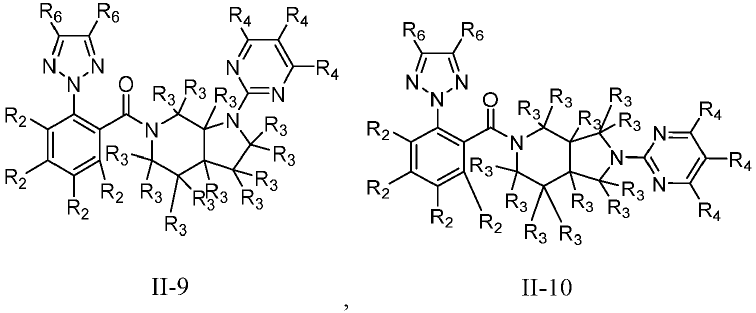 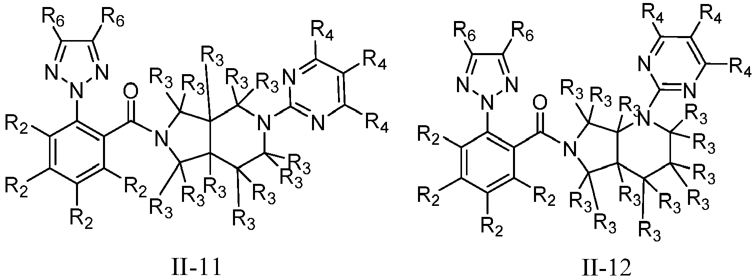 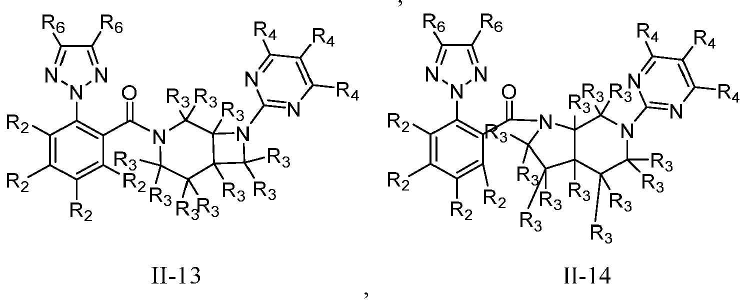 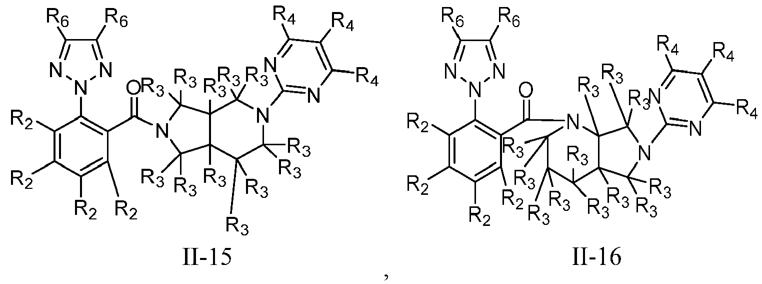
each R₆ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
preferably selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
preferably selected from the group consisting of H, fluorine, chlorine, bromine, iodine, cyano, methyl, ethyl, propyl, isopropyl, butyl and *tert*-butyl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, the compound is further represented by the following formula: 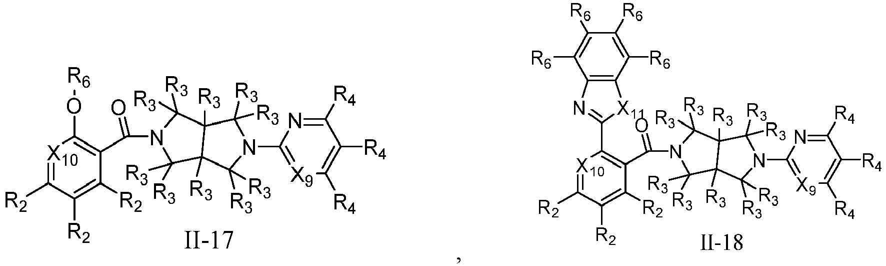 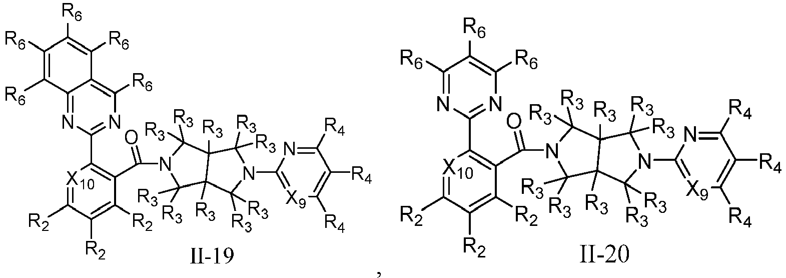 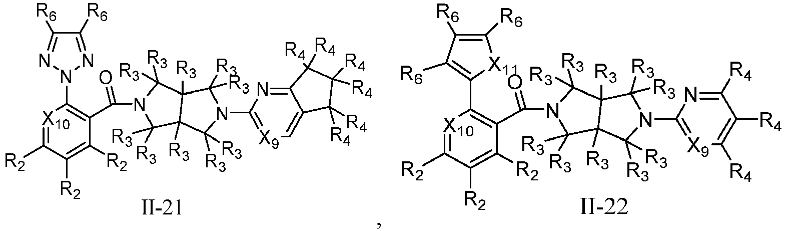 or 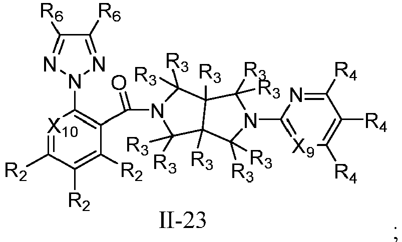 wherein:
X₉ is CR₄ or N;
X₁₀ is CR₂ or N;
X₁₁ is O or S;
the substituents R₂, R₃, R₄, R₆ are as defined above.

In some embodiments, the compound is further represented by the following formula: 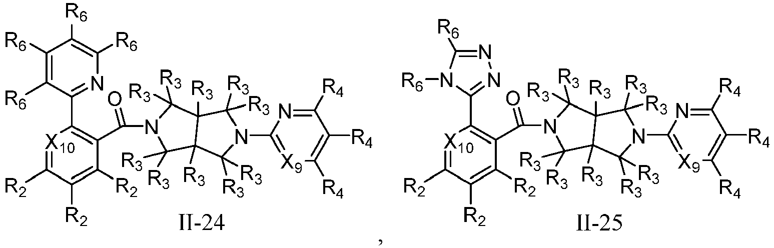 or 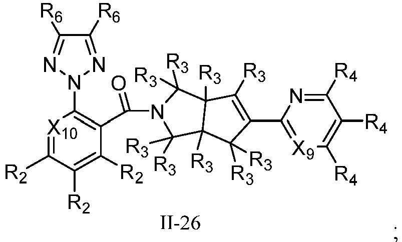 wherein:
X₉ is CR₄ or N;
X₁₀ is CR₂ or N;
the substituents R₂, R₃, R₄, R₆ are as defined above.

In formulas II-1, II-3, II-4, II-5, II-6, II-7, II-8, II-9, II-10, II-11, II-12, II-13, II-14, II-15, II-16, II-21, II-23, II-26, preferably at least one R₂ is halogen, the halogen is preferably located in the ortho or meta position of the triazole, and more preferably in the ortho position; the halogen is preferably fluorine.

In formula II-20, preferably at least one R₂ is halogen, the halogen is preferably located at the ortho or meta position of the pyrimidine, more preferably at the meta position of the pyrimidine, and/or at the ortho position of the connecting carbonyl; the halogen is preferably fluorine.

In some embodiments, the compound is further represented by the following formula:

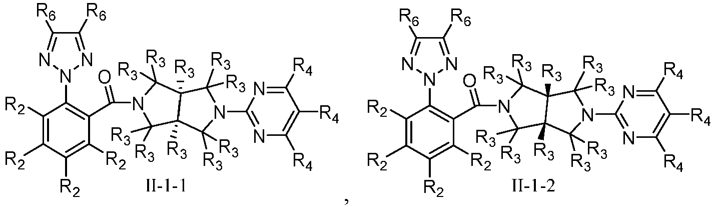

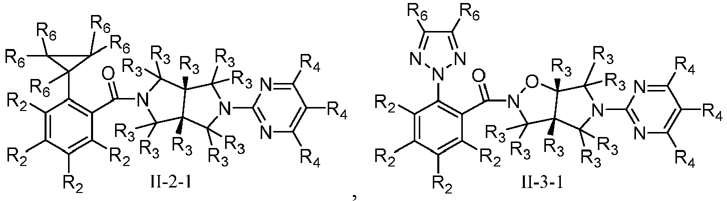

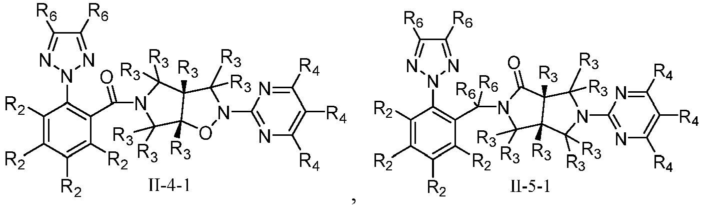

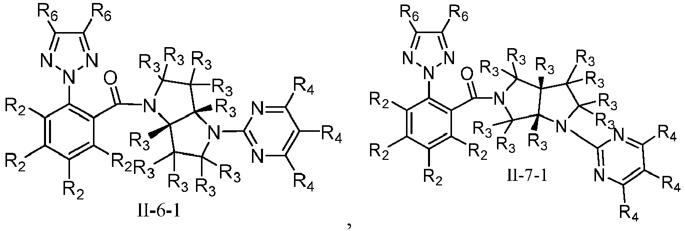

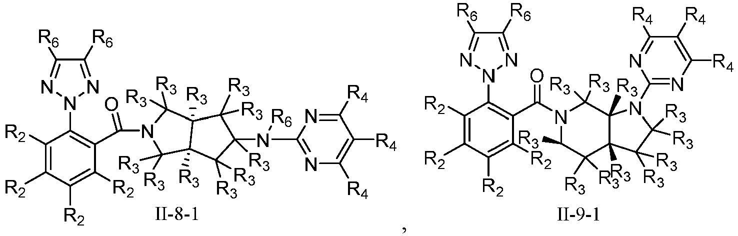

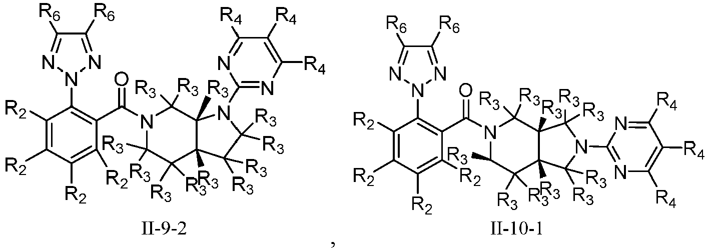

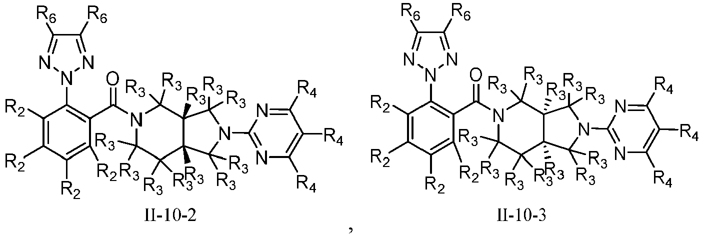

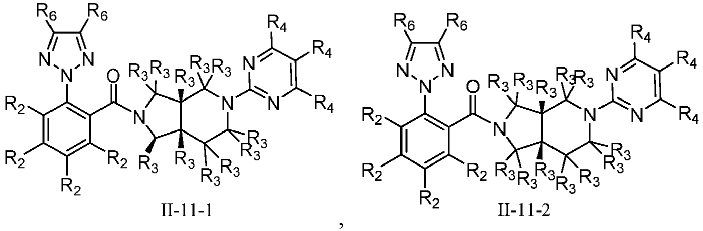

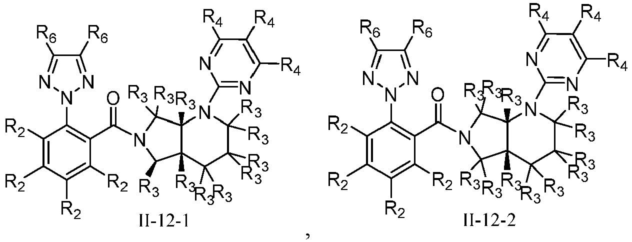

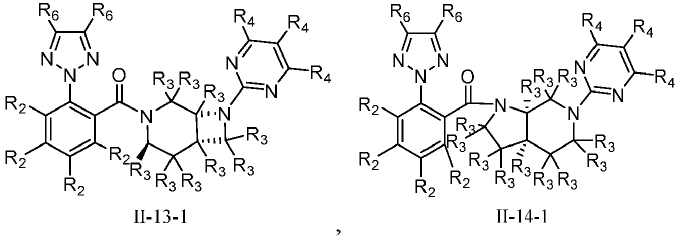

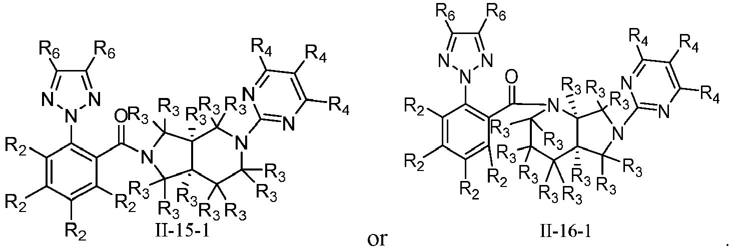

In some embodiments, the compound is further represented by the following formula:

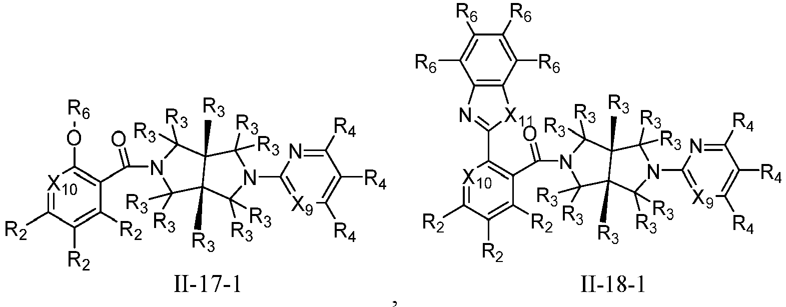

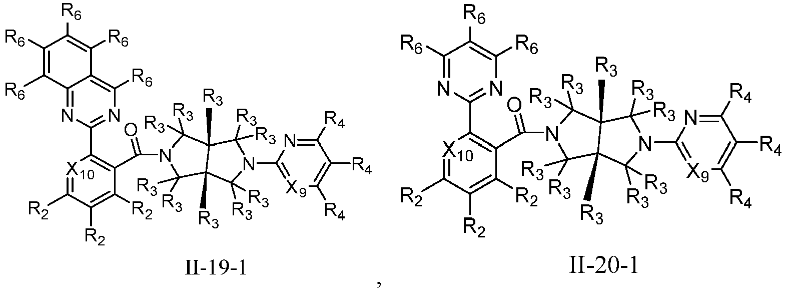

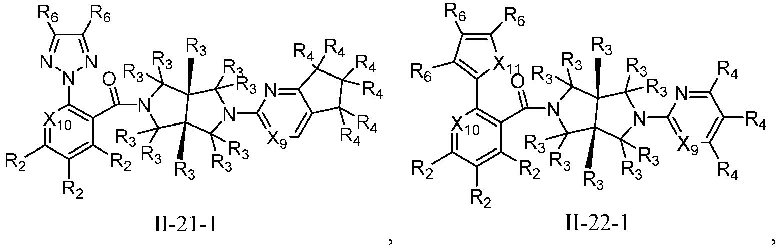

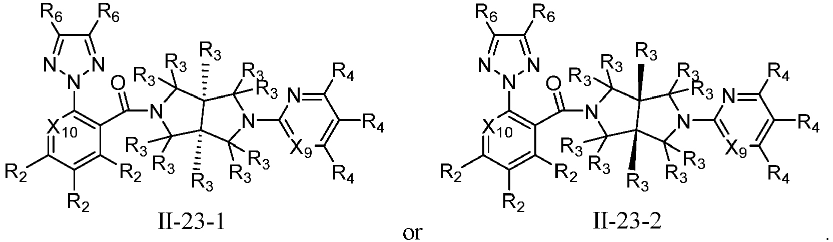

In some embodiments, the compound is further represented by the following formula:

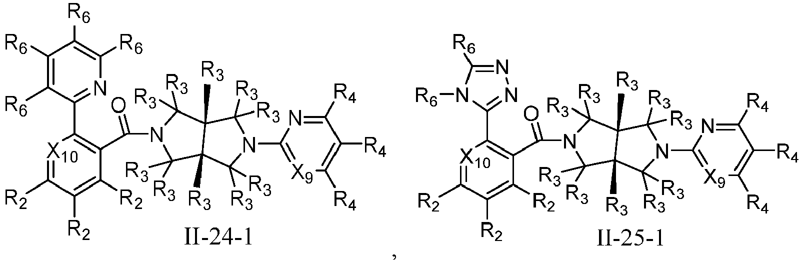

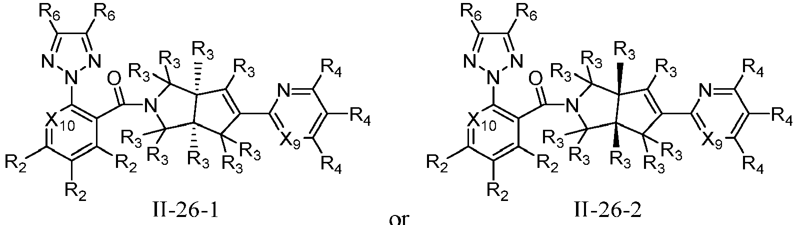

In any formula of the present invention, the substituents may also have the following definitions:
at least one of substituent R₄ is preferably selected from the group consisting of branched alkyl, linear alkyl containing multiple substituents, and cycloalkyl, the branched alkyl and cycloalkyl are each optionally further substituted by one or more substituents; the cycloalkyl is preferably cyclopropane;
preferably, the substituents of the branched alkyl and cycloalkyl, and the substituents of the branched alkyl containing multiple substituents are each independently selected from the group consisting of halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy, and more preferably halogen, amino, hydroxy, thiol and cyano;
at least two of the multiple substituents of the linear alkyl containing multiple substituents are connected to the carbon atom which is directly connected to ring C;
   and/or,
at least one of substituent R₄ is preferably selected from the group consisting of hydroxyalkyl and haloalkyl, each optionally further substituted, the hydroxyalkyl is preferably selected from the group consisting of 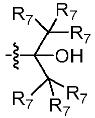 and 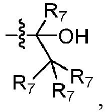 the haloalkyl can be preferably selected from the group consisting of 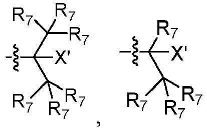 and 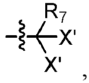 specifically 1-hydroxyethyl, 2-hydroxypropan-2-yl, 2-halopropan-2-yl and the like, the 2-halopropan-2-yl is preferably 2-fluoropropan-2-yl, the hydroxyalkyl and haloalkyl can be located in any position of multiple R₄ substitution positions in the formula, and preferably adjacent to N on the heterocycle;
   and/or,
at least one of substituent R₄ is preferably selected from the group consisting of thiolalkyl, aminoalkyl and cyanoalkyl, each optionally further substituted, the thiolalkyl is preferably selected from the group consisting of 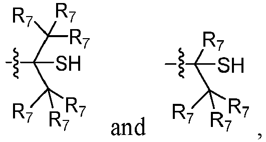 the aminoalkyl can be preferably selected from the group consisting of 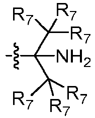 and 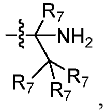 the cyanoalkyl can be preferably selected from the group consisting of 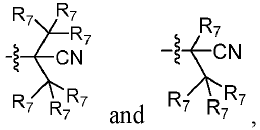 specifically 2-cyanopropan-2-yl, 2-aminopropan-2-yl and the like, the thiolalkyl, aminoalkyl and cyanoalkyl can be located in any position of multiple R₄ substitution positions in the formula, and preferably adjacent to N on the heterocycle;
   and/or,
at least one of substituent R₄ is preferably a group capable of forming hydrogen bonds, the group capable of forming hydrogen bonds is a group capable of forming hydrogen bonds with the substituents on the branched alkyl, linear alkyl containing multiple substituents, and cycloalkyl, or a group capable of forming hydrogen bonds with the hydroxy, halogen and the like of hydroxyalkyl and haloalkyl, or a group capable of forming hydrogen bonds with the thiol, amino and the like of thiolalkyl and aminoalkyl, specifically hydroxy, amino, carboxy, F and the like, the group capable of forming hydrogen bonds is preferably adjacent to the branched alkyl, linear alkyl containing multiple substituents, cycloalkyl, hydroxyalkyl, haloalkyl, thiolalkyl, aminoalkyl and cyanoalkyl in order to form hydrogen bonds, the formation of hydrogen bonds can maintain high selectivity while increasing the B/P value; when ring C is a 6-membered aryl or heteroaryl, the group capable of forming hydrogen bonds is preferably located at the para-position of the connecting point between ring C and ring B;
   and/or,
preferably at least one of substituent R₄ is an alkyl, which is optionally further substituted, specifically methyl, ethyl and the like; the alkyl can be located in any position of multiple R₄ substitution positions in the formula, and preferably located in the ortho, meta or para position of the above hydroxyalkyl and haloalkyl,
or located in the ortho, meta or para position of the above thiolalkyl, aminoalkyl and cyanoalkyl;
   and/or,
at least one of substituent R₆ is a cycloalkyl, optionally further substituted, for example 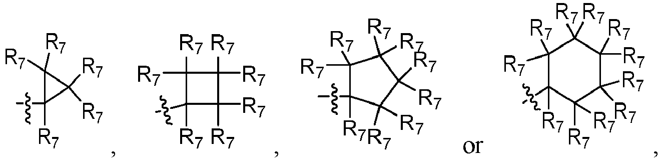 specifically cyclopropyl, cyclobutyl, cyclopentyl, cyclohexyl and the like, the cycloalkyl can be located in any position of multiple R₆ substitution positions in the formula;
   and/or,
substituent R₆ is a haloalkyl;
   and/or,
R₂ at the ortho position of the carbonyl can be a hydroxy;
the above haloalkyl can also be halomethyl, haloethyl, halopropyl, haloisopropyl, halobutyl, halo*tert*-butyl, haloisobutyl, haloisoamyl and the like, the halogen is preferably one or more selected from the group consisting of F, Cl, Br and I, wherein the haloalkyl comprises at least one halogen, and one, two or three halogens can be connected to the primary carbon of the haloalkyl, one or two halogens can be connected to the secondary carbon of the haloalkyl, and one halogen can be connected to the tertiary carbon of the haloalkyl, and one, two or three H which is not connected to halogen on the primary carbon of the haloalkyl can be replaced by R₇, one or two H which is not connected to halogen on the secondary carbon can be replaced by R₇, HH which is not connected to halogen on the tertiary carbon can be replaced by R₇, and the haloalkyl can specifically be trifluoromethyl, difluoromethyl, 1,1,2,2-tetrafluoroethyl, perfluoroethyl, 2-fluoropropan-2-yl and the like;
X' is halogen;
each R₇ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
preferably selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
preferably selected from the group consisting of hydrogen, fluorine, chlorine, bromine, iodine, cyano, methyl, ethyl, propyl, isopropyl, butyl and *tert*-butyl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

The present invention further relates to a compound of formula (III), a stereoisomer thereof or a pharmaceutically acceptable salt thereof: 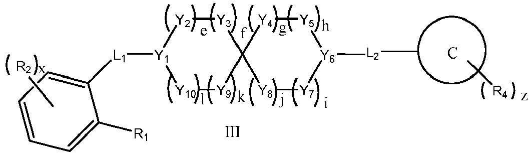 wherein:
Y₁ and Y₆ are each independently selected from the group consisting of N and CR₃;
Y₂, Y₃, Y₄, Y₅, Y₇, Y₈, Y₉ and Y₁₀ are each independently selected from the group consisting of -NR₃-, -C(R₃)₂-, -CO-, -O- and -S-;
ring C is selected from the group consisting of cycloalkyl, heterocyclyl, aryl and heteroaryl, and ring C is not a 5 membered heteroaryl;
L₁ and L₂ are each independently selected from the group consisting of a bond, -[C(R₅)₂]ₙ-, -[C(R₅)₂-O]ₙ-, -O-, -CO-, -COO-, -OOC-, -CON(R₅)-, -N(R₅)CO-, -N(R₅)- and -C(R₅)₂N(R₅)-; L₁ is preferably -CO-; and L₂ is preferably a bond;
R₁, R₂, R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
each R₅ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₁, R₂, R₃, R₄, R₅ can be connected to form a cycloalkyl, heterocyclyl, aryl or heteroaryl, the cycloalkyl, heterocyclyl, aryl or heteroaryl can be optionally further substituted;
e is selected from the group consisting of 0, 1 and 2;
f is selected from the group consisting of 0, 1 and 2;
g is selected from the group consisting of 0, 1 and 2;
h is selected from the group consisting of 0, 1 and 2;
i is selected from the group consisting of 0, 1 and 2;
j is selected from the group consisting of 0, 1 and 2;
k is selected from the group consisting of 0, 1 and 2;
l is selected from the group consisting of 0, 1 and 2;
x is selected from the group consisting of 0, 1, 2, 3 and 4;
z is selected from the group consisting of 0, 1, 2, 3, 4 and 5;
n is selected from the group consisting of 1, 2, 3, 4, 5 and 6.

In some embodiments, each R₁ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, C₃₋₆ cycloalkyl, 5 to 6 membered heteroaryl and C₁₋₃ haloalkoxy, the 5 to 6 membered heteroaryl and C₁₋₃ haloalkoxy are each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
more further preferably selected from the group consisting of 1H-1,2,3-triazol-1-yl, 2H-1,2,3-triazol-2-yl, cyclopropyl, 1H-1,2,3-triazol-1-yl, 2H-1,2,3-triazol-2-yl and cyclopropyl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, each R₂ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen and halogen, the halogen is preferably selected from the group consisting of fluorine, chlorine, bromine and iodine;
optionally, R₂ is preferably located at the meta or para position of R₁.

In some embodiments, Y₁ is independently selected from -CR₃-, Y₆ is independently selected from N, Y₉ is independently selected from -O-, Y₂, Y₃, Y₄, Y₅, Y₇, Y₈ and Y₁₀ are each independently selected from -C(R₃)₂-, e=1, f 1, g=0, h=1, i=1, j=0, k=1, l=1;
or
Y₁ and Y₆ are each independently selected from N, Y₄ is independently selected from -O-, Y₂, Y₃, Y₅, Y₇, Y₈, Y₉ and Y₁₀ are each independently selected from -C(R₃)₂-, e=1, f=0, g=1, h=2, i=1, j=0, k=0, l=1;
   or
Y₁ and Y₆ are each independently selected from N, Y₂, Y₃, Y₄, Y₅, Y₇, Y₈, Y₉ and Y₁₀ are each independently selected from -C(R₃)₂-, e=1, f=0, g=1, h=1, i=0, j=0, k=1, l=1;
   or
Y₁ and Y₆ are each independently selected from N, Y₂, Y₃, Y₄, Y₅, Y₇, Y₈, Y₉ and Y₁₀ are each independently selected from -C(R₃)₂-, e=1, f=0, g=2, h=1, i=0, j=0, k=1, l=1;
   and/or
each R₃ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl.

In some embodiments, the compound is further represented by the following formula: 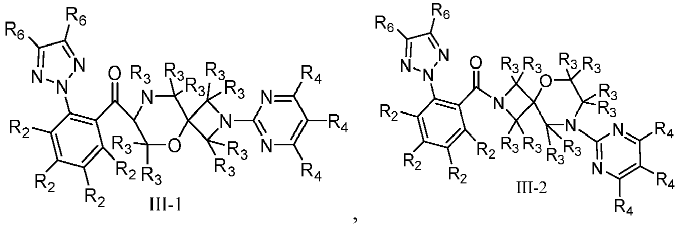 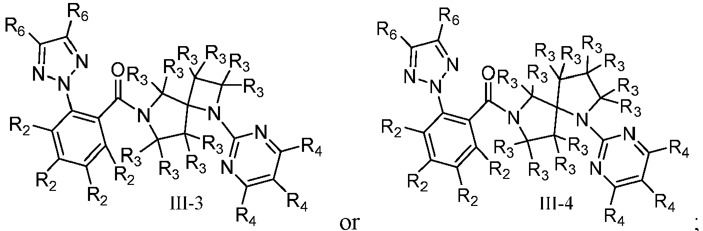 wherein:
each R₆ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
preferably selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
preferably selected from the group consisting of H, fluorine, chlorine, bromine, iodine, cyano, methyl, ethyl, propyl, isopropyl, butyl and tert-butyl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

The present invention further relates to a compound of formula (IV), a stereoisomer thereof or a pharmaceutically acceptable salt thereof: 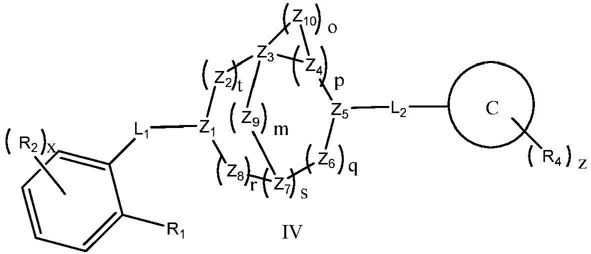 wherein:
Z₁ and Z₅ are each independently selected from the group consisting of N and CR₃;
Z₂, Z₆, Z₈, Z₉ and Z₁₀ are each independently selected from the group consisting of -NR₃-, -C(R₃)₂-, -CO-, -O- and -S-;
when m is 0, then Z₃ and Z₇ are each independently selected from the group consisting of -NR₃-, -C(R₃)₂-, -CO-, -O- and -S-;
when m is not 0, then Z₃ and Z₇ are each independently selected from the group consisting of N and CR₃;
when o is 0, then each Z₄ is independently selected from the group consisting of -NR₃-, -C(R₃)₂-, -CO-, -O- and -S-;
when o is not 0, then each Z₄ is independently selected from the group consisting of N and CR₃;
ring C is selected from the group consisting of cycloalkyl, heterocyclyl, aryl and heteroaryl, and ring C is not a 5 membered heteroaryl;
L₁, L₂ are each independently selected from the group consisting of a bond, -[C(R₅)₂]ₙ-, -[C(R₅)₂-O]ₙ-, -O-, -CO-, -COO-, -OOC-, -CON(R₅)-, -N(R₅)CO-, -N(R₅)-and -C(R₅)₂N(R₅)-; L₁ is preferably selected from the group consisting of -CO-, -CON(R₅)- and -N(R₅)CO-; L₂ is preferably selected from the group consisting of a bond and -N(R₅)-;
R₁, R₂, R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
each R₅ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₁, R₂, R₃, R₄, R₅ can be connected to form a cycloalkyl, heterocyclyl, aryl or heteroaryl, the cycloalkyl, heterocyclyl, aryl or heteroaryl can be optionally further substituted;
m is selected from the group consisting of 0, 1 and 2;
o is selected from the group consisting of 0, 1, 2, 3, 4, 5 and 6;
p is selected from the group consisting of 0 and 1;
q is selected from the group consisting of 0 and 1;
s is selected from the group consisting of 0 and 1;
r is selected from the group consisting of 0 and 1;
t is selected from the group consisting of 0 and 1;
x is selected from the group consisting of 0, 1, 2, 3 and 4;
z is selected from the group consisting of 0, 1, 2, 3, 4 and 5;
n is selected from the group consisting of 1, 2, 3, 4, 5 and 6.

In some embodiments, each R₁ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen, halogen, C₃₋₆ cycloalkyl, 5 to 6 membered heteroaryl and C₁₋₃ haloalkoxy, the 5 to 6 membered heteroaryl and C₁₋₃ haloalkoxy are each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
more further preferably selected from the group consisting of 1H-1,2,3-triazol-1-yl, 2H-1,2,3-triazol-2-yl, 2,2,2-trifluoroethoxy, 1H-1,2,3-triazol-1-yl, 2H-1,2,3-triazol-2-yl and 2,2,2-trifluoroethoxy, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, each R₂ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more preferably selected from the group consisting of hydrogen and halogen, the halogen is preferably selected from the group consisting of fluorine, chlorine, bromine and iodine;
optionally, R₂ is preferably located at the meta or para position of R₁.
In some embodiments,
m=0, o=1, p=1, q=0, r=0, s=1, t=0, Z₁ is independently selected from N, Z₃, Z₄, Z₅ are each independently selected from CR₃, Z₇, Z₁₀ are each independently selected from -C(R₃)₂-;
m=1, o=0, p=1, q=1, t=1, s=1, t=1, Z₁, Z₅ are each independently selected from N, Z₃, Z₇ are each independently selected from CR₃, Z₂, Z₄, Z₆, Z₈ are each independently selected from -C(R₃)₂-;
m=2, o=0, p=1, q=1, t=1, s=1, t=0, Z₁, Z₅ are each independently selected from N, Z₃, Z₇ are each independently selected from CR₃, Z₄, Z₆, Z₈, Z₉ are each independently selected from -C(R₃)₂-;
m=1, o=0, p=1, q=1, t=1, s=1, t=0, Z₁, Z₅ are each independently selected from N, Z₃, Z₇ are each independently selected from CR₃, Z₄, Z₆, Z₈, Z₉ are each independently selected from -C(R₃)₂-;
m=1, o=0, p=0, q=1, r=0, s=1, t=1, Z₁, Z₅ are each independently selected from N, Z₃, Z₇ are each independently selected from CR₃, Z₂, Z₆, Z₉ are each independently selected from -C(R₃)₂-;
m=0, o=0, p=0, q=0, r=1, s=1, t=1, Z₁, Z₅ are each independently selected from N, Z₂, Z₃, Z₇, Z₈ are each independently selected from -C(R₃)₂-;
m=0, o=0, p=0, q=0, r=1, s=1, t=1, Z₁ is independently selected from N, Z₅ is independently selected from CR₃, Z₂, Z₃, Z₇, Z₈ are each independently selected from -C(R₃)₂-;
m=0, o=0, p=0, q=0, r=1, s=1, t=1, Z₁ is independently selected from CR₃, Z₅ is independently selected from N, Z₂, Z₃, Z₇, Z₈ are each independently selected from -C(R₃)₂-;
m=0, o=0, p=0, q=1, r=1, s=1, t=1, Z₁ is independently selected from N, Z₅ is independently selected from CR₃, Z₂, Z₃, Z₆, Z₇, Z₈ are each independently selected from -C(R₃)₂-;
m=0, o=0, p=0, q=0, r=0, s=0, t=0, Z₁, Z₅ are each independently selected from CR₃, Z₃ is independently selected from -C(R₃)₂-,
m=0, o=0, p=0, q=0, r=0, s=1, t=0, Z₁ is independently selected from CR₃, Z₅ is independently selected from N, Z₃, Z₇ are each independently selected from -C(R₃)₂-,
   and/or
each R₃ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl.

In some embodiments, the compound is further represented by the following formula: 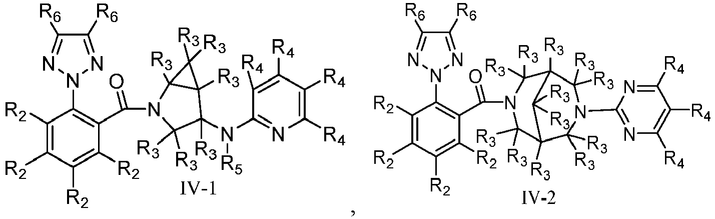 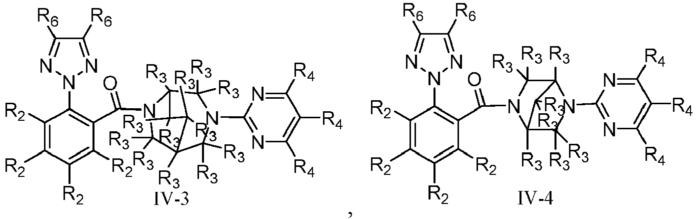 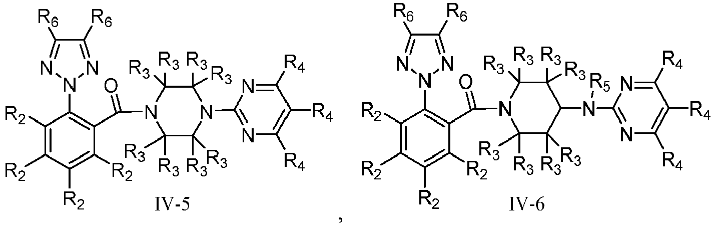 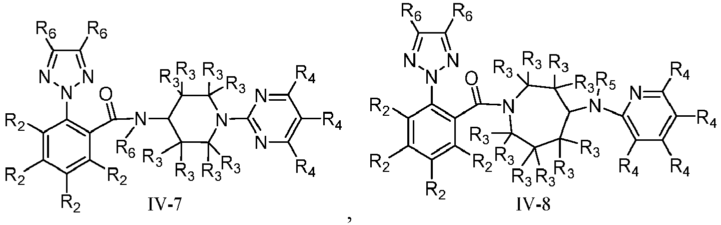 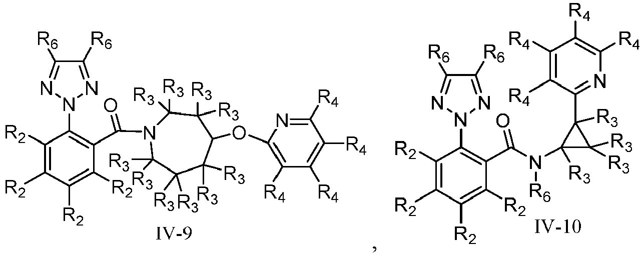 or 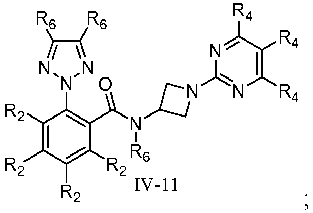 wherein:
each R₆ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
preferably selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy;
preferably selected from the group consisting of H, fluorine, chlorine, bromine, iodine, cyano, methyl, ethyl, propyl, isopropyl, butyl and tert-butyl, each optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

In some embodiments, the compound is further represented by the following formula:

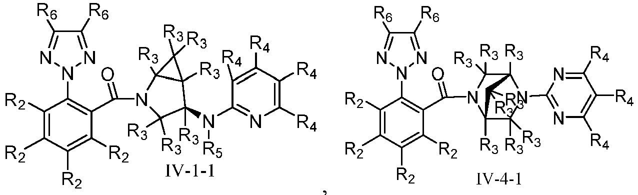

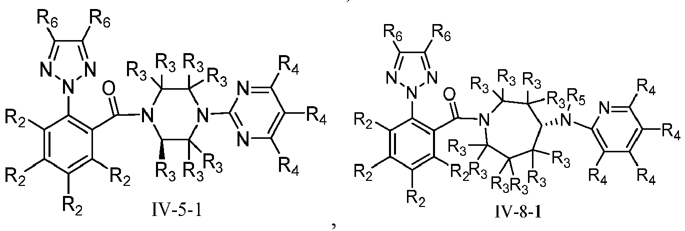

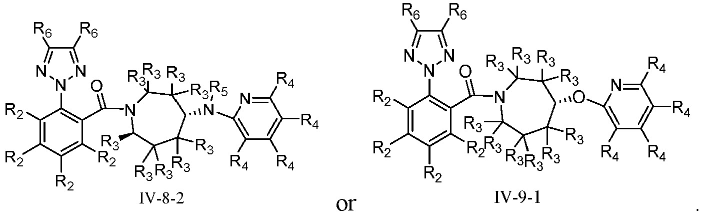

In the present invention, and/or 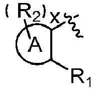 is preferably

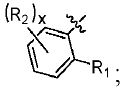

In the present invention, R₁ is preferably: 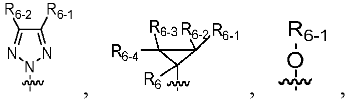 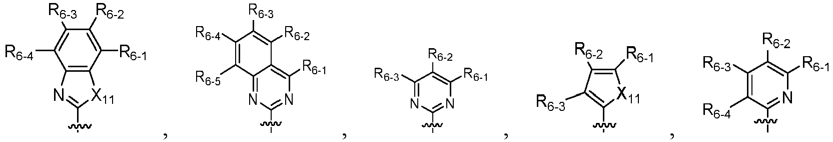 or 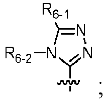 and/or 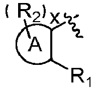 is preferably: 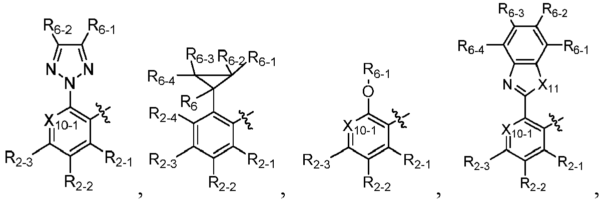 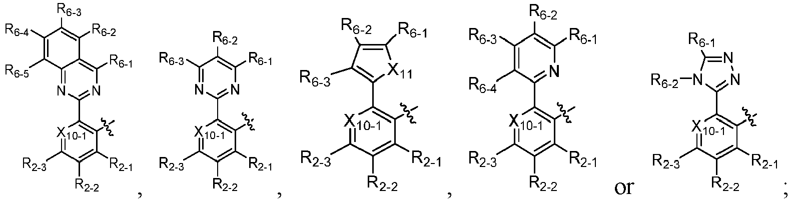 ring B is preferably: 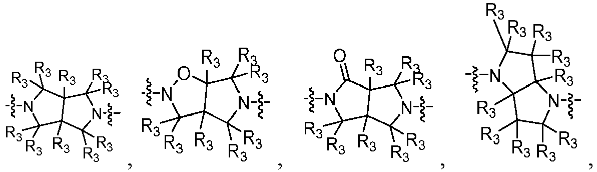 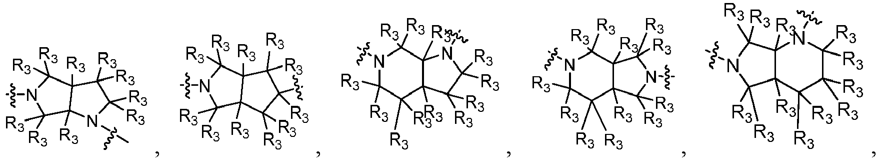 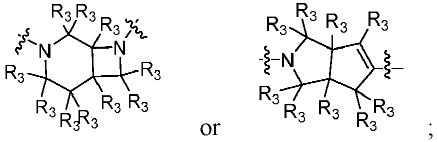 more preferably 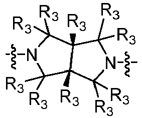 or 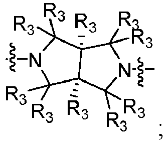 specifically 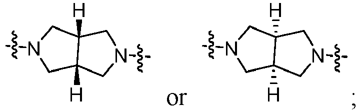 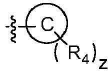 is preferably 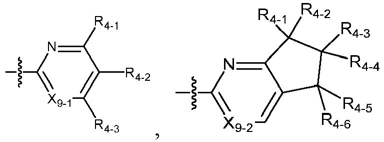 and the like; more preferably 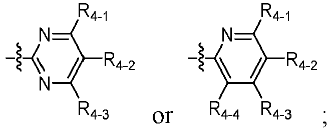 wherein:
X₉₋₁ is CR₄₋₄ or N;
X₉₋₂ is CR₄₋₇ or N;
X₁₀₋₁ is CR₂₋₄ or N;
X₁₁ is O or S;
the substituents R₂₋₁, R₂₋₂, R₂₋₃, R₂₋₄ are each independently as defined as R₂;
the substituents R₄₋₁, R₄₋₂, R₄₋₃, R₄₋₄, R₄₋₅, R₄₋₆, R₄₋₇ are each independently as defined as R₄;
the substituents R₆₋₁, R₂₋₂, R₆₋₃, R₆₋₄, R₆₋₅, R₆₋₆ are each independently as defined as R₆;
the substituent R₃ is as defined above.

The present invention also provides the following compound: 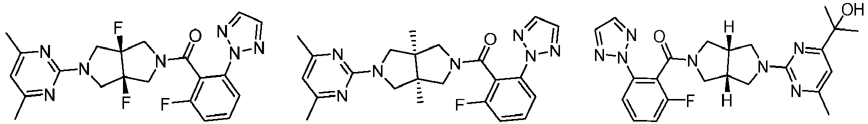 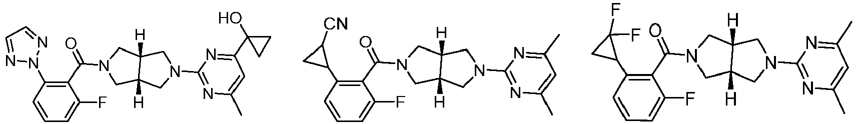 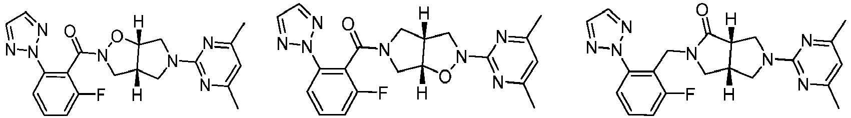 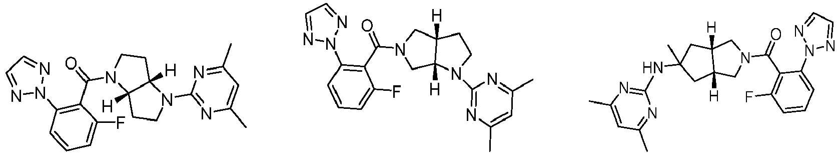 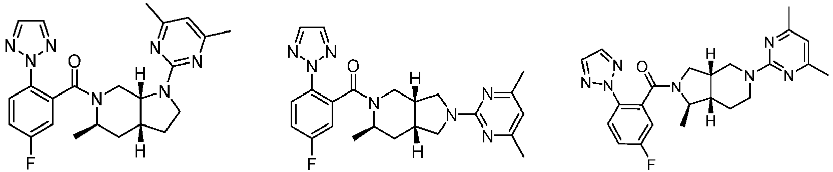 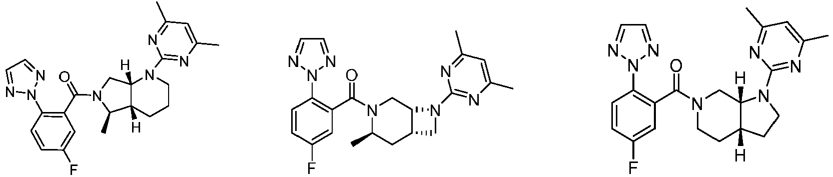 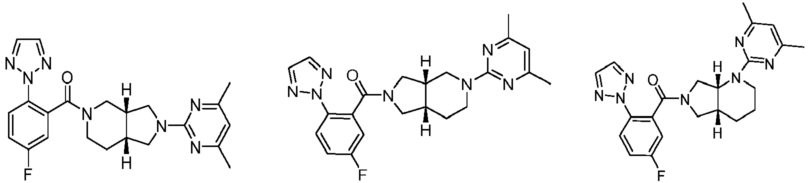 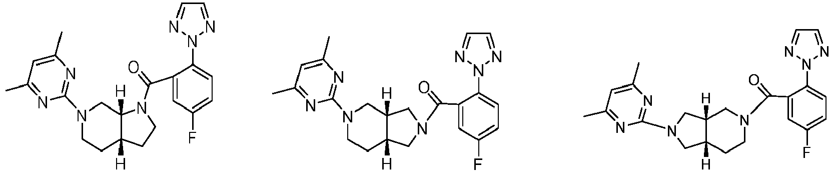 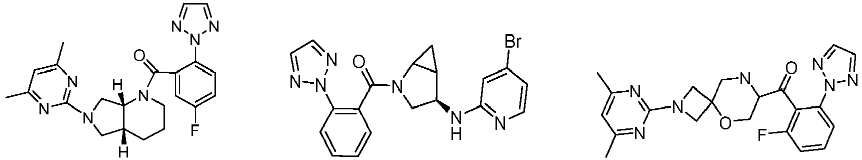 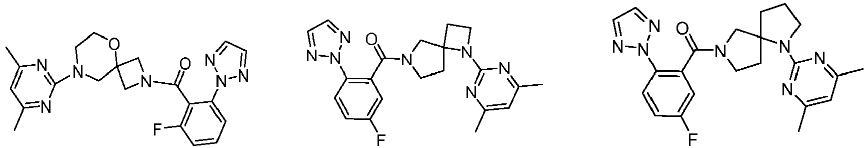 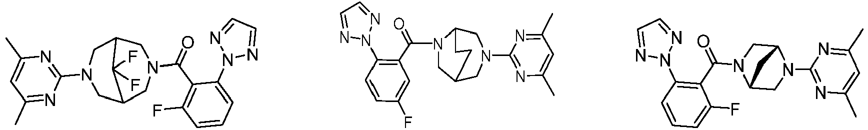 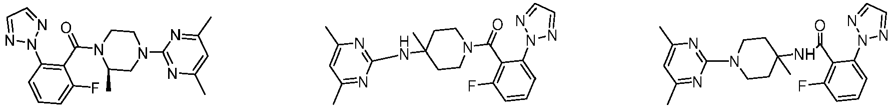 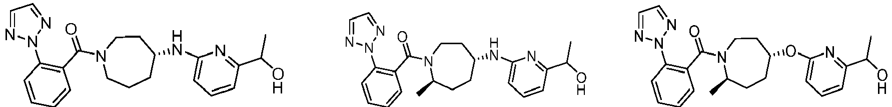 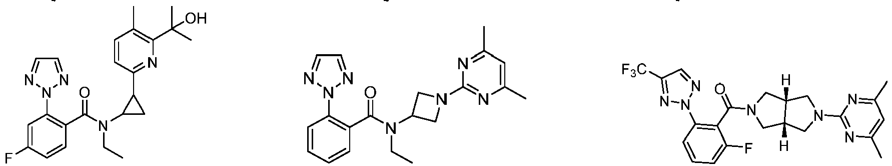 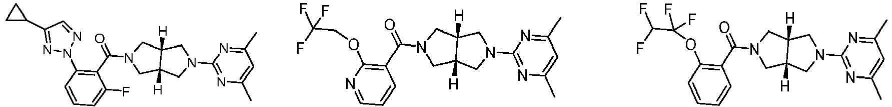 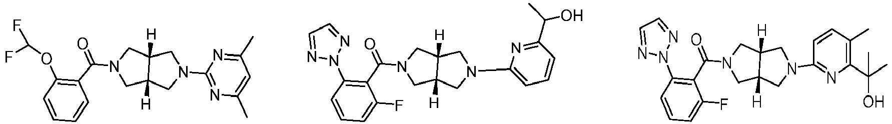 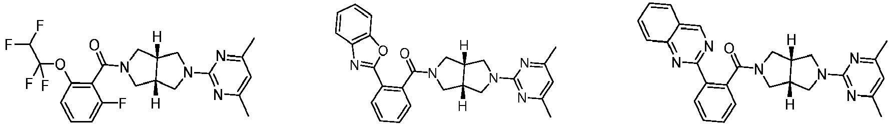 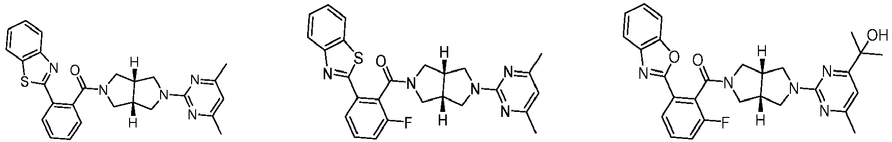 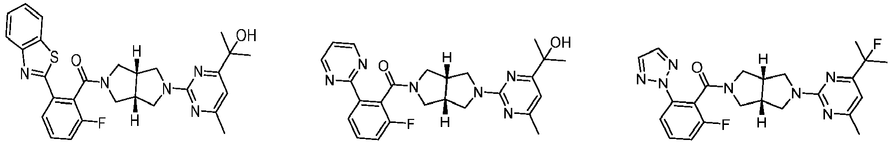 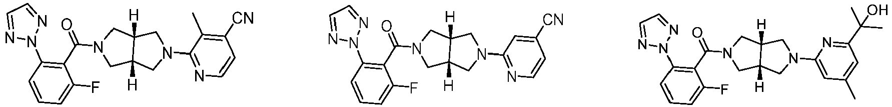 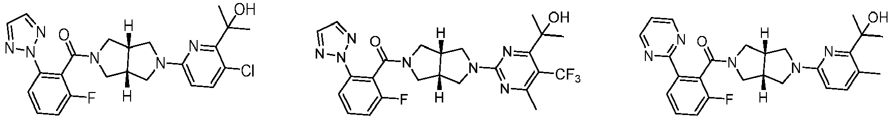 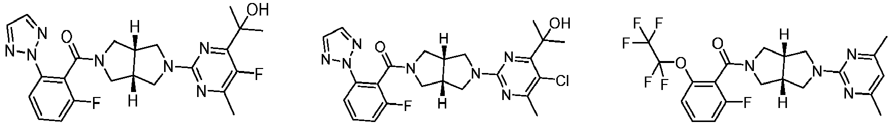 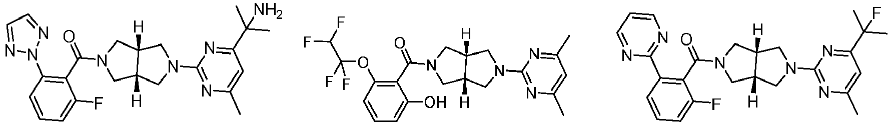 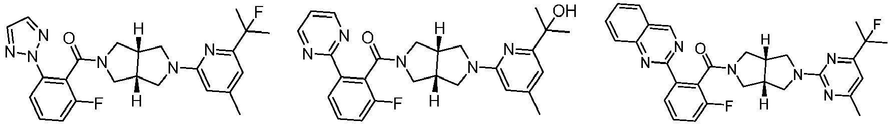 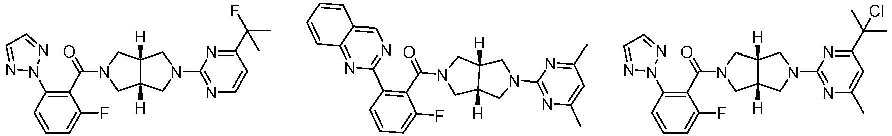 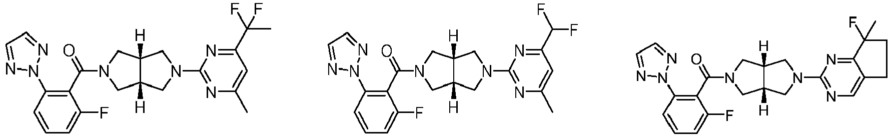 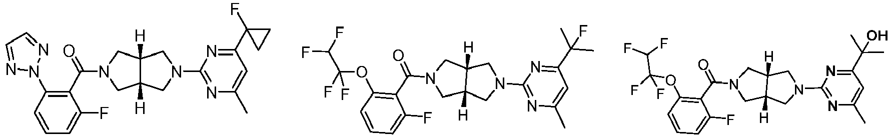 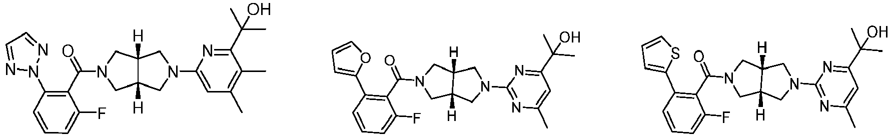 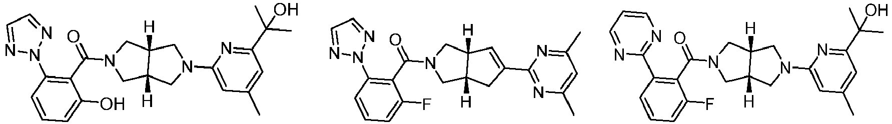 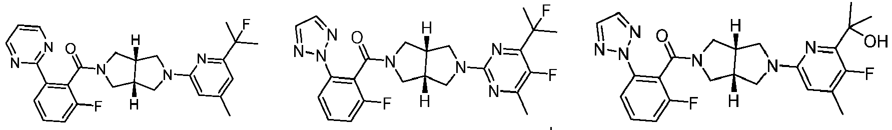 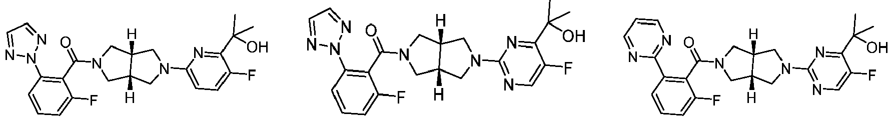 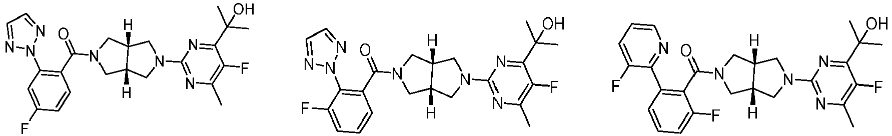 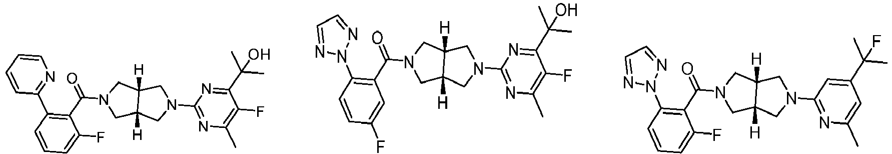 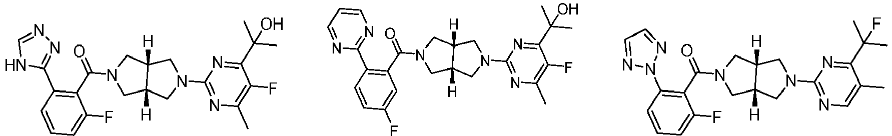 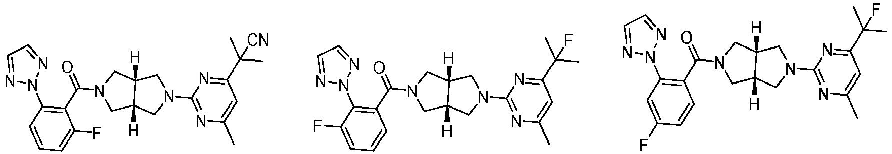 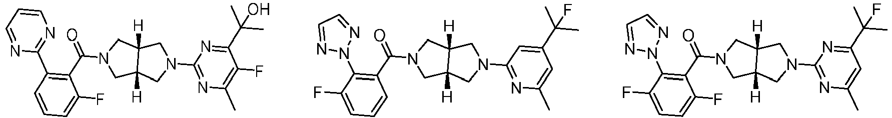 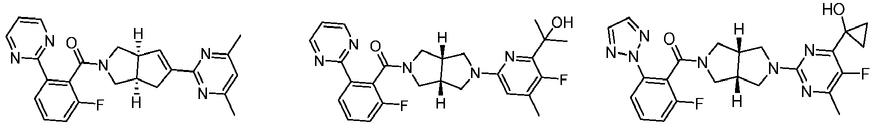 or

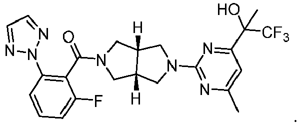

The present invention further provides an intermediate, a stereoisomer thereof or a pharmaceutically acceptable salt thereof, the intermediate, a stereoisomer thereof or a pharmaceutically acceptable salt thereof is used to prepare the compound of the present invention;
the intermediate is represented by formula II-17': 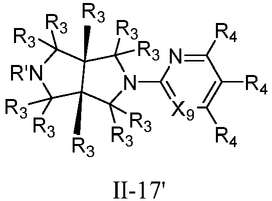
wherein, X₉, R₃, R₄ are as defined above;
R' is H or an amino protecting group, and the amino protecting group is preferably *tert*-butoxycarbonyl, benzyloxycarbonyl, *p*-toluenesulfonyl, trityl, formyl, trifluoroacetyl, and the like.

The structure of the intermediate is as follows:

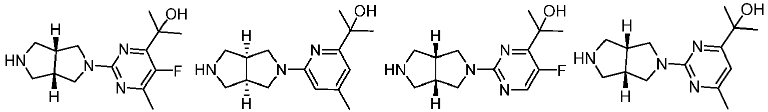

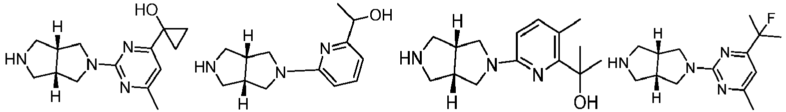

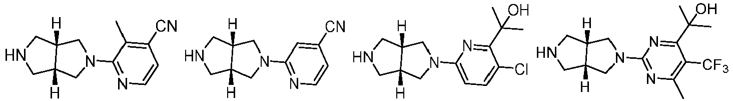

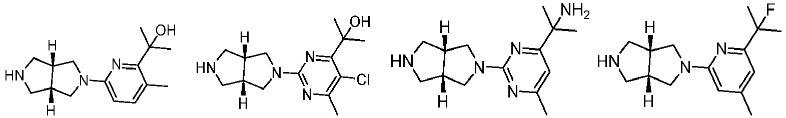

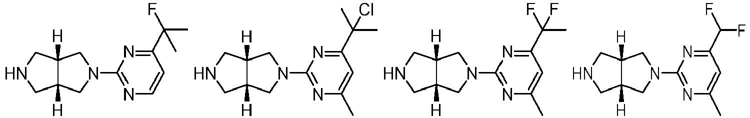

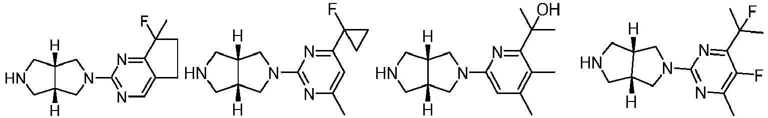

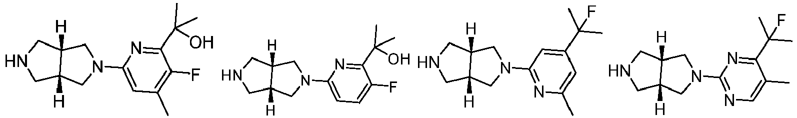

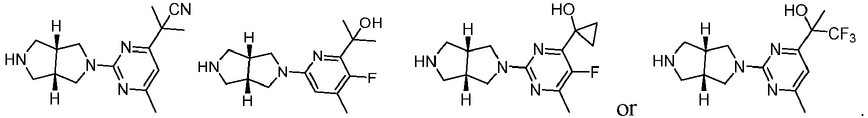

The present invention further relates to a pharmaceutical composition comprising a therapeutically effective dose of the compound, a stereoisomer thereof or a pharmaceutically acceptable salt thereof of the present invention, and one or more pharmaceutically acceptable carriers or excipients.

The present invention further relates to a use of the compound, a stereoisomer thereof or a pharmaceutically acceptable salt thereof of the present invention, or the pharmaceutical composition of the present invention in the preparation of an orexin receptor antagonist, preferably an OX2R selective receptor antagonist.

The present invention further relates to a use of the compound, a stereoisomer thereof or a pharmaceutically acceptable salt thereof of the present invention, or the pharmaceutical composition of the present invention in the preparation of a medicament for the treatment of nervous system disease; wherein the nervous system disease is preferably selected from the group consisting of insomnia, depression, anxiety and drug addiction, and more preferably selected from the group consisting of major depressive disorder (MDD), primary and secondary insomnia, and depression with insomnia.

The present invention further relates to a method for treating nervous system disease by using the compound, a stereoisomer thereof or a pharmaceutically acceptable salt thereof of the present invention, or the pharmaceutical composition of the present invention; the nervous system disease is preferably selected from the group consisting of insomnia, depression, anxiety and drug addiction, and more preferably selected from the group consisting of major depressive disorder (MDD), primary and secondary insomnia, and depression with insomnia.

### DEFINITIONS

Unless otherwise stated, the terms used in the specification and claims have the meanings described below.

In the present invention, alkyl refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof.

More preferably, the alkyl is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like.

The alkyl can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl. The alkyl of the present invention is preferably selected from the group consisting of methyl, ethyl, isopropyl, *tert*-butyl, haloalkyl, deuterated alkyl, alkoxy-substituted alkyl and hydroxy-substituted alkyl; the hydroxy-substituted alkyl can be 2-hydroxyisopropyl, 1-hydroxy ethyl.

In the present invention, cycloalkyl refers to a saturated or partially unsaturated monocyclic or polycyclic hydrocarbon substituent group having 3 to 20 carbon atoms, preferably 3 to 12 carbon atoms, more preferably 3 to 8 carbon atoms, and further preferably 3 to 6 carbon atoms.

Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

In the present invention, spiro cycloalkyl refers to a 5 to 20 membered polycyclic group with individual ring connected through one shared carbon atom (called a spiro atom), wherein the ring can contain one or more double bonds, but none of the ring has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include: 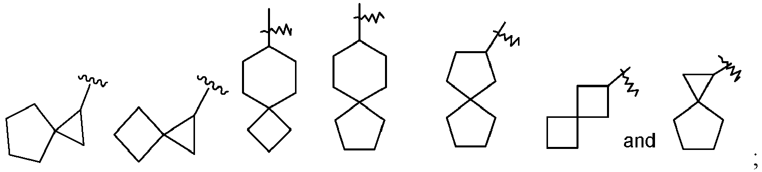 and also include spiro cycloalkyl in which a cycloalkyl and a heterocyclyl are connected through one spiro atom, non-limiting examples thereof include:

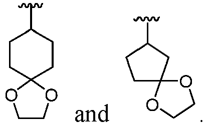

In the present invention, fused cycloalkyl refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered or 5-membered/6-membered bicyclic fused cycloalkyl. Non-limiting examples of fused cycloalkyl include:

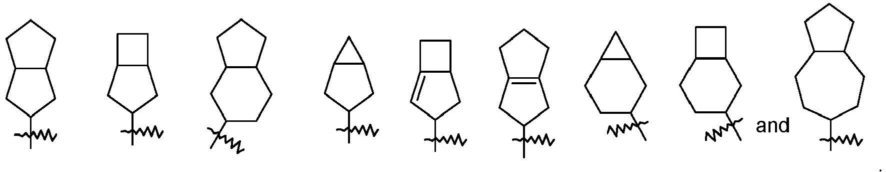

In the present invention, bridged cycloalkyl refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

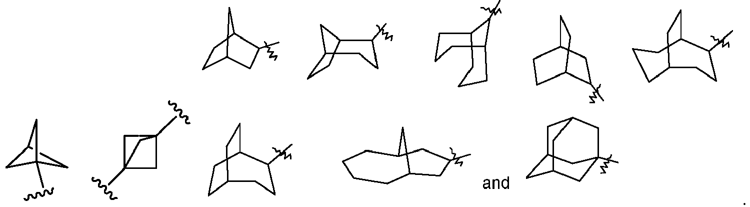

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

In the present invention, heterocyclyl refers to a 3 to 20 membered saturated or partially unsaturated monocyclic or polycyclic hydrocarbon group, wherein one or more ring atoms are heteroatoms selected from the group consisting of nitrogen, oxygen and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 10 ring atoms; and further preferably, 3 to 8 ring atoms. Non-limiting examples of monocyclic heterocyclyl include pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, amylene oxide group, imidazolidinyl, tetrahydrofuranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, 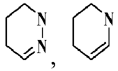 and the like; preferably pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, dihydropyrrolyl, tetrahydrofuranyl, pyrazolidinyl, morpholinyl, 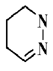 , 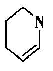 , piperazinyl and pyranyl; and more preferably dihydropyrrolyl, pyrrolidinyl, pyrrolidinonyl, piperidin-2-onyl, 3,4-dihydropyridin-2(1H)-onyl, 4,5-dihydropyridazin-3(2H)-onyl, azetidinyl, oxetanyl, amylene oxide group, morpholinyl, piperidinyl, piperazinyl, 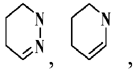 pyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

In the present invention, spiro heterocyclyl refers to a 5 to 20 membered polycyclic heterocyclyl group with individual rings connected through one shared atom (called a spiro atom), wherein one or more ring atoms are heteroatoms selected from the group consisting of nitrogen, oxygen and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

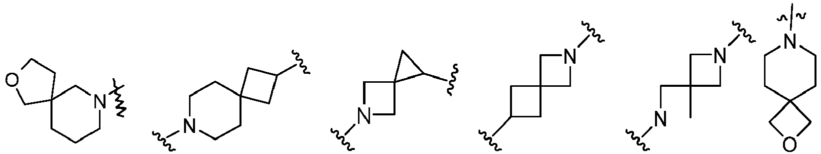

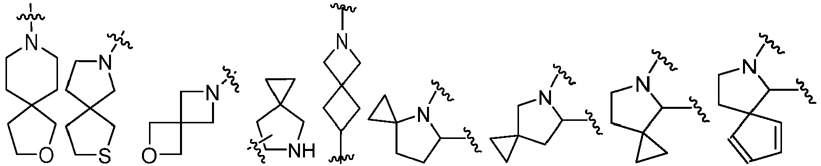

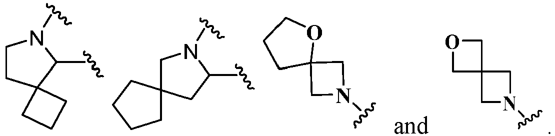

In the present invention, fused heterocyclyl refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of nitrogen, oxygen and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

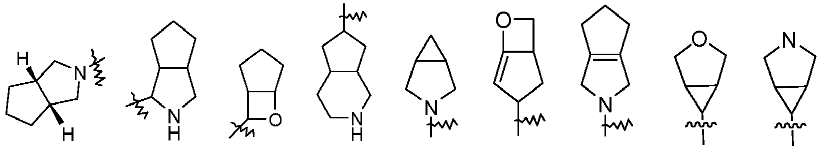

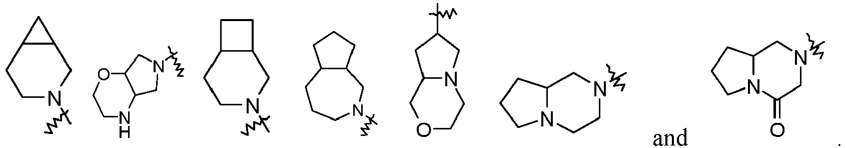

In the present invention, bridged heterocyclyl refers to a 5 to 14 membered polycyclic heterocyclyl group, wherein every two rings in the system share two disconnected atoms, wherein the rings can have one or more double bond(s), but none of the rings has a completely conjugated π-electron system, and one or more ring atoms are heteroatoms selected from the group consisting of nitrogen, oxygen and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

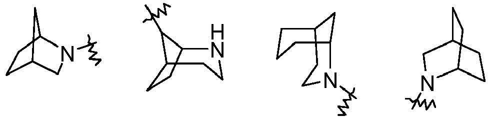

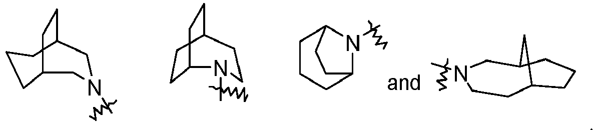

The heterocyclyl ring can be fused to the ring of aryl, heteroaryl or cycloalkyl, wherein the ring bound to the parent structure is heterocyclyl. Non-limiting examples include:

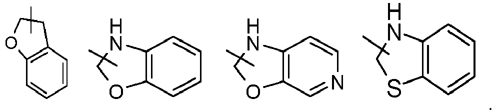

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

In the present invention, aryl refers to a 6 to 14 membered all-carbon monocyclic ring or polycyclic fused ring (*i.e*. each ring in the system shares an adjacent pair of carbon atoms with another ring in the system) having a conjugated π-electron system, preferably a 6 to 10 membered aryl, more preferably a 6 to 8 membered aryl, for example, phenyl and naphthyl, and preferably phenyl. The aryl ring can be fused to the ring of heteroaryl, heterocyclyl or cycloalkyl, wherein the ring bound to the parent structure is aryl. Non-limiting examples include:

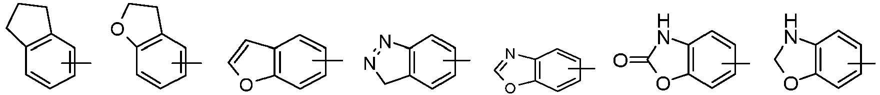

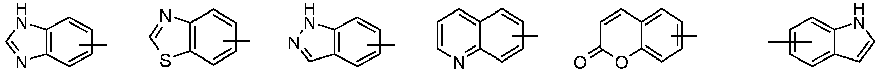

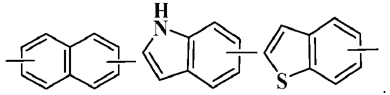

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

In the present invention, heteroaryl refers to a 5 to 14 membered heteroaromatic system having 1 to 4 heteroatoms selected from the group consisting of oxygen, sulfur and nitrogen. The heteroaryl is preferably a 5 to 10 membered heteroaryl, more preferably a 5 to 8 membered heteroaryl, and most preferably a 5 or 6 membered heteroaryl, for example pyrazinyl, pyridazinyl, imidazolyl, furyl, thienyl, thiazolyl, pyrazolyl, oxazolyl, isoxazolyl, pyrrolyl, triazolyl, tetrazolyl, pyridyl, pyrimidinyl, thiadiazole, oxadiazole, pyrazinyl and the like, and preferably pyrimidinyl, pyrazolyl, oxazolyl, isoxazolyl, oxadiazole, pyridine. The heteroaryl ring can be fused to the ring of aryl, heterocyclyl or cycloalkyl, wherein the ring bound to the parent structure is heteroaryl ring. Non-limiting examples thereof include:

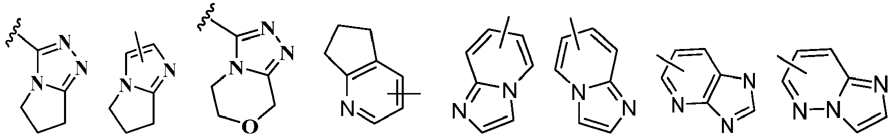

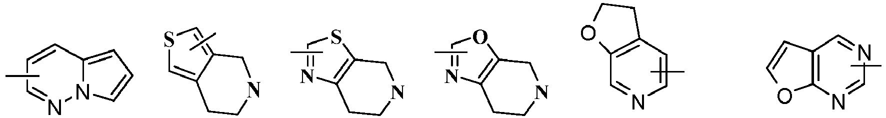

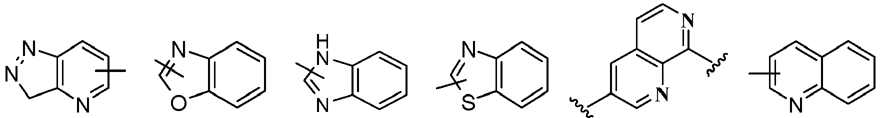

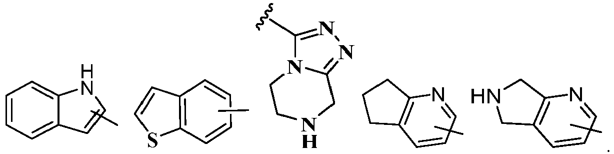

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

In the present invention, alkoxy refers to an -O-(alkyl) or an -O-(unsubstituted cycloalkyl) group, wherein the alkyl is as defined above and is preferably an alkyl having 1 to 8 carbon atoms, more preferably an alkyl having 1 to 6 carbon atoms, and most preferably an alkyl having 1 to 3 carbon atoms. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

Non-limiting examples of alkoxy also include: propan-2-oxyl and the like.

In the present invention, haloalkyl refers to an alkyl substituted by one or more halogen(s), wherein the alkyl is as defined above. Non-limiting examples of haloalkyl include: trifluoromethyl, trifluoroethyl.

Non-limiting examples of haloalkyl also include: difluoromethyl, 1,1,2,2-tetrafluoroethyl, perfluoroethyl and the like.

In the present invention, haloalkoxy refers to an alkoxy substituted by one or more halogen(s), wherein the alkoxy is as defined above.

The haloalkoxy can be fully or partially halogenated, the number of halogen can be 1, 2, 3, 4, 5, 6, 7, 8, 9, 10 and the like; the halogen is preferably F, Cl, Br, I; for example, the haloalkoxy can be trifluoromethoxy, difluoromethoxy, 1,1,2,2-tetrafluoroethoxy, perfluoroethoxy and the like.

In the present invention, hydroxyalkyl refers to an alkyl substituted by hydroxy(s), wherein the alkyl is as defined above.

In the present invention, alkenyl refers to a chain olefin, also known as alkene group. The alkenyl is preferably an alkenyl having 2 to 8 carbon atoms, more preferably an alkenyl having 2 to 6 carbon atoms, further preferably an alkenyl having 2 to 4 carbon atoms, and most preferably an alkenyl having 2 to 3 carbon atoms. Non-limiting examples of alkenyl include vinyl and allyl. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

In the present invention, alkynyl refers to a chain alkyne, also known as alkyne group, and refers to an unsaturated hydrocarbon group containing -C≡C-. The alkynyl is preferably an alkynyl having 2 to 8 carbon atoms, more preferably an alkynyl having 2 to 6 carbon atoms, further preferably an alkynyl having 2 to 4 carbon atoms, and most preferably an alkynyl having 2 to 3 carbon atoms. The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy or alkoxycarbonyl.

In the present invention, haloalkyl refers to an alkyl substituted by one or more halogen(s), wherein the alkyl is as defined above.

In the present invention, haloalkoxy refers to an alkoxy substituted by one or more halogen(s), wherein the alkoxy is as defined above.

In the present invention, hydroxyalkyl refers to an alkyl substituted by hydroxy(s), wherein the alkyl is as defined above.

In the present invention, hydroxy refers to an -OH group.

In the present invention, halogen refers to fluorine, chlorine, bromine or iodine.

In the present invention, amino refers to a -NH₂ group.

In the present invention, cyano refers to a -CN group.

In the present invention, nitro refers to a -NO₂ group.

In the present invention, carboxy refers to a -C(O)OH group.

The hydrogen atom of the present invention can be replaced by its isotope deuterium. Any of the hydrogen atoms of the compounds of the examples of the present invention can also be substituted by deuterium atom.

In the present invention, "optional" or "optionally" means that the event or circumstance described subsequently can, but need not, occur. For example, "the heterocyclyl optionally substituted by an alkyl" means that an alkyl can be, but need not be, present, and such a description includes the situation of the heterocyclyl being substituted by an alkyl and the heterocyclyl being not substituted by an alkyl.

In the present invention, "substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

In the present invention, "more" refers to 2 or more than 2, such as integers of 2, 3, 4, 5, 6, 7, 8.

In the present invention, pharmaceutical composition refers to a mixture of one or more of the compounds according to the present invention or physiologically/pharmaceutically acceptable salts or prodrugs thereof with other chemical components such as physiologically/pharmaceutically acceptable carriers and excipients. The purpose of the pharmaceutical composition is to facilitate administration of a compound to an organism, which is conducive to the absorption of the active ingredient so as to exert biological activity.

In the present invention, pharmaceutically acceptable salt refers to a salt of the compound of the present invention, which is safe and effective in mammals and has the desired biological activity.

### DETAILED DESCRIPTION OF THE INVENTION

The present invention will be further described with reference to the following examples, but the examples should not be considered as limiting the scope of the present invention.

### EXAMPLES

### Preparation of intermediates:

### Preparation of intermediate 1: 2-(2-chloro-6-methylpyrimidin-4-yl)propanol

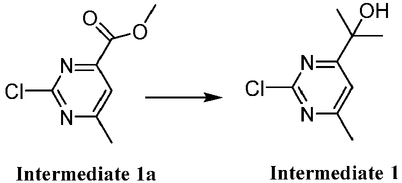

At -78°C, a solution of intermediate 1a (674 mg, 3.6 mmol) in THF (5 mL) was added to methylmagnesium bromide (5 mL, 1M in THF). The mixture was stirred at -78°C for 2 hours, then at room temperature for 12 hours. The reaction solution was quenched by slowly adding saturated ammonium chloride solution (20 mL), and extracted with dichloromethane (20 mL*3). The organic phases were combined, dried and concentrated. The residues were purified by silica gel column chromatography (petroleum ether/ethyl acetate system) to obtain intermediate 1 (362 mg, colorless oil) with a yield of 53.7%.
MS m/z (ESI): 187.1 [M+1]

### Preparation of intermediate 2: 1-(2-chloro-6-methylpyrimidin-4-yl)cyclopropan-1-ol

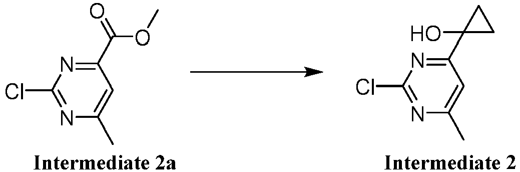

At 0°C, tetraisopropyl titanate (430 mg, 1.51 mmol) and ethylmagnesium bromide (9 mL, 1M in THF) were slowly added to a solution of intermediate 2a (560 mg, 3.00 mmol) in THF (5 mL), and the mixture was stirred at room temperature for 2 hours. The reaction solution was quenched by slowly adding saturated ammonium chloride solution (20 mL), and extracted with dichloromethane (20 mL*3). The organic phases were combined, dried and concentrated. The residues were purified by silica gel column chromatography (petroleum ether/ethyl acetate system) to obtain intermediate 2 (232 mg, colorless oil, 41.9%).
MS m/z (ESI): 185.1 [M+1]

### Preparation of intermediate 3: 2-(2-cyanocyclopropyl)-6-fluorobenzoic acid

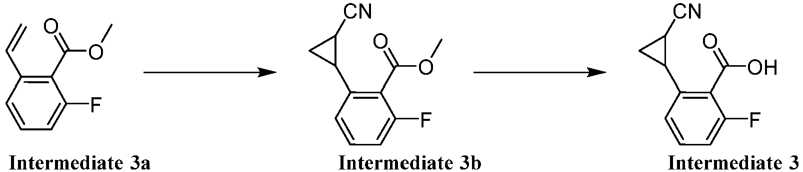

Intermediate 3a (900 mg, 5.0 mmol), FeTPPCl (105 mg, 0.15 mmol), and aminoethyl hydrochloride (920 mg, 10.0 mmol) were dissolved in dichloromethane (12.5 mL). A solution of sodium nitrite (1.04 g, 15.1 mmol) in water (12.5 mL) was slowly added, and the mixture was stirred at room temperature for 12 hours. The reaction solution was quenched by slowly adding water (20 mL), and extracted with dichloromethane (20 mL*3). The organic phases were combined, dried and concentrated. The residues were purified by silica gel column chromatography (petroleum ether/ethyl acetate system) to obtain methyl 2-(2-cyanocyclopropyl)-6-fluorobenzoate (intermediate 3b) (425 mg, pale yellow oil, 38.8%).
MS m/z (ESI): 220.1 [M+1]

At room temperature, lithium hydroxide monohydrate (210 mg, 5.0 mmol) was added to a solution of intermediate 3b (400 mg, 1.83 mmol) in THF (10 mL) and water (5 mL), and the mixture was stirred at room temperature for 2 hours. The reaction solution was adjusted to pH 5~6 with 1M dilute hydrochloric acid, and extracted with dichloromethane (20 mL*3). The organic phases were combined, dried and concentrated to obtain intermediate 3 (347 mg, pale yellow oil, 92.7%).
MS m/z (ESI): 206.0 [M+1]

### Preparation of intermediate 4: 2-(2,2-difluorocyclopropyl)-6-fluorobenzoic acid

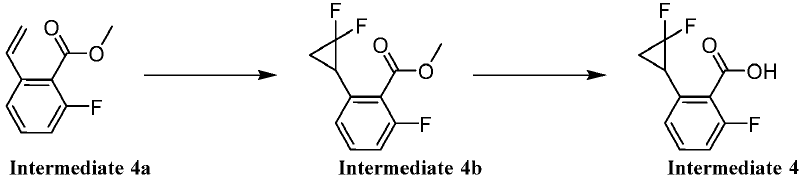

At room temperature, trimethyl(trifluoromethyl)silane (3.16 g, 22.25 mmol) was added to a solution of methyl 2-vinyl-6-fluoro-benzoate intermediate 4a (1.0 g, 5.56 mmol) and sodium iodide (167 mg, 1.12 mmol) in THF (10.0 mL), and the mixture was stirred at 65°C for 3 hours. The reaction solution was concentrated after cooling, and the residues were purified by silica gel column chromatography (petroleum ether/ethyl acetate system) to obtain methyl 2-(2,2-difluorocyclopropyl)-6-fluorobenzoate (intermediate 4b) (854 mg, pale yellow oil, 66.8%).
MS m/z (ESI): 231.2 [M+1]

At room temperature, lithium hydroxide monohydrate (420 mg, 10.0 mmol) was added to a solution of intermediate 4b (800 mg, 3.48 mmol) in THF (20 mL) and water (10 mL), and the mixture was stirred at room temperature for 2 hours. The reaction solution was adjusted to pH 5~6 with 1M dilute hydrochloric acid, and extracted with dichloromethane (30 mL*3). The organic phases were combined, dried and concentrated to obtain intermediate 4 (682 mg, pale yellow oil, 90.8%).
MS m/z (ESI): 217.0 [M+1]

### Preparation of intermediate 5: 2-(2,2-difluorocyclopropyl)benzoic acid

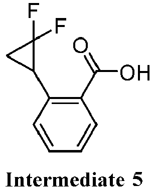

The preparation method of intermediate 5 was refered to the preparation method of intermediate 4, except that intermediate 4a was replaced with intermediate 5 (472 mg, pale yellow oil, 33.8%) to obtain methyl 2-vinylbenzoate.
MS m/z (ESI): 199.2 [M+1]

### Preparation of intermediate 6: 2-(benzooxazol-2-yl)benzoic acid

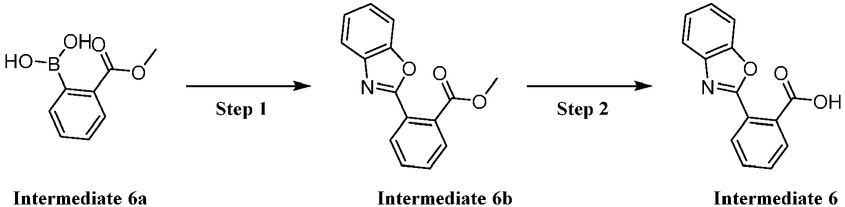

### Step 1

### Preparation of methyl 2-(benzooxazol-2-yl)benzoate

Intermediate 6a (1.8 g, 1.00 mmol), 2-chlorobenzoxazole (1.0 g, 6.51 mmol), Pd(dppf)Cl₂^{∗}DCM (531 mg, 0.65 mmol), potassium phosphate (2.76 g, 13.02 mmol), 1,4-dioxane (20 mL) and H₂O (5 mL) were added to a reactor. The mixture was stirred at 90°C for 12 hours under N₂ protection. The reaction solution was cooled, quenched with water, and extracted with ethyl acetate (30 mL*3). The organic phases were combined, dried and concentrated. The residues were purified by silica gel column chromatography to obtain intermediate 6b (800 mg, pale yellow oil) with a yield of 48.5%.
MS m/z (ESI): 254.1 [M+1]

### Step 2

### Preparation of 2-(benzooxazol-2-yl)benzoic acid

At room temperature, NaOH (2 g, 50.0 mmol) was added to a solution of intermediate 6b (800 mg, 3.16 mmol) in methanol (20 mL) and water (5 mL), and the mixture was stirred at room temperature for 2 hours. The reaction solution was adjusted to pH 5~6 with concentrated hydrochloric acid, and extracted with dichloromethane (30 mL*3). The organic phases were combined, dried and concentrated to obtain intermediate 6 (688 mg, pale yellow solid) with a yield of 91.0%.
MS m/z (ESI): 240.0 [M+1]

### Preparation of intermediate 7: 2-(benzooxazol-2-yl)-6-fluorobenzoic acid

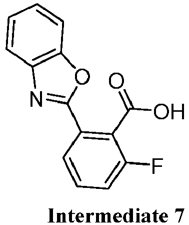

The synthetic method of intermediate 7 was refered to the synthetic method of intermediate 6, except that intermediate 6a was replaced with intermediate 7 (650 mg, pale yellow solid, yield 88.3%) to obtain methyl 2-fluoro-6-(4,4,5,5-tetramethyl-1,3-dioxaborolan-2-yl)benzoate.
MS m/z (ESI): 258.0 [M+1]

### Preparation of intermediate 8: 2-chloro-4-(2-fluoropropan-2-yl)-6-methylpyrimidine

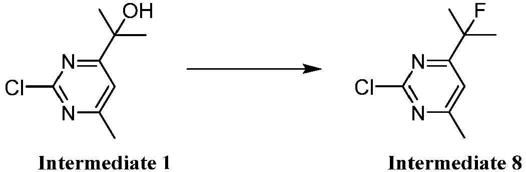

At -78°C, DAST (1.29 g, 8.01 mmol) was added to a solution of intermediate 1 (300 mg, 1.61 mmol) in dichloromethane (10 mL). The mixture was stirred at -78°C for 2 hours, then at room temperature for 12 hours. The reaction solution was quenched by slowly adding saturated sodium bicarbonate solution (50 mL), and extracted with dichloromethane (20 mL*3). The organic phases were combined, dried and concentrated. The residues were purified by silica gel column chromatography to obtain intermediate 8 (243 mg, colorless oil) with a yield of 80.1%.
MS m/z (ESI): 189.1 [M+1]

### Preparation of intermediate 9: 2-chloro-4-(2-chloropropan-2-yl)-6-methylpyrimidine

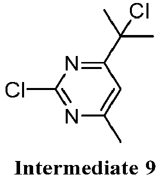

At -78°C, thionyl chloride (380 mg, 3.19 mmol) was added to a solution of intermediate 1 (300 mg, 1.61 mmol) in dichloromethane (10 mL), and the mixture was stirred at 20°C for 12 hours. The reaction solution was quenched by slowly adding saturated sodium bicarbonate solution (50 mL), and extracted with dichloromethane (20 mL*3). The organic phases were combined, dried and concentrated. The residues were purified by silica gel column chromatography to obtain intermediate 9 (208 mg, colorless oil) with a yield of 63.1%.
MS m/z (ESI): 206.0 [M+1]

### Preparation of intermediate 10: 2-chloro-4-(1,1-difluoroethyl)-6-methylpyrimidine

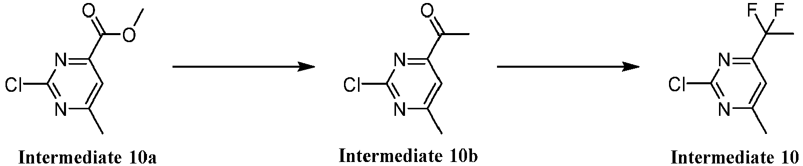

At -78°C, methylmagnesium bromide (5 mL, 1M in THF) was added to a solution of intermediate 10a (900 mg, 4.83 mmol) in THF (10 mL), and the mixture was stirred at -78°C for 2 hours. The reaction solution was quenched by slowly adding saturated ammonium chloride solution (20 mL), and extracted with dichloromethane (20 mL*3). The organic phases were combined, dried and concentrated. The residues were purified by silica gel column chromatography (petroleum ether/ethyl acetate system) to obtain 1-(2-chloro-6-methylpyrimidin-4-yl)ethan-1-one (intermediate 10b) (615 mg, colorless oil) with a yield of 74.7%.
MS m/z (ESI): 171.2 [M+1]

At -78°C, DAST (1.70 g, 10.56 mmol) was added to a solution of intermediate 10b (600 mg, 3.52 mmol) in dichloromethane (15 mL). The mixture was stirred at -78°C for 2 hours, then at room temperature for 12 hours. The reaction solution was quenched by slowly adding saturated sodium bicarbonate solution (50 mL), and extracted with dichloromethane (20 mL*3). The organic phases were combined, dried and concentrated. The residues were purified by silica gel column chromatography to obtain intermediate 10 (358 mg, colorless oil) with a yield of 52.8%.
MS m/z (ESI): 193.1 [M+1]

### Preparation of intermediate 11: 2-chloro-4-(difluoromethyl)-6-methylpyrimidine

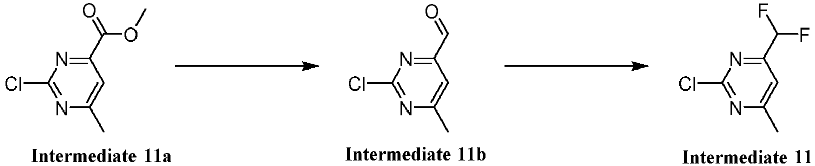

At -78°C, DIBAL-H (6 mL, 1M in toluene) was added to a solution of intermediate 11a (1.0 g, 5.36 mmol) in DCM (5 mL), and the mixture was stirred at -78°C for 3 hours. The reaction solution was quenched by slowly adding dilute hydrochloric acid (20 mL), and extracted with dichloromethane (20 mL*3). The organic phases were combined, dried and concentrated. The residues were purified by silica gel column chromatography (petroleum ether/ethyl acetate system) to obtain 2-chloro-6-methylpyrimidine-4-carbaldehyde (intermediate 11b) (636 mg, colorless oil) with a yield of 75.8%.
MS m/z (ESI): 157.0 [M+1]

At -78°C, DAST (1.54 g, 9.57 mmol) was added to a solution of intermediate 11b (500 mg, 3.19 mmol) in dichloromethane (15 mL). The mixture was stirred at -78°C for 2 hours, then at room temperature for 12 hours. The reaction solution was quenched by slowly adding saturated sodium bicarbonate solution (60 mL), and extracted with dichloromethane (20 mL*3). The organic phases were combined, dried and concentrated. The residues were purified by silica gel column chromatography to obtain intermediate 11 (373 mg, colorless oil) with a yield of 65.4%.
MS m/z (ESI): 179.1 [M+1]

### Preparation of intermediate 12: 2-chloro-7-fluoro-7-methyl-6,7-dihydro-5H-cyclopenta[d]pyrimidine

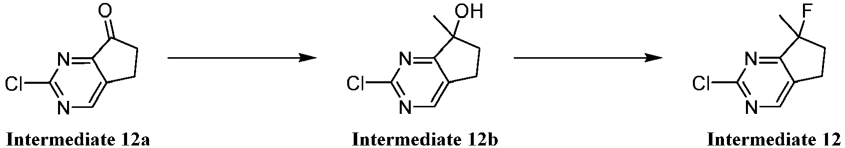

At -78°C, methylmagnesium bromide (6 mL, 1M in THF) was added to a solution of intermediate 12a (1.0 g, 5.93 mmol) in THF (15 mL), and the mixture was stirred at -78°C for 2 hours. The reaction solution was quenched by slowly adding saturated ammonium chloride solution (20 mL), and extracted with dichloromethane (20 mL*3). The organic phases were combined, dried and concentrated. The residues were purified by silica gel column chromatography (petroleum ether/ethyl acetate system) to obtain 2-chloro-7-methyl-6,7-dihydro-5H-cyclopenta[d]pyrimidin-7-ol (intermediate 12b) (863 mg, colorless oil) with a yield of 78.8%.
MS m/z (ESI): 185.1 [M+1]

At -78°C, DAST (1.35 g, 8.39 mmol) was added to a solution of intermediate 12b (500 mg, 2.71 mmol) in dichloromethane (15 mL). The mixture was stirred at -78°C for 2 hours, then at room temperature for 12 hours. The reaction solution was quenched by slowly adding saturated sodium bicarbonate solution (50 mL), and extracted with dichloromethane (20 mL*3). The organic phases were combined, dried and concentrated. The residues were purified by silica gel column chromatography to obtain intermediate 12 (342 mg, colorless oil) with a yield of 67.7%.
MS m/z (ESI): 187.0 [M+1]

### Preparation of intermediate 13: 2-chloro-4-(1-fluorocyclopropyl)-6-methylpyrimidine

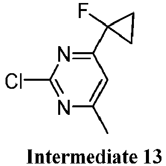

At -78°C, DAST (1.29 g, 8.01 mmol) was added to a solution of intermediate 2 (600 mg, 3.25 mmol) in dichloromethane (10 mL). The mixture was stirred at -78°C for 2 hours, then at room temperature for 12 hours. The reaction solution was quenched by slowly adding saturated sodium bicarbonate solution (50 mL), and extracted with dichloromethane (20 mL*3). The organic phases were combined, dried and concentrated. The residues were purified by silica gel column chromatography to obtain intermediate 13 (473 mg, colorless oil) with a yield of 78.0%.
MS m/z (ESI): 187.1 [M+1]

### Preparation of intermediate 14: (3 aR,6aS)-5-(4,6-dimethylpyrimidin-2-yl)-1,2,3,3 a,4, 6a-hexahydrocyclopenta[c]pyrrole

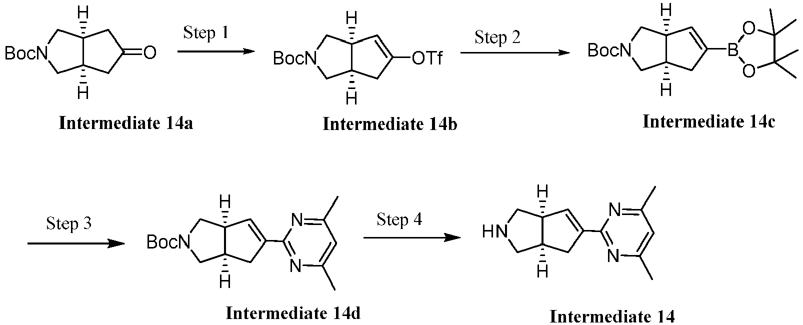

### Step 1

Intermediate 14a (10 g, 44.44 mmol) was dissolved in tetrahydrofuran (100 mL). At -78°C and under argon protection, LiHMDS (58 mL, 58 mmol, 1 M in THF) was slowly added and stirred at this temperature for 30 minutes. A solution of PhNTf₂ (21 g, 58.82 mmol) in tetrahydrofuran (50 mL) was added. The solution was reacted at -78°C for 1 hour, then at room temperature for 2 hours. The reaction was quenched by saturated aqueous ammonium chloride solution (50 mL) at 0°C, and extracted with ethyl acetate (100 mL). The organic layer was washed once with saturated aqueous sodium chloride (50 mL), dried over anhydrous sodium sulfate, filtered and concentrated. The concentrate was purified by column chromatography (petroleum ether: ethyl acetate=10:1 to 2:1) to obtain intermediate 14b (12 g, yield 75.6%), which was used directly in the next step.

### Step 2

Intermediate 14b (12 g, 33.61 mmol), 4,4,4',4',5,5,5',5'-octamethyl-2,2'-bis(1,3,2-dioxaborinane) (7.84 g, 30.87 mmol), Pd(dppf)Cl₂ (200 mg, 0.27 mmol) and potassium acetate (5.50 g, 56 mmol) were added to dioxane (200 mL). The reaction solution was stirred at 90°C for 12 hours under argon protection,, then cooled to room temperature. Ethyl acetate (200 mL) was added, the solution was washed with saturated aqueous sodium chloride (50 mL*2). The organic layer was dried over anhydrous sodium sulfate, and concentrated under reduced pressure. The concentrate was purified by column chromatography (petroleum ether:ethyl acetate=10:1 to 3:1) to obtain intermediate 14c (10 g, yield 88.8%).

¹H NMR (400 MHz, CDCl₃) δ 6.31 (s, 1H), 3.64 - 3.31 (m, 4H), 2.90 (dd, 2H), 2.61 (ddd, , 1H), 2.34 (d, 1H), 1.43 (d, 9H), 1.26 (d, 12H).

### Step 3

Intermediate 14c (2.00 g, 6.00 mmol), 2-chloro-4,6-dimethylpyrimidine (0.85 g, 6.00 mmol), tetrakistriphenylphosphine palladium (70 mg, 0.06 mmol) and potassium phosphate (2.54 g, 12.00 mmol) were added to dioxane (50 mL) and water (10 mL). The reaction solution was stirred under reflux for 16 hours under argon protection, then cooled to room temperature. Ethyl acetate (100 mL) was added, and the solution was washed with saturated aqueous sodium chloride (50 mL*2). The organic layer was dried over anhydrous sodium sulfate, and concentrated under reduced pressure. The concentrate was purified by column chromatography (petroleum ether: ethyl acetate=10:1 to 1:1) to obtain intermediate 14d (800 mg, yield 42.5%).
MS m/z (ESI): 316.1 [M+1]
¹H NMR (400 MHz, CDCl3) δ 6.84 (s, 1H), 6.78 (s, 1H), 3.71 (s, 1H), 3.54 (s, 3H), 3.04 (t, 3H), 2.81 (d, 1H), 2.47 (d, 6H), 1.41 (d, 9H).

### Step 4

At 0°C, TFA (3.0 mL) was slowly added to a solution of intermediate 14d (800 mg, 2.54 mmol) in DCM (6.0 mL), and the mixture was stirred at room temperature for 2 hours. The reaction solution was concentrated to obtain intermediate 14 (550 mg), which was directly used in the next step.
MS m/z (ESI): 216.1 [M+1]
¹H NMR (400 MHz, DMSO-*d*6) δ 7.04 (s, 1H), 6.64 (s, 1H), 3.43 (s, 2H), 2.94-2.39 (m, 7H), 2.38 (d, 6H).

### Preparation of intermediate 15: 2-(6-chloro-3-fluoro-4-methylpyridin-2-yl)propan-2-ol

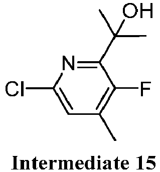

Compound 2-chloro-5-fluoro-4-methylpyridine (2.0 g, 13.74 mmol) was dissolved in tetrahydrofuran (5.0 mL), and cooled to -78°C. n-Butyllithium (1.6 M, 10.73 mL) was added, and the reaction solution was stirred for 1 hour. Acetone (798 mg, 13.74 mmol) was added, and the reaction solution was stirred at -78°C for 1 hour. After completion of the reaction, saturated aqueous ammonium chloride solution (5 ml) was added, and the solution was extracted with dichloromethane (10 mL*3). The organic phases were combined, dried, and concentrated to obtain a crude product. The crude product was purified by column chromatography (petroleum ether/ethyl acetate system) to obtain intermediate 15 (2.2 g, yield 78.6%).
MS m/z (ESI): 204.0 [M+1]

### Preparation of intermediate 16: 2-(5-fluoro-2-((3aR,6aS)-hexahydropyrrolo[3,4-c]pyrrol-2(1H)-yl)-6-methylpyrimidin-4-yl)-propan-2-ol

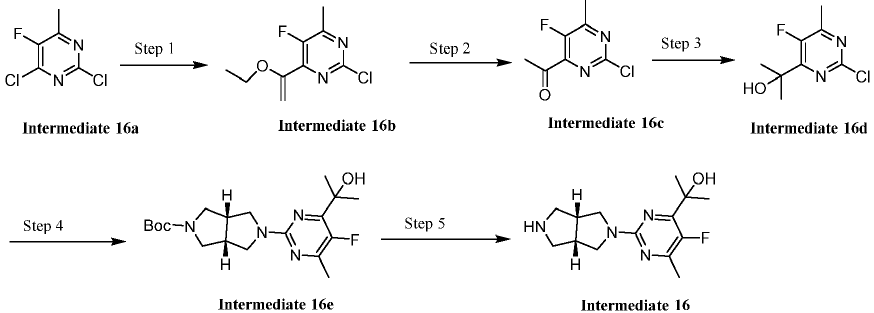

### Step 1

Tributyl-(1-ethoxy-vinyl)-stannane (2.7 mL, 7.99 mmol) and dichlorobis(triphenylphosphine)palladium(II) (100 mg, 0.142 mmol) were added to a solution of intermediate 16a (1.3 g, 7.18 mmol) in anhydrous DMF (15 mL) under nitrogen protection. The mixture was heated at 100°C for 16 hours, and then cooled. A saturated potassium fluoride solution (aq) was added, and the mixture was stirred at room temperature for 1 hour. After filtration through celite, the organic phase was washed thoroughly with water, extracted with ethyl acetate and concentrated. The crude product was purified by column chromatography (PE:EA=10:1) to obtain intermediate 16b (1.3 g, 83.5%).
MS m/z (ESI): 217.0 [M+1]

### Step 2

Intermediate 16b (1.3 g, 6.00 mmol) was dissolved in THF (10 mL), followed by addition of 3N HCl (5 mL). The mixture was stirred at room temperature for 1 hour. LCMS monitoring showed that the reaction was complete. The solution was adjusted to pH=7~8 with saturated NaHCO₃, and extracted with ethyl acetate. The organic phase was concentrated to obtain intermediate 16c (1.1 g, 97.2%), which was directly used in the next step.
MS m/z (ESI): 189.0 [M+1]
1HNMR (400 MHz, Chloroform-d) δ 2.69 (s, 3H), 2.62 (d, J = 2.8 Hz, 3H).

### Step 3

At 0°C, intermediate 16c (1.9 g, 10.07 mmol) and MeMgBr (3 M, 4.37 mL) were added to THF (40 mL). The reaction solution was stirred at 25°C for 1 hour, followed by addition of saturated NH₄Cl (20 ml). The solution was extracted with ethyl acetate (40 ml), and the combined extracts were dried over Na₂SO₄, and concentrated under reduced pressure. The resulting residues were purified by column chromatography (PE:EA=3:1) to obtain intermediate 16d (1.4 g, yield 67.9%).
MS m/z (ESI): 205.0[M+1]

### Step 4

*tert*-Butyl rac-(3aR,6aS)-2,3,3a,4,6,6a-hexahydro-1H-pyrrole[3,4-c]pyrrole-5-carboxylate (500 mg, 2.36 mmol), intermediate 16d (530.16 mg, 2.59 mmol) and cesium carbonate (1.55 g, 4.76 mmol) were added to DMF (10 mL). The reaction solution was stirred at 100°C for 3 hours, followed by addition of water (20 ml). The solution was extracted with dichloromethane (30mL*2), and the combined extracts were dried over Na₂SO₄, and concentrated under reduced pressure. The resulting residues were purified by column chromatography (PE:EA=3:1) to obtain intermediate 16e (720 mg, yield 80.3%).
MS m/z (ESI): 381.2 [M+1]

### Step 5

Intermediate 16e (1 g, 2.63 mmol) and trifluoroacetic acid (2.69 mmol, 2 mL) were added to DCM (3 mL). The reaction solution was stirred at 25°C for 3 hours, and then concentrated under reduced pressure to obtain intermediate 16 (700 mg, yield 95.0%).
MS m/z (ESI): 281.2 [M+1]

### Preparation of intermediate 17: 2-(6-(3aR,6aS)-hexahydropyrrolo[3,4-c]pyrrol-2(1H)-yl)-4-methylpyridin-2-yl)propan-2-ol

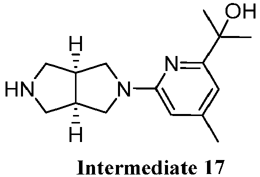

The preparation method of intermediate 17 was refered to step 3, step 4 and step 5 of the preparation method of intermediate 16, except that intermediate 16c was replaced with intermediate 17 (1.2 g, colorless oil, 53.7%) to obtained 6-chloro-4-methyl-2-acetyl-pyridine.
MS m/z (ESI): 262.2 [M+1]

### Preparation of intermediate 18: 2-(2-chloro-6-methylpyridin-4-yl)propan-2-ol

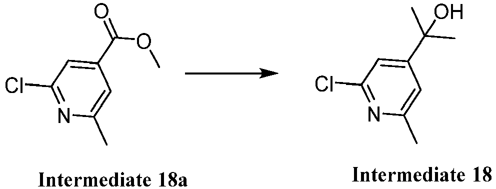

At -78°C, a solution of intermediate 18a (1g, 5.4 mmol) in THF (5 mL) was added to methylmagnesium bromide (16 mL, 1M in THF). The mixture was stirred at -78°C for 2 hours, then at room temperature for 12 hours. The reaction solution was quenched by slowly adding saturated ammonium chloride solution (20 mL), and extracted with dichloromethane (20 mL*3). The organic phases were combined, dried and concentrated. The residues were purified by silica gel column chromatography (petroleum ether/ethyl acetate system) to obtain intermediate 18 (800 mg, yield 80.0%).
MS m/z (ESI): 186.1 [M+1]

### Example 1

### Preparation of compound 1: ((3aR,6aS)-5-(4,6-dimethylpyrimidin-2-yl)-3a,6a-difluorohexahydropyrrolo[3,4-c]pyrro l-2(1H)-yl)(2-fluoro-6-(2H-1,2,3-triazol-2-yl)phenyl)methanone

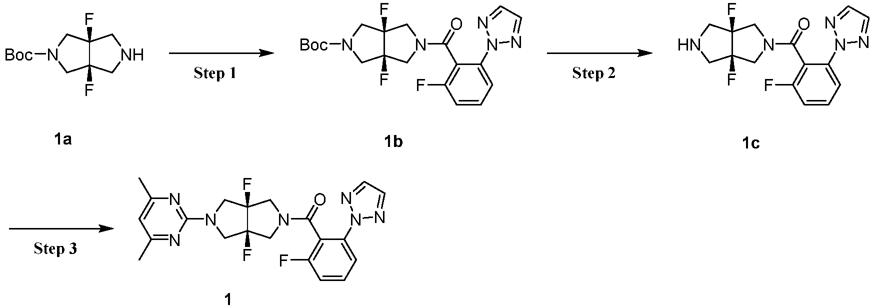

### Step 1

HATU (690 mg, 1.82 mmol) and DIPEA (550 mg, 4.26 mmol) were slowly added to a solution of 1a (300 mg, 1.21 mmol) (synthesized according to Bioorganic & Medicinal Chemistry Letters 27 (2017) 1458-1462) and 2-fluoro-6-(2H-1,2,3-triazol-2-yl)benzoic acid (275 mg, 1.33 mmol) in DMF (5.0 mL), and the mixture was stirred at room temperature for 2 hours. The reaction solution was diluted with 30 mL of ethyl acetate, and washed with water (30 mL*3). The organic phases were combined, dried and concentrated. The residues were purified by silica gel column chromatography (petroleum ether/ethyl acetate system) to obtain *tert*-butyl (3aR,6aS)-3a,6a-difluoro-5-(2-fluoro-6-(2H-1,2,3-triazol-2-yl)benzoyl)hexahydropyrrol o[3,4-c]pyrrole-2(1H)-carboxylate (compound 1b) (507 mg, pale yellow solid, 95.9%).
MS m/z (ESI): 438.2 [M+1]

### Step 2

At 0°C, TFA (3.0 mL) was slowly added to a solution of 1b (500 mg, 1.14 mmol) in DCM (6.0 mL), and the mixture was stirred at room temperature for 2 hours. The reaction solution was concentrated to obtain ((3aR,6aS)-3a,6a-difluorohexahydropyrrolo[3,4-c]pyrrol-2(1H)-yl)(2-fluoro-6-(2H-1,2, 3-triazol-2-yl)phenyl)methanone (compound 1c) (377 mg, light yellow oil), which was used directly in the next step.
MS m/z (ESI): 338.1 [M+1]

### Step 3

2-Chloro-4,6-dimethylpyrimidine (64 mg, 0.45 mmol) and cesium carbonate (196 mg, 0.60 mmol) were slowly added to a solution of 1c (100 mg, 0.30 mmol) in DMF (2.0 mL), and the mixture was stirred at 100°C for 12 hours. The reaction solution was cooled, diluted with 10 mL of ethyl acetate, and washed with water (10 mL*3). The organic phases were combined, dried and concentrated. The residues were purified by preparative HPLC to obtain ((3aR,6aS)-5-(4,6-dimethylpyrimidin-2-yl)-3a,6a-difluorohexahydropyrrolo[3,4-c]pyrro l-2(1H)-yl)(2-fluoro-6-(2H-1,2,3-triazol-2-yl)phenyl)methanone (compound 1) (43 mg, white solid, 32.7%).
MS m/z (ESI): 444.2 [M+1]

### Example 2

### Preparation of compound 2: ((3aR,6aS)-5-(4,6-dimethylpyrimidin-2-yl)-3a,6a-dimethylhexahydropyrrolo[3,4-c]pyrr ol-2(1H)-yl)(2-fluoro-6-(2H-1,2,3-triazol-2-yl)phenyl)methanone

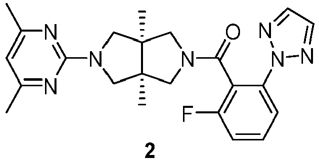

The preparation method of compound 2 was refered to the preparation method of Example 1, except that 1a was replaced with tert-butyl (3aR,6aS)-3a,6a-dimethylhexahydropyrrolo[3,4-c]pyrrole-2(1H)-carboxylate (synthesized according to Bioorganic & Medicinal Chemistry Letters 27 (2017) 1458-1462) to obtain Compound 2 (51 mg, white solid, 52.6%).
MS m/z (ESI): 436.2 [M+1]

### Example 3

### Preparation of compound 3: (2-fluoro-6-(2H-1,2,3-triazol-2-yl)phenyl)((3aR,6aS)-5-(4-(2-hydroxypropan-2-yl)-6-m ethylpyrimidin-2-yl)hexahydropyrrolo[3,4-c]pyrrol-2(1H)-yl)methanone

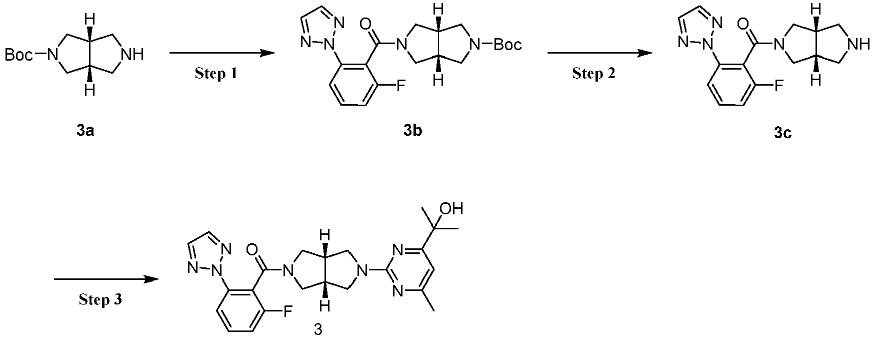

### Step 1

HATU (809 mg, 2.13 mmol) and DIPEA (550 mg, 4.26 mmol) were slowly added to a solution of 3a (300 mg, 1.42 mmol) and 2-fluoro-6-(2H-1,2,3-triazol-2-yl)benzoic acid (310 mg, 1.50 mmol) in DMF (5.0 mL), and the mixture was stirred at room temperature for 2 hours. The reaction solution was diluted with 30 mL of ethyl acetate, and washed with water (30 mL*3). The organic phases were combined, dried and concentrated. The residues were purified by silica gel column chromatography (petroleum ether/ethyl acetate system) to obtain tert-butyl (3aR,6aS)-5-(2-fluoro-6-(2H-1,2,3-triazol-2-yl)benzoyl)hexahydropyrrolo[3,4-c]pyrrole -2(1H)-carboxylate (compound 3b) (452 mg, pale yellow solid, 79.7%).
MS m/z (ESI): 402.1 [M+1]

### Step 2

At 0°C, TFA (3.0 mL) was slowly added to a solution of 3b (450 mg, 1.12 mmol) in DCM (6.0 mL), and the mixture was stirred at room temperature for 2 hours. The reaction solution was concentrated to obtain (2-fluoro-6-(2H-1,2,3-triazol-2-yl)phenyl)((3aR,6aS)-hexahydropyrrolo[3,4-c]pyrrol-2( 1H)-yl)methanone (compound 3c) (330 mg, pale yellow oil), which was used directly in the next step.
MS m/z (ESI): 302.0 [M+1]

### Step 3

Intermediate 1 (61.5 mg, 0.33 mmol), 3c (100 mg, 0.33 mmol), Pd₂(dba)₃ (18 mg, 0.02 mmol), BINAP (25 mg, 0.04 mmol), cesium carbonate (215 mg, 0.66 mmol) and dioxane (2 mL) were added to a round-bottomed flask, and the mixture was stirred at 100°C for 12 hours under nitrogen protection. The reaction solution was cooled, quenched with water (5 mL), and extracted with dichloromethane (10 mL*3). The organic phases were combined, dried and concentrated. The residues were purified by preparative HPLC to obtain (2-fluoro-6-(2H-1,2,3-triazol-2-yl)phenyl)((3aR,6aS)-5-(4-(2-hydroxypropan-2-yl)-6-m ethylpyrimidin-2-yl)hexahydropyrrolo[3,4-c]pyrrol-2(1H)-yl)methanone (compound 3) (32.4 mg, white solid, 21.8%).
MS m/z (ESI): 452.2 [M+1]

¹H NMR (400 MHz, DMSO-d6) δ 8.16 (s, 1H), 7.94 (s, 1H), 7.80 (dd, J = 17.2, 8.2 Hz, 1H), 7.66 (td, J = 8.3, 6.1 Hz, 1H), 7.44 (dt, J = 12.0, 8.6 Hz, 1H), 6.74 (d, J = 4.1 Hz, 1H), 5.10 (d, J = 4.0 Hz, 1H), 3.73 (dtd, J = 22.4, 7.2, 6.5, 3.6 Hz, 2H), 3.59 - 3.41 (m, 4H), 3.35 (d, J = 4.8 Hz, 1H), 3.16 - 2.90 (m, 3H), 2.28 (d, J = 3.5 Hz, 3H), 1.36 (d, J = 3.3 Hz, 6H).

### Example 4

### Preparation of compound 4: (2-fluoro-6-(2H-1,2,3-triazol-2-yl)phenyl)((3aR,6aS)-5-(4-(1-hydroxycyclopropyl)-6-m ethylpyrimidin-2-yl)hexahydropyrrolo[3,4-c]pyrrol-2(1H)-yl)methanone

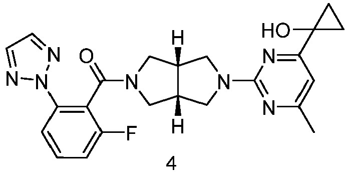

The preparation method of compound 4 was refered to the preparation method of Example 3, except that intermediate 1 was replaced with intermediate 2 to obtain Compound 4 (12 mg, white solid, 11.3%).
MS m/z (ESI): 450.1 [M+1]

### Example 5

### Preparation of compound 5: 2-(2-((3aR,6aS)-5-(4,6-dimethylpyrimidin-2-yl)octahydropyrrolo[3,4-c]pyrrole-2-carbo nyl)-3-fluorophenyl)cyclopropane-1-carbonitrile

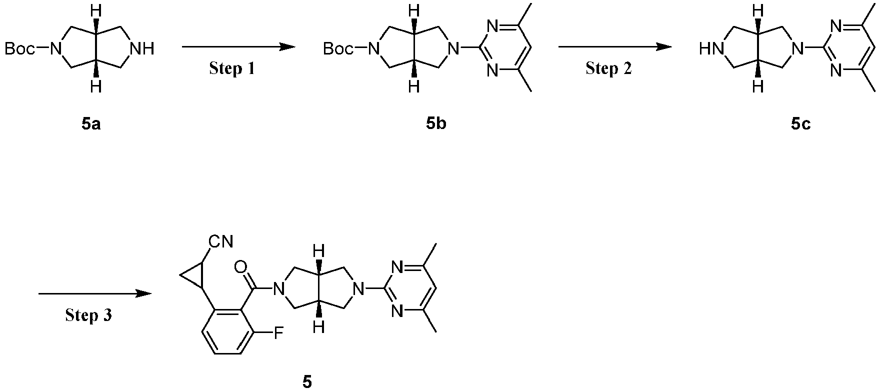

### Step 1

2-Chloro-4,6-dimethylpyrimidine (260 mg, 1.84 mmol) and cesium carbonate (910 mg, 2.80 mmol) were slowly added to a solution of 5a (300 mg, 1.42 mmol) in DMF (5.0 mL), and the mixture was stirred at 100°C for 12 hours. The reaction solution was cooled, diluted with 20 mL of ethyl acetate, and washed with water (20 mL*3). The organic phases were combined, dried and concentrated. The residues were purified by silica gel column chromatography (petroleum ether/ethyl acetate system) to obtain *tert*-butyl (3aR,6aS)-5-(4,6-dimethylpyrimidin-2-yl)hexahydropyrrolo[3,4-c]pyrrole-2(1H)-carbox ylate (compound 5b) (315 mg, pale yellow solid, 70.0%).
MS m/z (ESI): 319.2 [M+1]

### Step 2

At 0°C, TFA (3.0 mL) was slowly added to a solution of 5b (310 mg, 0.97 mmol) in DCM (6.0 mL), and the mixture was stirred at room temperature for 2 hours. The reaction solution was concentrated to obtain (3aR,6aS)-2-(4,6-dimethylpyrimidin-2-yl)octahydropyrrolo[3,4-c]pyrrole (compound 5c) (200 mg, pale yellow oil), which was used directly in the next step.
MS m/z (ESI): 219.2 [M+1]

### Step 3

HATU (210 mg, 0.56 mmol) and DIPEA (143 mg, 1.11 mmol) were slowly added to a solution of 5c (80 mg, 0.37 mmol) and intermediate 3 (85 mg, 0.41 mmol) in DMF (2.0 mL), and the mixture was stirred at room temperature for 2 hours. The reaction solution was diluted with 10 mL of ethyl acetate, and washed with water (8 mL*3). The organic phases were combined, dried and concentrated. The residues were purified by preparative HPLC to obtain compound 5 (36 mg, white solid, 24.2%).
MS m/z (ESI): 406.2 [M+1]

### Example 6

### Preparation of compound 6: (2-(2,2-difluorocyclopropyl)-6-fluorophenyl)((3aR,6aS)-5-(4,6-dimethylpyrimidin-2-yl) hexahydropyrrolo[3,4-c]pyrrol-2(1H)-yl)methanone

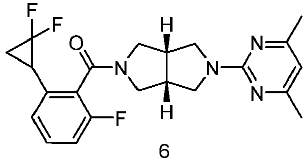

The preparation method of compound 6 was refered to the preparation method of Example 5, except that intermediate 3 was replaced with intermediate 4 to obtain Compound 6 (25 mg, white solid, 28.3%).
MS m/z (ESI): 417.2 [M+1]

### Example 7

### Preparation of compound 7: ((3aR,6aS)-5-(4,6-dimethylpyrimidin-2-yl)hexahydro-2H-pyrrolo[3,4-d]isoxazol-2-yl)( 2-fluoro-6-(2H-1,2,3-triazol-2-yl)phenyl)methanone

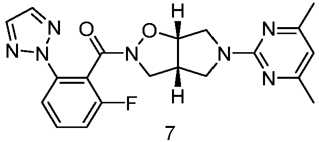

The preparation method of compound 7 was refered to the preparation method of Example 1, except that 1a was replaced with tert-butyl (3aR,6aS)-hexahydro-5H-pyrrolo[3,4-d]isoxazole-5-carboxylate to obtain Compound 7 (37 mg, white solid, 21.8%).
MS m/z (ESI): 410.2 [M+1]

### Example 8

### Preparation of compound 8: ((3aR,6aS)-2-(4,6-dimethylpyrimidin-2-yl)hexahydro-5H-pyrrolo[3,4-d]isoxazol-5-yl)( 2-fluoro-6-(2H-1,2,3-triazol-2-yl)phenyl)methanone

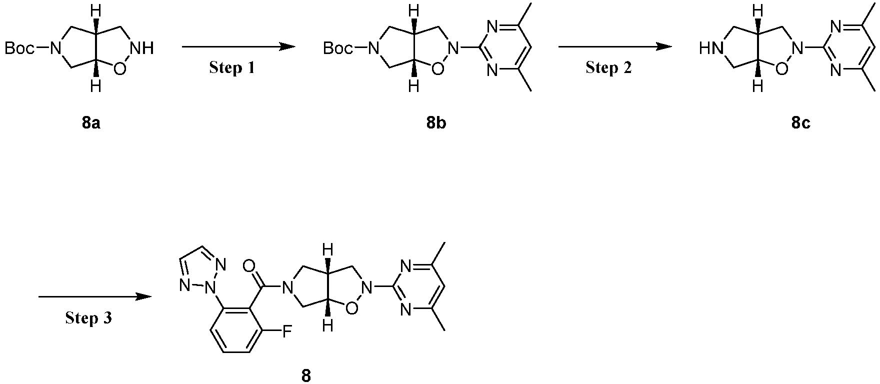

### Step 1

2-Chloro-4,6-dimethylpyrimidine (250 mg, 1.75 mmol) and cesium carbonate (766 mg, 2.35 mmol) were slowly added to a solution of 8a (250 mg, 1.17 mmol) in DMF (5.0 mL), and the mixture was stirred at 100°C for 12 hours. The reaction solution was cooled, diluted with 15 mL of ethyl acetate, and washed with water (15 mL*3). The organic phases were combined, dried and concentrated. The residues were purified by silica gel column chromatography (petroleum ether/ethyl acetate system) to obtain *tert*-butyl (3aR,6aS)-2-(4,6-dimethylpyrimidin-2-yl)hexahydro-5H-pyrrolo[3,4-d]isoxazole-5-carb oxylate (compound 8b) (238 mg, pale yellow solid, 63.7%).
MS m/z (ESI): 321.2 [M+1]

### Step 2

At 0°C, TFA (3.0 mL) was slowly added to a solution of 8b (230 mg, 0.72 mmol) in DCM (6.0 mL), and the mixture was stirred at room temperature for 2 hours. The reaction solution was concentrated to obtain (3 aR,6aS)-2-(4,6-dimethylpyrimidin-2-yl)hexahydro-2H-pyrrolo[3,4-d]isoxazole (compound 8c) (150 mg, pale yellow oil), which was used directly in the next step.
MS m/z (ESI): 221.1 [M+1]

### Step 3

HATU (210 mg, 0.56 mmol) and DIPEA (143 mg, 1.11 mmol) were slowly added to a solution of 8c (80 mg, 0.36 mmol) and 2-fluoro-6-(2H-1,2,3-triazol-2-yl)benzoic acid (80 mg, 0.39 mmol) in DMF (2.0 mL), and the mixture was stirred at room temperature for 2 hours. The reaction solution was diluted with 10 mL of ethyl acetate, and washed with water (8 mL*3). The organic phases were combined, dried and concentrated. The residues were purified by preparative HPLC to obtain compound 8 (41 mg, white solid, 27.6%).
MS m/z (ESI): 410.2 [M+1]

### Example 9

### Preparation of compound 9: (3aR,6aS)-5-(4,6-dimethylpyrimidin-2-yl)-2-(2-fluoro-6-(2H-1,2,3-triazol-2-yl)benzyl)h exahydropyrrolo[3,4-c]pyrrol-1(2H)-one

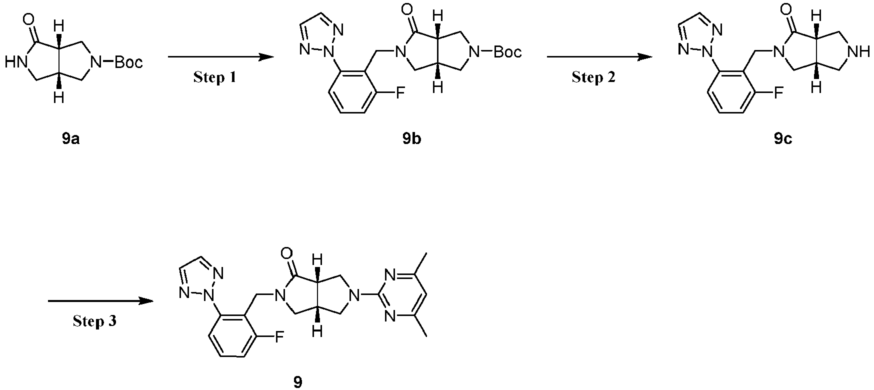

### Step 1

2-(2-(Bromomethyl)-3-fluorophenyl)-2H-1,2,3-triazole (850 mg, 3.35 mmol) and potassium carbonate (621 mg, 4.50 mmol) were slowly added to a solution of 9a (500 mg, 2.21 mmol) in DMF (8 mL), and the mixture was stirred at 80°C for 2 hours. The reaction solution was cooled, diluted with 30 mL of ethyl acetate, and washed with water (30 mL*3). The organic phases were combined, dried and concentrated. The residues were purified by silica gel column chromatography (petroleum ether/ethyl acetate system) to obtain tert-butyl (3 aS, 6aS)-5-(2-fluoro-6-(2H-1,2,3-triazol-2-yl)benzyl)-4-oxohexahydropyrrolo[3,4-c]py rrole-2(1H)-carboxylate (compound 9b) (766 mg, colorless liquid, 86.3%).
MS m/z (ESI): 402.2 [M+1]

### Step 2

At 0°C, TFA (5.0 mL) was slowly added to a solution of 9b (750 mg, 1.87 mmol) in DCM (10.0 mL), and the mixture was stirred at room temperature for 2 hours. The reaction solution was concentrated to obtain (3aR,6aS)-2-(2-fluoro-6-(2H-1,2,3-triazol-2-yl)benzyl)hexahydropyrrolo[3,4-c]pyrrol-1 (2H)-one (compound 9c) (552 mg, pale yellow oil), which was used directly in the next step.
MS m/z (ESI): 302.1 [M+1]

### Step 3

2-Chloro-4,6-dimethylpyrimidine (98 mg, 0.68 mmol) and cesium carbonate (221 mg, 0.68 mmol) were slowly added to a solution of 9c (100 mg, 0.33 mmol) in DMF (2.0 mL), and the mixture was stirred at 100°C for 12 hours. The reaction solution was cooled, diluted with 10 mL of ethyl acetate, and washed with water (10 mL*3). The organic phases were combined, dried and concentrated. The residues were purified by preparative HPLC to obtain compound 9 (23 mg, white solid, 17.0%).
MS m/z (ESI): 408.2 [M+1]

### Example 10

### Preparation of compound 10: ((3aS,6aS)-4-(4,6-dimethylpyrimidin-2-yl)hexahydropyrrolo[3,2-b]pyrrol-1(2H)-yl)(2-fl uoro-6-(2H-1,2,3-triazol-2-yl)phenyl)methanone

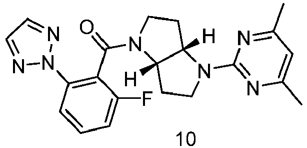

The preparation method of compound 10 was refered to the preparation method of Example 1, except that 1a was replaced with tert-butyl (3aS, 6aS)-hexahydropyrrolo[3,2-b]pyrrole-1(2H)-carboxylate to obtain Compound 10 (35 mg, white solid, 26.3%).
MS m/z (ESI): 408.2 [M+1]

### Example 11

### Preparation of compound 11: ((3aS,6aS)-1-(4,6-dimethylpyrimidin-2-yl)hexahydropyrrolo[3,4-b]pyrrol-5(1H)-yl)(2-fl uoro-6-(2H-1,2,3-triazol-2-yl)phenyl)methanone

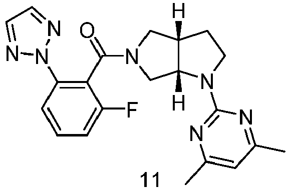

The preparation method of compound 11 was refered to the preparation method of Example 1, except that 1a was replaced with *tert*-butyl (3aS, 6aS)-hexahydropyrrolo[3,4-b]pyrrole-1(2H)-carboxylate to obtain Compound 11 (15 mg, white solid, 13.8%).
MS m/z (ESI): 408.1 [M+1]

### Example 12

### Preparation of compound 12: ((3 aR, 6aS)-5-((4,6-dimethylpyrimidin-2-yl)amino)-5-methylhexahydrocyclopenta[c]pyr rol-2(1H)-yl)(2-fluoro-6-(2H-1,2,3-triazol-2-yl)phenyl)methanone

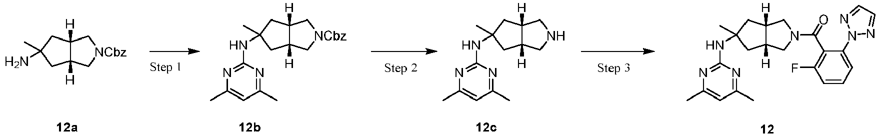

### Step 1

12a (500 mg, 1.82 mmol) and cesium carbonate (1.8 g, 5.52 mmol) were dissolved in N,N-dimethylformamide (5 mL), followed by addtion of 2-chloro-4,6-dimethylpyrimidine (260 mg, 1.82 mmol). The reaction solution was stirred at 100°C for 2 hours. Saturated brine (10 mL) was added to the reaction solution, and extracted with ethyl acetate (10 mL*3). The organic phases were combined, dried and concentrated. The residues were purified by silica gel column chromatography (petroleum ether/ethyl acetate system) to obtain benzyl (3aR,6aS)-5-((4,6-dimethylpyrimidin-2-yl)amino)-5-methylhexahydrocyclopenta[c]pyrr ole-2(1H)-carboxylate (compound 12b) (520 mg, 75.0%).
MS: m/z (ESI): 381.2 [M+1]

### Step 2

12b (0.5 g, 1.32 mmol) and palladium on carbon (100 mg) were dissolved in dichloromethane (5 mL). The reaction solution was stirred for 24 hours under hydrogen atmosphere, and then filtered. The filtrate was concentrated, and extracted with water and dichloromethane (20 mL*3). The organic phases were combined, dried, and concentrated to obtain (3aR,6aS)-N-(4,6-dimethylpyrimidin-2-yl)-5-methyloctahydrocyclopenta[c]pyrrol-5-am ine (compound 12c) (0.28 g, 86.5%).
MS: m/z (ESI): 247.2 [M+1]

### Step 3

12c (200 mg, 0.81 mmol) and triethylamine (164 mg, 1.62 mmol) were dissolved in tetrahydrofuran (5 mL), followed by addtion of 5-fluoro-2-(2H-1,2,3-triazol-2-yl)benzoyl chloride (218 mg, 0.97 mmol) at 0°C. The reaction solution was stirred overnight at room temperature. Saturated brine (10 mL) was added to the reaction solution, and extracted with ethyl acetate (10 mL*3). The organic phases were combined, dried and concentrated. The residues were purified by silica gel column chromatography (petroleum ether/ethyl acetate system) to obtain compound 12 (140 mg, 39.6%).
MS: m/z (ESI): 436.2 [M+1]

### Example 13

### Preparation of compound 13: ((3aR,5R,7aR)-1-(4,6-dimethylpyrimidin-2-yl)-5-methyloctahydro-6H-pyrrolo[2,3-c]py ridin-6-yl)(5-fluoro-2-(2H-1,2,3-triazol-2-yl)phenyl)methanone

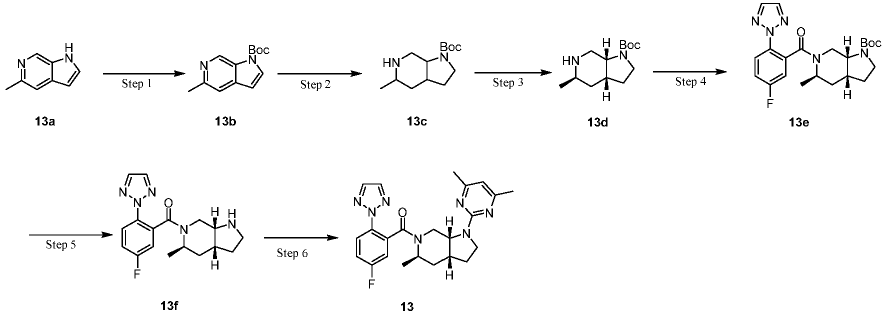

### Step 1

13a (1.0 g, 7.58 mmol) was dissolved in dichloromethane (20 mL), followed by addtion of di-*tert*-butyl dicarbonate (1.77 g, 7.62 mmol). The reaction solution was stirred at room temperature for 1 hour. The reaction solution was extracted with water and dichloromethane (20 mL*3). The organic layers were combined, dried over anhydrous sodium sulfate, filtered and concentrated. The residues were purified by silica gel column chromatography (petroleum ether/ethyl acetate system) to obtain tert-butyl 5-methyl-1H-pyrrolo[2,3-c]pyridine-1-carboxylate (compound 13b) (1.7 g, 96.7%).
MS m/z (ESI): 233.1 [M+1]

### Step 2

13b (1.5 g, 6.47 mmol) and platinum dioxide (0.22 g, 0.97 mmol) were dissolved in dichloromethane (5 mL) and acetic acid (1 mL). The reaction solution was stirred for 24 hours under hydrogen atmosphere (50 psi), and then filtered. The filtrate was concentrated, and extracted with water and dichloromethane (3x20 mL). The organic phases were combined, dried, and concentrated to obtain tert-butyl 5-methyloctahydro-1H-pyrrolo[2,3-c]pyridine-1-carboxylate (compound 13c) (1.48 g, 95.4%).
MS: m/z (ESI): 241.2 [M+1]

### Step 3

13c was resolved by SFC chiral preparation to obtain tert-butyl (3aR,5R,7aR)-5-methyloctahydro-1H-pyrrolo[2,3-c]pyridine-1-carboxylate (compound 13d) (850 mg, 57.4%).
MS: m/z (ESI): 241.2 [M+1]

### Step 4

13d (500 mg, 2.08 mmol) and triethylamine (530 mg, 5.2 mmol) were dissolved in tetrahydrofuran (5 mL), followed by addition of 5-fluoro-2-(2H-1,2,3-triazol-2-yl)benzoyl chloride (560 mg, 2.5 mmol) at 0°C. The reaction solution was stirred overnight at room temperature. Saturated brine (10 mL) was added to the reaction solution, and extracted with ethyl acetate (3x10 mL). The organic phases were combined, dried and concentrated. The residues were purified by silica gel column chromatography (petroleum ether/ethyl acetate system) to obtain *tert*-butyl (3aR,5R,7aR)-6-(5-fluoro-2-(2H-1,2,3-triazol-2-yl)benzoyl)-5-methyloctahydro-1H-pyr rolo[2,3-c]pyridine-1-carboxylate (compound 13e) (550 mg, 61.5%).
MS: m/z (ESI): 430.2 [M+1]

### Step 5

13e (500 mg, 1.17 mmol) was dissolved in dichloromethane (5 mL), followed by addition of trifluoroacetic acid (5 mL) at 0°C. The reaction solution was stirred at room temperature for 2 hours. The reaction solution was concentrated to obtain the crude product (5-fluoro-2-(2H-1,2,3-triazol-2-yl)phenyl)((3aR,5R,7aR)-5-methyloctahydro-6H-pyrrol o[2,3-c]pyridin-6-yl)methanone (compound 13f) (350 mg, crude product).
MS: m/z (ESI): 330.2 [M+1]

### Step 6

13f (100 mg, 0.30 mmol) and cesium carbonate (300 mg, 0.92 mmol) were dissolved in N,N-dimethylformamide (5 mL), followed by addition of 2-chloro-4,6-dimethylpyrimidine (43 mg, 0.30 mmol). The reaction solution was stirred at 100°C for 2 hours. Saturated brine (10 mL) was added to the reaction solution, and extracted with ethyl acetate (10 mL*3). The organic phases were combined, dried and concentrated. The residues were purified by silica gel column chromatography (petroleum ether/ethyl acetate system) to obtain compound 13 (90 mg, 68.1%).
MS: m/z (ESI): 436.2 [M+1]

### Example 14

### Preparation of compound 14: ((3aS,6R,7aR)-2-(4,6-dimethylpyrimidin-2-yl)-6-methyloctahydro-5H-pyrrolo[3,4-c]py ridin-5-yl)(5-fluoro-2-(2H-1,2,3-triazol-2-yl)phenyl)methanone

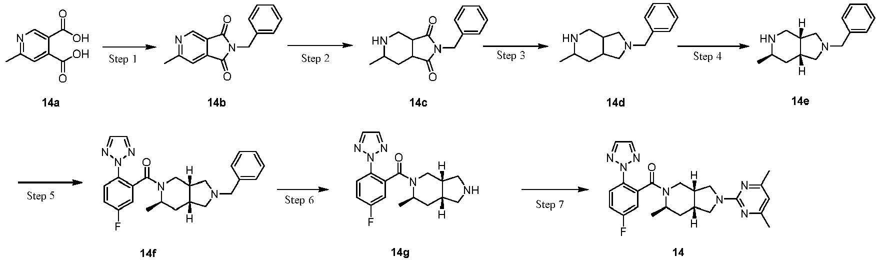

### Step 1

At room temperature, acetic anhydride (170 mL) was added dropwise to 14a (100 g, 552.5 mmol), and the reaction solution was stirred at 110°C for 4 hours. The reaction solution was concentrated under reduced pressure. Diethyl ether (200 mL) was added to the residues and filtered. The filter cake was washed with diethyl ether (100 ml*4) to obtain an acid anhydride compound intermediate. Benzylamine (76 mL) was added to the intermediate under an ice bath,, and the reaction solution was stirred at 110°C for 0.5 hour. Acetic anhydride (170 mL) was added dropwise to the reaction solution under an ice bath,, followed by stirring at 110°C for 2 hours. The reaction solution was cooled, followed by addition of ethanol (500 mL). The reaction solution was filtered, and the filter cake was washed with ethanol (100 ml*3), and collected to obtain 2-benzyl-6-methyl-1H-pyrrolo[3,4-c]pyridine-1,3(2H)-dione (compound 14b) (89.4 g, 64.2%).
MS: m/z (ESI): 253.1 [M+1]

### Step 2

The preparation method of compound 14c was refered to the preparation method of compound 13c of Example 13, except that compound 14b was used as starting material to obtain 2-Benzyl-6-methylhexahydro-1H-pyrrolo[3,4-c]pyridine- 11,3 (2H)-dione (compound 14c) (25.2 g, 52.7%).
MS: m/z (ESI): 259.1[M+1]

### Step 3

Lithium aluminum tetrahydride (1.8 g, 47.4 mmol) was dissolved in anhydrous tetrahydrofuran (5 mL), followed by addition of Example 18c (5.0 g, 19.4 mmol) at 0°C. The reaction solution was stirred at 70°C for 5 hours. Water (1.8 mL), 15% sodium hydroxide solution (1.8 mL) and water (5.4 mL) were added to the reaction solution successively. The reaction solution was filtered, and the filter cake was washed with ethyl acetate (30 ml*4). The filtrate was concentrated, and purified by silica gel column chromatography (petroleum ether/ethyl acetate system) to obtain 2-benzyl-6-methyloctahydro-1H-pyrrolo[3,4-c]pyridine (compound 14d) (3.8 g, 85.3%).
MS: m/z (ESI): 231.2 [M+1]

### Step 4

The preparation method of compound 14e was refered to the preparation method of compound 13d of Example 13, except that compound 14d was used as starting material to obtain (3aR,6R,7aR)-2-Benzyl-6-methyloctahydro-1H-pyrrolo[3,4-c]pyridine (compound 14e) (2.1 g, 55.3%).
MS: m/z (ESI): 231.2 [M+1]

### Step 5

The preparation method of compound 14f was refered to the preparation method of compound 13e of Example 13, except that compound 14e was used as starting material to obtain ((3aS,6R,7aR)-2-Benzyl-6-methyloctahydro-5H-pyrrolo[3,4-c]pyridin-5-yl)(5-fluoro-2-(2H-1,2,3-triazol-2-yl)phenyl)methanone (compound 14f) (0.3 g, 66.7%).
MS: m/z (ESI): 420.2 [M+1]

### Step 6

Compound 14f (0.3 g, 0.72 mmol) and palladium on carbon (100 mg) were dissolved in dichloromethane (5 mL). The reaction solution was stirred for 24 hours under hydrogen atmosphere, and then filtered. The filtrate was concentrated, and extracted with water and dichloromethane (20 mL*3). The organic phases were combined, dried, and concentrated to obtain (5-fluoro-2-(2H-1,2,3-triazol-2-yl)phenyl)((3aS,6R,7aR)-6-methyloctahydro-5H-pyrrol o[3,4-c]pyridin-5-yl)methanone (compound 14g) (0.2 g, 84.9%).
MS: m/z (ESI): 330.2 [M+1]

### Step 7

The preparation method of compound 4 was refered to the preparation method of compound 13 of Example 13, except that compound 14g was used as starting material to obtain Compound 14 (0.12 g, 45.5%).
MS: m/z (ESI): 436.2 [M+1]

### Example 15

### Preparation of compound 15: ((1R,3aS,7aS)-5-(4,6-dimethylpyrimidin-2-yl)-1-methyloctahydro-2H-pyrrolo[3,4-c]pyr idin-2-yl)(5-fluoro-2-(2H-1,2,3-triazol-2-yl)phenyl)methanone

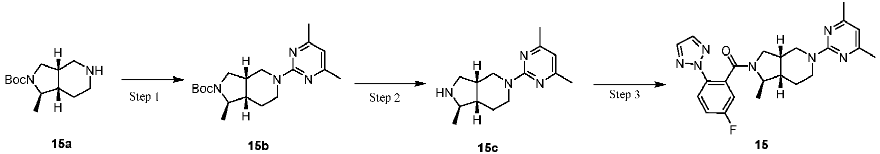

### Step 1

The preparation method of compound 15b was refered to the preparation method of compound 13 of Example 13, except that compound 15a was used as starting material to obtain tert-Butyl (1R,3aS,7aS)-5-(4,6-dimethylpyrimidin-2-yl)-1-methyloctahydro-2H-pyrrolo[3,4-c]pyri dine-2-carboxylate (compound 15b) (1.1 g, 56.5%).
MS: m/z (ESI): 347.2 [M+1]

### Step 2

The preparation method of compound 15c was refered to the preparation method of compound 13f of Example 13, except that compound 15b was used as starting material to obtain (1R,3aR,7aS)-5-(4,6-Dimethylpyrimidin-2-yl)-1-methyloctahydro-1H-pyrrolo[3,4-c]pyr idine (compound 15c) (960 mg, 86.5%).
MS: m/z (ESI): 247.2 [M+1]

### Step 3

The preparation method of compound 15 was refered to the preparation method of compound 13e of Example 13, except that compound 15c was used as starting material to obtain Compound 15 (110 mg, 66.5%).
MS: m/z (ESI): 436.2 [M+1]

### Example 16

### Preparation of compound 16: ((4aR, SR, 7aR)-1-(4,6-dimethylpyrimidin-2-yl)-5-methyloctahydro-6H-pyrrolo[3,4-b]py ridin-6-yl)(5-fluoro-2-(2H-1,2,3-triazol-2-yl)phenyl)methanone

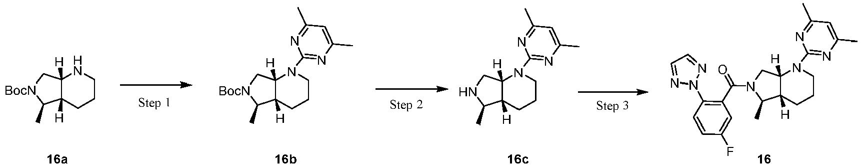

### Step 1

The preparation method of compound 16b was refered to the preparation method of compound 13 of Example 13, except that compound 16a was used as starting material to obtain tert-Butyl (4aR,5R,7aR)-1-(4,6-dimethylpyrimidin-2-yl)-5-methyloctahydro-6H-pyrrolo[3,4-b]pyr idine-6-carboxylate (compound 16b) (0.8 g, 44.5%).
MS: m/z (ESI): 347.2 [M+1]

### Step 2

The preparation method of compound 16c was refered to the preparation method of compound 13f of Example 13, except that compound 16b was used as starting material to obtain (4aR,5R,7aR)-1-(4,6-Dimethylpyrimidin-2-yl)-5-methyloctahydro-1H-pyrrolo[3,4-b]py ridine (compound 16c) (440 mg, 76.3%).
MS: m/z (ESI): 247.2 [M+1]

### Step 3

The preparation method of compound 16 was refered to the preparation method of compound 13e of Example 13, except that compound 16c was used as starting material to obtain Compound 16 (56 mg, 36.6%).
MS: m/z (ESI): 436.2 [M+1]

### Example 17

### Preparation of compound 17: ((1R,4R,6S)-8-(4,6-dimethylpyrimidin-2-yl)-4-methyl-3,8-diazabicyclo[4.2.0]octan-3-y l)(5-fluoro-2-(2H-1,2,3-triazol-2-yl)phenyl)methanone

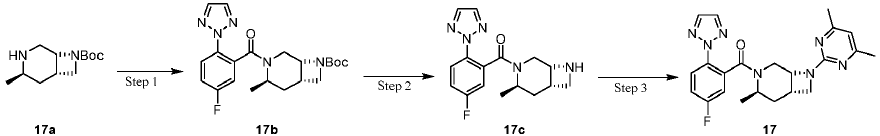

### Step 1

The preparation method of compound 17b was refered to the preparation method of compound 13e of Example 13, except that compound 16a was used as starting material to obtain tert-Butyl (1R,4R,6 S)-3-(5-fluoro-2-(2H-1,2,3-triazol-2-yl)b enzoyl)-4-methyl-3, 8-diazabicyclo[4. 2.0]octane-8-carboxylate (compound 17b) (1.3 g, 84.5%).
MS: m/z (ESI): 416.2 [M+1]

### Step 2

The preparation method of compound 17c was refered to the preparation method of compound 13f of Example 13, except that compound 17b was used as starting material to obtain (5-Fluoro-2-(2H-1,2,3-triazol-2-yl)phenyl)((1R,4R,6S)-4-methyl-3,8-diazabicyclo[4.2.0 ]octan-3-yl)methanone (compound 17c) (0.8 g, 56.3%).
MS: m/z (ESI): 316.2 [M+1]

### Step 3

The preparation method of compound 17 was refered to the preparation method of compound 13 of Example 13, except that compound 17c was used as starting material to obtain Compound 17 (88 mg, 26.6%).
MS: m/z (ESI): 422.2 [M+1]

### Example 18

### Preparation of compound 18: ((3 aR, 7aR)-1-(4,6-dimethylpyrimidin-2-yl)octahydro-6H-pyrrolo[2,3-c]pyridin-6-yl)(5-fluoro-2-(2H-1,2,3-triazol-2-yl)phenyl)methanone

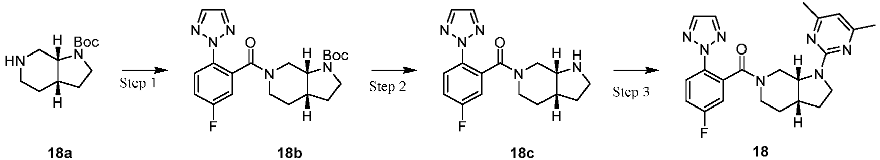

### Step 1

The preparation method of compound 18b was refered to the preparation method of compound 13e of Example 13, except that compound 18a was used as starting material to obtain tert-Butyl (3aS,7aR)-6-(5-fluoro-2-(2H-1,2,3-triazol-2-yl)benzoyl)octahydro-1H-pyrrolo[2,3-c]pyr idine-1-carboxylate (compound 18b) (0.8 g, 74.5%).
MS: m/z (ESI): 416.1 [M+1]

### Step 2

The preparation method of compound 18c was refered to the preparation method of compound 13f of Example 13, except that compound 18b was used as starting material to obtain (5-Fluoro-2-(2H-1,2,3-triazol-2-yl)phenyl)((3aR,7aR)-octahydro-6H-pyrrolo[2,3-c]pyri din-6-yl)methanone (compound 18c) (0.62 g, 46.3%).
MS: m/z (ESI): 316.2 [M+1]

### Step 3

The preparation method of compound 18 was refered to the preparation method of compound 13 of Example 13, except that compound 18c was used as starting material to obtain Compound 18 (55 mg, 36.6%).
MS: m/z (ESI): 422.1 [M+1]

### Example 19

### Preparation of compound 19: ((3aS,7aR)-2-(4,6-dimethylpyrimidin-2-yl)octahydro-5H-pyrrolo[3,4-c]pyridin-5-yl)(5-f luoro-2-(2H-1,2,3-triazol-2-yl)phenyl)methanone

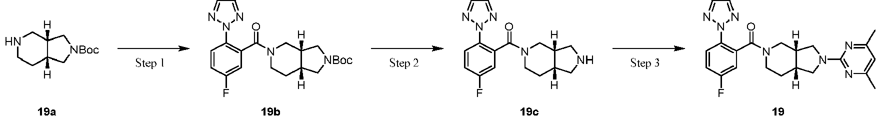

### Step 1

The preparation method of compound 19b was refered to the preparation method of compound 13e of Example 13, except that compound 19a was used as starting material to obtain (3aR,7aR)-5-(5-Fluoro-2-(2H-1,2,3-triazol-2-yl)benzoyl)octahydro-2H-pyrrolo[3,4-c]py ridine-2-carboxylic acid (compound 19b) (0.62 g, 63.4%).
MS: m/z (ESI): 416.2 [M+1]

### Step 2

The preparation method of compound 19c was refered to the preparation method of compound 13f of the example, except that compound 19b was used as starting material to obtain (5-Fluoro-2-(2H-1,2,3-triazol-2-yl)phenyl)((3aS,7aR)-octahydro-5H-pyrrolo[3,4-c]pyrid in-5-yl)methanone (compound 19c) (0.44 g, 56.3%).
MS: m/z (ESI): 316.1 [M+1]

### Step 3

The preparation method of compound 19 was refered to the preparation method of compound 13 of Example 13, except that compound 19c was used as starting material to obtain Compound 19 (35 mg, 32.6%).
MS: m/z (ESI): 422.2 [M+1]

### Example 20

### Preparation of compound 20: ((3aS,7aS)-5-(4,6-dimethylpyrimidin-2-yl)octahydro-2H-pyrrolo[3,4-c]pyridin-2-yl)(5-f luoro-2-(2H-1,2,3-triazol-2-yl)phenyl)methanone

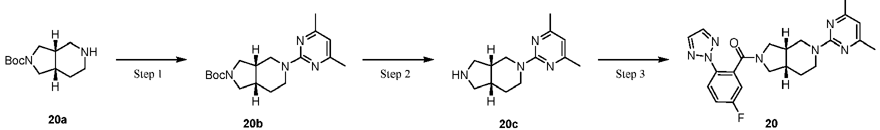

### Step 1

The preparation method of compound 20b was refered to the preparation method of compound 13 of Example 13, except that compound 20a was used as starting material to obtain tert-butyl (3aS,7aS)-5-(4,6-dimethylpyrimidin-2-yl)octahydro-2H-pyrrolo[3,4-c]pyridine-2-carbo xylate (compound 20b) (0.26 g, 56.7%).
MS: m/z (ESI): 333.1 [M+1]

### Step 2

The preparation method of compound 20c was refered to the preparation method of compound 13f of Example 13, except that compound 20b was used as starting material to obtain (3aR,7aS)-5-(4,6-Dimethylpyrimidin-2-yl)octahydro-1H-pyrrolo[3,4-c]pyridine (compound 20c) (0.18 g, 76.3%).
MS: m/z (ESI): 233.2 [M+1]

### Step 3

The preparation method of compound 20 was refered to the preparation method of compound 13e of Example 13, except that compound 20c was used as starting material to obtain Compound 20 (66 mg, 65.5%).
MS: m/z (ESI): 422.2 [M+1]

### Example 21

### Preparation of compound 21: ((4aR,7aR)-1-(4,6-dimethylpyrimidin-2-yl)octahydro-6H-pyrrolo[3,4-b]pyridin-6-yl)(5-fluoro-2-(2H-1,2,3-triazol-2-yl)phenyl)methanone

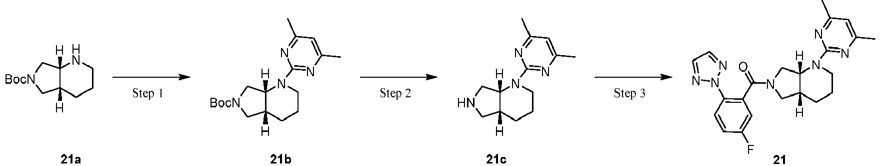

### Step 1

The preparation method of compound 21b was refered to the preparation method of compound 13 of Example 13, except that compound 21a was used as starting material to obtain tert-Butyl (4aR,7aR)-1-(4,6-dimethylpyrimidin-2-yl)octahydro-6H-pyrrolo[3,4-b]pyridine-6-carbo xylate (compound 21b) (0.23 g, 52.7%).
MS: m/z (ESI): 333.1 [M+1]

### Step 2

The preparation method of compound 21c was refered to the preparation method of compound 13f of Example 13, except that compound 21b was used as starting material to obtain (4aR,7aR)-1-(4,6-Dimethylpyrimidin-2-yl)octahydro-1H-pyrrolo[3,4-b]pyridine (compound 21c) (0.12 g, 66.3%).
MS: m/z (ESI): 233.2 [M+1]

### Step 3

The preparation method of compound 21 was refered to the preparation method of compound 13e of Example 13, except that compound 21c was used as starting material to obtain Compound 21 (45 mg, 55.5%).
MS: m/z (ESI): 422.1 [M+1]

### Example 22

### Preparation of compound 22: ((3aS,7aR)-6-(4,6-dimethylpyrimidin-2-yl)octahydro-1H-pyrrolo[2,3-c]pyridin-1-yl)(5-f luoro-2-(2H-1,2,3-triazol-2-yl)phenyl)methanone

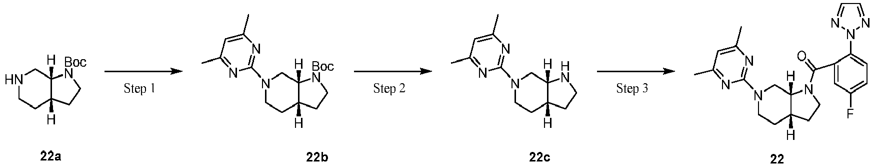

### Step 1

The preparation method of compound 22b was refered to the preparation method of compound 13 of Example 13, except that compound 22a was used as starting material to obtain tert-Butyl (3aS,7aR)-6-(4,6-dimethylpyrimidin-2-yl)octahydro-1H-pyrrolo[2,3-c]pyridine-1-carbo xylate (compound 22b) (0.34 g, 66.7%).
MS: m/z (ESI): 333.2 [M+1]

### Step 2

The preparation method of compound 22c was refered to the preparation method of compound 13f of Example 13, except that compound 22b was used as starting material to obtain (3aR,7aR)-6-(4,6-dimethylpyrimidin-2-yl)octahydro-1H-pyrrolo[2,3-c]pyridine (compound 22c) (0.18 g, 56.3%).
MS: m/z (ESI): 233.2 [M+1]

### Step 3

The preparation method of compound 22 was refered to the preparation method of compound 13e of Example 13, except that compound 22c was used as starting material to obtain Compound 22 (35 mg, 45.5%).
MS: m/z (ESI): 422.1 [M+1]

### Example 23

### Preparation of compound 23: ((3 aR, 7aR)-5-(4,6-dimethylpyrimidin-2-yl)octahydro-2H-pyrrolo[3,4-c]pyridin-2-yl)(5-fluoro-2-(2H-1,2,3-triazol-2-yl)phenyl)methanone

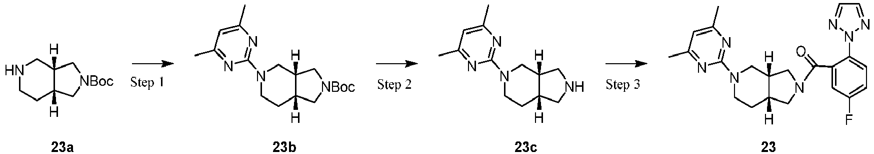

### Step 1

The preparation method of compound 23b was refered to the preparation method of compound 13 of Example 13, except that compound 23a was used as starting material to obtain tert-Butyl (3aR,7aR)-5-(4,6-dimethylpyrimidin-2-yl)octahydro-2H-pyrrolo[3,4-c]pyridine-2-carbo xylate (compound 23b) (0.38 g, 68.7%).
MS: m/z (ESI): 333.2 [M+1]

### Step 2

The preparation method of compound 23c was refered to the preparation method of compound 13f of Example 13, except that compound 23b was used as starting material to obtain (3aS,7aR)-5-(4,6-dimethylpyrimidin-2-yl)octahydro-1H-pyrrolo[3,4-c]pyridine (compound 23c) (0.21 g, 86.3%).
MS: m/z (ESI): 233.1 [M+1]

### Step 3

The preparation method of compound 23 was refered to the preparation method of compound 13e of Example 13, except that compound 23c was used as starting material to obtain Compound 23 (45 mg, 35.5%).
MS:m/z (ESI): 422.2 [M+1]

### Example 24

### Preparation of compound 24: ((3aR,7aS)-2-(4,6-dimethylpyrimidin-2-yl)octahydro-5H-pyrrolo[3,4-c]pyridin-5-yl)(5-f luoro-2-(2H-1,2,3-triazol-2-yl)phenyl)methanone

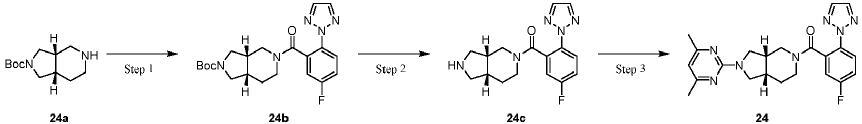

### Step 1

The preparation method of compound 24b was refered to the preparation method of compound 13e of Example 13, except that compound 24a was used as starting material to obtain tert-Butyl (3 aS, 7aS)-5-(5-fluoro-2-(2H-1,2,3-triazol-2-yl)benzoyl)octahydro-2H-pyrrolo[3,4-c]pyr idine-2-carboxylate (compound 24b) (0.8 g, 74.5%).
MS: m/z (ESI): 416.1 [M+1]

### Step 2

The preparation method of compound 24c was refered to the preparation method of compound 13f of Example 13, except that compound 24b was used as starting material to obtain (5-Fluoro-2-(2H-1,2,3-triazol-2-yl)phenyl)((3aR,7aS)-octahydro-5H-pyrrolo[3,4-c]pyrid in-5-yl)methanone (compound 24c) (0.62 g, 46.3%).
MS: m/z (ESI): 316.1 [M+1]

### Step 3

The preparation method of compound 24 was refered to the preparation method of compound 13 of Example 13, except that compound 24c was used as starting material to obtain Compound 24 (55 mg, 36.6%).
MS: m/z (ESI): 422.1 [M+1]

### Example 25

### Preparation of compound 25: ((4aR,7aR)-6-(4,6-dimethylpyrimidin-2-yl)octahydro-1H-pyrrolo[3,4-b]pyridin-1-yl)(5-fluoro-2-(2H-1,2,3-triazol-2-yl)phenyl)methanone

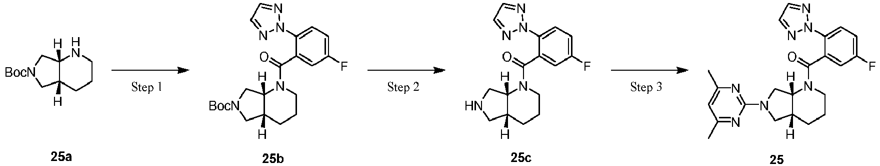

### Step 1

The preparation method of compound 25b was refered to the preparation method of compound 13e of Example 13, except that compound 25a was used as starting material to obtain tert-Butyl (4aR,7aR)-1-(5-fluoro-2-(2H-1,2,3-triazol-2-yl)benzoyl)octahydro-6H-pyrrolo[3,4-b]py ridine-6-carboxylate (compound 25b) (0.8 g, 74.5%).
MS: m/z (ESI): 416.2 [M+1]

### Step 2

The preparation method of compound 25c was refered to the preparation method of compound 13f of Example 13, except that compound 25b was used as starting material to obtain (5-Fluoro-2-(2H-1,2,3-triazol-2-yl)phenyl)((4aR,7aR)-octahydro-1H-pyrrolo[3,4-b]pyri din-1-yl)methanone (compound 25c) (0.62 g, 46.3%).
MS: m/z (ESI): 316.2 [M+1]

### Step 3

The preparation method of compound 25 was refered to the preparation method of compound 13 of Example 13, except that compound 25c was used as starting material to obtain Compound 25 (55 mg, 36.6%).
MS: m/z (ESI): 422.1 [M+1]

### Example 26

### Preparation of compound 26: (2-(2H-1,2,3-triazol-2-yl)phenyl)((4R)-4-((4-bromopyridin-2-yl)amino)-2-azabicyclo[3. 1.0]hexan-2-yl)methanone

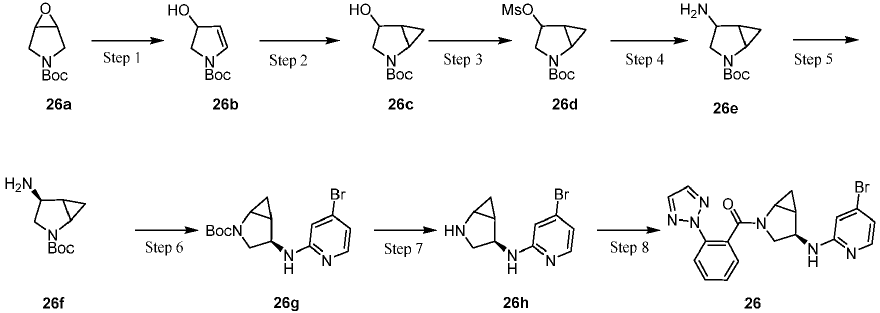

### Step 1

Compound 26a (1.0 g, 5.41 mmol) was dissolved in tetrahydrofuran (20 mL), followed by addition of LiHMDS (10.8 mL, 1.0M) at -50°C. The reaction solution was stirred at -50°C for 10 hours. Water (20 mL) was added to the reaction solution, and extracted with ethyl acetate (20 mL*3). The organic layers were combined, dried over anhydrous sodium sulfate, filtered and concentrated. The residues were purified by silica gel column chromatography (petroleum ether/ethyl acetate system) to obtain *tert*-butyl 3-hydroxy-2,3-dihydro-1H-pyrrole-1-carboxylate (compound 26b) (0.95 g, 95.0%).
MS m/z (ESI): 186.1 [M+1]

### Step 2

N-methyl-N-nitrosourea (500 mg, 4.85 mmol) was added to a mixed solution of 20% aqueous potassium hydroxide solution (2 ml) and diethyl ether (2 ml) in ice bath, and stirred for 1 hour in ice bath. The organic phase of the mixed solution was added to compound 26b (200 mg, 1.08 mmol) dissolved in ether (2 ml), followed by addition of palladium acetate (22 mg, 0.01 mmol). The reaction solution was stirred in ice bath for 30 minutes. The reaction solution was filtered, and the filter cake was washed with DCM (5 ml*3). The filtrate was concentrated, and purified by silica gel column chromatography (petroleum ether/ethyl acetate system) to obtain tert-butyl 4-hydroxy-2-azabicyclo[3.1.0]hexane-2-carboxylate (compound 26c) (182 mg, 84.6%).
MS: m/z (ESI): 200.2 [M+1]

### Step 3

Compound 26c (100 mg, 0.50 mmol) and triethylamine (100 mg, 1.0 mmol) were dissolved in dichloromethane (20 mL), followed by addition of methanesulfonyl chloride (85 mg, 0.75 mmol). The reaction solution was stirred at room temperature for 1 hour. The reaction solution was extracted with water and dichloromethane (3x20 mL). The organic layers were combined, dried over anhydrous sodium sulfate, filtered and concentrated. The residues were purified by silica gel column chromatography (petroleum ether/ethyl acetate system) to obtain *tert-*butyl 4-((methylsulfonyl)oxy)-2-azabicyclo[3.1.0]hexane-2-carboxylate (compound 26d) (110 mg, 79.0%).
MS m/z (ESI): 278.1 [M+1]

### Step 4

Compound 26d (100 mg, 0.36 mmol) was dissolved in a solution of ammonia in methanol (7M, 5 mL), and the reaction solution was stirred at 80°C for 1 hour. The reaction solution was concentrated, and extracted with water and dichloromethane (3x20 mL). The organic layers were combined, dried over anhydrous sodium sulfate, filtered and concentrated. The residues were purified by silica gel column chromatography (petroleum ether/ethyl acetate system) to obtain *tert*-butyl 4-amino-2-azabicyclo[3.1.0]hexane-2-carboxylate (compound 26e) (65 mg, 90.9%).
MS m/z (ESI): 199.1 [M+1]

### Step 5

Compound 26e was resolved by SFC chiral preparation to obtain tert-butyl (*4S*)-4-amino-2-azabicyclo[3.1.0]hexane-2-carboxylate (compound 26f) (30 mg, 46.2%).
MS: m/z (ESI): 199.1 [M+1]

### Step 6

Compound 26f (30 mg, 0.15 mmol) and cesium carbonate (150 mg, 0.45 mmol) were dissolved in N,N-dimethylformamide (5 mL), followed by addition of 4-bromo-2-chloropyridine (38 mg, 0.2 mmol). The reaction solution was stirred at 100°C for 2 hours. Saturated brine (10 mL) was added to the reaction solution, and extracted with ethyl acetate (5 mL*3). The organic phases were combined, dried and concentrated. The residues were purified by silica gel column chromatography (petroleum ether/ethyl acetate system) to obtain tert-butyl (4R)-4-(((4-bromopyridin-2-yl)amino)-2-azabicyclo[3 .1.0]hexane-2-carboxylate (compound 24g) (45 mg, 83.9%).
MS: m/z (ESI): 355.2 [M+1]

### Step 7

The preparation method of compound 26h was refered to the preparation method of compound 13f of Example 13, except that compound 26g was used as starting material to obtain (4R)-N-(4-Bromopyridin-2-yl)-2-azabicyclo[3.1.0]hexan-4-amine (compound 26h) (30 mg, 88.5%).
MS: m/z (ESI): 255.1 [M+1]

### Step 8

The preparation method of compound 26 was refered to the preparation method of compound 13e of Example 13, except that compound 26h was used as starting material to obtain Compound 26 (28 mg, 77.8%).
MS: m/z (ESI): 426.2 [M+1]

### Example 27

### Preparation of compound 27: (2-(4,6-dimethylpyrimidin-2-yl)-5-oxa-2,8-diazaspiro[3.5]nonan-8-yl)(2-fluoro-6-(2H-1 ,2,3-triazol)-2-yl)phenyl)methanone

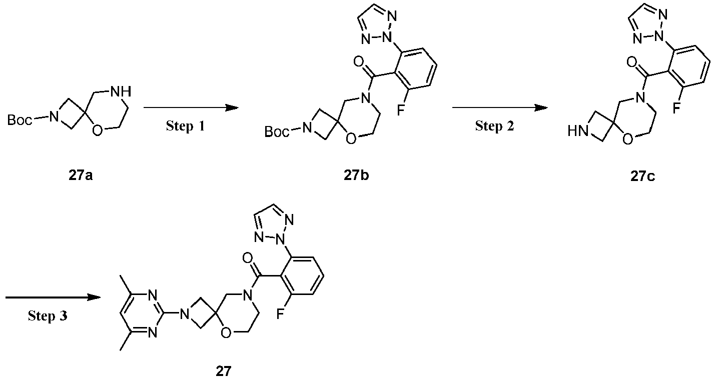

### Step 1

A mixture of 2-fluoro-6-(2H-1,2,3-triazol-2-yl)benzoic acid (1.0 g, 4.83 mmol), HATU (2.8 g, 7.2 mmol) and DIEA (1.25 g, 9.6 mmol) in DMF (30 mL) was stirred at room temperature for 10 minutes. 27a (1.1 g, 4.82 mmol) was added, and the resulting mixture was stirred at room temperature overnight. The reaction solution was cooled, diluted with 100 mL of ethyl acetate, and washed with water (50 mL*3). The organic phases were combined, dried and concentrated. The residues were purified by silica gel column chromatography (petroleum ether/ethyl acetate system) to obtain *tert-*butyl 8-(2-fluoro-6-(2H-1,2,3-triazol-2-yl)benzoyl)-5-oxa-2,8-diazaspiro[3.5]nonane-2-carbo xylate (compound 27b) (1.2 g, white solid, 59.6%).
MS m/z (ESI): 418.2 [M+1]

### Step 2

At 0°C, TFA (10 mL) was slowly added to a solution of 27b (1.2 g, 2.88 mmol) in DCM (20 mL), and the mixture was stirred at 25°C for 2 hours. The reaction solution was quenched with icy 5% NaHCO₃ (60 mL), and extracted with dichloromethane (50 mL*3). The organic phases were combined, dried and concentrated to obtain (2-fluoro-6-(2H-1,2,3-triazol-2-yl)phenyl)(5-oxa-2,8-diazaspiro[3.5]nonan-8-yl)methan one (compound 27c) (0.8 g, white solid, 87.7%).
MS m/z (ESI): 318.1 [M+1]

### Step 3

Cs₂CO₃ (1.6 g, 5 mmol) and 2-chloro-4,6-dimethylpyrimidine (1.1 g, 7.72 mmol) were added to a solution of 27c (0.8 g, 2.52 mmol) in DMF (20 mL), and the mixture was stirred at 90°C for 16 hours. The reaction solution was quenched by water (30 mL), and extracted with dichloromethane (30 mL*3). The organic phases were combined, dried and concentrated. The residues were purified by silica gel column chromatography (petroleum ether/ethyl acetate system) to obtain compound 27 (0.5 g, pale yellow solid, 29.3%).
MS m/z (ESI): 424.2 [M+1]

### Example 28

### Preparation of compound 28: (8-(4,6-dimethylpyrimidin-2-yl)-5-oxa-2,8-diazaspiro[3.5]nonan-2-yl)(2-fluoro-6-(2H-1 ,2,3-triazol)-2-yl)phenyl)methanone

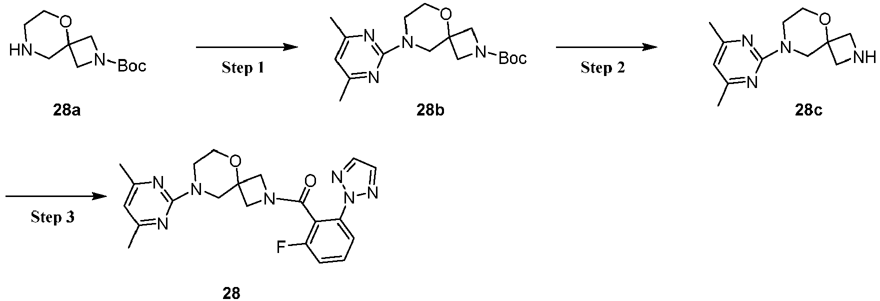

### Step 1

Cs₂CO₃ (2.9 g, 8.8 mmol) and 2-chloro-4,6-dimethylpyrimidine (0.69 g, 4.84 mmol) were added to a solution of 28a (1 g, 4.39 mmol) in DMF (20 mL), and the mixture was stirred at 90°C for 16 hours. The reaction solution was quenched by water (30 mL), and extracted with dichloromethane (30 mL*3). The organic phases were combined, dried and concentrated. The residues were purified by silica gel column chromatography (petroleum ether/ethyl acetate system) to obtain tert-butyl 8-(4,6-dimethylpyrimidin-2-yl)-5-oxa-2,8-diazaspiro[3.5]nonane-2-carboxylate (compound 28b) (1 g, pale yellow solid, 68.3%).
MS m/z (ESI): 335.2 [M+1]

### Step 2

TFA (10 mL) was slowly added to a solution of 28b (1 g, 2.99 mmol) in DCM (20 mL), and the mixture was stirred at 25°C for 2 hours. The reaction solution was quenched with icy 5% NaHCO₃ (60 mL), and extracted with dichloromethane (50 mL*3). The organic phases were combined, dried and concentrated to obtain 8-(4,6-dimethylpyrimidin-2-yl)-5-oxa-2,8-diazaspiro[3.5]nonane (compound 28c) (0.65 g, white solid), which was used directly in the next step.
MS m/z (ESI): 235.1 [M+1]

### Step 3

A mixture of 2-fluoro-6-(2H-1,2,3-triazol-2-yl)benzoic acid (1.0 g, 4.83 mmol), HATU (2.8 g, 7.2 mmol) and DIEA (1.25 g, 9.6 mmol) in DMF (30 mL) was stirred at room temperature for 10 minutes. 28c (1.1 g, 4.83 mmol) was added, and the resulting mixture was stirred at room temperature overnight. The reaction solution was cooled, diluted with 100 mL of ethyl acetate, and washed with water (3x 50 mL). The organic phases were combined, dried and concentrated. The residues were purified by silica gel column chromatography (petroleum ether/ethyl acetate system) to obtain compound 28 (1.2 g, white solid, 46.7%).
MS m/z (ESI): 424.2 [M+1]

### Example 29

### Preparation of compound 29: (1-(4,6-dimethylpyrimidin-2-yl)-1,6-diazaspiro[3.4]octan-6-yl)(5-fluoro-2-(2H-1,2,3-tri azol-2-yl)phenyl)methanone

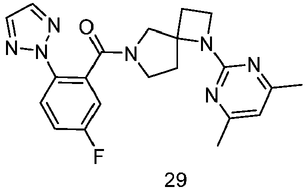

The preparation method of compound 29 was refered to the preparation method of Example 27.
MS: m/z (ESI): 408.1 [M+1]

### Example 30

### Preparation of compound 30: (1-(4,6-dimethylpyrimidin-2-yl)-1,7-diazaspiro[4.4]nonan-7-yl)(5-fluoro-2-(2H-1,2,3-tri azol-2-yl)phenyl)methanone

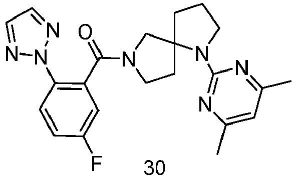

The preparation method of compound 30 was refered to the preparation method of Example 27.
MS m/z (ESI): 422.1 [M+1]

### Example 31

### Preparation of compound 31: (7-(4,6-dimethylpyrimidin-2-yl)-9,9-difluoro-3,7-diazabicyclo[3.3.1]nonan-3-yl)(2-fluo ro-6-(2H-1,2,3-triazol-2-yl)phenyl)methanone

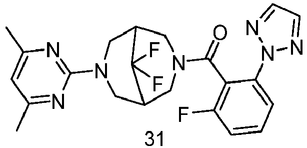

The preparation method of compound 31 was refered to the preparation method of Example 27.
MS m/z (ESI): 458.2 [M+1]

### Example 32

### Preparation of compound 32: (3-(4,6-dimethylpyrimidin-2-yl)-3,6-diazabicyclo[3.2.2]nonan-6-yl)(5-fluoro-2-(2H-1,2, 3-triazol-2-yl)phenyl)methanone

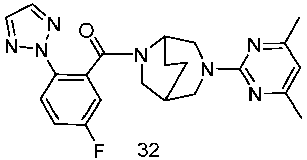

The preparation method of compound 32 was refered to the preparation method of Example 27.
MS m/z (ESI): 422.1 [M+1]

### Example 33

### Preparation of compound 33: (((1R,4R)-5-(4,6-dimethylpyrimidin-2-yl)-2,5-diazabicyclo[2.2.1]heptan-2-yl)(2-fluoro-6-(2H-1,2,3-triazol-2-yl)phenyl)methanone

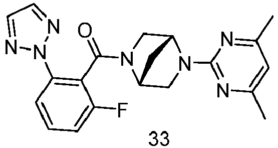

The preparation method of compound 33 was refered to the preparation method of Example 27.
MS m/z (ESI): 394.1 [M+1]

### Example 34

### Preparation of compound 34: (R)-(4-(4,6-dimethylpyrimidin-2-yl)-2-methylpiperazin-1-yl)(2-fluoro-6-(2H-1,2,3-triaz ol-2-yl)phenyl methanone

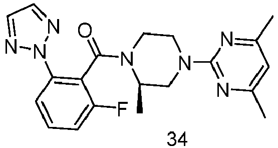

The preparation method of compound 34 was refered to the preparation method of Example 27.
MS m/z (ESI): 396.2 [M+1]

### Example 35

### Preparation of compound 35: (4-((4,6-dimethylpyrimidin-2-yl)amino)-4-methylpiperidin-1-yl)(2-fluoro-6-(2H-1,2,3-t riazol-2-yl)phenyl)methanone

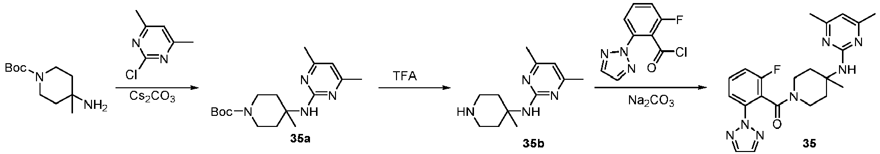

*tert*-Butyl 4-((4,6-dimethylpyrimidin-2-yl)amino)-4-methylpiperidine-1-carboxylate (compound 35a) (95 mg, 63.5%) was obtained by reference to the preparation method of compound 12b of Example 12 except for using tert-butyl 4-amino-4-methylpiperidine-1-carboxylate (100 mg, 0.467 mmol) and 2-chloro-4,6-dimethylpyrimidine (81 mg, 0.568 mmol) as starting materials.
MS: m/z (ESI): 321.2 [M+1]

4,6-Dimethyl-N-(4-methylpiperidin-4-yl)pyrimidin-2-amine (compound 35b) (65 mg, 99.5 %) was obtained by reference to the preparation method of compound 3c of Example 3 except for using tert-butyl 4-((4,6-dimethylpyrimidin-2-yl)amino)-4-methylpiperidine-1-carboxylate (95 mg, 0.297 mmol) as starting material.
MS: m/z (ESI): 221.1 [M+H]

Compound 35 (25.3 mg, 20.9%) was obtained by reference to the preparation method of compound 12 of Example 12 except for using 4,6-dimethyl-N-(4-methylpiperidin-4-yl)pyrimidin-2-amine (65 mg, 0.295 mmol) and 2-fluoro-6-(2H-1,2,3-triazol-2-yl)benzoyl chloride (80 mg, 0.355 mmol) as starting materials.
MS: m/z (ESI): 410.2 [M+H]

### Example 36

Preparation of compound 36: N-(1-(4,6-dimethylpyrimidin-2-yl)-4-methylpiperidin-4-yl)-2-fluoro-6-(2H-1,2,3-triazol -2-yl)benzamide

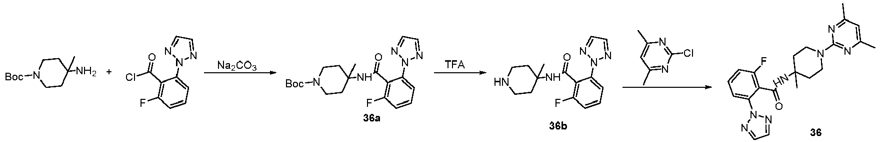

*tert*-Butyl 4-(2-fluoro-6-(2H-1,2,3-triazol-2-yl)benzamido)-4-methylpiperidine-1-carboxylate (compound 35a) (210 mg, 55.8%) was obtained by reference to the preparation method of compound 12 of Example 12 except for using *tert*-butyl 4-amino-4-methylpiperidine-1-carboxylate (200 mg, 0.935 mmol) and 2-fluoro-6-(2H-1,2,3-triazol-2-yl)benzoyl chloride (253 mg, 1.12 mmol) as starting materials.
MS: m/z (ESI): 404.1 [M+1]

2-Fluoro-N-(4-methylpiperidin-4-yl)-6-(2H-1,2,3-triazol-2-yl)benzamide (compound 35b) (150 mg, 95%) was obtained by reference to the preparation method of compound 3c of Example 3 except for using *tert*-butyl 4-(2-fluoro-6-(2H-1,2,3-triazol-2-yl)benzamido)-4-methylpiperidine-1-carboxylate (210 mg, 0.521 mmol) as starting material.
MS: m/z (ESI): 304.2 [M+H]

Compound 35 (19.3 mg, 17.9%) was obtained by reference to the preparation method of compound 12b of Example 12 except for using 2-fluoro-N-(4-methylpiperidin-4-yl)-6-(2H-1,2,3-triazol-2-yl)benzamide (80 mg, 0.264 mmol) and 2-chloro-4,6-dimethylpyrimidine (45 mg, 0.316 mmol) as starting materials.
MS: m/z (ESI): 410.2 [M+H]

### Example 37

### Preparation of compound 37: (2-(2H-1,2,3-triazol-2-yl)phenyl)(4-((6-(1-hydroxyethyl)pyridin-2-yl)amino)azepan-1-y l)methanone

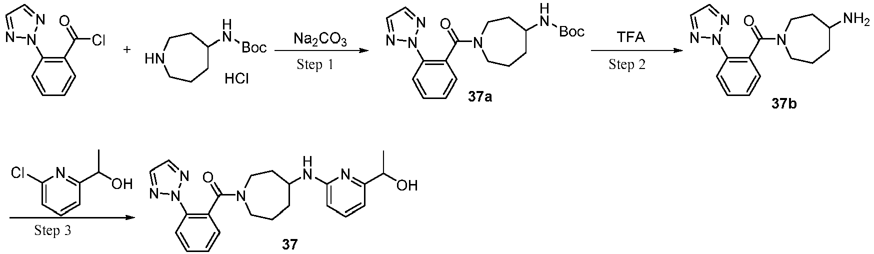

### Step 1

*tert*-Butyl (1-(2-(2H-1,2,3-triazol-2-yl)benzoyl)azepan-4-yl)carbamate (compound 37a) (105 mg, 68.0%) was obtained by reference to the preparation method of compound 12 of Example 12 except for using 4-*tert*-butoxycarbonyl-1H-azepine hydrochloride (100 mg, 0.401 mmol) and 2-(2H-1,2,3-triazol-2-yl)benzoyl chloride (96 mg, 0.463 mmol) as starting materials.
MS: m/z (ESI): 386.0 [M+1]

### Step 2

(2-(2H-1,2,3-Triazol-2-yl)phenyl)(4-aminoazepan-1-yl)methanone (compound 36b) (75 mg, 96.5%) was obtained by reference to the preparation method of compound 3c of Example 3 except for using compound 37a (105 mg, 0.273 mmol) as starting material.
MS: m/z (ESI): 286.1 [M+H]

### Step 3

Compound 37 (19.3 mg, 18.1%) was obtained by reference to the preparation method of compound 12b of Example 12 except for using 37b (75 mg, 0.263 mmol) and 2-chloro-6-(1-hydroxyethyl)pyridine (49 mg, 0.311 mmol) as starting materials.
MS: m/z (ESI): 407.2 [M+H]

### Example 38

### Preparation of compound 38: (2-(2H-1,2,3-triazol-2-yl)phenyl)(5-((6-(1-hydroxyethyl)pyridin-2-yl)amino)-2-methyla zepan-1 -yl)methanone

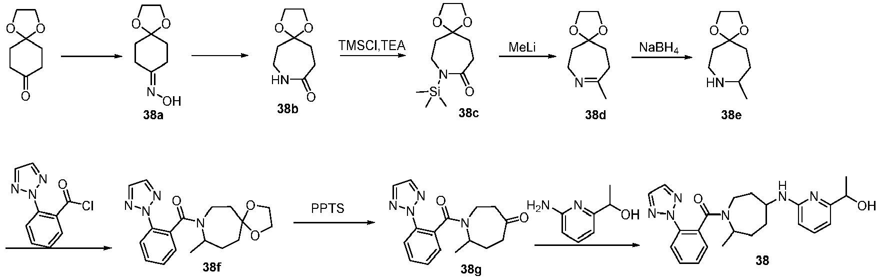

1,4-Cyclohexanedione monoethylene glycol ketal (10 g, 64.1 mmol) and hydroxylamine hydrochloride (8.85 g, 128 mmol) were dissolved in tetrahydrofuran (50 mL) and water (50 mL). Sodium carbonate (13.6 g, 128 mmol) were added in batches and reacted at room temperature for 1 hour. The reaction solution was extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure to obtain the crude product 4-oxime-cyclohexanone ethylene glycol acetal 38a (10.0 g), which was used directly in the next step.

Compound 38a (10.0 g, 58.5 mmol) was dissolved in tetrahydrofuran (50 mL), followed by addition of sodium hydroxide (4.68 g, 117.0 mmol) in water (50 mL). After about 1 hour, benzenesulfonyl chloride (10.3 g, 58.5 mmol) was slowly added dropwise, then the solution was warmed up to 50°C and reacted for 16 hours. The reaction solution was cooled to room temperature, poured into 100 mL of water, and extracted with dichloromethane (100 mL*3). The organic phases were combined, washed with water (100 mL) and saturated sodium chloride solution (100 mL) successively, dried over anhydrous sodium sulfate and filtered. The filtrate was concentrated under reduced pressure and purified by silica gel column chromatography (methanol/dichloromethane = 0 ~ 10 %) to obtain 1,4-dioxa-8-azaspiro[4.6]undecan-9-one (compound 38b) (6.6 g, 66%) as an off-white solid.
MS: m/z (ESI): 172.1 [M+H]

38b (6.6 g, 38.6 mmol) was dissolved in anhydrous toluene (80 mL), followed by addition of triethylamine (5.07 g, 50.2 mmol) and trimethylchlorosilane (4.61 g, 42.5 mmol). The mixture was reacted at room temperature for 16 hours. The reaction solution was filtered, and the filtrate was concentrated under reduced pressure to obtain 8-(trimethylsilyl)-1,4-dioxa-8-azaspiro[4.6]undecan-9-one (compound 38c) (7.3 g, 77.8%), which was used directly in the next step.

Compound 38c (7.3 g, 30.0 mmol) was dissolved in anhydrous tetrahydrofuran (100 mL). The solution was cooled to -30°C under nitrogen protection, followed by addition of 1 M methyllithium tetrahydrofuran solution (30 mL, 30.0 mmol) dropwise. The mixture was reacted at room temperature for 16 hours. The reaction solution was poured into 100 mL of saturated ammonium chloride, and extracted with methyl tert-butyl ether (100 mL*2). The organic phases were combined, washed with water (100 mL) and saturated sodium chloride solution (100 mL) successively, dried over anhydrous sodium sulfate and filtered. The filtrate was concentrated under reduced pressure to obtain 9-methyl-1,4-dioxa-8-azaspiro[4.6]undec-8-ene (compound 38d) (4.4 g, 86.6%), which was used directly in the next step.

Compound 38d (4.4 g, 26.0 mmol) was dissolved in anhydrous ethanol (100 mL), followed by slowly addition of sodium borohydride (1.48 g, 39.1 mmol) in batches. The mixture was reacted at room temperature for 4 hours. The reaction solution was poured into 10% potassium carbonate solution (100 mL), stirred for 30 minutes, and extracted with dichloromethane (100 mL*3). The organic phases were combined, washed with water (100 mL) and saturated sodium chloride solution (100 mL) successively, dried over anhydrous sodium sulfate and filtered. The filtrate was concentrated under reduced pressure and purified by silica gel column chromatography (methanol/dichloromethane = 0 ~ 10 %) to obtain 9-methyl-1,4-dioxa-8-azaspiro[4.6]undecane (compound 38e) (2.6 g, 58.4%).
MS: m/z (ESI): 172.1 [M+H]

(2-(2H-1,2,3-Triazol-2-yl)phenyl)(9-methyl-1,4-dioxa-8-azaspiro[4.6]undecan-8-yl )methanone (compound 38f) (230 mg, 57.5%) was obtained by reference to the preparation method of compound 12 of Example 12 except for using compound 38e (200 mg, 1.17 mmol) and 2-(2H-1,2,3-triazol-2-yl)benzoyl chloride (243 mg, 1.17 mmol) as starting materials.
MS: m/z (ESI): 343.2 [M+1]

Compound 38f (230 mg, 0.673 mmol) was dissolved in tetrahydrofuran (3 mL) and water (1 mL), followed by addition of pyridine 4-methylbenzenesulfonate (169 mg, 0.673 mmol). The mixture was heated to 60°C and reacted for 16 hours. The reaction solution was cooled to room temperature, followed by addition of 30 mL of ethyl acetate. The solution was washed with water (30 mL) and saturated sodium chloride solution (30 mL) successively, dried over anhydrous sodium sulfate and filtered. The filtrate was concentrated under reduced pressure to obtain 1-(2-(2H-1,2,3-triazol-2-yl)benzoyl)-7-methylazepan-4-one (compound 38g) (180 mg, 89.8%), which was used directly in the next step.
MS: m/z (ESI): 299.1 [M+1]

Compound 38g (180 mg, 0.604 mmol) was dissolved in toluene (5 mL), followed by addition of 1-(6-aminopyridin-2-yl)ethanol (83 mg, 0.601 mmol). The mixture was heated to 80°C and reacted for 4 hours. Sodium triacetoxyborohydride (255 mg, 1.20 mmol) was added, and reacted at room temperature for 16 hours. The reaction solution was poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the crude product was purified by reverse HPLC to obtain compound 38 (31.0 mg, 12.3%) as a white solid.
MS: m/z (ESI): 421.2 [M+1]

### Example 39

### Preparation of compound 39: (2-(2H-1,2,3-triazol-2-yl)phenyl)(5-((6-(1-hydroxyethyl)pyridin-2-yl)oxy)-2-methylaze pan-1-yl)methanone

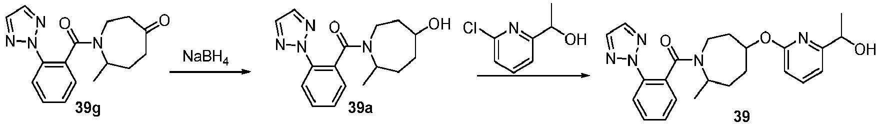

Compound 39g (100 mg, 0.336 mmol) was dissolved in tetrahydrofuran (2 mL), followed by addition of sodium borohydride (38 mg, 1.00 mmol). The mixture was reacted at room temperature for 3 hours. The reaction solution was poured into 30 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure to obtain the crude product (2-(2H-1,2,3-triazol-2-yl)phenyl)(4-hydroxyazepan-1-yl)methanone (compound 39a) (100 mg), which was used directly in the next step.
MS: m/z (ESI): 301.2 [M+1]

Compound 39a (100 mg, 0.333 mmol) was dissolved in anhydrous tetrahydrofuran (2 mL), followed by addition of sodium hydride (27 mg, 60% w.t., 0.675 mmol). The mixture was reacted at room temperature for 30 minutes. 1-(6-Chloropyridin-2-yl)ethan-1-ol (104 mg, 0.662 mmol) was added, and the mixture was reacted at room temperature for 2 hours. The reaction solution was poured into 30 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the crude product was purified by reverse HPLC to obtain compound 39 (11.3 mg, 8.1%) as a white solid.
MS: m/z (ESI): 422.2 [M+1]

### Example 40

### Preparation of compound 40: N-ethyl-4-fluoro-N-(2-(6-(2-hydroxypropan-2-yl)-5-methylpyridin-2-yl)cyclopropyl)-2-(2H-1,2,3-triazol-2-yl)benzamide

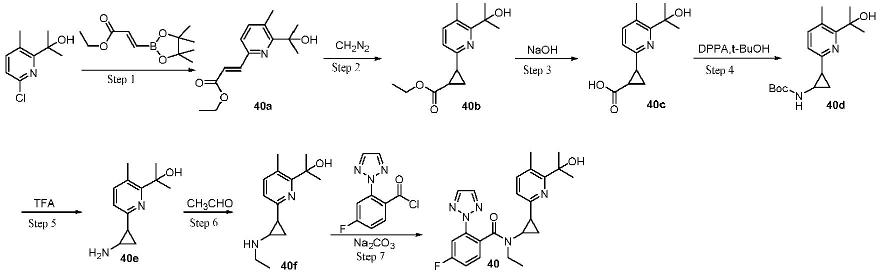

### Step 1

2-(6-Chloro-3-methylpyridin-2-yl)propanol (1.0 g, 5.39 mmol) was dissolved in toluene (10 mL) and water (10 mL), followed by addition of 2-(ethoxycarbonyl)vinylboronic acid pinacol ester (1.83 g, 8.09 mmol), Pd(dppf)Cl₂ (197 mg, 0.270 mmol) and sodium carbonate (1.14 g, 10.8 mmol). The mixture was purged with nitrogen three times, heated to 100°C and reacted for 2 hours. The reaction solution was cooled to room temperature, poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure and purified by silica gel column chromatography (ethyl acetate/petroleum ether = 30 % ~ 100 %) to obtain ethyl 3-(6-(2-hydroxypropan-2-yl)-5-methylpyridin-2-yl)acrylate (compound 40a) (800 mg, 59.4%) as an off-white solid.
MS: m/z (ESI): 250.1 [M+1]

### Step 2

Compound 40a (800 mg, 3.21 mmol) was dissolved in anhydrous dichloromethane (10 mL), and cooled to 0°C. A freshly prepared 1 M solution of diazomethane in dichloromethane (10 mL, 10 mmol) was added, and the mixture was reacted at room temperature for 4 hours. 2 mL of acetic acid was added and stirred for 10 minutes. The reaction solution was poured into 50 mL of water, and extracted with ethyl acetate (2x50 mL). The organic phases were combined, washed with water (50 mL) and saturated sodium chloride solution (50 mL) successively, dried over anhydrous sodium sulfate and filtered. The filtrate was concentrated under reduced pressure and purified by silica gel column chromatography (ethyl acetate/petroleum ether = 0 ~ 100 %) to obtain ethyl 2-(6-(2-hydroxypropan-2-yl)-5-methylpyridin-2-yl)cyclopropane-1-carboxylate (compound 40b) (560 mg, 66.3%).
MS: m/z (ESI): 264.2 [M+1]

### Step 3

Compound 40b (560 mg, 2.13 mmol) was dissolved in methanol (10 mL) and water (5 mL), followed by addition of sodium hydroxide (170 mg, 4.26 mmol). The mixture was reacted at room temperature for 2 hours. The reaction solution was poured into 50 mL of water, adjusted to pH 5 with dilute hydrochloric acid, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure to obtain the crude product 2-(6-(2-hydroxypropan-2-yl)-5-methylpyridin-2-yl)cyclopropane-1-carboxylic acid (compound 40c) (480 mg, 95.9%), which was used directly in the next step.
MS: m/z (ESI): 236.1 [M+1]

### Step 4

Compound 40c (480 mg, 2.04 mmol) was dissolved in tert-butanol (5 mL), followed by addition of triethylamine (69 mg, 0.681 mmol) and diphenylphosphoryl azide (842 mg, 3.06 mmol). The mixture was heated to 90°C and reacted for 6 hours. The reaction solution was cooled to room temperature, poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure and purified by silica gel column chromatography (ethyl acetate/petroleum ether = 0 ~ 80 %) to obtain tert-butyl (2-(6-(2-hydroxypropan-2-yl)-5-methylpyridin-2-yl)cyclopropyl)carbamate (compound 40d) (260 mg, 41.5%).
MS: m/z (ESI): 307.2 [M+1]

### Step 5

Compound 40d (260 mg, 0.850 mmol) was dissolved in dichloromethane (2 mL), followed by addition of trifluoroacetic acid (2 mL). The mixture was reacted at room temperature for 2 hours. The reaction solution was concentrated under reduced pressure to obtain the crude product 2-(6-(2-aminocyclopropyl)-3-methylpyridin-2-yl)propanol (compound 40e) (175 mg), which was used directly in the next step.
MS: m/z (ESI): 207.1 [M+1]

### Step 6

2-(6-(2-(Ethylamino)cyclopropyl)-3-methylpyridin-2-yl)propanol (compound 40f) (150 mg, 75.5%) was obtained by reference to the last step of the preparation method of Example 38 except for using compound 45e (175 mg, 0.850 mmol) and acetaldehyde (75 mg, 1.70 mmol) as starting materials.
MS: m/z (ESI): 235.2 [M+1]

### Step 7

Compound 40 (42.3 mg, 15.6%) was obtained by reference to the preparation method of compound 12 of Example 12 except for using compound 45f (150 mg, 0.641 mmol) and 4-fluoro-2-(2H-1,2,3-triazol-2-yl)benzoyl chloride (255 mg, 1.13 mmol) as starting materials.
MS: m/z (ESI): 424.2 [M+1]

### Example 41

### Preparation of compound 41: N-(1-(4,6-dimethylpyrimidin-2-yl)azetidin-3-yl)-N-ethyl-2-(2H-1,2,3-triazol-2-yl)benza mide

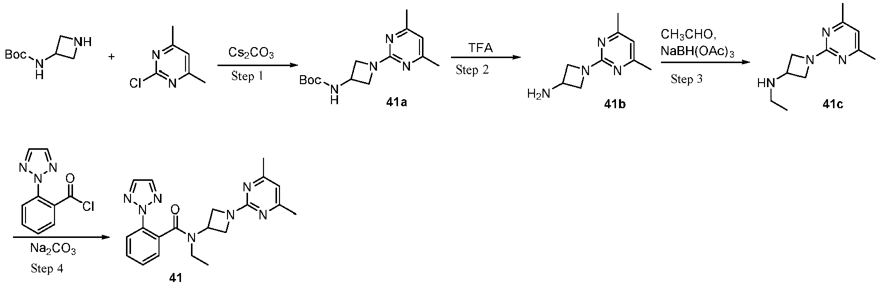

### Step 1

tert-Butyl (1-(4,6-dimethylpyrimidin-2-yl)azetidin-3-yl)carbamate (compound 41a) (390 mg, 80.4%) was obtained by reference to the preparation method of compound 1 of Example 1 except for using 3-N-*tert*-butoxycarbonylaminocyclobutylamine (300 mg, 1.74 mmol) and 2-chloro-4,6-dimethylpyrimidine (299 mg, 2.10 mmol) as starting materials.
MS: m/z (ESI): 279.1 [M+1]

### Step 2

1-(4,6-Dimethylpyrimidin-2-yl)azetidinamine (compound 41b) (250 mg) was obtained by reference to the preparation method of compound 3c of Example 3 except for using compound 41a (390 mg, 1.40 mmol) as starting material.
MS: m/z (ESI): 179.1 [M+H]

### Step 3

1-(4,6-Dimethylpyrimidin-2-yl)-N-ethylazetidinamine (compound 41c) (160 mg, 55.3%) was obtained by reference to the preparation method of compound 40f of Example 40 except for using compound 41b (250 mg, 1.40 mmol) as starting material.
MS: m/z (ESI): 207.2 [M+H]

### Step 4

Compound 41 (30.5 mg, 27.8%) was obtained by reference to the preparation method of compound 12 of Example 12 except for using compound 41c (60 mg, 0.291 mmol) and 2-(2H-1,2,3-triazol-2-yl)benzoyl chloride (72 mg, 0.348 mmol) as starting materials.
MS: m/z (ESI): 378.2 [M+H]

### Example 42

### Preparation of compound 42: ((3aR,6aS)-5-(4,6-dimethylpyrimidin-2-yl)hexahydropyrrolo[3,4-c]pyrrol-2(1H)-yl)(2-f luoro-6-(4-(trifluoromethyl)-2H-1,2,3-triazol-2-yl)phenyl)methanone

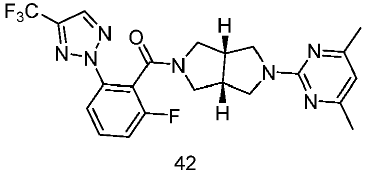

The preparation method of compound 42 was refered to the preparation method of Example 3.
MS m/z (ESI): 476.2 [M+1]

### Example 43

### Preparation of compound 43: (2-(4-cyclopropyl-2H-1,2,3-triazol-2-yl)-6-fluorophenyl)((3aR,6aS)-5-(4,6-dimethylpyri midin-2-yl)hexahydropyrrolo[3,4-c]pyrrol-2(1H)-yl)methanone

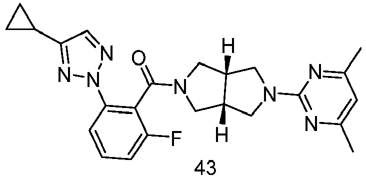

The preparation method of compound 43 was refered to the preparation method of Example 3.
MS m/z (ESI): 448.2 [M+1]

### Example 44

### Preparation of compound 44: ((3aR,6aS)-5-(4,6-dimethylpyrimidin-2-yl)hexahydropyrrolo[3,4-c]pyrrol-2(1H)-yl)(2-( 2,2,2-trifluoroethoxy)pyridin-3-yl)methanone

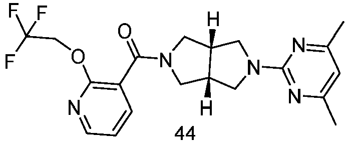

The preparation method of compound 44 was refered to the preparation method of Example 3.
MS m/z (ESI): 422.2 [M+1]

### Example 45

### Preparation of compound 45: ((3aR,6aS)-5-(4,6-dimethylpyrimidin-2-yl)hexahydropyrrolo[3,4-c]pyrrol-2(1H)-yl)(2-( 1, 1,2,2-tetrafluoroethoxy)phenyl)methanone

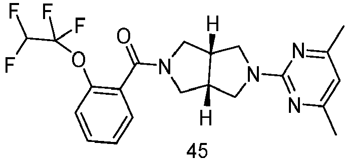

The preparation method of compound 45 was refered to the preparation method of Example 3.
MS m/z (ESI): 439.1 [M+1]
¹H NMR (400 MHz, DMSO-d6) δ7.56 - 7.38 (m, 4H), 6.71 (tt, J = 51.6, 2.8 Hz, 1H), 6.39 (s, 1H), 3.78 - 3.73 (m, 2H), 3.66 - 3.61 (m, 1H), 3.46 - 3.37 (m, 3H), 3.31 - 3.28 (m, 1H), 3.07 - 2.90 (m, 3H), 2.21 (s, 6H).

### Example 46

### Preparation of compound 46: (2-(difluoromethoxy)phenyl)((3aR,6aS)-5-(4,6-dimethylpyrimidin-2-yl)hexahydropyrro lo[3,4-c]pyrrol-2(1H)-yl)methanone

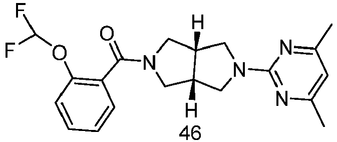

The preparation method of compound 46 was refered to the preparation method of Example 3.
MS m/z (ESI): 389.2 [M+1]

### Example 47

### Preparation of compound 47: (2-fluoro-6-(2H-1,2,3-triazol-2-yl)phenyl)((3aR,6aS)-5-(6-(1-hydroxyethyl)pyridin-2-yl )hexahydropyrrolo[3,4-c]pyrrol-2(1H)-yl)methanone

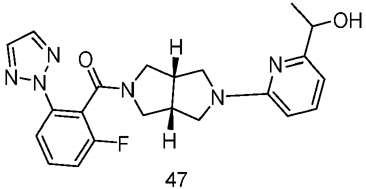

The preparation method of compound 47 was refered to the preparation method of Example 3.
MS m/z (ESI): 423.2 [M+1]

### Example 48

### Preparation of compound 48: (2-fluoro-6-(2H-1,2,3-triazol-2-yl)phenyl)((3aR,6aS)-5-(6-(2-hydroxypropan-2-yl)-5-m ethylpyridin-2-yl)hexahydropyrrolo[3,4-c]pyrrol-2(1H)-yl)methanone

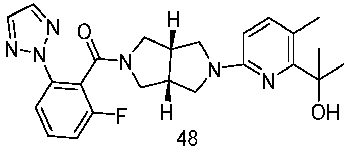

The preparation method of compound 48 was refered to the preparation method of Example 3.
MS m/z (ESI): 451.1 [M+1]
¹H NMR (400 MHz, CDCl3) δ 7.96 - 7.78 (m, 2H), 7.74 (s, 1H), 7.49 (dt, J = 14.2, 7.0 Hz, 1H), 7.28 (s, 1H), 7.21 - 7.03 (m, 1H), 6.22 (s, 1H), 4.12 - 3.86 (m, 1H), 3.85 - 2.92 (m, 9H), 2.33 (s, 3H), 1.76 - 1.28 (m, 6H).

### Example 49

### Preparation of compound 49: ((3aR,6aS)-5-(4,6-dimethylpyrimidin-2-yl)hexahydropyrrolo[3,4-c]pyrrol-2(1H)-yl)(2-f luoro-6-(1,1,2,2-tetrafluoroethoxy)phenyl)methanone

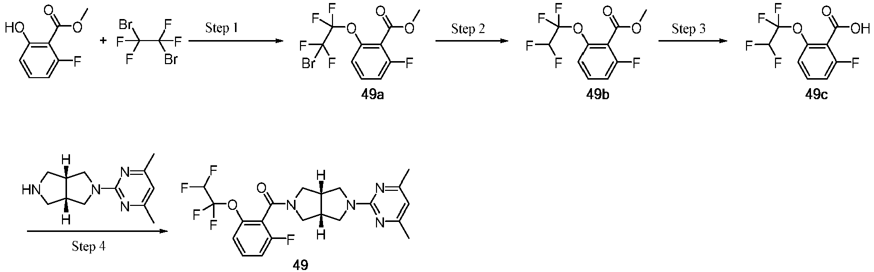

### Step 1

Methyl 2-fluoro-6-hydroxybenzoate (2 g, 11.8 mmol) was dissolved in anhydrous dimethyl sulfoxide (20 mL), followed by addition of 1,2-dibromotetrafluoroethane (5.74 g, 22.1 mmol) and cesium carbonate (5.75 g, 17.6 mmol). The mixture was reacted at 60°C for 16 hours under nitrogen atmosphere. The reaction solution was cooled to room temperature, poured into 100 mL of water, and extracted with methyl tert-butyl ether (80 mL*2). The organic phases were combined, washed with water (80 mL) and saturated sodium chloride solution (80 mL) successively, dried over anhydrous sodium sulfate and filtered. The filtrate was concentrated under reduced pressure to obtain methyl 2-(2-bromo-1,1,2,2-tetrafluoroethoxy)-6-fluorobenzoate (compound 49a) (4.0 g, yellow oil, 97.4%), which was used directly in the next step.
MS: m/z (ESI): 348.9 [M+1]

### Step 2

Compound 49a (4.0 g, 11.5 mmol) was dissolved in acetic acid (20 mL), and heated to 60°C. Zinc powder (2.25 g, 34.4 mmol) was added in batches, and reacted at 60°C for 1 hour. The reaction solution was cooled to room temperature and filtered. The filtrate was concentrated under reduced pressure to remove most of the solvent, and dissolved in 100 mL of ethyl acetate. The solution was washed with saturated sodium carbonate solution (50 mL * 2) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain methyl 2-fluoro-6-(1,1,2,2-tetrafluoroethoxy)benzoate (compound 49b) (2.5 g, colorless liquid, 80.8%).
MS: m/z (ESI): 271.0 [M+1]
¹H NMR (400 MHz, DMSO-*d*₆) δ 7.73 - 7.68 (m, 1H), 7.46 - 7.41 (m, 1H), 7.35 (d, *J* = 8.4 Hz, 1H), 6.81 (tt, *J* = 51.6, 2.9 Hz, 1H), 3.87 (s, 3H).

### Step 3

Compound 49b (800 mg, 2.96 mmol) was dissolved in methanol (8 mL) and water (2 mL), followed by addition of sodium hydroxide (355 mg, 8.88 mmol). The mixture was reacted at room temperature for 16 hours. The reaction solution was poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure to obtain 2-fluoro-6-(1,1,2,2-tetrafluoroethoxy)benzoic acid (compound 49c) (700 mg, white solid, 92.3%).
MS: m/z (ESI): 257.0 [M+1]

### Step 4

The preparation method of compound 49 was refered to the preparation method of compound 5 of Example 5 except that 5c (80 mg, 0.37 mmol) and 49c (94 mg, 0.37 mmol) were used as starting materials to obtain Compound 49 (75 mg, white solid, 44.8%).
MS: m/z (ESI): 457.2 [M+1]

### Example 50

### Preparation of compound 50: (2-(benzooxazol-2-yl)phenyl)((3 aR, 6aS)-5-(4, 6-dimethylpyrimidin-2-yl)hexahydropyrr olo[3,4-c]pyrrol-2(1H)-yl)methanone

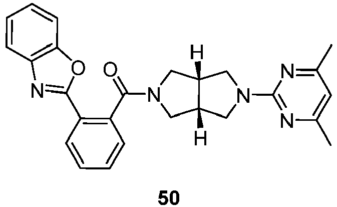

The synthetic method of compound 50 was refered to the synthetic method of Example 5, except that intermediate 3 was replaced with intermediate 6 to Compound 50 (28 mg, white solid, yield 26.5%).
MS m/z (ESI): 440.1 [M+1]
¹H NMR (400 MHz, DMSO-d6) δ 8.17 (d, J = 7.5 Hz, 1H), 7.80 - 7.75 (m, 1H), 7.73 - 7.62 (m, 3H), 7.50 (d, J = 7.2 Hz, 1H), 7.42 - 7.33 (m, 2H), 6.44 (s, 1H), 3.82 (td, J = 13.1, 12.4, 7.4 Hz, 2H), 3.66 - 3.61 (m, 2H), 3.49 (dd, J = 10.1, 3.7 Hz, 3H), 3.14 (dd, J = 10.8, 5.0 Hz, 1H), 3.08 (dd, J = 9.8, 4.5 Hz, 1H), 2.99 (q, J = 6.4, 5.9 Hz, 1H), 2.24 (s, 6H).

### Example 51

### Preparation of compound 51: ((3aR,6aS)-5-(4,6-dimethylpyrimidin-2-yl)hexahydropyrrolo[3,4-c]pyrrol-2(1H)-yl)(2-( quinazolin-2-yl)phenyl)methanone

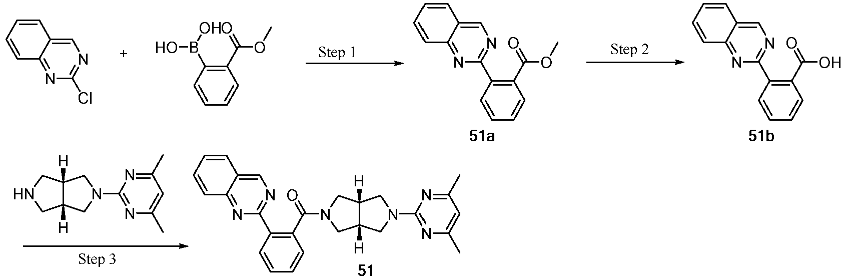

### Step 1

2-Chloroquinazoline (500 mg, 3.04 mmol) was dissolved in 1,4-dioxane (8 mL) and water (2 mL), followed by addition of 2-(methoxycarbonyl)phenylboronic acid (656.04 mg, 3.64 mmol), Pd(dppf)Cl₂ (111 mg, 0.152 mmol) and cesium carbonate (1.48 g, 4.54 mmol). The mixture was purged with nitrogen three times, and reacted in microwave at 100°C for 1 hour. The reaction solution was cooled to room temperature, poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by silica gel column chromatography (petroleum ether/ethyl acetate system) to obtain methyl 2-(quinazolin-2-yl)benzoate (compound 51a) (140 mg, white solid, 17.4%), which was used directly in the next step.
MS: m/z (ESI): 265.1 [M+1]

### Step 2

Compound 51a (140 mg, 0.53 mmol) was dissolved in methanol (3 mL) and water (1 mL), followed by addition of sodium hydroxide (64 mg, 1.60 mmol). The mixture was heated to 50°C and reacted for 2 hours. The reaction solution was cooled to room temperature, poured into 50 mL of water, adjusted to pH 5 with dilute hydrochloric acid, and extracted with ethyl acetate (30 mL*2). The organic phases were combined, washed with saturated sodium chloride solution (30 mL), dried over anhydrous sodium sulfate and filtered. The filtrate was concentrated under reduced pressure to obtain 2-(quinazolin-2-yl)benzoic acid (compound 51b) (130 mg, white solid, 98.1%), which was used directly in the next step.
MS: m/z (ESI): 251.1 [M+1]

### Step 3

The preparation method of compound 51 was refered to the preparation method of compound 1, except that 1c (80 mg, 0.37 mmol) and 51b (91 mg, 0.36 mmol) were used as starting materials to obtain Compound 51 (4.8 mg, white solid, 2.9%).
¹H NMR (400 MHz, DMSO-d₆) δ 9.63 (s, 1H), 8.33 - 8.31 (m, 1H), 8.11 (d, *J* = 8.0 Hz, 1H), 7.86 (s, 2H), 7.75 - 7.71 (m, 1H), 7.60 - 7.58 (m, 2H), 7.44 - 7.42 (m, 1H), 6.40 (s, 1H), 3.84 - 3.72 (m, 2H), 3.64 - 3.55 (m, 3H), 3.47 - 3.44 (m, 2H), 3.15 - 3.08 (m, 2H), 3.00 - 2.94 (m, 1H), 2.21 (s, 6H).
MS: m/z (ESI): 451.0 [M+1]

### Example 52

### Preparation of compound 52: (2-(benzo[d]thiazol-2-yl)phenyl)((3aR,6aS)-5-(4,6-dimethylpyrimidin-2-yl)hexahydrop yrrolo[3,4-c]pyrrol-2(1H)-yl)methanone

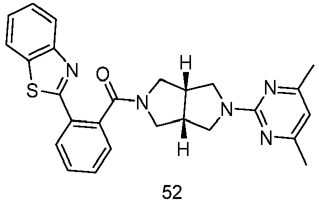

The preparation method of compound 52 was refered to the preparation method of Example 5.
MS m/z (ESI): 456.2 [M+1]
¹H NMR (400 MHz, DMSO-d6) δ 8.15 - 8.10 (m, 1H), 8.01 - 7.90 (m, 2H), 7.61 (ddt, J = 10.0, 7.4, 3.6 Hz, 2H), 7.46 (ddd, J = 12.7, 6.8, 3.0 Hz, 3H), 6.38 (s, 1H), 3.77 (dt, J = 10.7, 5.1 Hz, 2H), 3.62 (dd, J = 11.5, 7.3 Hz, 2H), 3.49 (d, J = 5.0 Hz, 3H), 3.13 (d, J = 5.0 Hz, 1H), 3.08 - 2.93 (m, 2H), 2.21 (s, 6H).

### Example 53

### Preparation of compound 53: (2-(benzo[d]thiazol-2-yl)-6-fluorophenyl)((3aR,6aS)-5-(4,6-dimethylpyrimidin-2-yl)hex ahydropyrrolo[3,4-c]pyrrol-2(1H)-yl)methanone

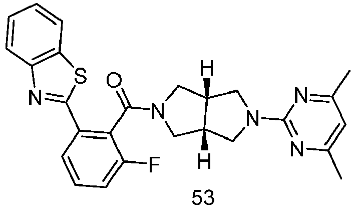

The preparation method of compound 53 was refered to the preparation method of Example 5.
MS m/z (ESI): 474.2 [M+1]
¹H NMR (400 MHz, DMSO-d6) δ 8.21 - 8.10 (m, 1H), 8.02 (dd, J = 39.4, 8.1 Hz, 1H), 7.83 (dd, J = 41.4, 7.7 Hz, 1H), 7.71 - 7.55 (m, 2H), 7.54 - 7.35 (m, 2H), 6.42 (d, J = 9.3 Hz, 1H), 3.87 - 3.51 (m, 7H), 3.17 - 2.94 (m, 3H), 2.23 (d, J = 9.9 Hz, 6H).

### Example 54

### Preparation of compound 54: (2-(benzo[d]oxazol-2-yl)-6-fluorophenyl)((3aR,6aS)-5-(4-(2-hydroxypropan-2-yl)-6-me thylpyrimidin-2-yl)hexahydropyrrolo[3,4-c]pyrrol-2(1H)-yl)methanone

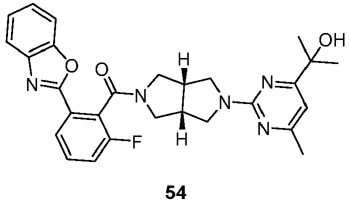

The synthetic method of compound 54 was refered to the synthetic method of Example 5.
MS m/z (ESI): 502.0 [M+1]
¹H NMR (400 MHz, DMSO-d6) δ 8.07 (dd, J = 28.5, 7.8 Hz, 1H), 7.84 (t, J = 6.8 Hz, 1H), 7.75 - 7.67 (m, 2H), 7.62 (t, J = 8.8 Hz, 1H), 7.52 - 7.41 (m, 1H), 7.39 - 7.33 (m, 1H), 6.74 (d, J = 14.5 Hz, 1H), 5.09 (s, 1H), 3.99 - 3.90 (m, 1H), 3.80 (dd, J = 12.4, 6.4 Hz, 1H), 3.69 (d, J = 12.2 Hz, 2H), 3.64 - 3.51 (m, 3H), 3.15 - 3.07 (m, 2H), 2.97 (s, 1H), 2.29 (s, 3H), 1.37 (s, 6H).

### Example 55

### Compound 55: (2-(benzo[d]thiazol-2-yl)-6-fluorophenyl)((3 aR,6aS)-5-(4-(2-hydroxypropan-2-yl)-6-me thylpyrimidin-2-yl)hexahydropyrrolo[3,4-c]pyrrol-2(1H)-yl)methanone

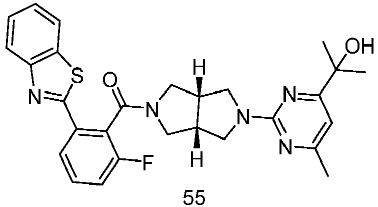

The synthetic method of compound 55 was refered to the synthetic method of Example 5.
MS: m/z (ESI): 518.2 [M+1]
¹H NMR (400 MHz, DMSO-d6) δ 8.20 - 8.04 (m, 1H), 7.89 (t, J = 8.3 Hz, 1H), 7.78 (d, J = 7.8 Hz, 1H), 7.66 (q, J = 7.4, 7.0 Hz, 1H), 7.60 - 7.49 (m, 2H), 7.46 - 7.37 (m, 1H), 6.74 (d, J = 6.9 Hz, 1H), 3.92 - 3.56 (m, 6H), 3.17 - 2.95 (m, 4H), 2.32 - 2.20 (m, 3H), 1.34 (d, J = 18.5 Hz, 6H).

### Example 56

### Preparation of compound 56: (2-fluoro-6-(pyrimidin-2-yl)phenyl)((3R,6S)-5-(4-(2-hydroxypropan-2-yl)-6-methylpyri midin-2-yl)hexahydropyrrolo[3,4-c]pyrrol-2(1H)-yl)methanone

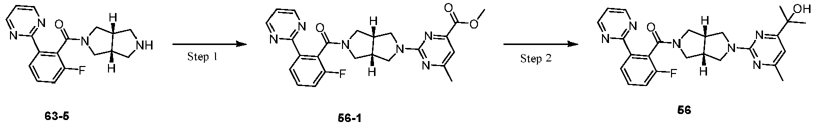

### Step 1

The synthetic method of compound 56-1 was refered to the synthetic method of compound 63, except that compound 63-5 and methyl 2-chloro-6-methylpyrimidine-4-carboxylate were used as starting materials to obtain methyl 2-((3*R*,6*S*)-5-(2-fluoro-6-(pyrimidin-2-yl)benzoyl)hexahydropyrrolo[3,4-c]pyrrol-2(1H) -yl)-6-methylpyrimidine-4-carboxylate (compound 56-1) (85 mg, 41.1%).
MS: m/z (ESI): 463.2 [M+1]

### Step 2

The synthetic method of compound 56 was refered to the synthetic method of compound 60-2, except that compound 56-1 was used as starting material to obtain (2-Fluoro-6-(pyrimidin-2-yl)phenyl)((3*R*,6*S*)-5-(4-(2-hydroxypropan-2-yl)-6-methylpyri midin-2-yl)hexahydropyrrolo[3,4-c]pyrrol-2(1H)-yl)methanone (compound 56) (18 mg, 41.1%).
MS: m/z (ESI): 463.2 [M+1]
¹H NMR (400 MHz, DMSO) δ 8.92 (d, J = 4.9 Hz, 1H), 8.79 (d, J = 4.8 Hz, 1H), 8.14 - 7.89 (m, 1H), 7.60 (dd, J = 13.9, 7.9 Hz, 1H), 7.56 - 7.27 (m, 2H), 6.76 (s, 1H), 3.86 - 3.63 (m, 2H), 3.61 - 3.44 (m, 4H), 3.21 (dd, J = 10.6, 4.3 Hz, 2H), 3.12 - 2.92 (m, 2H), 2.28 (d, J = 15.8 Hz, 3H), 1.37 (s, 6H).

### Example 57

### Preparation of compound 57: (2-fluoro-6-(2H-1,2,3-triazol-2-yl)phenyl)((3aR,6aS)-5-(4-(2-fluoropropan-2-yl)-6-meth ylpyrimidin-2-yl)hexahydropyrrolo[3,4-c]pyrrol-2(1H)-yl)methanone

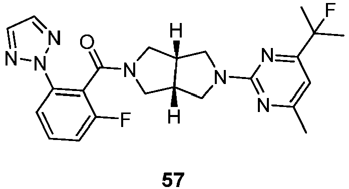

The synthetic method of compound 57 was refered to the synthetic method of Example 1, except that 2-chloro-4,6-dimethylpyrimidine was replaced with intermediate 8 to obtain Compound 57 (27 mg, white solid, yield 23.6%).
MS m/z (ESI): 454.1 [M+1]
¹H NMR (400 MHz, DMSO-d6) δ 8.08 (s, 1H), 7.87 (s, 1H), 7.73 (dd, J = 15.3, 8.2 Hz, 1H), 7.59 (td, J = 8.3, 6.1 Hz, 1H), 7.41 - 7.32 (m, 1H), 6.56 (t, J = 2.6 Hz, 1H), 3.72 - 3.61 (m, 2H), 3.56 - 3.36 (m, 5H), 3.05 - 2.84 (m, 3H), 2.24 (d, J = 3.9 Hz, 3H), 1.51 (d, J = 22.1 Hz, 6H).

### Example 58

### Preparation of compound 58: 2-((3aR,6aS)-5-(2-fluoro-6-(2H-1,2,3-triazol-2-yl)benzoyl)hexahydropyrrolo[3,4-c]pyrr ol-2(1H)-yl)-3-methylisonicotinonitrile

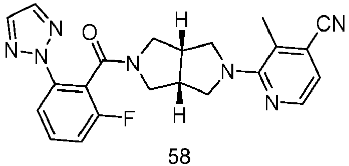

The synthetic method of compound 58 was refered to the synthetic method of Example 3.
MS: m/z (ESI): 418.2 [M+1]
¹H NMR (400 MHz, DMSO-d6) δ 8.17 (d, J = 7.5 Hz, 2H), 8.07 (s, 1H), 7.81 (t, J = 8.9 Hz, 1H), 7.67 (td, J = 8.2, 6.0 Hz, 1H), 7.45 (q, J = 8.1 Hz, 1H), 7.07 (dd, J = 15.1, 5.0 Hz, 1H), 3.69 (ddt, J = 29.2, 10.9, 6.9 Hz, 4H), 3.52 (q, J = 7.9, 7.3 Hz, 2H), 3.04 (ddt, J = 23.7, 12.1, 5.7 Hz, 4H), 2.47 (d, J = 5.4 Hz, 3H).

### Example 59

### Preparation of compound 59: 2-((3aR,6aS)-5-(2-fluoro-6-(2H-1,2,3-triazol-2-yl)benzoyl)hexahydropyrrolo[3,4-c]pyrr ol-2(1H)-yl)isonicotinonitrile

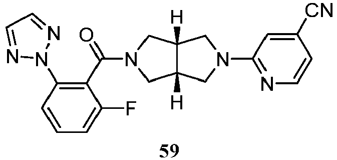

The synthetic method of Example 59 was refered to the synthetic method of Example 3, except that intermediate 1 was replaced with 2-chloroisonicotinonitrile to obtain Example 59 (31 mg, white solid, yield 23.8%).
MS m/z (ESI): 404.1 [M+1]
¹H NMR (400 MHz, DMSO-d6) δ 8.27 (d, J = 5.3 Hz, 1H), 8.16 (s, 1H), 8.01 (s, 1H), 7.80 (dd, J = 18.0, 8.2 Hz, 1H), 7.66 (tdd, J = 8.2, 6.1, 1.6 Hz, 1H), 7.44 (dt, J = 11.2, 8.7 Hz, 1H), 6.97 - 6.80 (m, 2H), 3.71 (dt, J = 12.9, 5.8 Hz, 2H), 3.60 - 3.49 (m, 2H), 3.43 - 3.31 (m, 3H), 3.17 - 2.96 (m, 3H).

### Example 60

### Preparation of compound 60: (2-fluoro-6-(2H-1,2,3-triazol-2-yl)phenyl)((3R,6S)-5-(6-(2-hydroxypropan-2-yl)-4-meth ylpyridin-2-yl)hexahydropyrrolo[3,4-c]pyrrol-2(1H)-yl)methanone

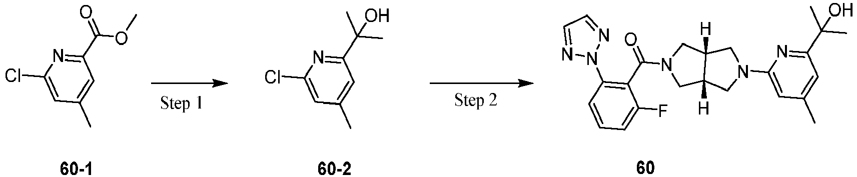

### Step 1

Compound 60-1 (100.00 mg, 539.08 µmol) was dissolved in tetrahydrofuran (5.0 mL), followed by addition of methylmagnesium bromide (3 M, 538.77 µL). The reaction solution was stirred at 0°C for 0.5 hour. Saturated ammonium chloride solution (5 ml) was added to the reaction solution, and the solution was extracted with dichloromethane (3* 10 mL). The organic phases were combined, dried, and concentrated to obtain a crude product. The crude product was purified by column chromatography (petroleum ether/ethyl acetate system) to obtain the product 2-(6-chloro-4-methylpyridin-2-yl)propan-2-ol (compound 60-2) (90 mg, 90.0%).
MS m/z (ESI): 186.1 [M+1]

### Step 2

Compound 60-2 (65.95 mg, 0.36 mmol), compound 3c (100 mg, 0.33 mmol), 4,5-bisdiphenylphosphine-9,9-dimethylxanthene (34.22 mg, 59.20 µmol) and palladium acetate (6.63 mg, 29.61 µmol) were dissolved in 1,4-dioxane (2 mL), followed by addition of potassium tert-butoxide (33.16 mg, 296.07 µmol). The reaction solution was stirred in microwave at 100°C for 1 hour. Saturated brine (10 ml) was added to the reaction solution, and the solution was extracted with dichloromethane (3* 10 mL). The organic phases were combined, dried, and concentrated to obtain a crude product. The crude product was purified to obtain (2-fluoro-6-(2H-1,2,3-triazol-2-yl)phenyl)((*3R,6S*)-5-(6-(2-hydroxypropan-2-yl)-4-meth ylpyridin-2-yl)hexahydropyrrolo[3,4-c]pyrrol-2(1H)-yl)methanone (compound 60) (8 mg, yield 6.00%).
MS m/z (ESI): 451.1 [M+1]
¹H NMR (400 MHz, DMSO) δ 7.95 (s, 1H), 7.80 (dd, J = 18.7, 8.2 Hz, 1H), 7.73 - 7.55 (m, 1H), 7.40 (m, 1H), 7.02 (dd, J = 58.3, 50.0 Hz, 2H), 6.66 (d, J = 6.9 Hz, 1H), 6.11 (s, 1H), 3.87 - 3.48 (m, 7H), 3.27 - 2.90 (m, 3H), 2.21 (d, J = 4.9 Hz, 3H), 1.38 (d, J = 6.3 Hz, 6H).

### Example 61

### Preparation of compound 61: ((3R,6S)-5-(5-chloro-6-(2-hydroxypropan-2-yl)pyridin-2-yl)hexahydropyrrolo[3,4-c]pyr rol-2(1H)-yl)(2-fluoro-6-(2H-1,2,3-triazol-2-yl)phenyl)methanone

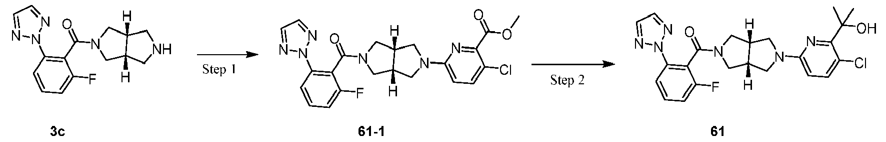

### Step 1

The synthetic method of compound 61-1 was refered to the synthetic method of compound 60, except that compound 3c was used as starting material to obtain methyl 3-chloro-6-((*3R,6S*)-5-(2-fluoro-6-(2H-1,2,3-triazol-2-yl)benzoyl)hexahydropyrrolo[3,4 -c]pyrrol-2(1H)-yl)picolinate (compound 61-1) (90 mg, 88.7%).
MS: m/z (ESI): 471.2 [M+1]

### Step 2

The synthetic method of compound 61 was refered to the synthetic method of intermediate 1, except that compound 61-1 was used as starting material to obtain ((*3R,6S*)-5-(5-Chloro-6-(2-hydroxypropan-2-yl)pyridin-2-yl)hexahydropyrrolo[3,4-c]py rrol-2(1H)-yl)(2-fluoro-6-(2H-1,2,3-triazol-2-yl)phenyl)methanone (compound 61) (38 mg, 38.6%).
MS: m/z (ESI): 471.2 [M+1]
¹H NMR (400 MHz, DMSO) δ 8.16 (s, 1H), 7.98 (s, 1H), 7.80 (dd, J = 20.4, 8.2 Hz, 1H), 7.72 - 7.52 (m, 2H), 7.44 (dt, J = 12.6, 8.7 Hz, 1H), 6.44 (dd, J = 8.7, 4.4 Hz, 1H), 5.72 (d, J = 23.8 Hz, 1H), 3.80 - 3.65 (m, 2H), 3.64 - 3.42 (m, 6H), 3.19 - 2.98 (m, 2H), 1.53 (d, J = 4.2 Hz, 6H).

### Example 62

### Preparation of compound 62: ((3 aR, 6aS)-5-(5-trifluoromethyl-4-(2-hydroxypropan-2-yl)-6-methylpyrimidin-2-yl)hex ahydropyrrolo[3,4-c]pyrrol-2(1H)-yl)(2-fluoro-6-(2H-1,2,3-triazol-2-yl)phenyl)methano ne

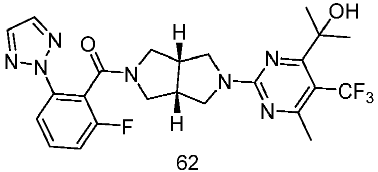

The preparation method of compound 62 was refered to the preparation method of Example 3.
MS m/z (ESI): 520.2 [M+1]

### Example 63

### Preparation of compound 63: (2-fluoro-6-(pyrimidin-2-yl)phenyl)((3R,6S)-5-(6-(2-hydroxypropan-2-yl)-5-methylpyri din-2-yl)hexahydropyrrolo[3,4-c]pyrrol-2(1H)-yl)methanone

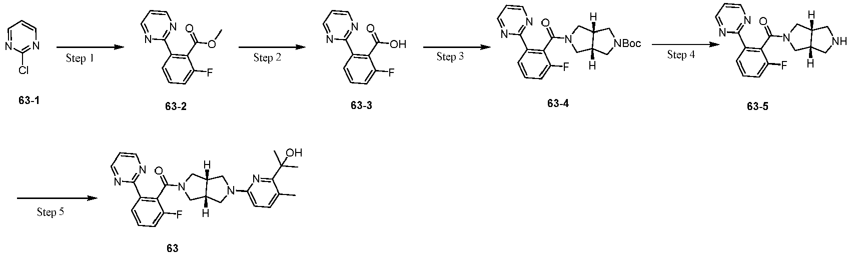

### Step 1

Compound 63-1 (0.2 g, 1.75 mmol), methyl 2-fluoro-6-(4,4,5,5-tetramethyl-1,3,2-dioxaborolan-2-yl)benzoate (586.94 mg, 2.10 mmol) and potassium phosphate (1.11 g, 5.24 mmol) were dissolved in 1,4-dioxane (4.0 mL) and H₂O (1.0 mL), followed by addition of [1,1'-bis(diphenylphosphino)ferrocene]dichloropalladium dichloromethane complex (142.49 mg, 174.62 µmol). The reaction solution was stirred at 100°C for 16 hours. Saturated brine (10 ml) was added to the reaction solution, and the solution was extracted with dichloromethane (3* 20 mL). The organic phases were combined, dried, and concentrated to obtain a crude product. The crude product was purified by column chromatography (petroleum ether/ethyl acetate system) to obtain methyl 2-fluoro-6-(pyrimidin-2-yl)benzoate (compound 63-2) (0.2 g, 49.1%).
MS m/z (ESI): 233.1 [M+1]

### Step 2

Compound 63-2 (0.8 g, 3.45 mmol) was dissolved in water (2 mL) and methanol (2 mL), followed by addition of sodium hydroxide (413.39 mg, 10.33 mmol). The reaction solution was stirred at 70°C for 16 hours. 2 mol/L hydrochloric acid solution (10 ml) was added to the reaction solution to adjust the pH to acidic, and the solution was extracted with ethyl acetate (3*20 mL). The organic phases were combined, dried, and concentrated to obtain the crude product 2-fluoro-6-(pyrimidin-2-yl)benzoic acid (compound 63-3) (260 mg, 34.6%). The crude product was directly used in the next step without purification.
MS m/z (ESI): 219.1 [M+1]

### Step 3

Compound 63-3 (0.2 g, 917.43 µmol) and N,N-dimethylformamide (0.1 ml) were dissolved in dichloromethane (2 mL), followed by addition of oxalyl chloride (232.70 mg, 1.83 mmol). The reaction solution was stirred at room temperature for 0.5 hour, and concentrated to obtain the crude product. The crude product tert-butyl (3R,6S)-2,3,3a,4,6,6a-hexahydro-1H-pyrrolo[3,4-c]pyrrole-5-carboxylate (192.88 mg, 909.81 µmol) was dissolved in dichloromethane (2 mL), followed by addition of N-ethyl-N-isopropylpropan-2-amine (352.29 mg, 2.73 mmol). The reaction solution was stirred at room temperature for 1 hour. Saturated brine (10 ml) was added to the reaction solution, and the solution was extracted with dichloromethane (3* 10 mL). The organic phases were combined, dried, and concentrated to obtain a crude product. The crude product was purified by column chromatography (petroleum ether/ethyl acetate system) to obtain *tert*-butyl (*3R,6S*)-5-(2-fluoro-6-(pyrimidin-2-yl)benzoyl)hexahydropyrrolo[3,4-c]pyrrole-2(1H)-c arboxylate (compound 63-4) (0.2 g, 53.4%).
MS m/z (ESI): 413.2 [M+1]

### Step 4

Compound 63-4 (200.00 mg, 485.43 µmol) was dissolved in hydrochloric acid/1,4-dioxane (4 M, 2 mL), and then the reaction solution was stirred at room temperature for 1 hour. The reaction solution was filtered, the filter cake was collected and dried to obtain (2-fluoro-6-(pyrimidin-2-yl)phenyl)((*3R,6S*)-hexahydropyrrolo[3,4-c]pyrrol-2(1H)-yl)m ethanone (compound 63-5) (120 mg, 79.2%).
MS m/z (ESI): 313.2 [M+1]

### Step 5

The synthetic method of compound 63 was refered to the synthetic method of compound 60, except that compound 63-5 and compound 2-(6-chloro-3-methylpyridin-2-yl)-propan-2-ol were used as starting materials to obtain (2-Fluoro-6-(pyrimidin-2-yl)phenyl)((*3R,6S*)-5-(6-(2-hydroxypropan-2-yl)-5-methylpyri din-2-yl)hexahydropyrrolo[3,4-c]pyrrol-2(1H)-yl)methanone (compound 63) (1.8 mg, 2.6%).
MS: m/z (ESI): 462.2 [M+1]

### Example 64

### Preparation of compound 64: ((3aR,6aS)-5-(5-fluoro-4-(2-hydroxypropan-2-yl)-6-methylpyrimidin-2-yl)hexahydropy rrolo[3,4-c]pyrrol-2(1H)-yl)(2-fluoro-6-(2H-1,2,3-triazol-2-yl)phenyl)methanone

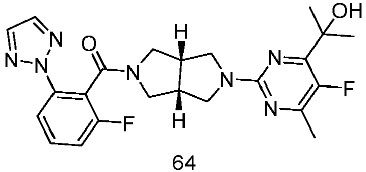

The synthetic method of compound 64 was refered to the synthetic method of Example 3.
MS m/z (ESI): 470.2 [M+1]

### Example 65

### Preparation of compound 65: ((3 aR, 6aS)-5-(5-chloro-4-(2-hydroxypropan-2-yl)-6-methylpyrimidin-2-yl)hexahydropy rrolo[3,4-c]pyrrol-2(1H)-yl)(2-fluoro-6-(2H-1,2,3-triazol-2-yl)phenyl)methanone

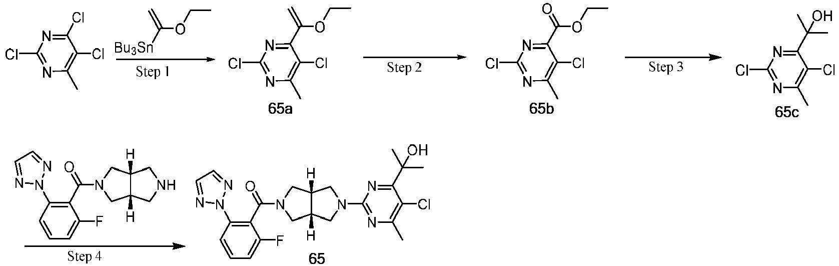

### Step 1

2,4,5-Trichloro-6-methylpyrimidine (900 mg, 4.56 mmol) was dissolved in anhydrous N,N-dimethylformamide (20 mL), followed by addition of tributyl(1-ethoxyvinyl)stannane (1.81 g, 5.01 mmol) and bistriphenylphosphine palladium dichloride (160 mg, 0.228 mmol). The mixture was purged with nitrogen three times, heated to 100°C and reacted for 4 hours. The reaction solution was cooled to room temperature and poured into 100 mL of water. The mixture was precipitated with potassium fluoride, filtered and washed with ethyl acetate (80 mL). The mixture was partitioned, and the aqueous phase was extracted with ethyl acetate (50 mL). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain 2,5-dichloro-4-(1-ethoxyvinyl)-6-methylpyrimidine (compound 65a) (540 mg, colorless liquid, 50.9%).
¹H NMR (400 MHz, CDCl₃) δ 4.76 (d, *J* = 3.2 Hz, 1H), 4.61 (d, *J* = 3.2 Hz, 1H), 3.95 (q, *J* = 7.0 Hz, 2H), 2.64 (s, 3H), 1.40 (t, *J*= 7.0 Hz, 3H).
MS:m/z (ESI): 234.0 [M+1]

### Step 2

Compound 65a (600 mg, 2.58 mmol) was dissolved in 1,4-dioxane (10 mL) and water (2 mL), and cooled to 0°C. Sodium periodate (1.10 g, 5.14 mmol) and potassium permanganate (81 mg, 0.513 mmol) were added, and reacted at room temperature for 1 hour. The reaction solution was filtered, and the filter cake was washed with 50 mL of ethyl acetate. The organic phase was washed with water (30 mL) and saturated sodium chloride solution (30 mL), dried over anhydrous sodium sulfate and filtered. The filtrate was concentrated under reduced pressure, and the residues were purified by silica gel column chromatography (petroleum ether/ethyl acetate system) to obtain ethyl 2,5-dichloro-6-methylpyrimidine-4-carboxylate (compound 65b) (490 mg, colorless liquid, 81.3%), which was used directly in the next step.
MS: m/z (ESI): 236.0 [M+1]

### Step 3

Compound 65b (490 mg, 2.09 mmol) was dissolved in anhydrous tetrahydrofuran (10 mL). The solution was cooled to -78°C under nitrogen protection, followed by addition of 1 M solution of methylmagnesium bromide in tetrahydrofuran (6.3 mL, 6.3 mmol). The mixture was reacted at -78°C for 1 hour, and then slowly warmed to room temperature for 1 hour. The reaction solution was poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain 2-(2,5-dichloro-6-methylpyrimidin-4-yl)propan-2-ol (compound 65c) (370 mg, yellow oil, 80.3%).
MS: m/z (ESI): 222.0 [M+1]

### Step 4

The preparation method of compound 65 was refered to the preparation method of compound 1, except that 1c (80 mg, 0.266 mmol) and 77c (70 mg, 0.317 mmol) were used as starting materials to obtain Compound 65 (21 mg, white solid, 16.3%).
MS: m/z (ESI): 486.7 [M+1]

### Example 66

### Preparation of compound 66: ((3aR,6aS)-5-(4,6-dimethylpyrimidin-2-yl)hexahydropyrrolo[3,4-c]pyrrol-2(1H)-yl)(2-f luoro-6-(perfluoroethoxy)phenyl)methanone

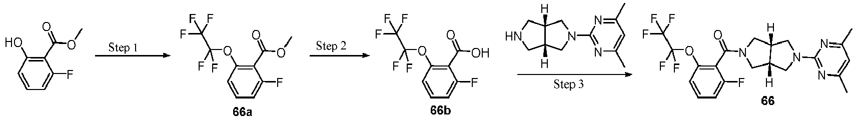

### Step 1

A three-necked flask was baked three times under nitrogen protection. Under nitrogen protection, silver trifluoromethanesulfonate (1.21 g, 4.70 mmol), tetramethylammonium fluoride (438 mg, 4.70 mmol), selective fluorine reagent (833 mg, 2.35 mmol) and methyl 2-fluoro-6-hydroxybenzoate (200 mg, 1.18 mmol) were added, followed by addition of toluene (10 mL), trimethyl(perfluoroethyl)silane (904 mg, 4.70 mmol) and 2-fluoropyridine (457 mg, 4.70 mmol). The mixture was reacted at room temperature for 16 hours. The reaction solution was filtered, and the filtrate was concentrated under reduced pressure. The resulting residues were purified by silica gel column chromatography (petroleum ether/ethyl acetate system) to obtain methyl 2-fluoro-6-(perfluoroethoxy)benzoate (compound 66a) (60 mg, colorless liquid, 17.7%), which was used directly in the next step.

### Step 2

The preparation method of compound 66b was refered to the preparation method of compound 48c of Example 48, except that 66a (60 mg, 0.208 mmol) was used as starting material to obtain 2-fluoro-6-(perfluoroethoxy)benzoic acid (compound 66b) (55 mg, white solid, 96.8%).
MS: m/z (ESI): 273.0 [M-1]

### Step 3

The preparation method of compound 66 was refered to the preparation method of compound 48 of Example 48, except that 5c (50 mg, 0.229 mmol) and 66b (55 mg, 0.201 mmol) were used as starting materials to obtain Compound 66b (31 mg, white solid, 32.5%).
MS: m/z (ESI): 475.2 [M+1]

### Example 67

### Preparation of compound 67: (2-fluoro-6-(2H-1,2,3-triazol-2-yl)phenyl)((3aR,6aS)-5-(4-(2-aminopropan-2-yl)-6-met hylpyrimidin-2-yl)hexahydropyrrolo[3,4-c]pyrrol-2(1H)-yl)methanone

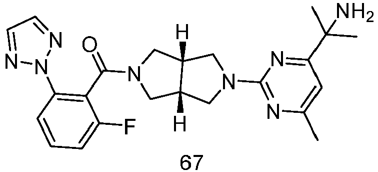

The preparation method of compound 67 was refered to the preparation method of Example 3.
MS m/z (ESI): 451.2 [M+1]

### Example 68

### Preparation of compound 68: ((3R,6S)-5-(4,6-dimethylpyrimidin-2-yl)hexahydropyrrolo[3,4-c]pyrrol-2(1H)-yl)(2-hyd roxy-6-(1,1,2,2-tetrafluoroethoxy)phenyl)methanone

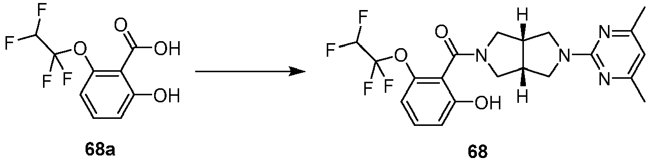

Compound 68a (50.0 mg, 0.20 mmol), (*3R,6S*)-2-(4,6-dimethylpyrimidin-2-yl) octahydropyrrolo[3,4-c]pyrrole (46.00 mg, 0.21 mmol) and N,N,N',N'-tetramethylchloroformamidine hexafluorophosphate (115 mg, 0.41 mmol) were dissolved in acetonitrile (2.0 mL), followed by addition of N-methylimidazole (35 mg, 0.43 mmol). The reaction solution was stirred at 50°C for 1 hour. Saturated sodium chloride (5 ml) was added to the reaction solution, and the solution was extracted with ethyl acetate (3*10 mL). The organic phases were combined, dried, and concentrated to obtain a crude product. The crude product was purified by column chromatography (petroleum ether/ethyl acetate system) to obtain the target product ((*3R,6S*)-5-(4,6-dimethylpyrimidin-2-yl)hexahydropyrrolo[3,4-c]pyrrol-2(1H)-yl)(2-hyd roxy-6-(1,1,2,2-tetrafluoroethoxy)phenyl)methanone (compound 68) (32 mg, 35.8%).
MS m/z (ESI): 455.2 [M+1]

### Example 69

### Preparation of compound 69: (2-fluoro-6-(pyrimidin-2-yl)phenyl)((3R,6S)-5-(4-(2-fluoropropan-2-yl)-6-methylpyrimi din-2-yl)hexahydropyrrolo[3,4-c]pyrrol-2(1H)-yl)methanone

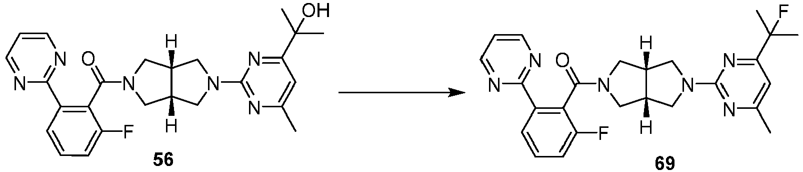

The synthetic method of compound 69 was refered to the synthetic method of intermediate 8, except that compound 56 was used as starting material to obtain (2-fluoro-6-(pyrimidin-2-yl)phenyl)((*3R,6S*)-5-(4-(2-fluoropropan-2-yl)-6-methylpyrimi din-2-yl)hexahydropyrrolo[3,4-c]pyrrol-2(1H)-yl)methanone (compound 69) (41 mg, 38.6%).
MS: m/z (ESI): 465.2 [M+1]

### Example 70

### Preparation of compound 70: (2-fluoro-6-(2H-1,2,3-triazol-2-yl)phenyl)((3R,6S)-5-(6-(2-fluoropropan-2-yl)-4-methyl pyridin-2-yl)hexahydropyrrolo[3,4-c]pyrrol-2(1H)-yl)methanone

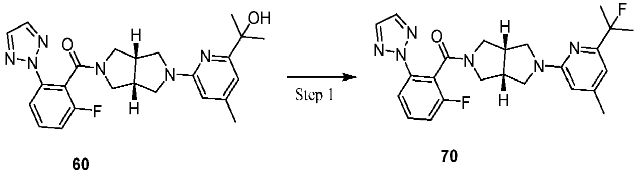

### Step 1

The synthetic method of compound 70 was refered to the synthetic method of intermediate 8, except that compound 60 was used as starting material to obtain (2-fluoro-6-(2H-1,2,3-triazol-2-yl)phenyl)((*3R,6S*)-5-(6-(2-fluoropropan-2-yl)-4-methyl pyridin-2-yl)hexahydropyrrolo[3,4-c]pyrrol-2(1H)-yl)methanone (compound 70) (33 mg, 38.6%).
MS: m/z (ESI): 453.2 [M+1]

### Example 71

### Preparation of compound 71: (2-fluoro-6-(pyrimidin-2-yl)phenyl)((3aR,6aS)-5-(6-(2-hydroxypropan-2-yl)-4-methylp yridin-2-yl)hexahydropyrrolo[3,4-c]pyrrol-2(1H)-yl)methanone

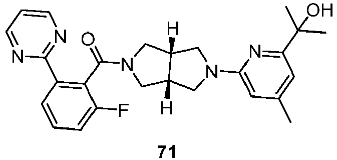

The preparation method of compound 71 was refered to the preparation method of Example 60.
MS m/z (ESI): 462.2 [M+1]

### Example 72

### Preparation of compound 72: (2-fluoro-6-(quinazolin-2-yl)phenyl)((3aR,6aS)-5-(4-(2-fluoropropan-2-yl)-6-methylpyr imidin-2-yl)hexahydropyrrolo[3,4-c]pyrrol-2(1H)-yl)methanone

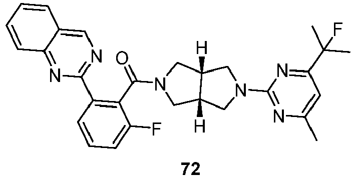

The synthetic method of compound 72 was refered to the synthetic method of Example 57.
MS: m/z (ESI): 515.2 [M+1]

### Example 73

### Preparation of compound 73: (2-fluoro-6-(2H-1,2,3-triazol-2-yl)phenyl)((3aR,6aS)-5-(4-(2-fluoropropan-2-yl)pyrimid in-2-yl)hexahydropyrrolo[3,4-c]pyrrol-2(1H)-yl)methanone

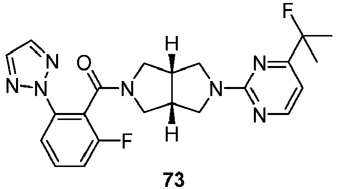

The synthetic method of compound 73 was refered to the synthetic method of Example 57.
MS: m/z (ESI): 440.2 [M+1]

### Example 74

### Preparation of compound 74: ((3aR,6aS)-5-(4,6-dimethylpyrimidin-2-yl)hexahydropyrrolo[3,4-c]pyrrol-2(1H)-yl)(2-f luoro-6-(quinazolin-2-yl)phenyl)methanone

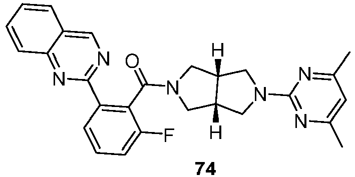

The synthetic method of compound 74 was refered to the synthetic method of Example 51.
MS m/z (ESI): 469.2 [M+1]

### Example 75

### Preparation of compound 75: ((3aR,6aS)-5-(4-(2-chloropropan-2-yl)-6-methylpyrimidin-2-yl)hexahydropyrrolo[3,4-c ]pyrrol-2(1H)-yl)(2-fluoro-6-(2H-1,2,3-triazol-2-yl)phenyl)methanone

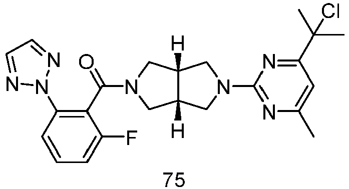

The synthetic method of compound 75 was refered to the synthetic method of Example 3.
MS m/z (ESI): 470.0 [M+1]

### Example 76

### Preparation of compound 76:

((3aR,6aS)-5-(4-(1,1-difluoroethyl)-6-methylpyrimidin-2-yl)hexahydropyrrolo[3,4-c]py rrol-2(1H)-yl)(2-fluoro-6-(2H-1,2,3-triazol-2-yl)phenyl)methanone

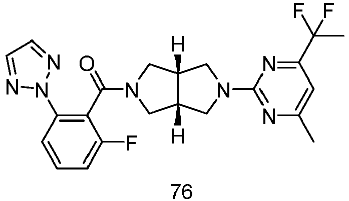

The synthetic method of compound 76 was refered to the synthetic method of Example 3.
MS m/z (ESI): 458.1 [M+1]

### Example 77

### Preparation of compound 77: ((3aR,6aS)-5-(4-(difluoromethyl)-6-methylpyrimidin-2-yl)hexahydropyrrolo[3,4-c]pyrr ol-2(1H)-yl)(2-fluoro-6-(2H-1,2,3-triazol-2-yl)phenyl)methanone

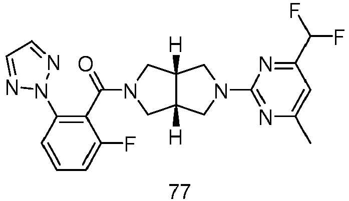

The synthetic method of compound 77 was refered to the synthetic method of Example 3.
MS m/z (ESI): 444.1 [M+1]

### Example 78

### Preparation of compound 78: (2-fluoro-6-(2H-1,2,3-triazol-2-yl)phenyl)((3aR,6aS)-5-(7-fluoro-7-methyl-6,7-dihydro-5H-cyclopenta[d]pyrimidin-2-yl)hexahydropyrrolo[3,4-c]pyrrol-2(1H)-yl)methanone

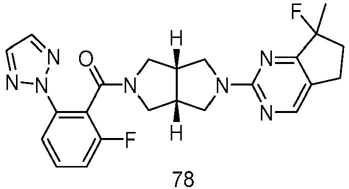

The synthetic method of compound 78 was refered to the synthetic method of Example 3.
MS m/z (ESI): 452.1 [M+1]

### Example 79

### Preparation of compound 79: (2-fluoro-6-(2H-1,2,3-triazol-2-yl)phenyl)((3aR,6aS)-5-(4-(1-fluorocyclopropyl)-6-met hylpyrimidin-2-yl)hexahydropyrrolo[3,4-c]pyrrol-2(1H)-yl)methanone

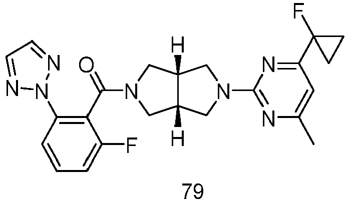

The synthetic method of compound 79 was refered to the synthetic method of Example 3.
MS m/z (ESI): 452.1 [M+1]

### Example 80

### Preparation of compound 80: (2-fluoro-6-(1,1,2,2-tetrafluoroethoxy)phenyl)((3aR,6aS)-5-(4-(2-fluoropropan-2-yl)-6-methylpyrimidin-2-yl)hexahydropyrrolo[3,4-c]pyrrol-2(1H)-yl)methanone

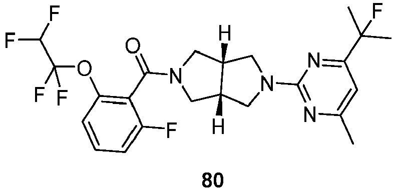

The preparation method of compound 80 was refered to the preparation method of Example 57.
MS m/z (ESI): 503.2 [M+1]

### Example 81

### Preparation of compound 81: (2-fluoro-6-(1,1,2,2-tetrafluoroethoxy)phenyl)((3aR,6aS)-5-(4-(2-hydroxypropan-2-yl)-6-methylpyrimidin-2-yl)hexahydropyrrolo[3,4-c]pyrrol-2(1H)-yl)methanone

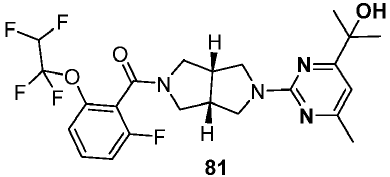

The preparation method of compound 81 was refered to the preparation method of Example 3.
MS m/z (ESI): 501.2 [M+1]

### Example 82

### Preparation of compound 82: (2-fluoro-6-(2H-1,2,3-triazol-2-yl)phenyl)((3aR,6aS)-5-(6-(2-hydroxypropan-2-yl)-4,5-dimethylpyridin-2-yl)hexahydropyrrolo[3,4-c]pyrrol-2(1H)-yl)methanone

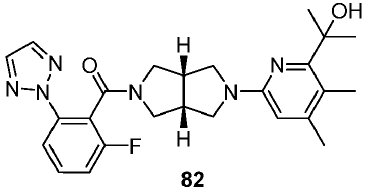

The preparation method of compound 82 was refered to the preparation method of Example 3.
MS m/z (ESI): 465.2 [M+1]

### Example 83

### Preparation of compound 83: (2-fluoro-6-(furan-2-yl)phenyl)((3 aR,6aS)-5-(4-(2-hydroxypropan-2-yl)-6-methylpyrimi din-2-yl)hexahydropyrrolo[3,4-c]pyrrol-2(1H)-yl)methanone

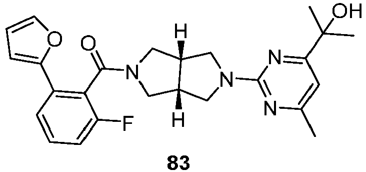

The synthetic method of compound 83 was refered to the synthetic method of Example 3.
MS m/z (ESI): 451.2 [M+1]

### Example 84

### Preparation of compound 84: (2-fluoro-6-(thiophen-2-yl)phenyl)((3aR,6aS)-5-(4-(2-hydroxypropan-2-yl)-6-methylpy rimidin-2-yl)hexahydropyrrolo[3,4-c]pyrrol-2(1H)-yl)methanone

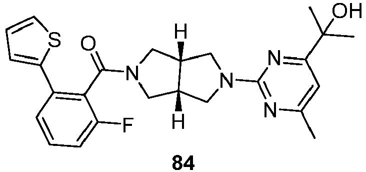

The synthetic method of compound 84 was refered to the synthetic method of Example 3.
MS m/z (ESI): 467.2 [M+1]

### Example 85

### Preparation of compound 85:

(2-hydroxy-6-(2H-1,2,3-triazol-2-yl)phenyl)((*3R,6S*)-5-(6-(2-hydroxypropan-2-yl)-4-me thylpyridin-2-yl)hexahydropyrrolo[3,4-c]pyrrol-2(1H)-yl)methanone

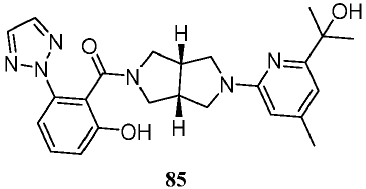

The synthetic method of compound 85 was refered to the synthetic method of Example 60.
MS m/z (ESI): 449.2 [M+1]

### Example 86

### Preparation of compound 86: ((3aR,6aS)-5-(4,6-dimethylpyrimidin-2-yl)-3,3a,4,6a-tetrahydrocyclopenta[c]pyrrol-2(1 H)-yl)(2-fluoro-6-(2H-1,2,3-triazol-2-yl)phenyl)methanone

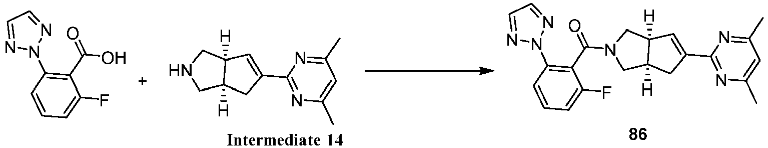

2-Fluoro-6-(triazol-2-yl)benzoic acid (60 mg, 0.29 mmol) and intermediate 14 (62.36 mg, 0.29 mmol) were added to MeCN (10 mL). Tetramethylchlorourea hexafluorophosphate (162.19 mg, 578.05 µmol) and 1-methylimidazole (47.56 mg, 0.58 mmol) were added at 25°C. The reaction solution was stirred at 25°C for 2 hours, followed by addition of water (20 ml). The solution was extracted with dichloromethane (30 mL*2), and the organic phases were combined, dried over Na₂SO₄, and concentrated under reduced pressure. The resulting crude product was purified by preparative HPLC to obtain compound 86 (50 mg, yield 42.7%).
MS m/z (ESI): 405.2 [M+1]
¹H NMR (400 MHz, DMSO-d6) δ 8.15 (s, 1H), 7.87 - 7.72 (m, 2H), 7.65 (dq, J = 8.3, 6.0 Hz, 1H), 7.47 - 7.34 (m, 1H), 7.09 (d, J = 9.2 Hz, 1H), 6.80 - 6.53 (m, 1H), 3.82 - 3.61 (m, 2H), 3.53 (s, 1H), 3.31 - 3.20 (m, 2H), 3.10 - 2.91 (m, 2H), 2.87 - 2.69 (m, 1H), 2.39 (d, J = 3.2 Hz, 6H).

### Example 87

### Preparation of compound 87: (2-fluoro-6-(pyrimidin-2-yl)phenyl)((3aR,6aS)-5-(6-(2-hydroxypropan-2-yl)-4-methylp yridin-2-yl)hexahydropyrrolo[3,4-c]pyrrol-2(1H)-yl)methanone

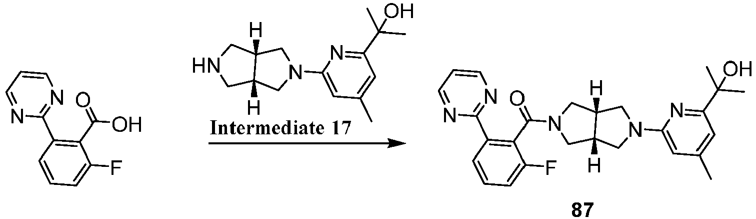

2-Fluoro-6-pyrimidin-2-ylbenzoic acid (50 mg, 0.23 mmol) and intermediate 17 (65.88 mg, 0.25 mmol) were added to MeCN (5 mL). Tetramethylchlorourea hexafluorophosphate (128.33 mg, 0.46 mmol) and 1-methylimidazole (56.45 mg, 0.69 mmol) were added at 25°C. The reaction solution was stirred at 25°C for 2 hours, followed by addition of water (20 ml). The solution was extracted with dichloromethane (30 mL*2), and the combined extracts were dried over Na₂SO₄, and concentrated under reduced pressure. The resulting crude product was purified by preparative HPLC to obtain compound 87 (64 mg, yield 60.5%).
MS m/z (ESI): 462.2 [M+1]
¹H NMR (400 MHz, DMSO-d6) δ 8.93 (d, J = 4.9 Hz, 1H), 8.82 (d, J = 4.9 Hz, 1H), 8.05 (dd, J = 25.0, 7.8 Hz, 1H), 7.62 (q, J = 7.7 Hz, 1H), 7.54 - 7.38 (m, 2H), 6.85 (d, J = 9.8 Hz, 1H), 6.68 (d, J = 22.0 Hz, 1H), 3.80 (ddd, J = 37.8, 12.6, 7.8 Hz, 4H), 3.61 - 3.51 (m, 3H), 3.18 (dt, J = 10.8, 5.1 Hz, 3H), 2.36 (d, J = 3.4 Hz, 3H), 1.50 (d, J = 5.2 Hz, 6H).

### Example 88

### Preparation of compound 88: (2-fluoro-6-(pyrimidin-2-yl)phenyl)((3aR,6aS)-5-(6-(2-fluoropropan-2-yl)-4-methylpyri din-2-yl)hexahydropyrrolo[3,4-c]pyrrol-2(1H)-yl)methanone

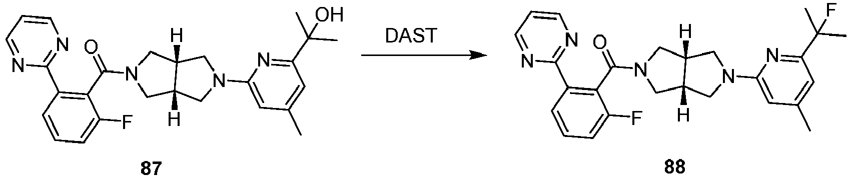

Compound 87 (45 mg, 0.1 mmol) and DAST (23.57 mg, 0.15 mmol) were added to DCM (5 mL) at 0°C. The reaction solution was stirred at 25°C for 0.5 hour, and water (20 ml) was added after completion of the reaction. The solution was extracted with dichloromethane (30 mL*2), and the organic phases were combined, dried over Na₂SO₄, and concentrated under reduced pressure. The resulting crude product was purified by preparative HPLC to obtain compound 88 (31 mg, yield 68.6%).
MS: m/z (ESI): 464.2 [M+1]
¹H NMR (400 MHz, DMSO-d6) δ 8.92 (d, J = 4.9 Hz, 1H), 8.76 (d, J = 4.9 Hz, 1H), 8.03 (dd, J = 31.4, 7.8 Hz, 1H), 7.67 - 7.33 (m, 3H), 6.56 (d, J = 3.8 Hz, 1H), 6.20 (d, J = 5.4 Hz, 1H), 3.85 - 3.65 (m, 2H), 3.59 - 3.47 (m, 3H), 3.27 - 2.90 (m, 5H), 2.23 (s, 3H), 1.60 (dd, J = 22.0, 3.0 Hz, 6H).

### Example 89

### Preparation of compound 89: ((3 aR, 6aS)-5-(5-fluoro-4-(2-fluoropropan-2-yl)-6-methylpyrimidin-2-yl)hexahydropyrr olo[3,4-c]pyrrol-2(1H)-yl)(2-fluoro-6-(2H-1,2,3-triazol-2-yl)phenyl)methanone

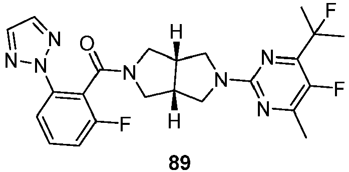

The synthetic method of compound 89 was refered to the synthetic method of Example 88, except that compound 87 was replaced with compound 64 as starting material to obtain Compound 89 (30 mg, yield 66%) .
MS: m/z (ESI): 472.2 [M+1]
¹H NMR (400 MHz, DMSO-d6) δ 8.16 (s, 1H), 7.96 (s, 1H), 7.80 (dd, J = 14.7, 8.2 Hz, 1H), 7.66 (td, J = 8.3, 6.0 Hz, 1H), 7.44 (q, J = 9.2 Hz, 1H), 3.76 - 3.64 (m, 2H), 3.53 (ddd, J = 23.2, 11.1, 7.2 Hz, 5H), 3.17 - 2.94 (m, 3H), 2.36 - 2.23 (m, 3H), 1.67 (dd, J = 21.9, 4.0 Hz, 6H).

### Example 90

### Preparation of compound 90: ((3aR,6aS)-5-(5-fluoro-6-(2-hydroxypropan-2-yl)-4-methylpyridin-2-yl)hexahydropyrro lo[3,4-c]pyrrol-2(1H)-yl)(2-fluoro-6-(2H-1,2,3-triazol-2-yl)phenyl)methanone

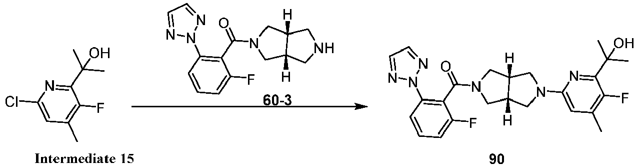

Compound 60-3 (100 mg, 0.33 mmol), intermediate 15 (75 mg, 0.37 mmol), 4,5-bisdiphenylphosphine-9,9-dimethylxanthene (34.22 mg, 59.14 µmol) and trisdibenzylideneacetone dipalladium (27 mg, 29.48 µmol) were dissolved in 1,4-dioxane (2 mL), followed by addition of cesium carbonate (283 mg, 868.58 µmol). The reaction solution was stirred in microwave at 100°C for 1 hour. After completion of the reaction, saturated brine (10 ml) was added, and the solution was extracted with dichloromethane (10 mL*3). The organic phases were combined, dried, and concentrated to obtain a crude product. The crude product was purified by preparative HPLC to obtain compound 90 (52 mg, yield 33.4%).
MS m/z (ESI): 469.2 [M+1]
¹H NMR (400 MHz, DMSO) δ 8.07 (d, J = 71.2 Hz, 2H), 7.92 - 7.75 (m, 1H), 7.66 (dt, J = 14.4, 7.2 Hz, 1H), 7.55 - 7.28 (m, 1H), 6.31 (d, J = 3.0 Hz, 1H), 5.40 (d, J = 20.6 Hz, 1H), 3.78 - 3.39 (m, 7H), 3.28 - 2.91 (m, 3H), 2.22 (s, 3H), 1.44 (d, J = 3.3 Hz, 6H).

### Example 91

### Preparation of compound 91: ((3 aR, 6aS)-5-(5-fluoro-6-(2-hydroxypropanyl)pyridin-2-yl)hexahydropyrrolo[3,4-c]pyrr ol-2(1H)-yl)(2-fluoro-6-(2H-1,2,3-triazol-2-yl)phenyl)methanone

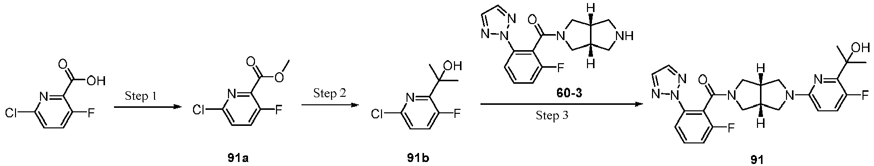

### Step 1

2-Chloro-5-fluoropyridine-6-carboxylic acid (500 mg, 2.85 mmol) was dissolved in anhydrous dichloromethane (10 mL), followed by addition of N,N-dimethylformamide (21 mg, 0.287 mmol). The mixture was cooled to 0°C, oxalyl chloride (723 mg, 5.70 mmol) was slowly added dropwise. The mixture was warmed up to room temperature and reacted for 2 hours. The reaction solution was concentrated under reduced pressure, dissolved in anhydrous dichloromethane (10 mL), and cooled to 0°C. Triethylamine (574 mg, 5.67 mmol) and methanol (454 mg, 14.2 mmol) were added, and reacted at 0°C for 1 hour. The reaction solution was poured into 50 mL of water, and extracted with dichloromethane (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain compound 91a (520 mg, yield 96.3%).
MS m/z (ESI): 190.0 [M+1]
¹H NMR (400 MHz, DMSO-*d*₆) δ 8.08 - 8.03 (m, 1H), 7.88 (dd, *J* = 8.8, 3.2 Hz, 1H), 3.90 (s, 3H).

### Step 2

Compound 91a (300 mg, 1.58 mmol) was dissolved in anhydrous tetrahydrofuran (5 mL). The solution was cooled to -78°C under nitrogen atmosthere, followed by addition of a solution of methylmagnesium chloride in tetrahydrofuran (1 M, 4 mL) dropwise. After completion of the addition, the mixture was reacted at -78°C for 2 hours. The reaction solution was poured into 50 mL of saturated ammonium chloride solution, and extracted with ethyl acetate (40 mL*2). The organic phases were combined, washed with water (40 mL) and saturated sodium chloride solution (40 mL) successively, dried over anhydrous sodium sulfate and filtered. The filtrate was concentrated under reduced pressure, and the residues were purified by silica gel column chromatography (petroleum ether/ethyl acetate system) to obtain compound 91b (260 mg, yield 86.6 %).
MS m/z (ESI): 190.1 [M+1]

### Step 3

Compound 60-3 (100 mg, 0.332 mmol) was dissolved in anhydrous N,N-dimethylformamide (2 mL), followed by addition of compound 91b (69 mg, 0.364 mmol) and cesium carbonate (216 mg, 0.663 mmol). The mixture was reacted under microwave at 180°C for 2 hours. The reaction solution was cooled to room temperature, poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by reverse preparative HPLC (acetonitrile/water system) to obtain compound 91 (31 mg, yield 20.6%).
MS m/z (ESI): 455.2 [M+1]
¹H NMR (400 MHz, DMSO-*d*₆) δ 8.16 (s, 1H), 7.98 (s, 1H), 7.84 - 7.77 (m, 1H), 7.69 - 7.63 (m, 1H), 7.49 - 7.40 (m, 2H), 6.39 (dd, *J* = 8.8, 2.0 Hz, 1H), 5.31 (d, *J* = 16.0 Hz, 1H), 3.74 - 3.65 (m, 2H), 3.59 - 3.38 (m, 4H), 3.28 - 3.25 (m, 1H), 3.16 - 2.98 (m, 3H), 1.45 (s, 6H).

### Example 92

### Preparation of compound 92: ((3 aR, 6aS)-5-(5-fluoro-4-(2-hydroxypropan-2-yl)pyrimidin-2-yl)hexahydropyrrolo[3,4-c]pyrrol-2(1H)-yl)(2-fluoro-6-(2H-1,2,3-triazol-2-yl)phenyl)methanone

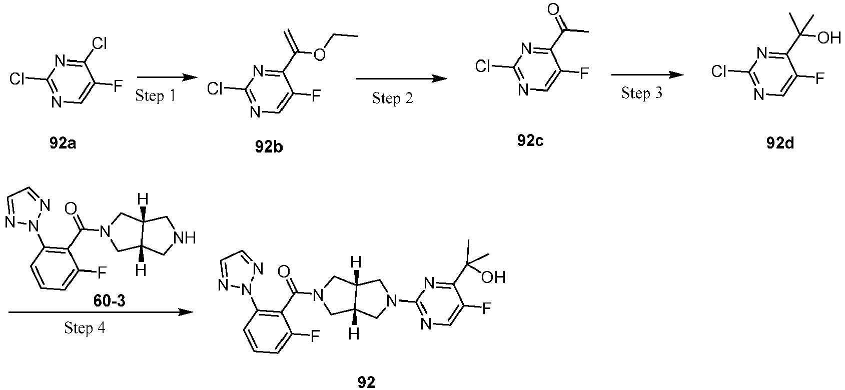

### Step 1

Tributyl(1-ethoxyvinyl)stannane (11.90 g, 32.95 mmol) and compound 92a (5 g, 29.95 mmol) were dissolved in DMF (5 mL), followed by addition of bistriphenylphosphinepalladium(II) chloride (1.05 g, 1.50 mmol). The reaction solution was stirred at 100°C for 2 hours. After completion of the reaction, saturated aqueous potassium fluoride solution (10 ml) was added, stirred for 1 hour and filtered. The filter cake was washed with dichloromethane (10 mL*3). The organic phases were combined, dried, and concentrated to obtain the crude product. The crude product was purified by column chromatography (petroleum ether/ethyl acetate system) to obtain compound 92b (5.6 g, yield 92.3%). MS m/z (ESI): 203.0 [M+1]

### Step 2

Compound 92b (5.6 g, 27.64 mmol) was dissolved in tetrahydrofuran (2 mL), followed by addition of hydrochloric acid (2 mL, 27.64 mmol). The reaction solution was stirred at 50°C for 2 hours. Saturated sodium chloride (10 mL) was added, and the solution was extracted with dichloromethane (10 mL*3). The organic phases were combined, dried, and concentrated to obtain a crude product. The crude product was purified by column chromatography (petroleum ether/ethyl acetate system) to obtain compound 92c (4.4 g, yield 91.2%).
MS m/z (ESI): 175.0 [M+1]

### Step 3

Compound 92c (4.4 g, 25.21 mmol) was dissolved in tetrahydrofuran (5 mL), followed by addition of methylmagnesium bromide (3 M, 9.24 mL) at -78°C. The reaction solution was stirred at -78°C for 15 minutes. Saturated ammonium chloride solution (10 ml) was added, and the solution was extracted with dichloromethane (10 mL*3). The organic phases were combined, dried, and concentrated to obtain a crude product. The crude product was purified by column chromatography (petroleum ether/ethyl acetate system) to obtain 92d (2.2 g, yield 45.8%).
MS m/z (ESI): 191.0 [M+1]

### Step 4

The synthetic method of compound 92 was refered to the synthetic method of compound 60, except that compound 60-3 and compound 92d were used as starting materials to obtain Compound 92 (38 mg, yield 42.6%).
MS: m/z (ESI): 456.2 [M+1]
¹H NMR (400 MHz, DMSO) δ 8.21 (t, J = 23.7 Hz, 1H), 7.99 (d, J = 73.9 Hz, 2H), 7.73 (dd, J = 17.8, 8.2 Hz, 1H), 7.59 (dd, J = 14.6, 8.1 Hz, 1H), 7.37 (dd, J = 19.5, 8.8 Hz, 1H), 5.13 (d, J = 9.5 Hz, 1H), 3.91 - 3.34 (m, 7H), 3.14 - 2.87 (m, 3H), 1.39 (d, J = 4.2 Hz, 6H).

### Example 93

### Preparation of compound 93: ((3 aR, 6aS)-5-(5-fluoro-4-(2-hydroxypropan-2-yl)pyrimidin-2-yl)hexahydropyrrolo[3,4-c]pyrrol-2(1H)-yl)(2-fluoro-6-(pyrimidin-2-yl)phenyl)methanone

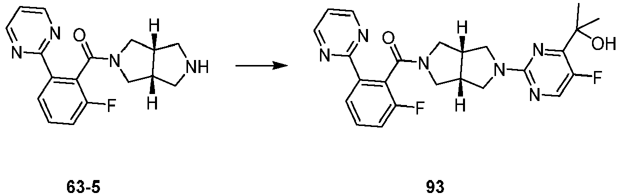

The synthetic method of compound 93 was refered to the synthetic method of compound 60, except that compound 63-5 and 92d were used as starting materials to obtain Compound 93 (22 mg, yield 28.6%).
MS: m/z (ESI): 467.2 [M+1]
¹H NMR (400 MHz, DMSO) δ 8.86 (dd, J = 48.0, 4.9 Hz, 2H), 8.32 (s, 1H), 8.02 (dd, J = 35.7, 7.8 Hz, 1H), 7.60 (dd, J = 14.0, 7.9 Hz, 1H), 7.44 (ddd, J = 26.7, 12.9, 4.9 Hz, 2H), 5.33 - 5.10 (m, 1H), 3.89 - 3.46 (m, 7H), 3.23 - 2.95 (m, 3H), 1.46 (s, 6H).

### Example 94

### Preparation of compound 94: (4-fluoro-2-(2H-1,2,3-triazol-2-yl)phenyl)((cis)-5-(5-fluoro-4-(2-hydroxypropan-2-yl)-6 -methylpyrimidin-2-yl)hexahydropyrrolo[3,4-c]pyrrol-2(1H)-yl)methanone

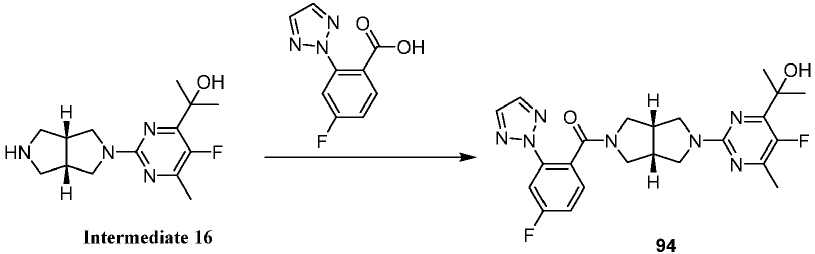

Intermediate 16 (80 mg, 0.285 mmol) was dissolved in acetonitrile (2 mL), followed by addition of 4-fluoro-2-(triazol-2-yl)benzoic acid (59 mg, 0.285 mmol), N-methylmorpholine (36 mg, 0.356 mmol) and N,N,N',N'-tetramethylchloroformamidine hexafluorophosphate (120 mg, 0.428 mmol). The reaction solution was reacted at room temperature for 2 hours. After completion of the reaction, the reaction solution was poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by reverse preparative HPLC (acetonitrile/water system) to obtain compound 94 (16.3 mg, yield 12.2%).
MS m/z (ESI): 470.2 [M+1]
¹H NMR (400 MHz, DMSO-d6) δ 8.03 (s, 2H), 7.74 (dd, *J* = 9.6, 2.0 Hz, 1H), 7.60 - 7.56 (m, 1H), 7.43 - 7.38 (m, 1H), 5.17 (s, 1H), 3.75 - 3.65 (m, 2H), 3.60 - 3.56 (m, 1H), 3.48 - 3.44 (m, 3H), 3.34 - 3.29 (m, 1H), 3.02 - 2.93 (m, 3H), 2.31 (s, 3H), 1.46 (s, 6H).

### Example 95

### Preparation of compound 95: (3-fluoro-2-(2H-1,2,3-triazol-2-yl)phenyl)((cis)-5-(5-fluoro-4-(2-hydroxypropan-2-yl)-6 -methylpyrimidin-2-yl)hexahydropyrrolo[3,4-c]pyrrol-2(1H)-yl)methanone

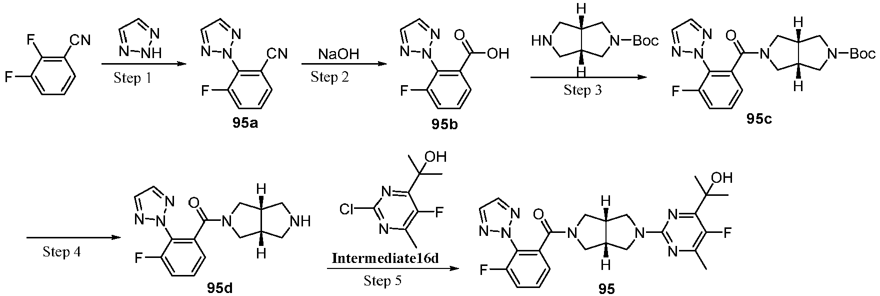

### Step 1

2,3-Difluorobenzonitrile (12.0 g, 86.3 mmol) was dissolved in N,N-dimethylformamide (100 mL), followed by addition of 1,2,3-triazole (5.96 g, 86.3 mmol) and cesium carbonate (28.1 g, 86.3 mmol). The reaction solution was heated to 120°C for 2 hours. After completion of the reaction, the reaction solution was cooled to room temperature, poured into 200 mL of water, and extracted with methyl *tert*-butyl ether (100 mL*2). The organic phases were combined, washed with water (100 mL) and saturated sodium chloride solution (100 mL) successively, dried over anhydrous sodium sulfate and filtered. The filtrate was concentrated under reduced pressure, and the residues were purified by silica gel column chromatography (petroleum ether/ethyl acetate system) to obtain compound 95a (6.5 g, yield 40.0 %).
MS m/z (ESI): 189.0 [M+1]
¹H NMR (400 MHz, CDCl₃) δ 8.00 (s, 2H), 7.67 - 7.65 (m, 1H), 7.62 - 7.55 (m, 2H).

### Step 2

Compound 95a (1.5 g, 7.97 mmol) was dissolved in 1,4-dioxane (10 mL) and water (30 mL), followed by addition of sodium hydroxide (3.19 g, 79.8 mmol). The mixture was heated to 110°C and reacted for 4 hours. The reaction solution was cooled to 0°C, adjusted to about pH 1 with dilute hydrochloric acid, and extracted with ethyl acetate (100 mL*2). The organic phases were combined, washed with water (100 mL) and saturated sodium chloride solution (100 mL) successively, dried over anhydrous sodium sulfate and filtered. The filtrate was concentrated under reduced pressure to obtain compound 95b (1.5 g, yield 90.8 %).
MS m/z (ESI): 208.0 [M+1]
¹H NMR (400 MHz, CDCl₃) δ 7.89 (s, 2H), 7.87 (d, *J* = 7.6 Hz, 1H), 7.63 - 7.58 (m, 1H), 7.52 - 7.49 (m, 1H).

### Step 3

(cis)-2-Boc-hexahydropyrrolo[3,4-c]pyrrole (120 mg, 0.565 mmol) was dissolved in anhydrous acetonitrile (2 mL), followed by addition of compound 95b (117 mg, 0.565 mmol), N,N,N',N'-tetramethylchloroformamidine hexafluorophosphate (238 mg, 0.848 mmol) and N-methylmorpholine (86 mg, 0.850 mmol). The reaction solution was reacted at room temperature for 2 hours. The reaction solution was poured into 30 mL of water, and extracted with ethyl acetate (30 mL*2). The organic phases were combined, washed with water (80 mL) and saturated sodium chloride solution (80 mL) successively, dried over anhydrous sodium sulfate and filtered. The filtrate was concentrated under reduced pressure, and the residues were purified by silica gel column chromatography (petroleum ether/ethyl acetate system) to obtain compound 95c (210 mg, yield 92.6%).
MS m/z (ESI): 402.2 [M+1]
¹H NMR (400 MHz, DMSO-*d*₆) δ 8.07 (s, 2H), 7.71 - 7.66 (m, 1H), 7.63 - 7.58 (m, 1H), 7.42 (d, *J* = 7.6 Hz, 1H), 3.49 - 3.42 (m, 3H), 3.40 - 3.36 (m, 1H), 3.23 - 3.19 (m, 1H), 3.16 - 3.01 (m, 3H), 2.87 - 2.78 (m, 2H), 1.41 (s, 9H).

### Step 4

Compound 95c (80 mg, 0.199 mmol) was dissolved in anhydrous dichloromethane (2 mL), followed by addition of hydrochloric acid dioxane solution (4 M, 2 mL). The mixture was reacted at room temperature for 2 hours. The reaction solution was concentrated under reduced pressure to obtain compound 95d (60 mg, yield 99.9%).
MS m/z (ESI): 302.1 [M+1]

### Step 5

Compound 95d (60 mg, 0.199 mmol) was dissolved in anhydrous N,N-dimethylformamide (2 mL), followed by addition of intermediate 16d (45 mg, 0.220 mmol) and cesium carbonate (130 mg, 0.399 mmol). The reaction solution was heated to 110°C for 2 hours. The reaction solution was cooled to room temperature, poured into 30 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by reverse preparative HPLC (acetonitrile/water system) to obtain compound 95 (31.3 mg, yield 33.5%).
MS m/z (ESI): 470.2 [M+1]
¹H NMR (400 MHz, DMSO-*d*₆) δ 8.00 (s, 2H), 7.70 - 7.65 (m, 1H), 7.62 - 7.57 (m, 1H), 7.42 (d, *J* = 7.6 Hz, 1H), 5.16 (s, 1H), 3.73 - 7.69 (m, 1H), 3.64 - 3.59 (m, 1H), 3.57 - 3.49 (m, 2H), 3.43 - 3.34 (m, 2H), 3.29 - 3.25 (m, 1H), 3.13 - 3.09 (m, 1H), 2.99 - 2.93 (m, 2H), 2.32 (d, *J* = 2.8 Hz, 3H), 1.46 (s, 6H).

### Example 96

### Preparation of compound 96: ((3aR,6aS)-5-(5-fluoro-4-(2-hydroxypropan-2-yl)-6-methylpyrimidin-2-yl)hexahydropy rrolo[3,4-c]pyrrol-2(1H)-yl)(2-fluoro-6-(3-fluoropyridin-2-yl)phenyl)methanone

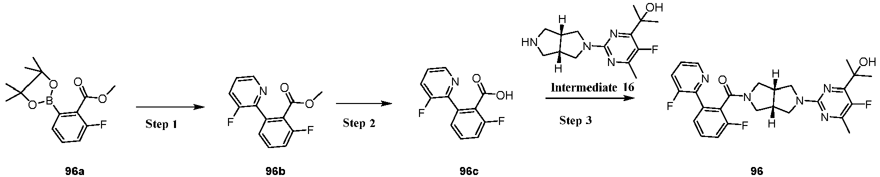

### Step 1

2-Bromo-3-fluoropyridine (250 mg, 1.42 mmol), compound 96a (477.48 mg, 1.70 mmol), Pd(dppf)Cl₂ (57.96 mg, 79.21 µmol) and K₃PO₄ (2 M, 1.42 mL) were added to 1,4-dioxane (10 mL) under N₂ atmosphere. The reaction solution was stirred at 100°C for 4 hours, followed by addition of water (20 ml). The solution was extracted with dichloromethane (30 mL*2), and the combined extracts were dried over Na₂SO₄, and concentrated under reduced pressure. The resulting crude product was purified to obtain compound 96b (0.17 g, yield 48.0%).
MS m/z (ESI): 250.1 [M+1]

### Step 2

Sodium hydroxide (480 mg, 12 mmol) was added to a mixed solution of compound 96b (150 mg, 0.6 mmol) in water (5.0 mL) and methanol (5.0 mL). The mixture was reacted at 25°C for 3 hours. 2 N HCl was added until pH=2, and the solution was extracted with ethyl acetate (20 mL*2). The organic phase was dried over anhydrous sodium sulfate, filtered and concentrated under reduced pressure to obtain compound 96c (120 mg, yield 84.8%).
MS m/z (ESI): 236.0 [M+1]

### Step 3

Tetramethylchlorourea hexafluorophosphate (280 mg, 1.00 mmol) and N-methylimidazole (164 mg, 2.00 mmol) were added to a solution of 96c (120 mg, 0.51 mmol) and intermediate 19 (140 mg, 0.50 mmol) in acetonitrile (3.0 mL). The mixture was stirred at 25°C for 30 minutes. 20 mL of water was added to the reaction solution, and extracted with ethyl acetate (20 mL*2). The organic phases were combined, dried over anhydrous sodium sulfate, filtered and concentrated. The resulting crude product was purified by preparative HPLC to obtain compound 96 (60 mg, yield 24.1%).
MS m/z (ESI): 498.2 [M+1]
¹H NMR (400 MHz, DMSO-d6) δ 8.41 (dd, J = 57.0, 4.6 Hz, 1H), 7.83 (dt, J = 22.2, 9.5 Hz, 1H), 7.62 - 7.35 (m, 4H), 5.17 (d, J = 14.6 Hz, 1H), 3.81 - 3.55 (m, 5H), 3.49 (d, J = 3.4 Hz, 2H), 3.19 - 2.97 (m, 3H), 2.32 (dd, J = 13.5, 2.8 Hz, 3H), 1.46 (d, J = 9.8 Hz, 6H).

### Example 97

### Preparation of compound 97: ((3aR,6aS)-5-(5-fluoro-4-(2-hydroxypropan-2-yl)-6-methylpyrimidin-2-yl)hexahydropy rrolo[3,4-c]pyrrol-2(1H)-yl)(2-fluoro-6-(pyridin-2-yl)phenyl)methanone

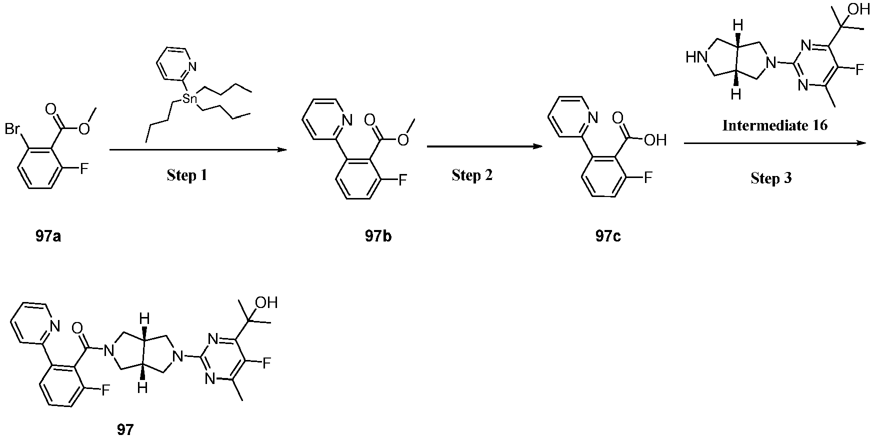

### Step 1

CuI (653.81 mg, 3.43 mmol), Pd(PPh₃)₄ (1.98 g, 1.71 mmol) and CsF (5.21 g, 34.30 mmol) were added to a solution of compound 97a (4 g, 17.16 mmol) and 2-(tributylstannyl)pyridine (6.97 g, 18.93 mmol) in DMF (60 mL) under N₂ atomsphere. The mixture was reacted at 120°C for 5 hours. The reaction solution was cooled to room temperature, followed by addition of 150 mL of water and extracting with ethyl acetate (100 mL*2). The organic phases were combined, dried over anhydrous sodium sulfate, filtered and concentrated. The resulting crude product was purified to obtain compound 97b (2.8 g, yield 70.5%).
MS m/z (ESI): 232.1 [M+1]

### Step 2

Sodium hydroxide (688.98 mg, 17.22 mmol) was added to a mixed solution of compound 97b (800 mg, 3.46 mmol) in water (5.0 mL) and methanol (5.0 mL). The mixture was reacted at 25°C for 3 hours. 2 N HCl was added until pH=2, and the solution was extracted with ethyl acetate (20 mL*2). The organic phase was dried over anhydrous sodium sulfate, filtered and concentrated under reduced pressure to obtain compound 97c (650 mg, yield 86.5%).
MS m/z (ESI): 218.0 [M+1]

### Step 3

Tetramethylchlorourea hexafluorophosphate (257 mg, 0.92 mmol) and N-methylimidazole (188 mg, 2.29 mmol) were added to a solution of 97c (100 mg, 0.46 mmol) and intermediate 16 (129 mg, 0.46 mmol) in acetonitrile (3.0 mL). The mixture was stirred at 25°C for 30 minutes. 20 mL of water was added to the reaction solution, and extracted with ethyl acetate (20 mL*2). The organic phases were combined, dried over anhydrous sodium sulfate, filtered and concentrated. The resulting crude product was purified by preparative HPLC to obtain compound 97 (40 mg, yield 18.1%).
MS m/z (ESI): 480.2 [M+1]
¹H NMR (400 MHz, DMSO-d6) δ 8.64 - 8.44 (m, 1H), 7.87 (dq, J = 21.9, 7.1, 6.6 Hz, 1H), 7.76 - 7.48 (m, 3H), 7.43 - 7.19 (m, 2H), 5.16 (s, 1H), 3.67 (dddd, J = 38.5, 25.2, 11.5, 7.2 Hz, 5H), 3.23 - 2.95 (m, 5H), 2.32 (dd, J = 11.6, 2.8 Hz, 3H), 1.46 (d, J = 11.0 Hz, 6H).

### Example 98

### Preparation of compound 98: (5-fluoro-2-(2H-1,2,3-triazol-2-yl)phenyl)((cis)-5-(5-fluoro-4-(2-hydroxypropan-2-yl)-6 -methylpyrimidin-2-yl)hexahydropyrrolo[3,4-c]pyrrol-2(1H)-yl)methanone

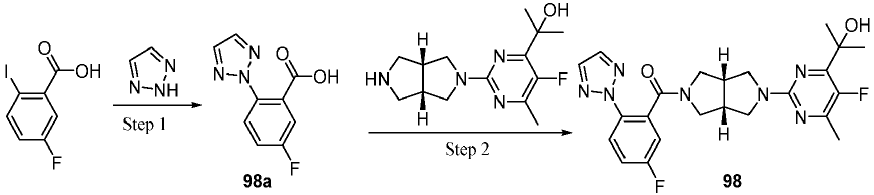

### Step 1

5-Fluoro-2-iodobenzoic acid (300 mg, 1.13 mmol) was dissolved in anhydrous N,N-dimethylformamide (4 mL), followed by addition of 1,2,3-triazole (117 mg, 1.69 mmol), cuprous iodide (258 mg, 1.35 mmol), *trans*-N,N'-dimethyl-1,2-cyclohexanediamine (193 mg, 0.97 mmol) and cesium carbonate (735 mg, 2.26 mmol). The mixture was reacted in microwave at 120°C for 15 minutes. The reaction solution was cooled to room temperature, poured into 50 mL of water, which was then adjusted to pH 5 with dilute hydrochloric acid, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by reverse C18 column chromatography (acetonitrile/water system) to obtain compound 98a (150 mg, yield 64.2%).
MS m/z (ESI): 208.0 [M+1]

### Step 2

The preparation method of compound 98 was refered to the preparation method of step 3 of compound 97, except that compound 97c was replaced with compound 98a to obtain Compound 98 (60 mg, yield 30%).
MS m/z (ESI): 470.2 [M+1]
¹H NMR (400 MHz, DMSO-*d*₆) δ 7.98 (s, 2H), 7.93 - 7.89 (m, 1H), 7.49 - 7.46 (m, 1H), 7.37 - 7.35 (m, 1H), 5.16 (s, 1H), 3.81 - 3.57 (m, 3H), 3.42 - 3.37 (m, 4H), 3.04 - 2.94 (m, 3H), 2.30 (s, 3H), 1.46 (s, 6H).

### Example 99

### Preparation of compound 99: (2-fluoro-6-(2H-1,2,3-triazol-2-yl)phenyl)((3aR,6aS)-5-(4-(2-fluoropropan-2-yl)-6-meth ylpyridin-2-yl)hexahydropyrrolo[3,4-c]pyrrol-2(1H)-yl)methanone

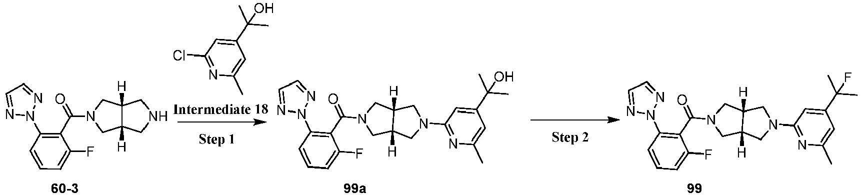

### Step 1

Intermediate 18 (61 mg, 0.33 mmol), compound 60-3 (100 mg, 0.33 mmol), Pd₂(dba)₃ (18 mg, 0.02 mmol), BINAP (25 mg, 0.04 mmol), cesium carbonate (215 mg, 0.66 mmol) and dioxane (2 mL) were added to a round-bottomed flask, and the mixture was stirred at 100°C for 12 hours under nitrogen atomsphere. The reaction solution was cooled, quenched by water (5 mL), and extracted with dichloromethane (10 mL*3). The organic phases were combined, dried, and concentrated. The residues were purified by preparative HPLC to obtain 99a (75 mg, yield 50.7%).
MS m/z (ESI): 451.2 [M+1]

### Step 2

Compound 99a (75 mg, 0.17 mmol) and DAST (48 mg, 0.30 mmol) were added to DCM (5 mL) at 0°C. The reaction solution was stirred at 25°C for 0.5 hour, followed by addition of water (20 ml). The solution was extracted with dichloromethane (30 mL*2), and the combined extracts were dried over Na₂SO₄, and concentrated under reduced pressure. The resulting crude product was purified by preparative HPLC to obtain compound 99 (50 mg, yield 66.4%).
MS m/z (ESI): 453.2 [M+1]
¹H NMR (400 MHz, DMSO-d6) δ 8.15 (s, 1H), 7.95 (s, 1H), 7.79 (dd, J = 17.7, 8.2 Hz, 1H), 7.65 (tdd, J = 8.2, 6.2, 1.8 Hz, 1H), 7.48 - 7.39 (m, 1H), 6.45 (d, J = 6.0 Hz, 1H), 6.19 (s, 1H), 3.81 - 3.42 (m, 7H), 3.21 - 2.98 (m, 3H), 2.29 (s, 3H), 1.72 - 1.44 (m, 6H)

### Example 100

### Preparation of compound 100: ((3aR,6aS)-5-(5-fluoro-4-(2-hydroxypropan-2-yl)-6-methylpyrimidin-2-yl)hexahydropy rrolo[3,4-c]pyrrol-2(1H)-yl)(2-fluoro-6-(4H-1,2,4-triazol-3-yl)phenyl)methanone

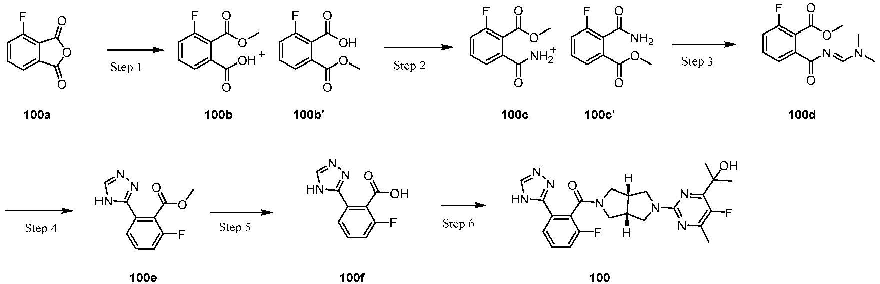

### Step 1

Compound 100a (5.0 g, 30.1 mmol) was dissolved in methanol (10 mL), and the reaction solution was stirred at 80°C for 16 hours. The reaction solution was concentrated to obtain the crude product mixture of compound 100b and compound 100b' (5.96 g, yield 99.9%).
MS: m/z (ESI): 199.1[M+1]

### Step 2

The mixture of compound 100b and compound 100b' (5.96 g, 30.1 mmol) were dissolved in dichloromethane (10 mL), followed by addition of oxalyl chloride (3.84 g, 30.25 mmol) and N,N-dimethylformamide (94.40 mg, 1.29 mmol). The reaction solution was stirred at 100°C for 1 hour, then concentrated. Dichloromethane (10 mL) was added, followed by addition of N-trimethylsilyl-N-trimethylsilyl-methylamine (2.44 g, 15.12 mmol). The reaction solution was stirred for 16 hours at room temperature. Methanol (10 ml) was added to the reaction solution, and a large amount of solids were precipitated. The solution was extracted with dichloromethane (5 mL*3). The organic phases were combined, dried, and concentrated to obtain the crude product mixture of compound 100c and compound 100c' (5.6 g, yield 94.4%).
MS: m/z (ESI): 198.1[M+1]

### Step 3

The mixture of compound 100c and compound 100c' (2.0 g, 10.1 mmol) was dissolved in N,N-dimethylformamide dimethyl acetal (10 mL), and the reaction solution was stirred at room temperature for 16 hours. The reaction solution was concentrated to obtain the crude product, which was purified by column chromatography (petroleum ether/ethyl acetate system) to obtain compound 100d (210 mg, yield 8.2%).
MS: m/z (ESI): 253.2[M+1]

### Step 4

Compound 100d (0.15 g, 594.65 µmol) was dissolved in acetic acid (2 mL), followed by addition of hydrazine hydrate (1.19 mmol, 0.2 mL, 0.85% purity). The reaction solution was stirred at room temperature for 72 hours. Saturated NaCl (10 mL) was added, and the solution was extracted with dichloromethane (5 mL*3). The organic phases were combined, dried, and concentrated to obtain the crude product. The crude product was purified by column chromatography (petroleum ether/ethyl acetate system) to obtain compound 100e (120 mg, yield 91.2%).
MS: m/z (ESI): 222.1[M+1]

### Step 5

Compound 100e (120 mg, 542.52 µmol) was dissolved in water (2 mL) and methanol (1 mL), followed by addition of sodium hydroxide (21.70 mg, 542.5 µmol). The reaction solution was stirred at room temperature for 36 hours. 2M hydrochloric acid was added to the reaction solution to adjust the pH of the reaction solution to 1-2, and extracted with dichloromethane (5 mL*3). The organic phases were combined, dried, and concentrated to obtain the crude product compound 100f (70 mg, yield 62.3%).
MS: m/z (ESI): 208.0[M+1]

### Step 6

The synthetic method of compound 100 was refered to the preparation method of step 3 of compound 97, except that compound 97c was replaced with compound 100f to obtain Compound 100 (22 mg, yield 38.7%).
MS: m/z (ESI): 470.2 [M+1]
¹H NMR (400 MHz, DMSO-d6) δ 8.40 (d, J = 83.8 Hz, 2H), 7.78 (dd, J = 11.4, 7.9 Hz, 1H), 7.48 (td, J = 8.1, 6.0 Hz, 1H), 7.29 (dd, J = 18.0, 8.8 Hz, 1H), 3.81 - 3.43 (m, 7H), 3.06 - 2.76 (m, 3H), 2.23 (d, J = 2.7 Hz, 3H), 1.38 (s, 6H).

### Example 101

### Preparation of compound 101: (5-fluoro-2-(pyrimidin-2-yl)phenyl)((cis)-5-(5-fluoro-4-(2-hydroxypropan-2-yl)-6-meth ylpyrimidin-2-yl)hexahydropyrrolo[3,4-c]pyrrol-2(1H)-yl)methanone

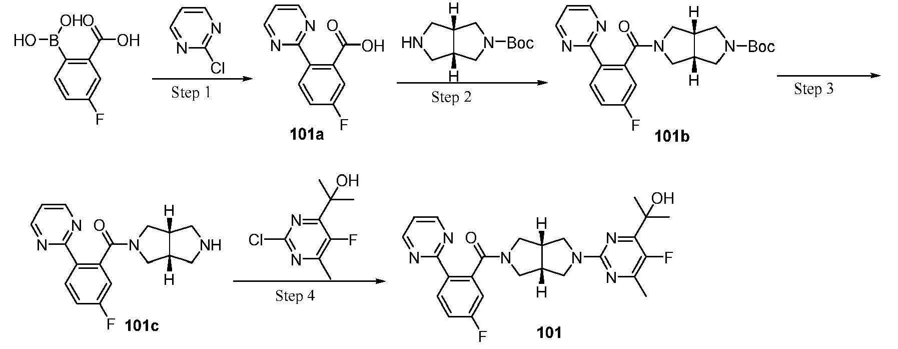

### Step 1

2-Chloropyrimidine (280 mg, 2.44 mmol) was dissolved in anhydrous N,N-dimethylformamide (4 mL) and water (1 mL), followed by addition of 2-borono-5-fluorobenzoic acid (300 mg, 1.63 mmol), tetrakis(triphenylphosphine)palladium (86 mg, 0.074 mmol) and cesium carbonate (451 mg, 1.38 mmol). The reaction solution was heated to 100°C for 2 hours under nitrogen protection. The reaction solution was cooled to room temperature, followed by addition of 15 mL of water and filtering. The filtrate was concentrated under reduced pressure, then 10 mL of ethanol was added. Inorganic salts was removed by filtration. The filtrate was concentrated under reduced pressure to obtain the crude product compound 101a (260 mg, yield 73.1%), which was used directly in the next step.
MS m/z (ESI): 219.1 [M+1]

### Step 2

The preparation method of compound 101b was refered to the preparation method of compound 95c.
MS m/z (ESI): 413.2 [M+1]

### Step 3

The preparation method of compound 101c was refered to the preparation method of compound 95d.
MS m/z (ESI): 313.1 [M+1]

### Step 4

The preparation method of compound 101 was refered to the preparation method of compound 95.
MS m/z (ESI): 481.2 [M+1]
¹H NMR (400 MHz, DMSO-*d*₆) δ 8.81 (d, *J* = 4.8 Hz, 2H), 8.19 (dd, *J* = 8.8, 5.6 Hz, 1H), 7.42 - 7.37 (m, 2H), 7.31 - 7.29 (m, 1H), 5.16 (s, 1H), 3.78 - 3.67 (m, 2H), 3.62 - 3.46 (m, 5H), 3.09 - 2.95 (m, 3H), 2.31 (d, *J* = 2.4 Hz, 3H), 1.46 (s, 6H).

### Example 102

### Preparation of compound 102: (2-fluoro-6-(2H-1,2,3-triazol-2-yl)phenyl)((cis)-5-(4-(2-fluoropropan-2-yl)-5-methylpyr imidin-2-yl)hexahydropyrrolo[3,4-c]pyrrol-2(1H)-yl)methanone

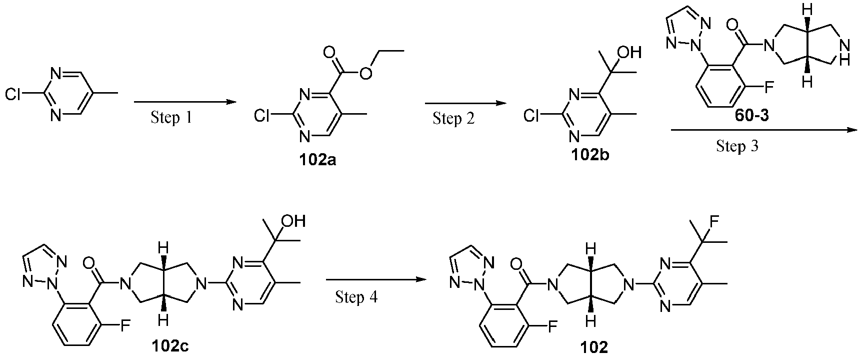

### Step 1

Ethyl pyruvate (2.71 g, 23.3 mmol) was added to a three-necked flask and cooled to -10°C under nitrogen atmosphere, followed by addition of acetic acid (10 mL). Hydrogen peroxide (794 mg) was slowly added dropwise over about 20 minutes, and stirred for 10 minutes. 2-Chloro-5-methylpyrimidine (1.0 g, 7.78 mmol) was dissolved in toluene (30 mL) and water (10 mL) and cooled to -10°C, followed by addition of ferrous sulfate heptahydrate (6.49 g, 23.3 mmol) and concentrated sulfuric acid (2.29 g). The above ethyl pyruvate reaction solution was added dropwise over about 1 hour under nitrogen protection,, then reacted at -10°C for 1 hour. The reaction solution was poured into 100 mL of ice water, and adjusted to pH 7 with 1 M sodium hydroxide solution. The solution was filtered, and the filtrate was extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain the crude product compound 102a (1.1 g, yield 70.5 %).
MS m/z (ESI): 201.0 [M+1]

### Step 2

Compound 102a (700 mg, 3.49 mmol) was dissolved in anhydrous tetrahydrofuran (10 mL). The solution was cooled to -78°C under nitrogen atmosphere, followed by addition of a solution of methylmagnesium chloride in tetrahydrofuran (3 M, 3.5 mL). After completion of the addition, the mixture was reacted at -78°C for 1 hour, and then slowly warmed to room temperature for 1 hour. The reaction solution was poured into 50 mL of saturated ammonium chloride solution, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain compound 102b (180 mg, yield 27.6%).
MS m/z (ESI): 187.0 [M+1]

### Step 3

Compound 60-3 (150 mg, 0.498 mmol) was dissolved in anhydrous N,N-dimethylformamide (3 mL), followed by addition of compound 118b (93 mg, 0.498 mmol) and cesium carbonate (324 mg, 0.994 mmol). The mixture was heated to 100°C for 2 hours. The reaction solution was cooled to room temperature, poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by silica gel column chromatography (petroleum ether/ethyl acetate system) to obtain compound 102c (130 mg, yield 57.8%).
MS m/z (ESI): 452.2 [M+1]
¹H NMR (400 MHz, DMSO-*d*₆) δ 8.16 (s, 1H), 8.06 (d, *J* = 3.2 Hz, 1H), 7.95 (s, 1H), 7.83 - 7.77 (m, 1H), 7.69 - 7.63 (m, 1H), 7.48 - 7.40 (m, 1H), 5.23 (d, *J* = 11.5 Hz, 1H), 3.77 - 3.66 (m, 2H), 3.60 - 3.53 (m, 1H), 3.50 - 3.39 (m, 3H), 3.35 - 3.34 (m, 1H), 3.14 - 2.95 (m, 3H), 2.29 (s, 3H), 1.44 (d, *J* = 3.8 Hz, 6H).

### Step 4

Compound 102c (90 mg, 0.199 mmol) was dissolved in anhydrous dichloromethane (3 mL). The solution was cooled to -78°C under nitrogen atmosphere, followed by slowly addition of diethylaminosulfur trifluoride (96 mg, 0.596 mmol) dropwise. After completion of the addition, the mixture was reacted at -78°C for 1 hour. The mixture was slowly warmed up to room temperature and reacted for 1 hour. The reaction solution was poured into 50 mL of saturated sodium bicarbonate solution, and extracted with dichloromethane (30 mL*2). The organic phases were combined, washed with saturated sodium chloride solution (30 mL), dried over anhydrous sodium sulfate and filtered. The filtrate was concentrated under reduced pressure, and the residues were purified by reverse preparative HPLC (acetonitrile/water system) to obtain compound 102 (25.9 mg, yield 28.7%).
MS m/z (ESI): 454.2 [M+1]
¹H NMR (400 MHz, DMSO-*d*₆) δ 8.17 (s, 1H), 8.16 (s, 1H), 7.95 (s, 1H), 7.83 - 7.75 (m, 1H), 7.69 - 7.64 (m, 1H), 7.48 - 7.40 (m, 1H), 3.77 - 3.69 (m, 2H), 3.60 - 3.54 (m, 1H), 3.48 - 3.34 (m, 4H), 3.16 - 2.95 (m, 3H), 2.21 (d, *J* = 5.6 Hz, 3H), 1.64 (d, *J* = 22.0 Hz, 6H).

### Example 103

### Preparation of compound 103: 2-(2-((cis)-5-(2-fluoro-6-(2H-1,2,3-triazol-2-yl)benzoyl)hexahydropyrrolo[3,4-c]pyrrol-2(1H)-yl)-6-methylpyrimidin-4-yl)-2-methylpropanenitrile

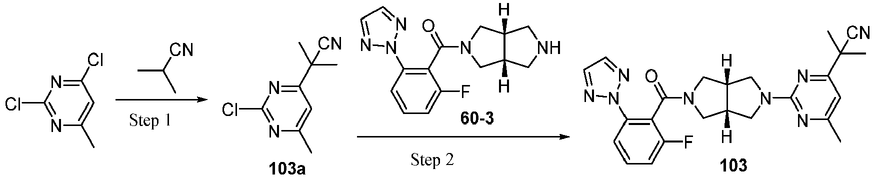

### Step 1

Isobutyronitrile (509 mg, 7.37 mmol) was dissolved in anhydrous tetrahydrofuran (10 mL). The solution was cooled to -78°C under nitrogen atmosphere, followed by addition of a solution of lithium diisopropylamide in tetrahydrofuran (2 M, 3.4 mL). After completion of the addition, the mixture was reacted at -78°C for 30 minutes, and then slowly warmed to about 0°C and reacted for 30 minutes. The solution was cooled to -78°C, followed by addition of a solution of 2,4-dichloro-6-methylpyrimidine in tetrahydrofuran (1.0 g, 6.13 mmol). After completion of the addition, the mixture was reacted at -78°C for 1 hour, and then slowly warmed to room temperature for 30 minutes. The reaction solution was poured into 50 mL of saturated ammonium chloride solution, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain compound 103a (80 mg, yield 6.7%).
MS m/z (ESI): 196.1 [M+1]

### Step 2

The preparation method of compound 103 was refered to the preparation method of compound 102c, except that compound 102b was replaced with compound 103a to obtain Compound 103 (34 mg, yield 54%).
MS m/z (ESI): 461.2 [M+1]
¹H NMR (400 MHz, DMSO-*d*₆) δ 8.16 (s, 1H), 7.96 (s, 1H), 7.83 - 7.77 (m, 1H), 7.69 - 7.64 (m, 1H), 7.48 - 7.40 (m, 1H), 6.70 (d, *J* = 5.6 Hz, 1H), 3.82 - 3.70 (m, 2H), 3.58 - 3.42 (m, 4H), 3.39 - 3.34 (m, 1H), 3.15 - 3.02 (m, 3H), 2.32 (s, 3H), 1.63 (s, 6H).

### Example 104

### Preparation of compound 104: (3-fluoro-2-(2H-1,2,3-triazol-2-yl)phenyl)((cis)-5-(4-(2-fluoropropan-2-yl)-6-methylpyr imidin-2-yl)hexahydropyrrolo[3,4-c]pyrrol-2(1H)-yl)methanone

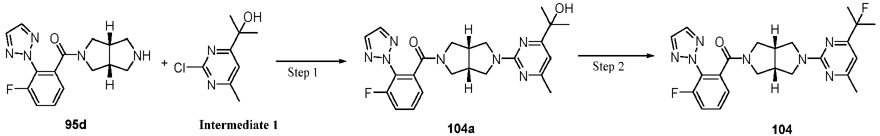

### Step 1

The preparation method of compound 104a was refered to the preparation method of compound 102c.
MS m/z (ESI): 452.2 [M+1]
¹H NMR (400 MHz, DMSO-*d*₆) δ 7.98 (s, 2H), 7.70 - 7.65 (m, 1H), 7.62 - 7.57 (m, 1H), 7.42 (d, *J* = 7.6 Hz, 1H), 6.75 (s, 1H), 5.11 (s, 1H), 3.75 - 3.70 (m, 1H), 3.65 - 3.60 (m, 1H), 3.57 - 3.49 (m, 2H), 3.43 - 3.35 (m, 2H), 3.29 - 3.25 (m, 1H), 3.14 - 3.10 (m, 1H), 2.98 - 2.91 (m, 2H), 2.29 (s, 3H), 1.38 (s, 6H).

### Step 2

The preparation method of compound 104 was refered to the preparation method of compound 102.
MS m/z (ESI): 454.1 [M+1]
¹H NMR (400 MHz, DMSO-*d*₆) δ 7.98 (s, 2H), 7.70 - 7.65 (m, 1H), 7.62 - 7.58 (m, 1H), 7.42 (d, *J* = 7.6 Hz, 1H), 6.63 (d, *J* = 1.6 Hz, 1H), 3.75 - 3.71 (m, 1H), 3.66 - 3.61 (m, 1H), 3.58 - 3.49 (m, 2H), 3.42 - 3.39 (m, 2H), 3.29 - 3.25 (m, 1H), 3.14 - 3.10 (m, 1H), 2.99 - 2.92 (m, 2H), 2.32 (s, 3H), 1.59 (d, *J* = 22.0 Hz, 6H).

### Example 105

### Preparation of compound 105: (4-fluoro-2-(2H-1,2,3-triazol-2-yl)phenyl)((3R,6S)-5-(4-(2-fluoropropan-2-yl)-6-methyl pyrimidin-2-yl)hexahydropyrrolo[3,4-c]pyrrol-2(1H)-yl)methanone

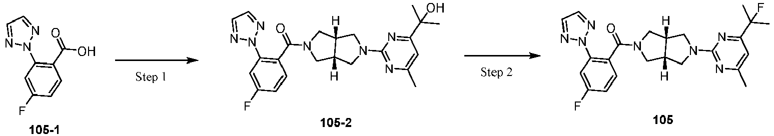

### Step 1

2-(2-((*3R,6S*)-Hexahydropyrrolo[3,4-c]pyrrol-2(1H)-yl)-6-methylpyrimidin-4-yl)pr opan-2-ol (126.64 mg, 0.48 mmol), compound 121-1 (100 mg, 0.48 mmol) and 1-methylimidazole (80.24 mg, 0.96 mmol) were dissolved in MeCN (2 mL), followed by addition of N,N,N',N'-tetramethylchloroformamidine hexafluorophosphate (270.32 mg, 0.98 mmol). The reaction solution was stirred at room temperature for 1 hour. Saturated NaCl (10 ml) was added to the reaction solution, and the solution was extracted with dichloromethane (5 mL*3). The organic phases were combined, dried, and concentrated to obtain a crude product. The crude product was purified by column chromatography (petroleum ether/ethyl acetate system) to obtain the target molecule (4-fluoro-2-(2H-1,2,3-triazol-2-yl)phenyl)((*3R,6S*)-5-(4-(2-hydroxypropan-2-yl)-6-meth ylpyrimidin-2-yl)hexahydropyrrolo[3,4-c]pyrrol-2(1H)-yl)methanone (compound 105-1) (160 mg, 73.4%).
MS m/z (ESI): 452.2 [M+1]

### Step 2

The synthetic method of compound 105 was refered to the synthetic method of step 2 of compound 99, except that compound 105-2 was used as starting material to obtain (4-fluoro-2-(2H-1,2,3-triazol-2-yl)phenyl)((*3R,6S*)-5-(4-(2-fluoropropan-2-yl)-6-methyl pyrimidin-2-yl)hexahydropyrrolo[3,4-c]pyrrol-2(1H)-yl)methanone (compound 105) (45 mg, 32.6%).
MS m/z (ESI): 454.2 [M+1]
¹H NMR (400 MHz, DMSO) δ 8.01 (s, 2H), 7.74 (dd, J = 9.7, 2.4 Hz, 1H), 7.58 (dd, J = 8.5, 6.0 Hz, 1H), 7.40 (td, J = 8.4, 2.5 Hz, 1H), 6.65 (t, J = 19.6 Hz, 1H), 3.84 - 3.44 (m, 6H), 3.35 (d, J = 12.1 Hz, 1H), 2.98 (dd, J = 19.1, 14.1 Hz, 3H), 2.31 (s, 3H), 1.58 (d, J = 22.0 Hz, 6H).

### Example 106

### Preparation of compound 106: ((3aR,6aS)-5-(5-fluoro-4-(2-hydroxypropan-2-yl)-6-methylpyrimidin-2-yl)hexahydropy rrolo[3,4-c]pyrrol-2(1H)-yl)(2-fluoro-6-(pyrimidin-2-yl)phenyl)methanone

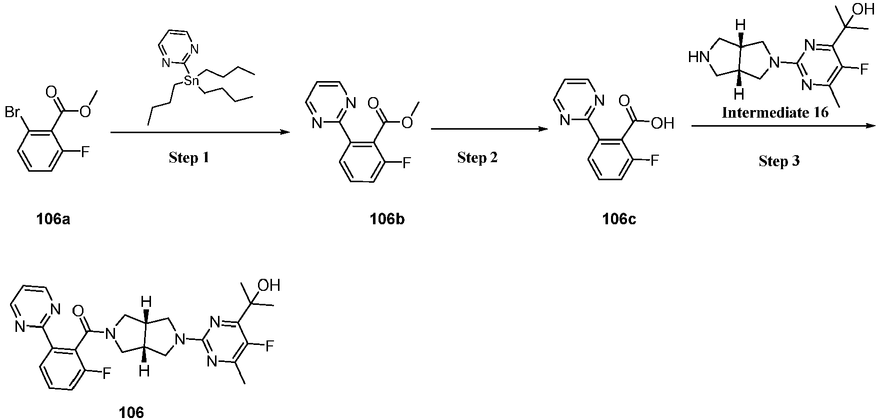

### Step 1

CuI (653.81 mg, 3.43 mmol), Pd(PPh₃)₄ (1.98 g, 1.71 mmol) and CsF (5.21 g, 34.30 mmol) were added to a solution of compound 106a (4 g, 17.16 mmol) and 2-(tributylstannyl)pyrimidine (6.97 g, 18.88 mmol) in DMF (60 mL) under N₂ atmosphere. The mixture was reacted at 120°C for 5 hours. The reaction solution was cooled to room temperature, followed by addition of 150 mL of water and extracting with ethyl acetate (100 mL*2). The organic phases were combined, dried over anhydrous sodium sulfate, filtered and concentrated. The resulting crude product was purified to obtain compound 106b (2.8 g, yield 70.3%).
MS m/z (ESI): 233.1 [M+1]

### Step 2

Sodium hydroxide (688.98 mg, 17.22 mmol) was added to a mixed solution of compound 106b (800 mg, 3.45 mmol) in water (5.0 mL) and methanol (5.0 mL). The mixture was reacted at 25°C for 3 hours. 2 N HCl was added until pH=2, and the solution was extracted with ethyl acetate (20 mL*2). The organic phase was dried over anhydrous sodium sulfate, filtered and concentrated under reduced pressure to obtain compound 106c (650 mg, yield 86.5%).
MS m/z (ESI): 219.0 [M+1]

### Step 3

Tetramethylchlorourea hexafluorophosphate (257 mg, 0.92 mmol) and N-methylimidazole (188 mg, 2.29 mmol) were added to a solution of 106c (100 mg, 0.46 mmol) and intermediate 19 (129 mg, 0.46 mmol) in acetonitrile (3.0 mL). The mixture was stirred at 25°C for 30 minutes. 20 mL of water was added to the reaction solution, and extracted with ethyl acetate (20 mL*2). The organic phases were combined, dried over anhydrous sodium sulfate, filtered and concentrated. The resulting crude product was purified by preparative HPLC to obtain compound 106 (40 mg, yield 18.2%).
MS m/z (ESI): 481.2 [M+1]
¹H NMR (400 MHz, DMSO-d6) δ 8.85 (dd, J = 42.9, 4.9 Hz, 2H), 8.01 (dd, J = 36.2, 7.8 Hz, 1H), 7.59 (td, J = 8.1, 5.8 Hz, 1H), 7.51 - 7.37 (m, 2H), 5.15 (s, 1H), 3.85 - 3.47 (m, 7H), 3.09 (dddd, J = 36.1, 22.9, 11.1, 7.0 Hz, 3H), 2.36 - 2.24 (m, 3H), 1.44 (d, J = 3.3 Hz, 6H).

### Example 107

### Preparation of compound 107: (3-fluoro-2-(2H-1,2,3-triazol-2-yl)phenyl)((3aR,6aS)-5-(4-(2-fluoropropan-2-yl)-6-meth ylpyridin-2-yl)hexahydropyrrolo[3,4-c]pyrrol-2(1H)-yl)methanone

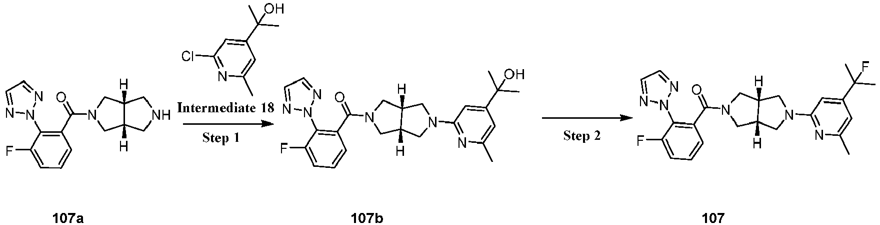

### Step 1

Intermediate 18 (61 mg, 0.33 mmol), 107a (100 mg, 0.33 mmol), Pd₂(dba)₃ (18 mg, 0.02 mmol), BINAP (25 mg, 0.04 mmol), cesium carbonate (215 mg, 0.66 mmol) and dioxane (2 mL) were added to a round-bottomed flask, and the mixture was stirred at 100°C for 12 hours under nitrogen atmosphere. The reaction solution was cooled, quenched by water (5 mL), and extracted with dichloromethane (10 mL*3). The organic phases were combined, dried, and concentrated. The residues were purified by preparative HPLC to obtain compound 107b (75 mg, yield 50.7%).
MS m/z (ESI): 451.2 [M+1]

### Step 2

Compound 107b (75 mg, 0.17 mmol) and DAST (48 mg, 0.30 mmol) were added to DCM (5 mL) at 0°C. The reaction solution was stirred at 25°C for 0.5 hour, followed by addition of water (20 ml). The solution was extracted with dichloromethane (30 mL*2), and the combined extracts were dried over Na₂SO₄, and concentrated under reduced pressure. The resulting crude product was purified by preparative HPLC to obtain compound 107 (50 mg, yield 66.4%).
MS m/z (ESI): 453.2 [M+1]
¹H NMR (400 MHz, DMSO-d6) δ 8.00 (s, 2H), 7.71 - 7.57 (m, 2H), 7.42 (d, J = 7.5 Hz, 1H), 6.48 (s, 1H), 6.22 (s, 1H), 3.71 - 3.41 (m, 5H), 3.30 - 2.91 (m, 5H), 2.33 (s, 3H), 1.63 (d, J = 1.4 Hz, 3H), 1.58 (d, J = 1.5 Hz, 3H).

### Example 108

### Preparation of compound 108: (3, 6-difluoro-2-(2H-1,2,3-triazol-2-yl)phenyl)((3 aR,6aS)-5-(4-(2-fluoropropan-2-yl)-6-methylpyrimidin-2-yl)hexahydropyrrolo[3,4-c]pyrrol-2(1H)-yl)methanone

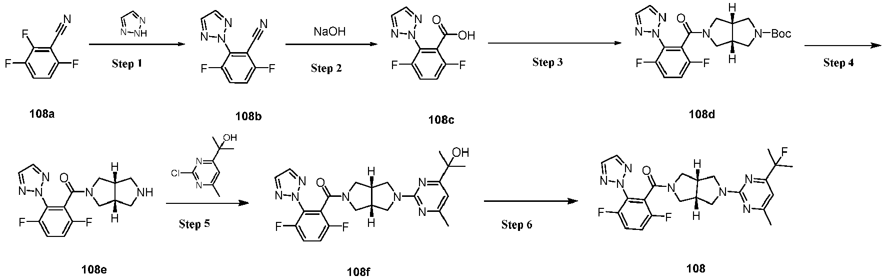

### Step 1

Potassium carbonate (5.28 g, 38.2 mmol) was added to a solution of intermediate 108a (2.0 g, 12.7 mmol) and 2H-1,2,3-triazole (0.88 g, 12.7 mmol) in DMF (30 mL). The mixture was heated to 70°C and stirred for 3 hours. The reaction solution was cooled to room temperature, followed by addition of 150 mL of water and extracted with ethyl acetate (50 mL*3). The organic phases were combined, dried over anhydrous sodium sulfate, filtered and concentrated. The resulting crude product was purified to obtain compound 108b (0.88 g, yield 33.5%).
MS m/z (ESI): 207.0 [M+1]
¹H NMR (400 MHz, DMSO-d6) δ 8.36 (s, 2H), 8.07 (td, *J* = 9.7, 4.9 Hz, 1H), 7.90 - 7.82(m, 1H).

### Step 2

NaOH (1.94 g, 48.5 mmol) was added to a mixed solution of intermediate 108b (1.0 g, 4.85 mmol) in 1,4-dioxane (5.0 mL) and water (15 mL). The mixture was heated to 100°C and stirred for 15 hours. After cooling to room temperature, 2 N HCl was added to the reaction solution until pH 2-3, and the aqueous phase was extracted with dichloromethane (20 mL*2). The organic phases were combined, dried over anhydrous sodium sulfate, filtered and concentrated to obtain compound 108c (0.89 g, yield 81.5%).
MS m/z (ESI): 226.0 [M+1]

### Step 3

The synthetic method of compound 108d was refered to the synthetic method of compound 95c, except that compound 95b was replaced with compound 108c to obtain Compound 108d (1.30 g, yield 81.2%).
MS m/z (ESI): 420.2 [M+1]

### Step 4

The synthetic method of compound 108e was refered to the synthetic method of compound 95d, except that compound 95c was replaced with compound 108d to obtain Compound 108e (0.98 g, yield 98.9%).
MS m/z (ESI): 320.1 [M+1]

### Step 5

The synthetic method of compound 108f was refered to the synthetic method of compound 107b, except that compound 107a was replaced with compound 108e to obtain Compound 108f (1.20 g, yield 82.7%).
MS m/z (ESI): 470.2 [M+1]

### Step 6

At 0°C, DAST (93 mg, 0.577 mmol) was added dropwise to a solution of compound 108f (180 mg, 0.383 mmol) in dichloromethane (5.0 mL). The reaction solution was kept at 0°C and reacted for 15 minutes. The reaction solution was slowly poured into 20 mL of water, and extracted with dichloromethane (20 mL*2). The organic phases were combined, dried over anhydrous sodium sulfate, filtered and concentrated. The resulting crude product was purified by preparative HPLC to obtain compound 108 (88 mg, yield 48.7%).
MS m/z (ESI): 472.2 [M+1]
¹H NMR (400 MHz, DMSO-d6) δ 8.18 (s, 1H), 7.94 (s, 1H), 7.76 - 7.57 (m, 2H), 6.64 (dd, *J* = 4.3, 1.9 Hz, 1H), 3.80 - 3.42 (m, 6H), 3.32 - 2.95 (m, 4H), 2.32 (d, *J* = 11.9 Hz, 3H), 1.69 - 1.52 (m, 6H).

### Example 109

### Preparation of compound 109: ((3aR,6aS)-5-(4,6-dimethylpyrimidin-2-yl)-3,3a,4,6a-tetrahydrocyclopenta[c]pyrrol-2(1 H)-yl)(2-fluoro-6-(pyrimidin-2-yl)phenyl)methanone

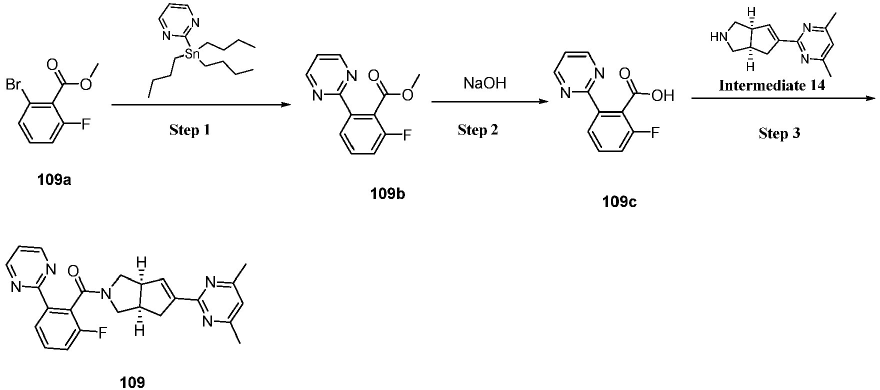

### Step 1

Cuprous iodide (653.81 mg, 3.43 mmol), Pd(PPh₃)₄ (1.98 g, 1.71 mmol) and CsF (5.21 g, 34.30 mmol) were added to a solution of compound 109a (4 g, 17.16 mmol) and 2-(tributylstannyl)pyrimidine (6.97 g, 18.88 mmol) in DMF (60 mL) under N₂ atmosphere. The reaction solution was reacted at 115°C for 5 hours. The reaction solution was cooled to room temperature, followed by addition of 150 mL of water and extracting with ethyl acetate (100 mL*2). The organic phases were combined, dried over anhydrous sodium sulfate, filtered and concentrated. The resulting crude product was purified to obtain compound 109b (2.8 g, yield 70.3%).
MS m/z (ESI): 233.1 [M+1]

### Step 2

Sodium hydroxide (688.98 mg, 17.22 mmol) was added to a mixed solution of compound 109b (800 mg, 3.45 mmol) in water (5.0 mL) and methanol (5.0 mL). The mixture was reacted at 25°C for 3 hours. 2 N HCl was added until pH=2, and the solution was extracted with ethyl acetate (20 mL*2). The organic phase was dried over anhydrous sodium sulfate, filtered and concentrated under reduced pressure to obtain compound 109c (650 mg, yield 86.5%).
MS m/z (ESI): 219.0 [M+1]

### Step 3

Tetramethylchlorourea hexafluorophosphate (257 mg, 0.92 mmol) and N-methylimidazole (188 mg, 2.29 mmol) were added to a solution of compound 109c (100 mg, 0.46 mmol) and intermediate 14 (132 mg, 0.61 mmol) in acetonitrile (3.0 mL). The mixture was stirred at 25°C for 30 minutes. 20 mL of water was added to the reaction solution, and extracted with ethyl acetate (20 mL*2). The organic phases were combined, dried over anhydrous sodium sulfate, filtered and concentrated. The resulting crude product was purified by preparative HPLC to obtain compound 109 (20 mg, yield 10.5%).
MS m/z (ESI): 416.2 [M+1]
¹H NMR (400 MHz, DMSO-d6) δ 8.90 (d, *J* = 4.9 Hz, 1H), 8.69 (dd, *J* = 24.8, 4.9 Hz, 1H), 8.12 - 7.90 (m, 1H), 7.67 - 7.37 (m, 2H), 7.30 - 7.02 (m, 2H), 6.86 - 6.65 (m, 1H), 3.95 - 3.47 (m, 4H), 3.23 - 2.55 (m, 4H), 2.49 - 2.34 (m, 6H).

### Example 110

### Preparation of compound 110: ((3aR,6aS)-5-(5-fluoro-6-(2-hydroxypropan-2-yl)-4-methylpyridin-2-yl)hexahydropyrro lo[3,4-c]pyrrol-2(1H)-yl)(2-fluoro-6-(pyrimidin-2-yl)phenyl)methanone

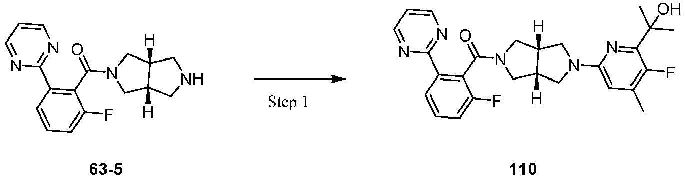

The synthetic method of compound 110 was refered to the synthetic method of compound 60, except that compound 63-5 and intermediate 15 were used as starting materials to obtain Compound 110 (45 mg, 32.6%).
MS: m/z (ESI): 480.2 [M+1]

### Example 111

### Preparation of compound 111: (2-fluoro-6-(2H-1,2,3-triazol-2-yl)phenyl)((cis)-5-(4-(1-fluorocyclopropyl)-6-methylpyr imidin-2-yl)hexahydropyrrolo[3,4-c]pyrrol-2(1H)-yl)methanone

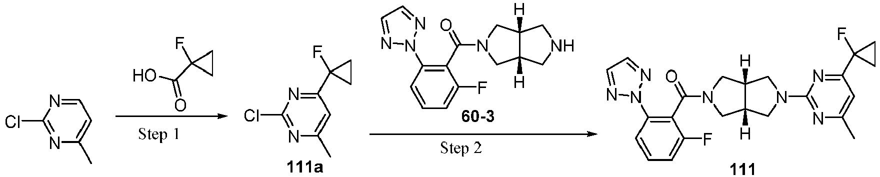

### Step 1

2-Chloro-4-methylpyrimidine (300 mg, 2.33 mmol) was dissolved in acetonitrile (5 mL), followed by addition of 1-fluorocyclopropanecarboxylic acid (485 mg, 4.66 mmol). The solution was heated to 80°C under nitrogen atmosphere, followed by addition of 1 mL of aqueous silver nitrate (792 mg, 4.66 mmol) solution at one time. 4 mL of aqueous ammonium persulfate (1.06 g, 4.65 mmol) solution was slowly added dropwise over about 1 hour, and the reaction solution was reacted at 80°C for 3 hours. The reaction solution was cooled to room temperature, and poured into 50 mL of water. 5 mL of ammonia was added, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain compound 111a (150 mg, yield 34.4%).
MS m/z (ESI): 187.0 [M+1]
¹H NMR (400 MHz, DMSO-*d*₆) δ 7.63 (s, 1H), 2.52 (s, 3H), 1.70 - 7.62 (m, 2H), 1.43 - 1.38 (m, 2H).

### Step 2

Compound 60-3 (100 mg, 0.332 mmol) was dissolved in N,N-dimethylformamide (3 mL), followed by addition of compound 111a (68 mg, 0.364 mmol) and cesium carbonate (216 mg, 0.663 mmol). The mixture was heated to 110°C for 1 hour. The reaction solution was cooled to room temperature, poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure, and the residues were purified by reverse preparative HPLC (acetonitrile/water system) to obtain compound 111 (122.5 mg, yield 81.8%).
MS m/z (ESI): 452.2 [M+1]
¹H NMR (400 MHz, DMSO-*d*6) δ 8.15 (s, 1H), 7.95 (s, 1H), 7.82 - 7.77 (m, 1H), 7.69 - 7.63 (m, 1H), 7.47 - 7.40 (m, 1H), 6.72 (s, 1H), 3.78 - 3.64 (m, 2H), 3.55 - 3.40 (m, 4H), 3.26 - 3.20 (m, 1H), 3.15 - 2.92 (m, 3H), 2.31 (s, 3H), 1.48 - 1.24 (m, 4H).

### Example 112

### Preparation of compound 112: (2-fluoro-6-(2H-1,2,3-triazol-2-yl)phenyl)((cis)-5-(4-methyl-6-(1,1,1-trifluoro-2-hydrox ypropan-2-yl)pyrimidin-2-yl)hexahydropyrrolo[3,4-c]pyrrol-2(1H)-yl)methanone

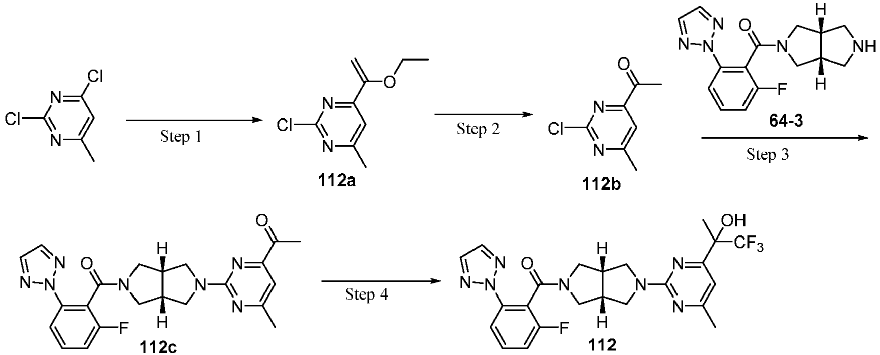

### Step 1

2,4-Dichloro-6-methylpyrimidine (2.0 g, 12.3 mmol) was dissolved in N,N-dimethylformamide (30 mL), followed by addition of tributyl(1-ethoxyvinyl)stannane (4.43 g, 12.3 mmol) and bistriphenylphosphine palladium dichloride (215 mg, 0.306 mmol). The mixture was purged with nitrogen three times, heated to 100°C and reacted for 4 hours. The reaction solution was cooled to room temperature, poured into 100 mL of water, precipitated with potassium fluoride, filtered and washed with ethyl acetate (80 mL). The mixture was partitioned, and the aqueous phase was extracted with ethyl acetate (50 mL). The organic phases were combined, washed with water (50 mL) and saturated sodium chloride solution (50 mL) successively, dried over anhydrous sodium sulfate and filtered. The filtrate was concentrated under reduced pressure, and the residues were purified by silica gel column chromatography (petroleum ether/ethyl acetate system) to obtain compound 112a (2.0 g, yield 82.1 %).
MS m/z (ESI): 199.1 [M+1]
¹H NMR (400 MHz, DMSO-*d*₆) δ 7.55 (s, 1H), 5.53 (d, *J* = 2.0 Hz, 1H), 4.71 (d, *J* = 2.0 Hz, 1H), 3.97 (q, *J* = 7.2 Hz, 2H), 2.51 (s, 3H), 1.37 (t, *J* = 7.2 Hz, 3H).

### Step 2

Compound 112a (800 mg, 4.03 mmol) was dissolved in anhydrous tetrahydrofuran (15 mL), followed by addition of hydrochloric acid (2 M, 5 mL). The mixture was reacted at room temperature for 2 hours. The reaction solution was poured into 50 mL of water, and extracted with ethyl acetate (50 mL*2). The organic phases were combined, washed with water (50 mL) and saturated sodium chloride solution (50 mL) successively, dried over anhydrous sodium sulfate and filtered. The filtrate was concentrated under reduced pressure to obtain the crude product compound 112b (600 mg, yield 87.3 %).
MS m/z (ESI): 171.0 [M+1]
¹H NMR (400 MHz, DMSO-*d*₆) δ 7.84 (s, 1H) , 2.60 (s, 3H), 2.59 (s, 3H).

### Step 3

Compound 64-3 (150 mg, 0.498 mmol) was dissolved in 1,4-dioxane (3 mL), followed by addition of compound 112b (102 mg, 0.598 mmol) and cesium carbonate (324 mg, 0.994 mmol). The mixture was heated to 100°C for 2 hours. The reaction solution was cooled to room temperature, poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by silica gel column chromatography (petroleum ether/ethyl acetate system) to obtain compound 112c (100 mg, yield 46.1%).
MS m/z (ESI): 436.2 [M+1]

### Step 4

Compound 112c (80 mg, 0.184 mmol) was dissolved in anhydrous tetrahydrofuran (3 mL), followed by addition of cesium fluoride (28 mg, 0.184 mmol). The solution was cooled to 0°C, followed by slowly adding (trifluoromethyl)trimethylsilane (131 mg, 0.921 mmol) dropwise. The reaction solution was warmed up to room temperature and reacted for 1 hour. The reaction solution was poured into 50 mL of water, and extracted with ethyl acetate (30 mL*2). The organic phases were combined, washed with water (30 mL) and saturated sodium chloride solution (30 mL) successively, dried over anhydrous sodium sulfate and filtered. The filtrate was concentrated under reduced pressure, and the residues were purified by reverse preparative HPLC (acetonitrile/water system) to obtain compound 112 (54.4 mg, yield 58.6%).
MS m/z (ESI): 506.2 [M+1]
¹H NMR (400 MHz, DMSO-*d*₆) δ 8.16 (s, 1H), 7.92 (s, 1H), 7.83 - 7.77 (m, 1H), 7.69 - 7.64 (m, 1H), 7.48 - 7.40 (m, 1H), 6.85 (d, *J* = 4.0 Hz, 1H), 6.58 (s, 1H), 3.80 - 3.69 (m, 2H), 3.59 - 3.42 (m, 4H), 3.37 - 3.34 (m, 1H), 3.11 - 2.97 (m, 3H), 2.32 (s, 3H), 1.61 (s, 3H).

### Biological Assay and Evaluation

The present invention is further illustrated below in combination with the following test examples, which are not intended to limit the scope of the present invention.

### I. Cell function experiment

Test Example 1. Determination of the effects of compound of the present invention on calcium flux in cells stably expressing OX1 and OX2 receptors
1. Experimental objective
   To determine the inhibitory effect of the compounds on calcium flux in CHO-K1/human OX1R and CHO-K1/human OX2R cells.
2. Experimental instruments and reagents
   2.1 Instruments
      384-well cell culture plate (Corning: 3764);
      Plate reader FLIPR Tetra (Molecular Device).
   2.2 Reagents
      DMEM, high glucose (Gibco: 12100);
      Fetal bovine serum (Biosera: FB-1058/500);
      P/S (Biosera: XC-A4122);
      5X Matrigel (Corning: 354230);
      HBSS (Sigma: H1387);
      HEPES (Invitrogen: 15630080);
      Fluo-8 AM (AAT Bioquest: 21080);
      Probenecid (Sigma: P8761);
      Pluronic F-127 (Sigma: P2443-250G);
      1000X Fluo-8 AM (2 mM): Fluo-8 AM was dissolved in DMSO, shaked for 1 to 2 minutes, aliquoted and stored at -20°C;
      Complete medium: DMEM + 10% FBS + 1X P/S;
      Cell inoculation medium: DMEM + 10% FBS + 1X PS;
      Assay buffer: 1X HBSS + 20mM HEPES +1 mM Probenecid + 0.025% Pluronic F-127;
      1X Matrigel: 5X Matrigel was diluted with DMEM;
      Cell lines: CHO-K1/human OX1R and CHO-K1/human OX2R cell lines.
3. Experimental process
   1) CHO-K1/human OX1R and CHO-K1/human OX2R cell lines were cultured separately in complete medium at 37°C, 5% CO₂ to 70%-90% confluence;
   2) A 384-well cell culture plate was coated with 1X Matrigel (5uL per well) at room temperature for 10-30 minutes;
   3) The cells were digested and resuspended in cell inoculation medium. CHO-K1/human OX1R and CHO-K1/human OX2R cell lines were inoculated into two 384-well cell culture plates respectively (8,000 cells/well/20 µL). The culture plates were incubated at 37°C, 5% CO₂ for 24 hours;
   4) The cell culture plates were taken out of the CO₂ incubator and equilibrated at room temperature for 10 minutes;
   5) 1000X Fluo-8 AM was diluted with assay buffer 1 equilibrated to room temperature to obtain 1X Fluo-8 AM at a concentration of 2 µM;
   6) The culture medium in the cell culture plate was removed, and 20 µL of 1X Fluo-8 AM was added to each well. The plates were centrifuged at room temperature at 300 rpm for 60 seconds, and incubated at room temperature in the dark for 1 hour;
   7) Determination of EC80 of OX1 and OX2 receptors agonists (OX-A for OX1R assays, OX-B for OX2R assays): OX1 and OX2 receptors agonists working solutions were diluted and prepared respectively. The diluted OX1 and OX2 receptors agonists were added using FLIPR Tetra to the experimental wells of the 384-well cell culture plates corresponding to CHO-K1/human OX1R and CHO-K1/human OX2R cell lines. The data were collected, and EC80 of OX-A and OX-B was obtained according to the following experimental data processing method;
   8) Steps 1 to 7 were repeated. Positive compounds (suvorexant and seltorexant) and test compounds were formulated. Diluted compounds (11 concentration points) were added to corresponding experimental wells of two 384-well cell culture plates using FLIPR Tetra. After 15 minutes, OX1 and OX2 receptors agonists were added to the plates of the two cell lines respectively according to the previously obtained EC80. The data were collected, and the IC50 of the positive compounds and the test compounds was determined.
4. Experimental data processing method
   Fluorescence signal value (RFU) was collected with FLIPR Tetra. The maximum RFU value was used to calculate the percentage inhibition (activation) based on the readings of the Low control (DMSO control) and High control (100 nM positive compound) experimental groups {% inhibition (activation) rate = (RFUsample - RFUlow control) / (RFUhigh control - RFUlow control)×100}. IC50 values of compounds were calculated using Prism8 to fit percent inhibition (activation) rates and 11-point concentration data to parametric nonlinear logic formulas.
5. Experimental results:

| Compound No. | OX1R IC₅₀ (nM) | OX2R IC₅₀ (nM) | Selectivity multiple |
|---|---|---|---|
| Seltorexant | 725 | 26.8 | 27 |
| 3 | 3699 | 44.4 | 83 |
| 43 | 10193 | 99.9 | 102 |
| 45 | 4329 | 52.2 | 83 |
| 48 | 24403 | 74.5 | 328 |
| 50 | 5840 | 71.1 | 82 |
| 56 | 3321 | 8.2 | 405 |
| 57 | 3049 | 9.7 | 314 |
| 60 | 13470 | 55.2 | 244 |
| 85 | 13714 | 58.5 | 234 |
| 91 | 3160 | 35.9 | 88 |
| 93 | 1062 | 8.7 | 122 |
| 94 | 1204 | 25.2 | 48 |
| 95 | 1324 | 27.0 | 49 |
| 97 | 263 | 7.26 | 36 |

The compounds of the present invention show a good inhibitory effect on calcium flux in cells stably expressing OX1 and OX2 receptors. Besides, the inhibitory effect of the compounds on the OX2 receptor is significantly better than that on the OX1 receptor, indicating good selectivity.

### II. Pharmacokinetic assay

### Pharmacokinetic assay in rats

### 1. Study obj ective

SD rats were used as test animals. The pharmacokinetic behavior of the compounds of the present invention was studied in rat body (plasma) by orally administration at a dose of 5 mg/kg.

### 2. Experimental protocol

### 2.1 Test compounds

Compounds of the Examples of the present invention, prepared by the applicant.

### 2.2 Test animals

Male SD rats (3 rats per group), purchased from Shanghai Jiesijie Laboratory Animal Co., LTD, with Certificate No.: SCXK (Shanghai) 2013-0006 N0.311620400001794.

### 2.3 Formulation of the compound

Formulation of the compound for oral administration: 20%HP-β-CD in Water
20 g of HP-β-CD powder was weighed, dissolved in 100 mL of purified water, mixed well by vortex, and sonicated to obtain a clear solution.

The compound of the Example was weighed, and added into a 20 mL glass flask. The solution was added, and sonicated for 10 minutes to obtain a colorless clear solution with a concentration of 0.5 mg/mL.

### 2.4 Administration

After an overnight fast, 3 male SD rats were administered p.o. with the test compound;
The dose of p.o. was 5 mg/kg, and the administration volume was 10 mL/kg.

### 2.5 Sample collection

Blood collection: 0.2 mL of blood was taken from the jugular vein of the rat before administration and at 0.25, 0.5, 1, 2, 4, 6, 8, 24 hours after administration. The samples were stored in EDTA-K₂ anticoagulant tubes, and centrifuged for 6 minutes at 4°C, 6000 rpm to separate the plasma. The plasma samples were stored at -80°C. The rat was fed 4 hours after the administration.

Brain tissue collection: After the experimental animals were euthanized by CO₂, brain tissues were collected at 0.25 h, 0.5 h, 1 h, 2 h, 4 h, 6 h, 8 h, 24 h after the administration. The brain tissue was rinsed with pre-cooled PBS, dried, weighed, and stored at -80°C.

### 2.6 Sample process

1) 160 µL of acetonitrile was added to 40 µL of the plasma sample for precipitation, and then the mixture was centrifuged at 3500 × g for 5 to 20 minutes.
2) The resulting supernatant was taken to analyze the concentration of the test compound by LC/MS/MS. LC/MS/MS analytical instrument: AB Sciex API 4000 Qtrap.

### 2.7 Liquid chromatography analysis

• Liquid chromatography condition: Shimadzu LC-20AD pump
• Chromatographic column: Agilent ZORBAX XDB-C18 (50×2.1 mm, 3.5 µm); mobile phase: eluent A was 0.1% aqueous formic acid solution, eluent B was acetonitrile
• Flow rate: 0.4 mL/min
• Elution time: 0-4.0 minutes, the eluent is as follows:

| Time/minute | Eluent A | Eluent B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

### 3. Experimental results and analysis

The main parameters of pharmacokinetics were calculated by WinNonlin 6.1. The results of pharmacokinetic test in rats are shown below

| No. | Tₘₐₓ (h) | AUC_{0-∞} (ng/mL*h) | Cₘₐₓ (ng/mL) |
|---|---|---|---|
| | | Brain | Brain |
| Seltorexant | 0.25 | 79 | 130 |
| Example 57 | 0.5 | 279 | 110 |
| Example 93 | 0.6 | 130 | 124 |
| Example 95 | 0.25 | 821 | 394 |
| Example 97 | 0.5 | 335 | 420 |

### 4. Experimental conclusion

The data show that in the pharmacokinetic assay in rats, the compounds of the Examples of the present invention show a high exposure after oral administration, especially a high exposure in the brain.

### III. Evaluation of the effect of the compound of the present invention on the sleep structure of rats by telemetry electroencephalography and electromyography sleep techniques

### 1. Experimental objective

This experiment evaluated the effect of the compound of the present invention on the sleep structure of SD rats using the telemetry electroencephalography and electromyography system of DSI, Inc.

### 2. Experimental instruments and reagents

### 2.1. Main instruments

DSI telemetry pressure measurement system: implant model: TL11M2-F40-EET, Data Sciences International. HD-S02, Data Sciences International.
Data acquisition software: Ponemah Software 5.0, Data Sciences International.
Data analysis software: NeuroScore, Data Sciences International.
Precision electronic balance: Sartorius AG, Germany, batch number 35292154
Electronic balance: Changzhou Keyuan Electronic Instrument Co., Ltd., (Su) No. 00000409
Ultrasonic instrument: Kunshan Ultrasonic Instrument Co., Ltd., KQ3200DE
Constant temperature magnetic stirrer: Shanghai Sile Instrument Co., Ltd., type 85-2
Vortex mixer: Shanghai Kanghe Optoelectronics Instrument Co., Ltd., H-101

### 2.2. Main reagents

HP-β-CD

### 3. Experimental process

### 3.1. Purchase and adaptation of the test animals

SD rats, weighed 190-210 g (5-6W) when they arrived at the facility. After the animals arrived at the facility, they were housed in an animal enclosure with strictly controlled environmental conditions for an adaptation period of 7-9 days. During the adaptation period, the test animals were placed in a 12h light/dark environment to adapt to the rhythm time, and the health status of the animals was monitored every day.

### 3.2. Grouping

At the facility, all animals were grouped by body weight, adaptively housed, and implanted with electrodes by surgery. The number of rats was at least 6 rats in each group, which met the requirements of statistical testing and pharmacodynamic guidelines.

### 3.3. Experimental procedure

1) Implantation of electrodes by surgery: The test animals were placed in a 12h light/dark environment for an adaptation period of 7-9 days (lights off: 07:00; lights on: 19:00). On the day of the experiment, the animal was anesthetized with Zoletil (i.p., 20 mg/kg) combined with Xylazine (i.p., 8 mg/kg). After anesthesia, the animal was fixed by brain stereotaxic instrument. The skin was prepared at the surgical area of the head. The skull was drilled, and the electrodes were implanted. At the same time, two electromyography electrodes were respectively inserted into the neck muscles in parallel, and the two ends were fixed with sutures to prevent their ends from touching each other. Then, the implant is placed subcutaneously on the back, and the surgical wound is sutured and sterilized.
2) Postoperative care: After surgery, the rats were carefully placed in a clean recovery cage, and placed in a lateral position to ensure unobstructed airway. 12h automatic alternation of light/dark (lights off: 07:00, lights on: 19:00), constant temperature 20-26°C, relative humidity 40-70%. The animals were subjected to 3 days of care after surgery. The surgical incision was treated with cephradine powder locally, 4-8 mg/kg of gentamicin was subcutaneously administered, and 0.1 ml of meloxicam was subcutaneously injected to each animal for 3 consecutive days. The experiment was performed after 7-10 days of recovery, and the animals were randomly grouped according to body weight.
3) Administration regimen and parameters monitoring: 7-10 days after surgery, basic electroencephalography and electromyography recordings were performed. After the basic electroencephalography and electromyography recordings, the administration was conducted for 7 consecutive days. On day 1 and day 7 of the administration, at 11:00, the electroencephalography and electromyography recordings were conducted 1 hour before the administration. The administration was conducted at 12:00, and the electroencephalography and electromyography recordings were conducted within 24 hours after the administration.
4) Experimental parameters: Latency duration changes of NREM and REM between different groups after the administration; duration changes of Wake, NREM and REM within 24 hours after the administration.

### 4. Data collection and analysis

Raw data were collected by DSI system Ponemah software, and analyzed by NeuroScore software and GraphPad Prism software. The experimental data were represented by mean ± standard error (Mean ± SEM), and analyzed by ANOVA, with P < 0.05 indicating a significant difference.

### 5. Experimental results

The waking/sleeping state distribution of the animals in the vehicle control group was in line with the circadian rhythm. The solvent has no significant effect on the sleep structure of the animals. The distribution of different waking/sleep stages conformed to the situation reported in the literature. The model was successful and stable.

The different sleep stages of each animal in the vehicle group and the administration group at 1 hour before the administration and after the administration were analyzed. The average value of each group was calculated in units of 1 h, and the distribution of different sleep stages duration of each group was analyzed.

At the light off stage, the total duration in different sleep stages of the animals at 1 hour, 3 hour, 5 hour, 7 hour after the administration was measured to analyze the efficacy among different administration groups.

At the light on stage, the total duration in different sleep stages of the animals at 1 hour, 3 hour, 5 hour, 7 hour after lighting on was measured to analyze the efficacy among different administration groups.

### 6. Experimental conclusion

The compounds of the Examples of the present invention can significantly reduce the NREM/REM latency and total awake duration at a low dose. The compounds can significantly increase the total NREM duration, and has no effect on the total REM duration, indicating a low risk of drowsiness. It can be seen that the compound of the present invention has a good sleep-promoting effect on rats, and low risk of drowsiness.

### IV. CYP enzyme single point inhibition assay

### 1. Experimental objective

The human liver microsome incubation system was used to rapidly predict the inhibitory effect of the compounds on CYP450 enzyme subtypes by using single-point method.

### 2. Experimental process

### 2.1 Formulation of solution

NADPH (reduced nicotinamide adenine dinucleotide phosphate) was weighed, to which 100 mM phosphate buffer was added to obtain a final concentration of 2.5 mM. 4 mL of 100 mM phosphate buffer was added to 50 µL of 20 mg/mL microsomes, and mixed well to obtain 0.25 mg/mL microsomes.

Formulation of test compound reaction solution:
The compound of the example to be tested was weighed, diluted to 10 mM with DMSO, and then diluted to 100 µM with 100 mM phosphate buffer.

### 2.2 Experimental procedure

1. 40 µL of liver microsomes, 10 µL of substrate, and 10 µL of the test compound were added to a 96-well plate, and pre-incubated for 3 minutes.
2. 40 µL of NADPH was added.
3. 300 µL of acetonitrile stop solution containing an internal standard was added at 20 minutes.
4. The sample was injected by centrifuge.

### 3. Experimental results:

| **Compounds** | **IC₅₀ (µM)** | | | |
|---|---|---|---|---|
| | **1A2** | **2C9** | **2C19** | **2D6** |
| Seltorexant | 46.3 | >100 | 9.1 | 61.6 |
| 60 | >100 | / | / | / |
| 93 | >100 | >100 | 82.7 | >100 |
| 95 | >100 | >100 | 82.7 | >100 |

| | | | | |
|---|---|---|---|---|
| Note: "j" means not tested. | | | | |

## Claims

1. A compound, a stereoisomer thereof or a pharmaceutically acceptable salt thereof, **characterized in that** the compound is represented by any of the following formulas: 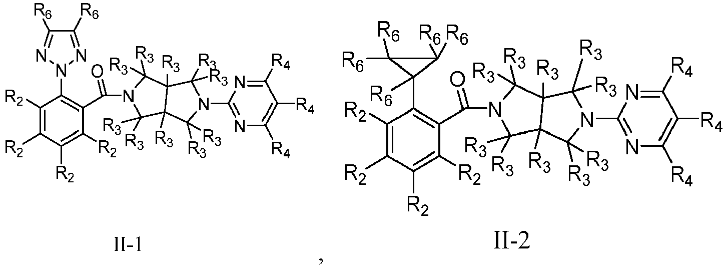 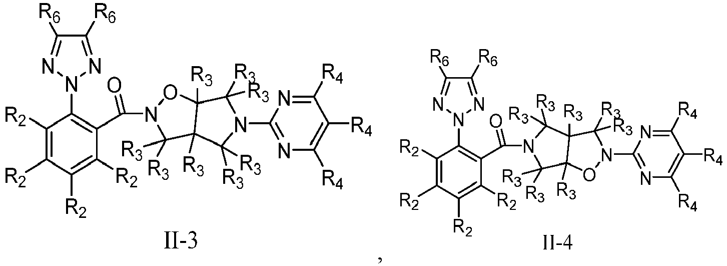 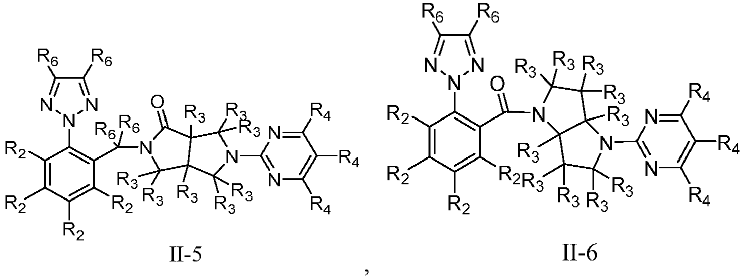 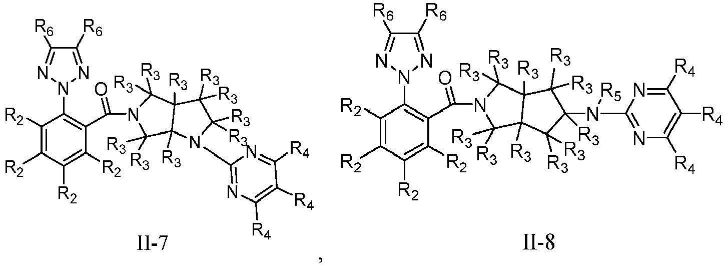 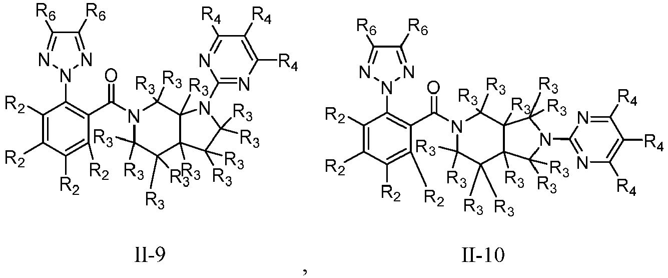 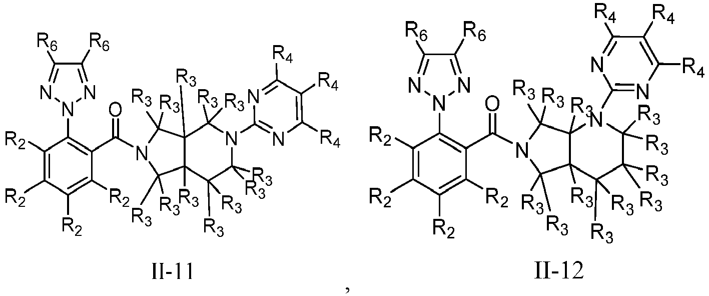 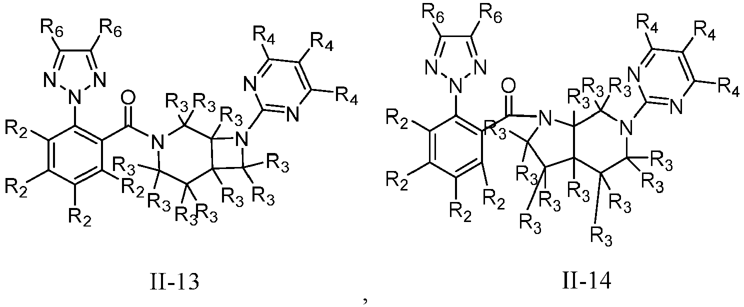 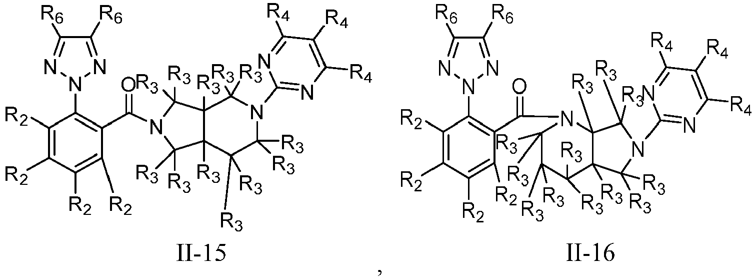 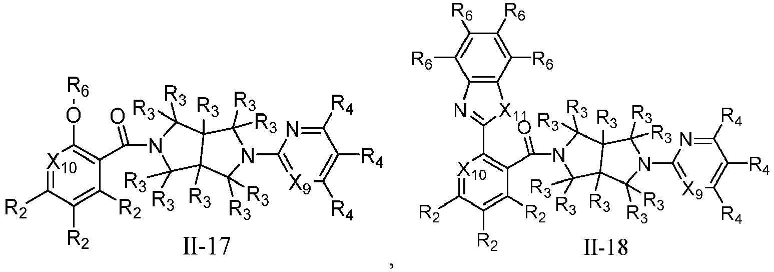 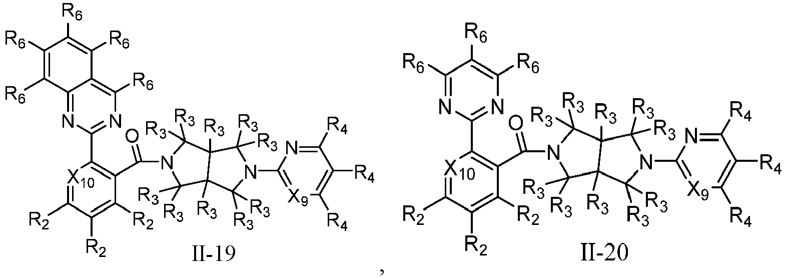 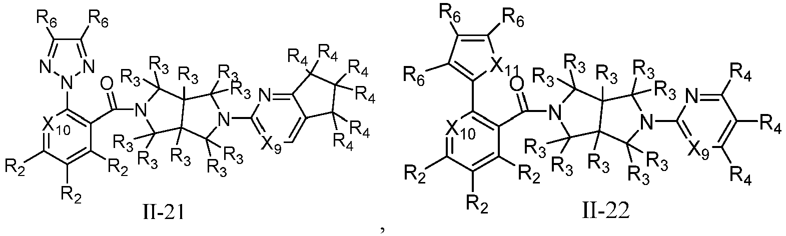 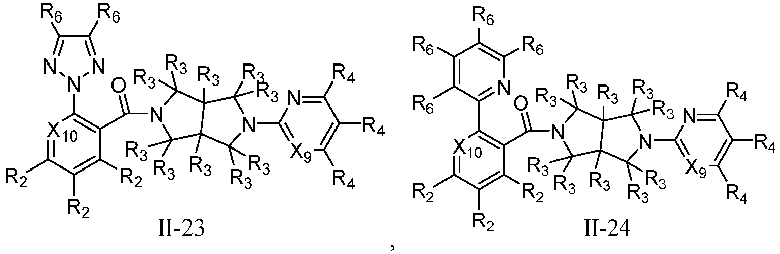 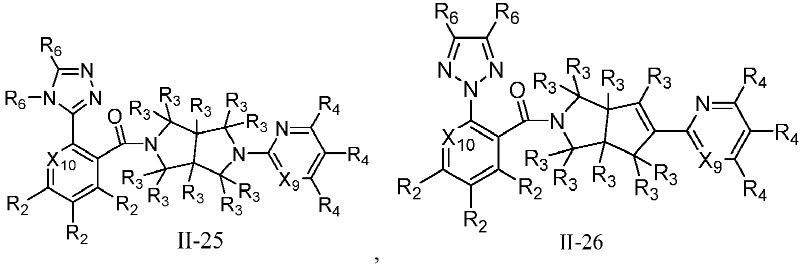 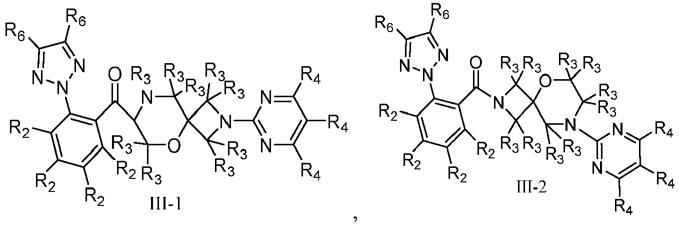 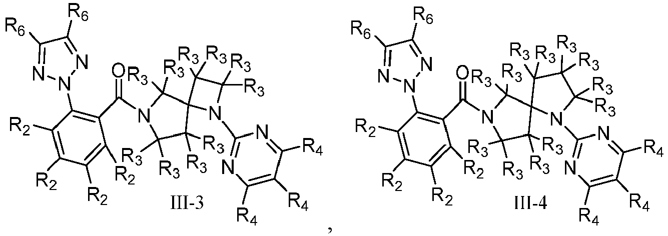 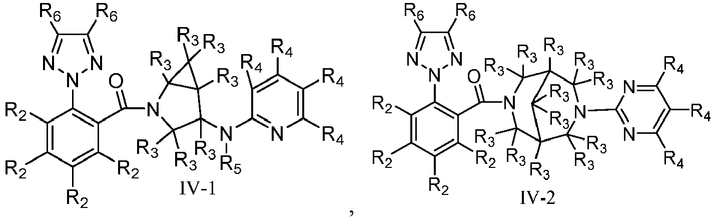 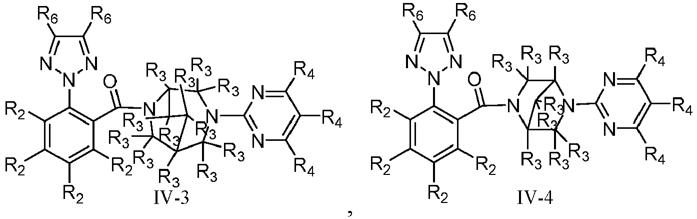 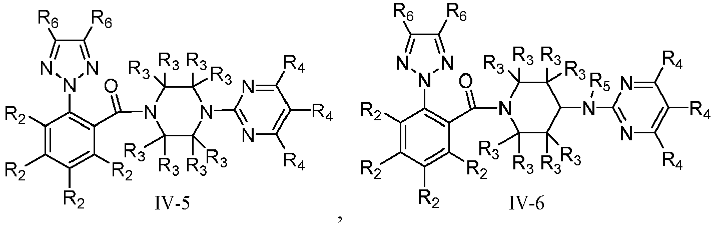 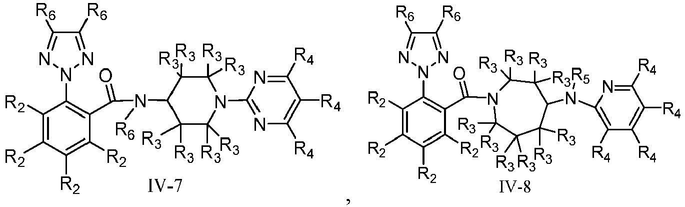 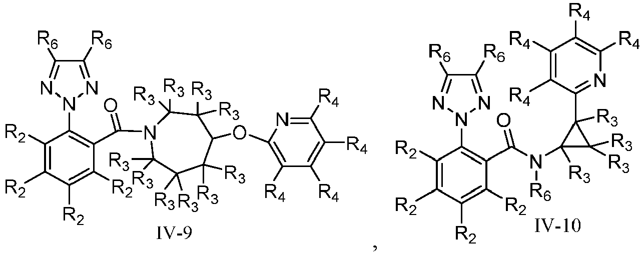 or 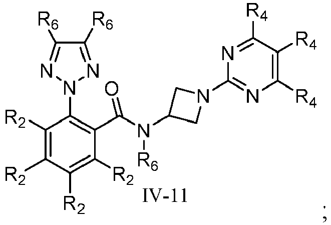
X₉ is CR₄ or N;
X₁₀ is CR₂ or N;
X₁₁ is O or S;
R₂, R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₂, R₃, R₄ can be connected to form a cycloalkyl, heterocyclyl, aryl or heteroaryl, the cycloalkyl, heterocyclyl, aryl or heteroaryl can be optionally further substituted;
preferably R₂, R₃, R₄ are each independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
each R₆ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
preferably each R₆ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

2. The compound, a stereoisomer thereof or a pharmaceutically acceptable salt thereof according to claim 1, **characterized in that** the compound is further represented by the following formulas:

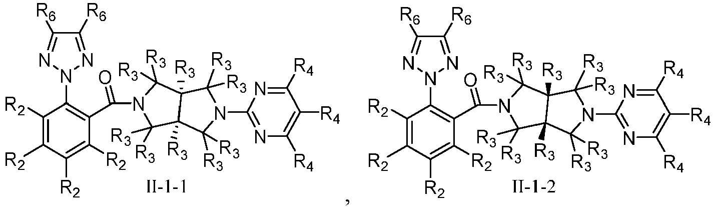

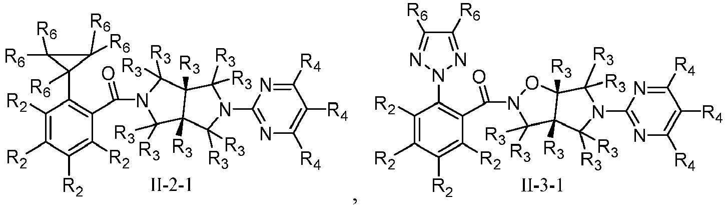

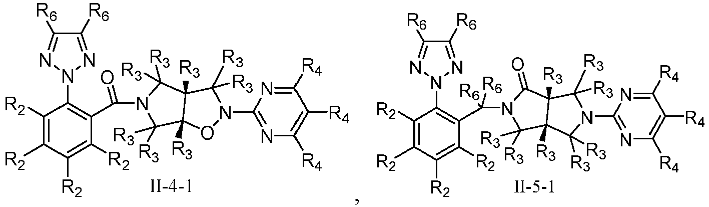

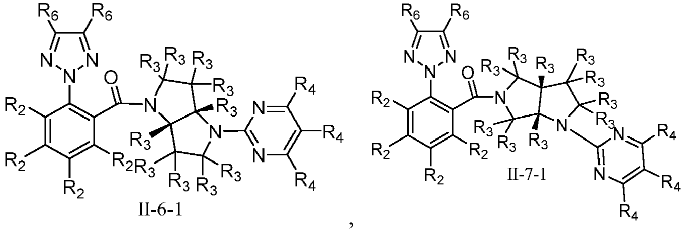

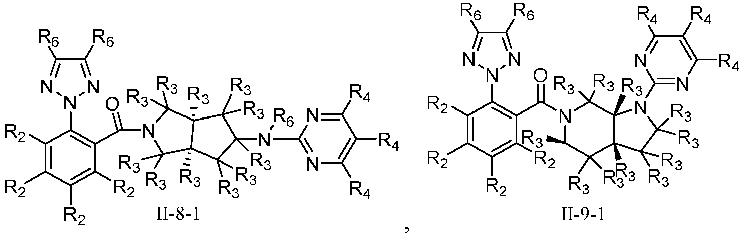

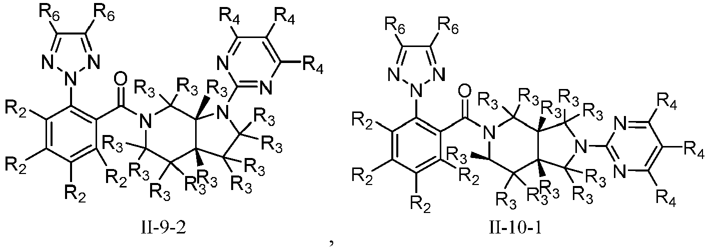

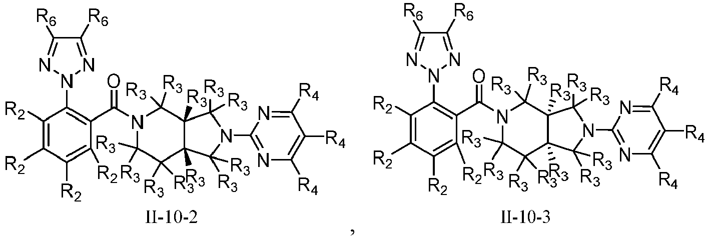

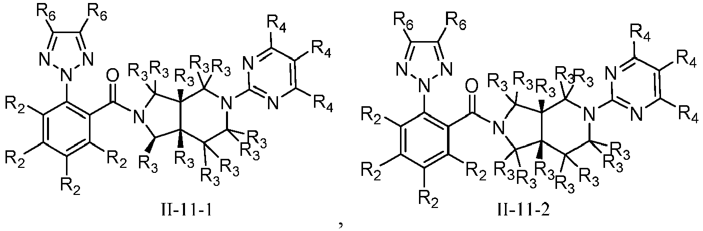

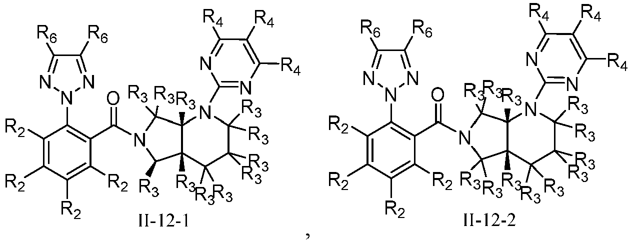

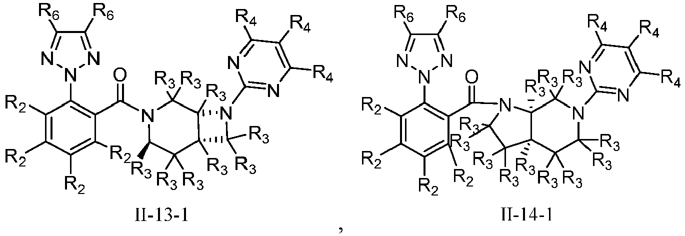

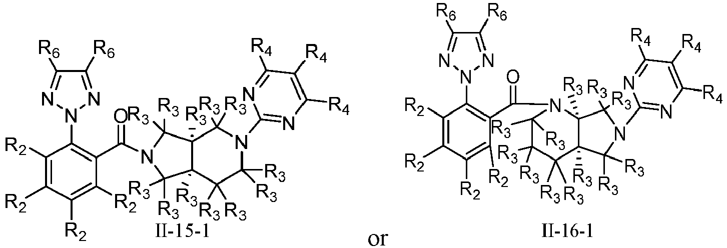

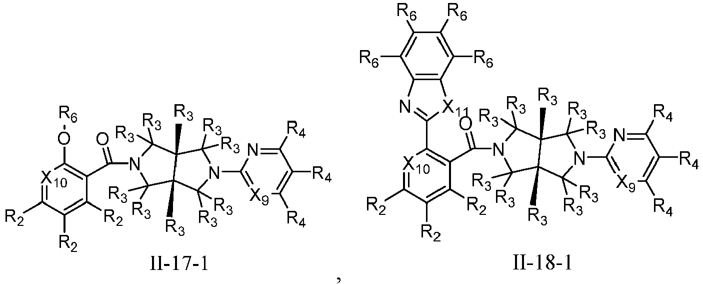

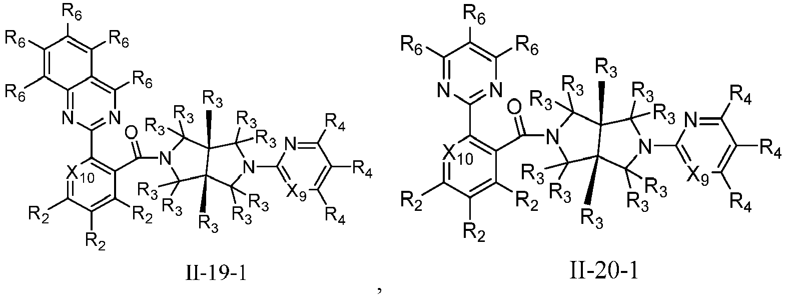

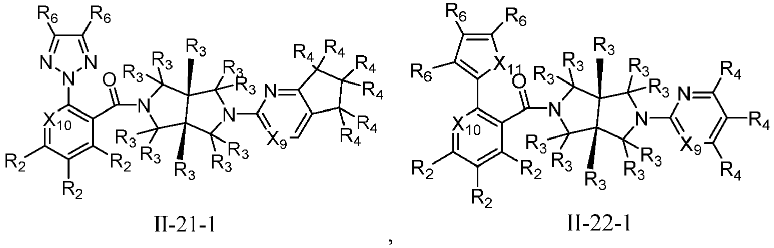

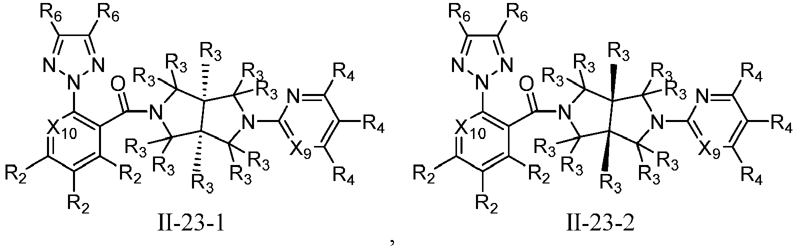

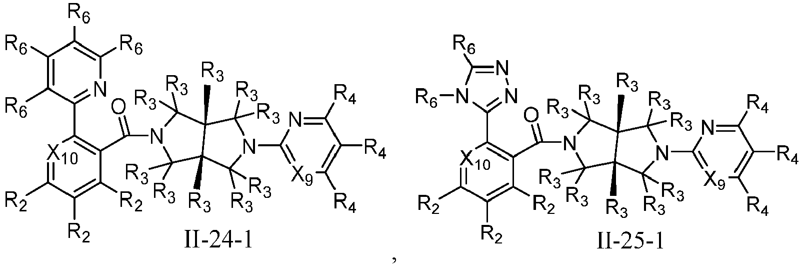

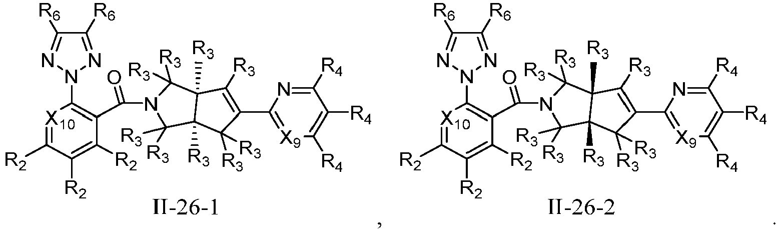

3. The compound, a stereoisomer thereof or a pharmaceutically acceptable salt thereof according to claim 1, **characterized in that** the compound is further represented by the following formulas:

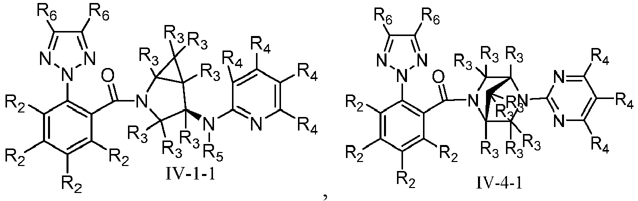

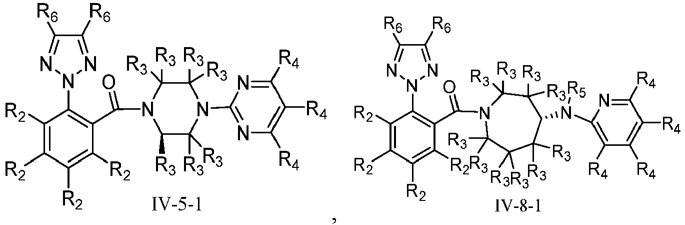

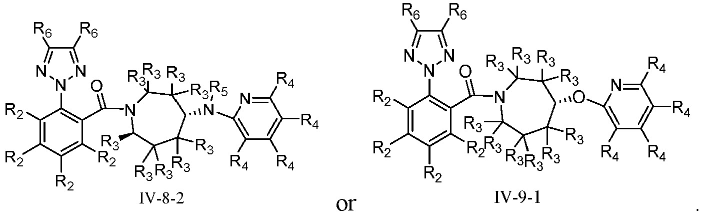

4. The compound, a stereoisomer thereof or a pharmaceutically acceptable salt thereof according to any one of claims 1 to 3, **characterized in that**,
each R₆ is independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
more further preferably selected from the group consisting of hydrogen, halogen, cyano and C₁₋₆ alkyl, the C₁₋₆ alkyl is optionally further substituted by one or more substituents selected from the group consisting of deuterium, halogen, amino, nitro, hydroxy, thiol, cyano, carboxy, sulfonic group, oxo, thioxo, C₁₋₃ alkyl, C₁₋₃ hydroxyalkyl and C₁₋₃ alkoxy.

5. The compound, a stereoisomer thereof or a pharmaceutically acceptable salt thereof according to any one of claims 1 to 4, **characterized in that**,
at least one of substituent R₄ is preferably selected from the group consisting of branched alkyl, linear alkyl containing multiple substituents, and cycloalkyl, the branched alkyl and cycloalkyl are each optionally further substituted by one or more substituents; the cycloalkyl is preferably cyclopropane.

6. The compound, a stereoisomer thereof or a pharmaceutically acceptable salt thereof according to any one of claims 1 to 4, **characterized in that**,
at least one of substituent R₄ is preferably selected from the group consisting of hydroxyalkyl and haloalkyl, which can be optionally further substituted, the hydroxyalkyl is preferably selected from the group consisting of 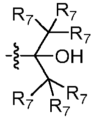 and 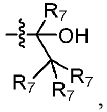 the haloalkyl is preferably selected from the group consisting of 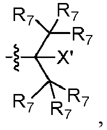 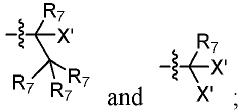
X' is halogen;
each R₇ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted.

7. The compound, a stereoisomer thereof or a pharmaceutically acceptable salt thereof according to any one of claims 1 to 4, **characterized in that**,
at least one of substituent R₄ is preferably selected from the group consisting of thiolalkyl, aminoalkyl and cyanoalkyl, which can be optionally further substituted, the thiolalkyl is preferably selected from the group consisting of 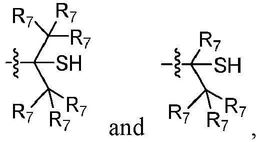 the aminoalkyl is preferably selected from the group consisting of 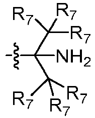 and 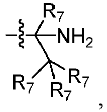 the cyanoalkyl is preferably selected from the group consisting of 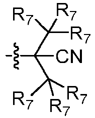 and 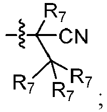
each R₇ is independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted.

8. The compound, a stereoisomer thereof or a pharmaceutically acceptable salt thereof according to any one of claims 6 to 7, **characterized in that**,
the hydroxyalkyl, haloalkyl, thiolalkyl, aminoalkyl or cyanoalkyl is adjacent to N on the heterocycle.

9. The compound, a stereoisomer thereof or a pharmaceutically acceptable salt thereof according to any one of claims 1 to 8, **characterized in that**,
the compound is shown in formulas II-1, II-3, II-4, II-5, II-6, 11-7, 11-8, II-9, 11-10, II-11, II-12, II-13, II-14, II-15, II-16, II-21, II-23 or II-26;
at least one substitutent R₂ is halogen, the halogen is preferably located in the ortho or meta position of the triazole, and more preferably in the ortho position;
the halogen is preferably fluorine.

10. The compound, a stereoisomer thereof or a pharmaceutically acceptable salt thereof according to any one of claims 1 to 8, **characterized in that**,
the compound is shown in formula II-20;
at least one substitutent R₂ is halogen, the halogen is preferably located at the ortho or meta position of the pyrimidine, more preferably at the meta position of the pyrimidine, and/or at the ortho position of the connecting carbonyl;
the halogen is preferably fluorine.

11. The compound, a stereoisomer thereof or a pharmaceutically acceptable salt thereof according to any one of claims 1 to 4, **characterized in that** the structure of the compound is as follows: 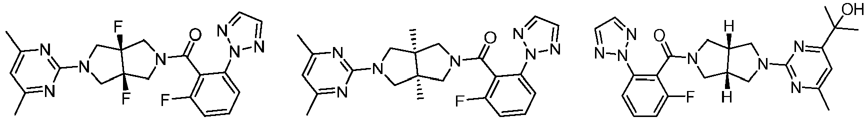 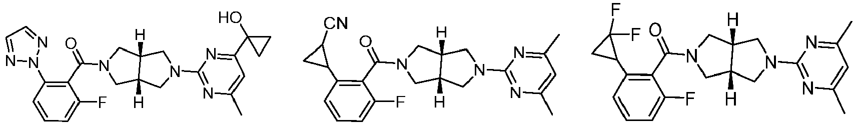 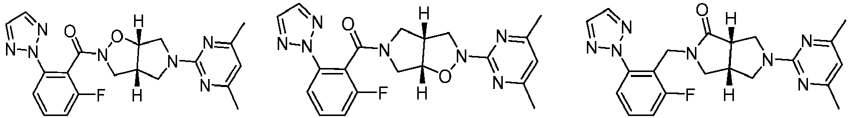 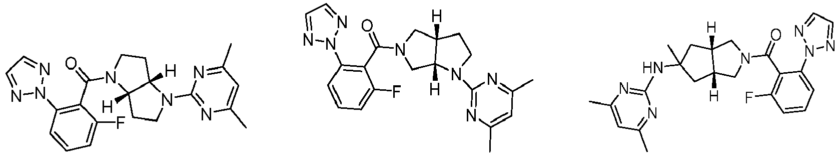 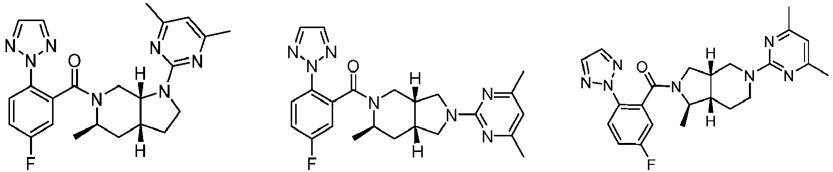 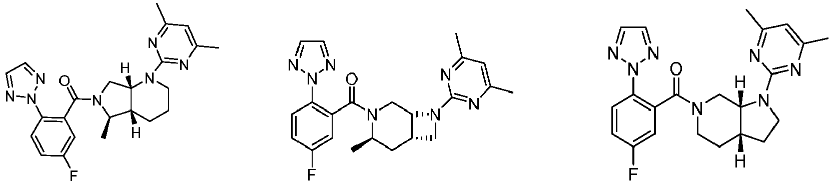 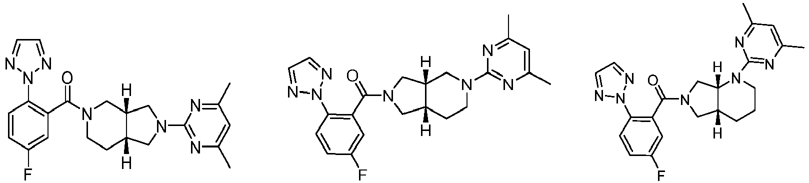 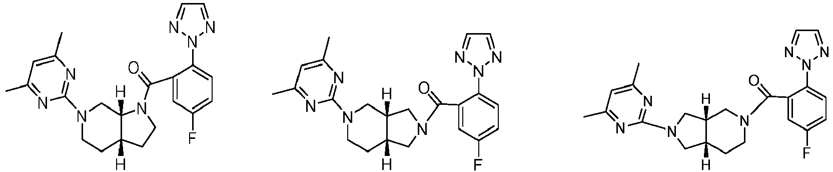 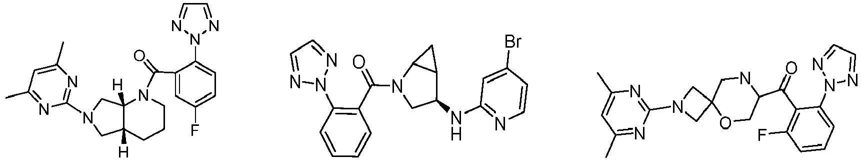 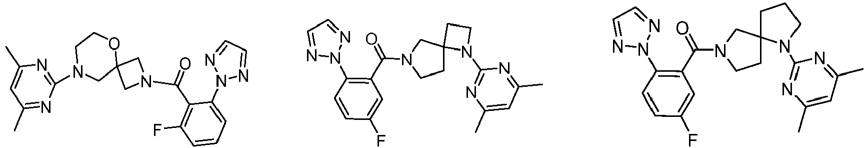 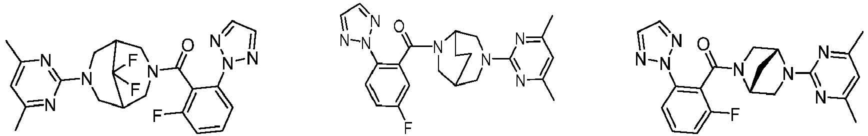 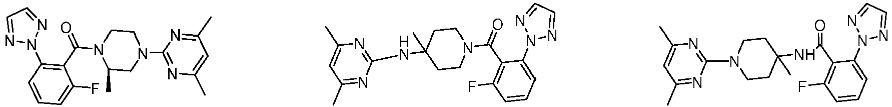 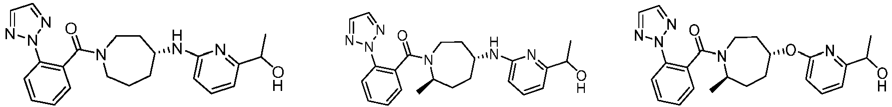 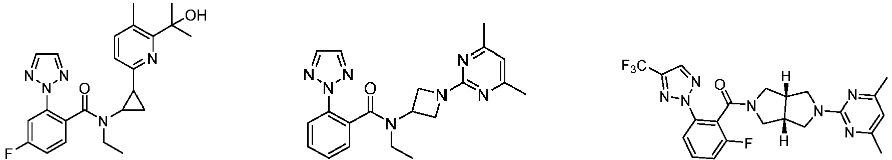 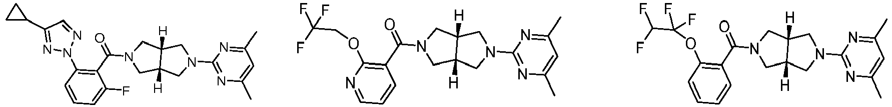 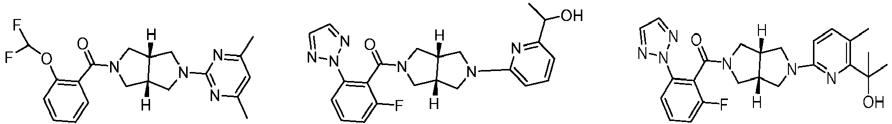 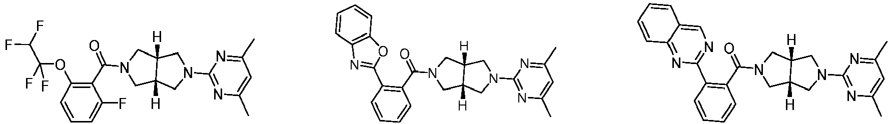 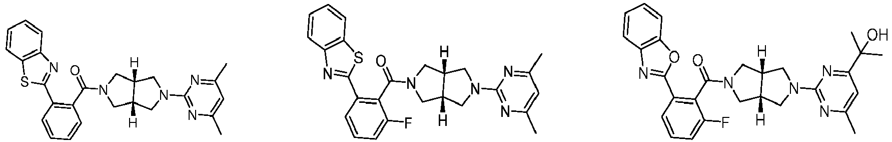 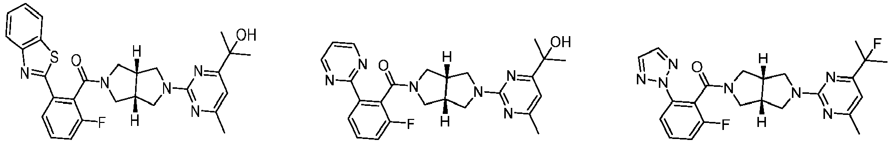 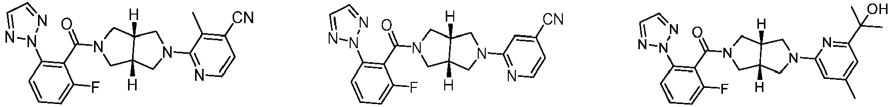 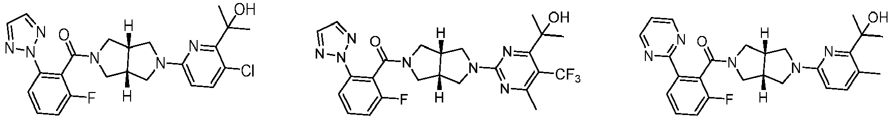 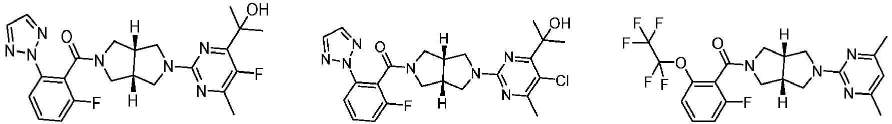 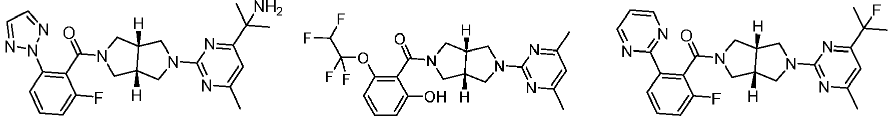 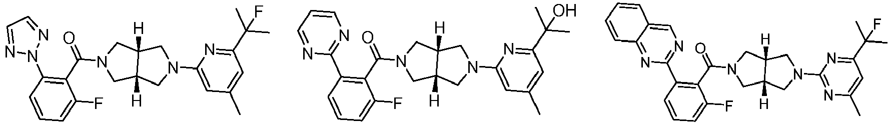 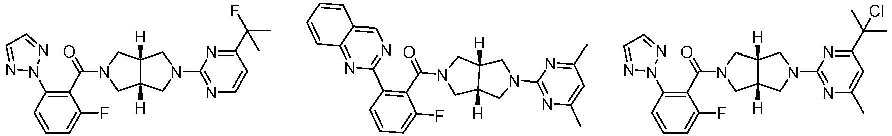 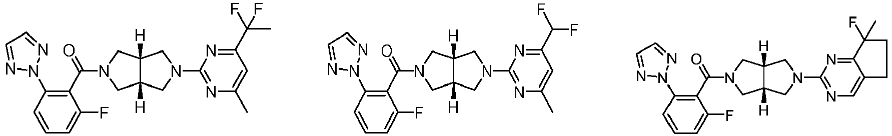 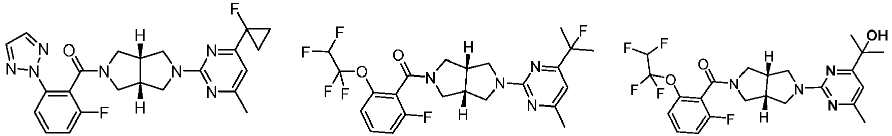 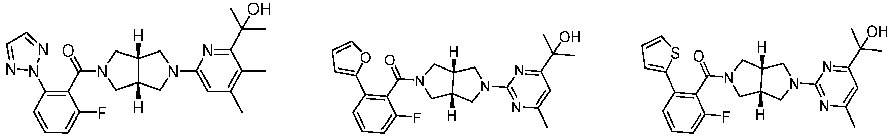 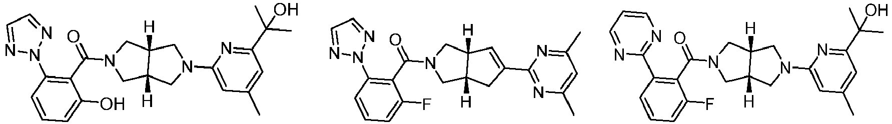 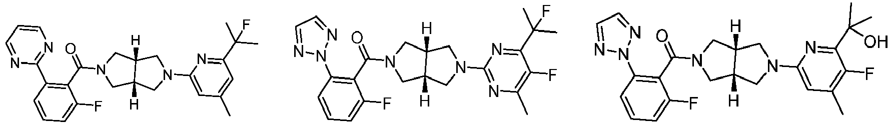 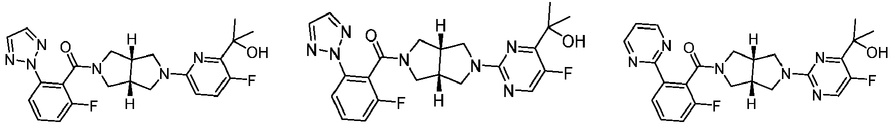 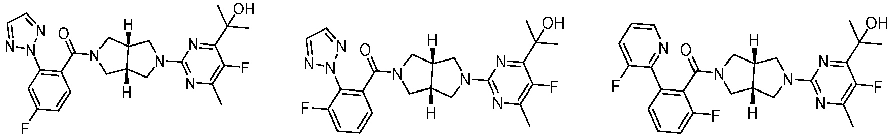 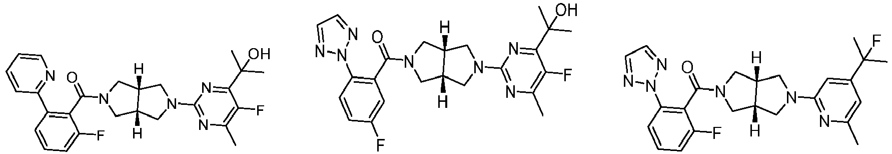 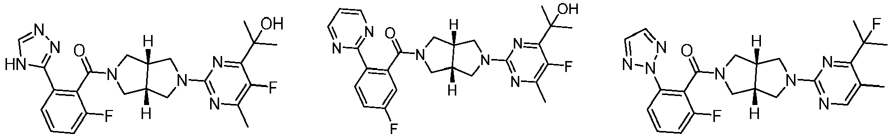 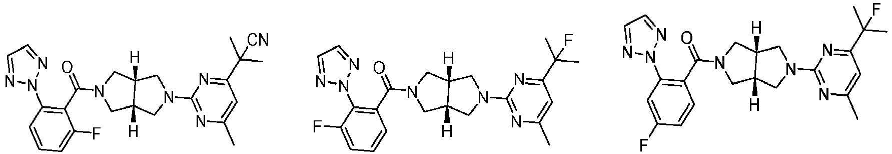 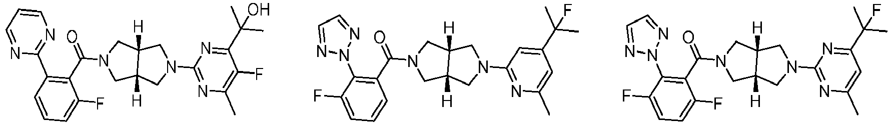 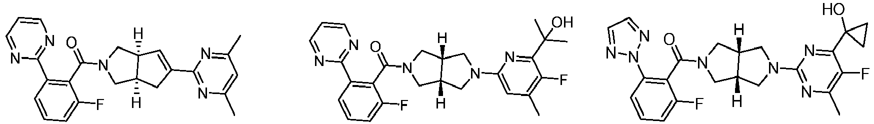 or

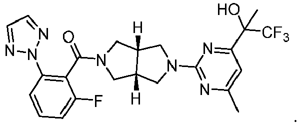

12. A pharmaceutical composition comprising a therapeutically effective dose of the compound, a stereoisomer thereof or a pharmaceutically acceptable salt thereof according to any one of claims 1 to 11, and one or more pharmaceutically acceptable carriers or excipients.

13. Use of the compound, a stereoisomer thereof or a pharmaceutically acceptable salt thereof according to any one of claims 1 to 11 or the pharmaceutical composition according to claim 12 in the preparation of an orexin receptor antagonist, preferably an OX2R selective receptor antagonist.

14. Use of the compound, a stereoisomer thereof or a pharmaceutically acceptable salt thereof according to any one of claims 1 to 11 or the pharmaceutical composition according to claim 12 in the preparation of a medicament for the treatment of nervous system disease; wherein the nervous system disease is preferably selected from the group consisting of insomnia, depression, anxiety and drug addiction, and more preferably selected from the group consisting of major depressive disorder, primary and secondary insomnia, and depression with insomnia.

15. An intermediate, a stereoisomer thereof or a pharmaceutically acceptable salt thereof, **characterized in that** the intermediate is represented by formula 11-17': 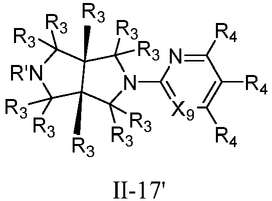
wherein X₉ is CR₄ or N;
R₃, R₄ are each independently selected from the group consisting of hydrogen, deuterium, halogen, amino, nitro, hydroxy, thiol, cyano, carboxy, sulfonic group, oxo, thioxo, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl, the amino, alkyl, deuterated alkyl, haloalkyl, hydroxyalkyl, alkoxy, haloalkoxy, hydroxyalkoxy, alkenyl, alkynyl, cycloalkyl, heterocyclyl, aryl and heteroaryl can be each optionally further substituted;
optionally, any two or more of R₃, R₄ can be connected to form a cycloalkyl, heterocyclyl, aryl or heteroaryl, the cycloalkyl, heterocyclyl, aryl or heteroaryl can be optionally further substituted;
preferably R₃, R₄ are each independently selected from the group consisting of hydrogen, deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl, the amino, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₁₋₈ hydroxyalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₈ alkyl, C₁₋₈ deuterated alkyl, C₁₋₈ haloalkyl, C₁₋₈ hydroxyalkyl, C₁₋₈ alkoxy, C₁₋₈ haloalkoxy, C₂₋₈ alkenyl, C₂₋₈ alkynyl, C₃₋₁₂ cycloalkyl, 3 to 12 membered heterocyclyl, C₆₋₁₄ aryl and 5 to 14 membered heteroaryl;
more preferably selected from the group consisting of hydrogen, deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl, the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ hydroxyalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl are each optionally further substituted by one or more substituents selected from the group consisting of deuterium, halogen, amino, nitro, hydroxy, thiol, cyano, carboxy, sulfonic group, oxo, thioxo, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₈ cycloalkyl, 3 to 8 membered heterocyclyl, C₆₋₁₀ aryl and 5 to 10 membered heteroaryl;
R' is H or an amino protecting group, and the amino protecting group is preferably tert-butoxycarbonyl, benzyloxycarbonyl, p-toluenesulfonyl, trityl, formyl or trifluoroacetyl.

16. The intermediate, a stereoisomer thereof or a pharmaceutically acceptable salt thereof according to claim 15, **characterized in that** the structure of the intermediate is as follows:
